# EUROPÄISCHE PATENTSCHRIFT

(11) **EP 3 127 991 B1**
(45) Veröffentlichungstag und Bekanntmachung des Hinweises auf die Patenterteilung: **15.04.2020**
(21) Anmeldenummer: 16001729.9
(22) Anmeldetag: 04.08.2016
(51) Int. Cl.: C09K 19/30

(54) **FLÜSSIGKRISTALLINES MEDIUM**
LIQUID CRYSTALLINE MEDIUM
MILIEU CRISTALLIN LIQUIDE

(30) Priorität: 05.08.2015 DE 102015009955
(43) Veröffentlichungstag der Anmeldung: 08.02.2017
(73) Patentinhaber: Merck Patent GmbH, 64293 Darmstadt (DE)
(72) Erfinder: Goetz, Achim, 64665 Alsbach-Haehnlein (DE); Hock, Christian, 63814 Mainaschaff (DE); Lietzau, Lars, 64380 Rossdorf (DE); Goebel, Mark, Winchester, SO23 8GJ (GB)

(56) Entgegenhaltungen:
- EP-A1- 0 062 470
- EP-A1- 0 733 692
- DE-A1- 3 139 130
- JP-A- 2001 226 675
- PAVLUCHENKO A I ET AL: "SYNTHESIS AND PROPERTIES OF LIQUID CRYSTALS WITH FLUORINATED TERMINAL SUBSTITUENTS", MOLECULAR CRYSTALS AND LIQUID CRYSTALS, GORDON AND BREACH, LONDON, GB, vol. 209, 1 January 1991 (1991-01-01), pages 225-235, XP002922261, ISSN: 0026-8941

## Beschreibung

Die vorliegende Erfindung betrifft ein flüssigkristallines Medium (FK-Medium), dessen Verwendung für elektrooptische Zwecke und dieses Medium enthaltende FK-Anzeigen.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur. Daneben gibt es auch Zellen, die mit einem elektrischen Feld parallel zur Substrat- und Flüssigkristallebene arbeiten, wie beispielsweise die IPS-Zellen ("in-plane switching"). Vor allem die TN-, STN-, FFS- (Fringe Field Switching)- und IPS- Zellen, sind derzeit kommerziell interessante Einsatzgebiete für die erfindungsgemäßen Medien.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nichtlinearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Addressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Für TV- und Videoanwendungen werden Displays mit schnellen Schaltzeiten benötigt, um Multimedia-Inhalte, wie z. B. Filme und Videospiele, realitätsnah wiedergeben zu können. Solche geringen Schaltzeiten lassen sich besonders dann realisieren, wenn Flüssigkristallmedien mit geringen Werten für die Viskosität, insbesondere der Rotationsviskosität γ₁ und mit einer hohen optischen Anisotropie (Δn) verwendet werden.

Zur Realisierung von 3D-Effekten mittels Shutterbrillen werden insbesondere schnell schaltende Mischungen mit niedrigen Rotationsviskositäten und einer entsprechend hohen optischen Anisotropie (Δn) eingesetzt. Elektrooptische Linsensysteme, mit denen eine 2-dimensionale Darstellung eines Displays in eine 3-dimensionale autostereoskopische Darstellung geschaltet werden kann, können unter Verwendung von Mischungen mit hoher optischer Anisotropie (Δn) realisiert werden.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleine Schwellenspannung.

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Eine der wichtigsten Eigenschaften von modernen LCDs ist die korrekte Wiedergabe von bewegten Bildern. Ist die Schaltgeschwindigkeit des verwendeten flüssigkristallinen Mediums zu langsam, ruft dies ungewünschte Artefakte bei der Darstellung solcher Inhalte hervor. Die physikalischen Parameter, die im Wesentlichen die Schaltzeit einer Flüssigkristallmischung bestimmen, sind die Rotationsviskosität γ₁ und die elastischen Konstanten. Letztere sind auch besonders wichtig, um einen guten Schwarzzustand des LCDs zu gewährleisten. Im Allgemeinen beobachtet man jedoch, dass mit einer Erhöhung der elastischen Konstanten auch der Klärpunkt der Mischung und somit die Rotationsviskosität der Mischung erhöht wird, so dass keine Verbesserung der Schaltzeit möglich ist. Insbesondere bei FK-Anzeigen für TV- und Video-Anwendungen (z.B. LCD-TV, Monitore, PDAs, Notebooks, Spielkonsolen) ist eine deutliche Verringerung der Schaltzeiten gewünscht. Eine Verringerung der Schichtdicke d ("cell gap") des FK-Mediums in der FK-Zelle führt theoretisch zu schnelleren Schaltzeiten, erfordert jedoch FK-Medien mit einer höheren Doppelbrechung Δn, um eine ausreichende optische Verzögerung (Retardation = d·Δn) zu gewährleisten. Die aus dem Stand der Technik bekannten FK-Materialien mit hoher Doppelbrechung besitzen jedoch im Allgemeinen gleichzeitig auch eine hohe Rotationsviskosität, was sich wiederum negativ auf die Schaltzeiten auswirkt.

Es besteht somit immer noch ein großer Bedarf nach Flüssigkristallmedien mit guten Reliability-Eigenschaften, wie z.B. hoher VHR (Voltage Holding Ratio), die diese Nachteile nicht oder nur in geringerem Maße zeigen.

Der Erfindung liegt die Aufgabe zugrunde, Medien insbesondere für derartige MFK-, TN-, STN-, OCB-, positive VA-, FFS-, HB (= high brightness)-FFS-, PS (=polymer stabilized)-FFS, IPS-, PS-IPS-Anzeigen bereitzustellen, welche die oben angegebenen gewünschten Eigenschaften besitzen und die oben angegebenen Nachteile nicht oder nur in geringerem Maße zeigen. Insbesondere sollten die FK-Medien schnelle Schaltzeiten und niedrige Rotationsviskositäten bei gleichzeitig relativer hoher Doppelbrechung aufweisen. Darüber hinaus sollten die FK-Medien einen hohen Klärpunkt und eine sehr gute Tieftemperaturstabilität (LTS = low temperature stability) zeigen.

Es wurde nun gefunden, dass diese Aufgabe gelöst werden kann, wenn man FK-Medien enthaltend eine oder mehrere Verbindungen der Formel I verwendet.

JP 2001226675 A, DE 3139130 A1, EP 0062470 A1, EP 0733692 A1 und Pavluchenko et al., Mol. Cryst. Liq. Cryst. 1991, 209, 225-235 offenbaren Verbindungen der Formel I, offenbaren aber keine erfindungsgemäßen Medien oder legen diese nahe.

Gegenstand der Erfindung ist ein flüssigkristallines Medium, dadurch gekennzeichnet, dass es eine oder mehrere Verbindungen der Formel I, 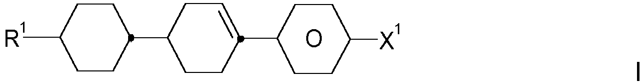 worin
- R¹: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 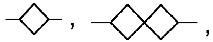 -O-, -CO-O-, -O-CO- so ersetzt sein können, dass O-Atome nicht direkt miteinander verknüpft sind, und worin auch ein oder mehrere H-Atome durch Halogen ersetzt sein können, und
- X¹: einen Alkylrest mit 1 bis 5 C-Atomen, OCF₃, CF₃, CHF₂, OCHF₂, OCF₂CF₃, CCF₂CHFCF₃, OCF=CF₂, OCH=CF₂ oder F
bedeuten,
sowie weitere Verbindungen wie in Anspruch 1 definiert enthält.

Die Verbindungen der Formel I führen zu LC-Mischungen mit den oben angegebenen gewünschten Eigenschaften, insbesondere zu LC-Mischungen mit einer sehr niedrigen Rotationsviskosität. Die erfindungsgemäßen Mischungen weisen sehr große elastische Konstanten auf und ermöglichen damit sehr gute Schaltzeiten. Weiterhin sind die erfindungsgemäßen Mischungen bei mindestens -20 °C stabil und zeigen keine Neigung zur Kristallisation. Die Rotationsviskositäten γ₁ liegen in der Regel bei < 120 mPa · s. Weiterhin zeichnen sich die erfindungsgemäßen Mischungen durch ein sehr gutes Verhältnis von Rotationsviskosität γ₁ und Klärpunkt, niedrige γ₁/K₁₁-Werte, welche zu schnelleren Schaltzeiten führen, einen hohen Klärpunkt und einen breiten nematischen Phasenbereich aus. Weiterhin sind die Verbindungen der Formel I gut in flüssigkristallinen Medien löslich.

Die Verbindungen der Formel I besitzen einen breiten Anwendungsbereich und zeichnen sich insbesondere durch ihre sehr großen elastischen Konstanten aus. In Abhängigkeit von der Auswahl der Substituenten können sie als Basismaterialien dienen, aus denen flüssigkristalline Medien zum überwiegenden Teil zusammengesetzt sind; es können aber auch den Verbindungen der Formel I flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Rotationsviskosität zu optimieren. Das Ergebnis sind erfindungsgemäße LC-Mischungen, die einen guten Schwarzzustand des Displays, der für den Kontrast des Displays ausschlaggebend ist, aufgrund hoher elastischer Konstanten unterstützen und gleichzeitig sehr gute Schaltzeiten ermöglichen.

Vorzugsweise bedeutet R¹ in den Verbindungen der Formel I und den Unterformeln einen geradkettigen Alkylrest, insbesondere mit 3-5 C-Atomen. In einer weiteren bevorzugten Ausführungsform können in dem Alkylrest auch ein oder mehrere CH₂-Gruppen durch -CH=CH- ersetzt sein.

Besonders bevorzugte Verbindungen der Formel I werden nachfolgend genannt: 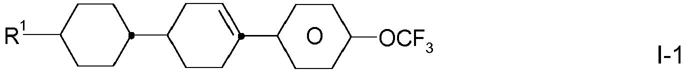 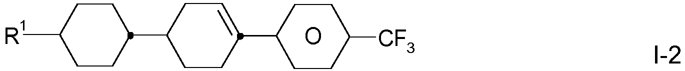 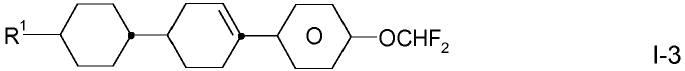 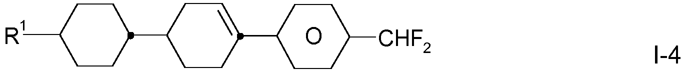 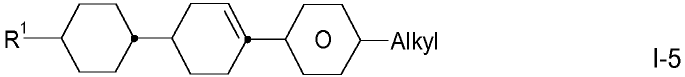 worin R¹ die in Anspruch 1 angegebenen Bedeutungen besitzt, vorzugsweise geradkettiges Alkyl oder Alkenyl mit bis zu 5 C-Atomen, und "Alkyl" ein geradkettiger Alkylrest mit 1-5 C-Atomen bedeutet.

Ganz besonders bevorzugt sind die Verbindungen der Formel I-1, I-2 und I-5.

Ganz besonders bevorzugte Verbindungen werden nachfolgend genannt:

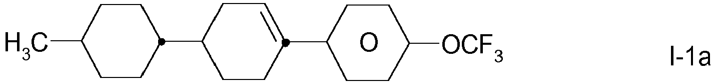

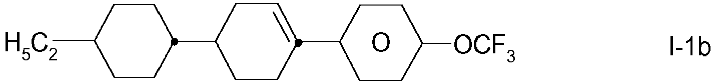

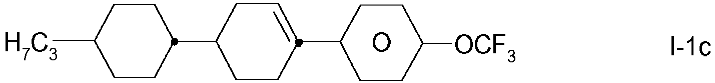

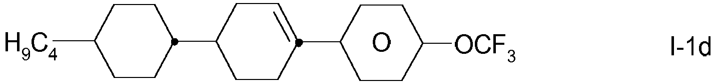

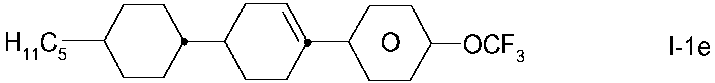

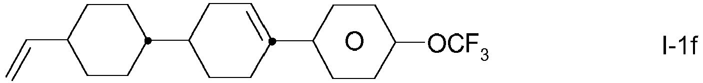

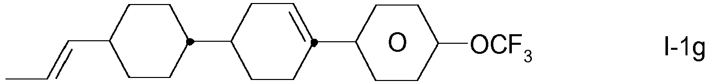

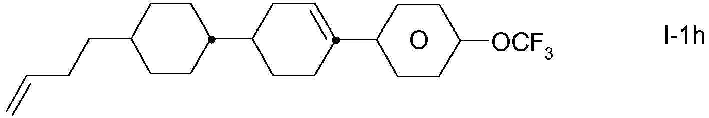

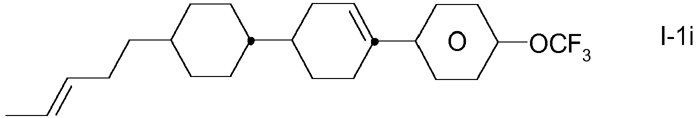

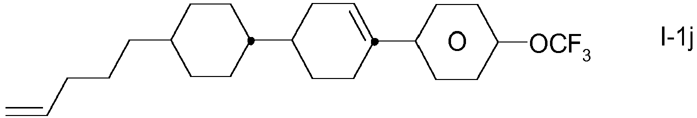

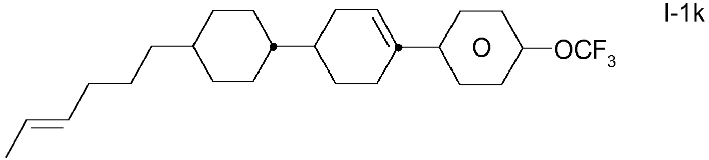

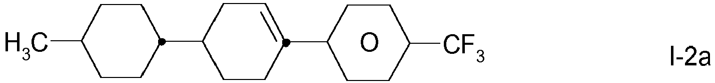

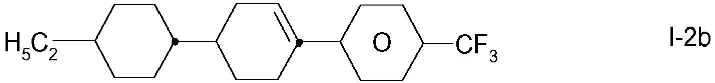

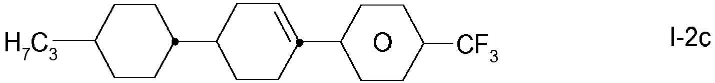

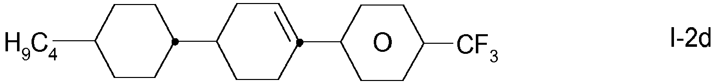

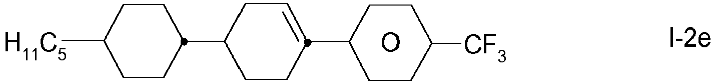

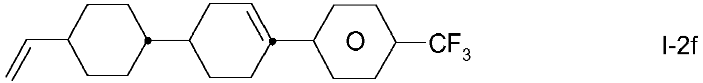

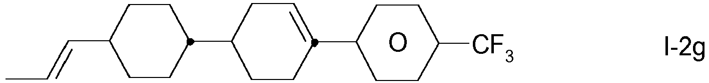

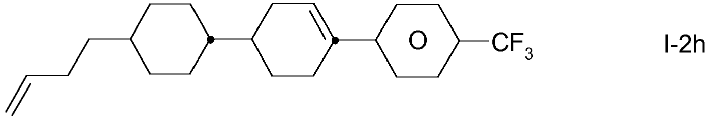

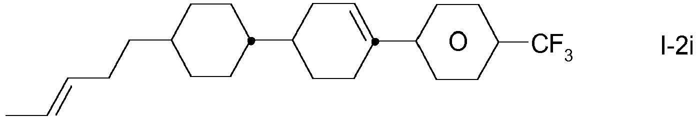

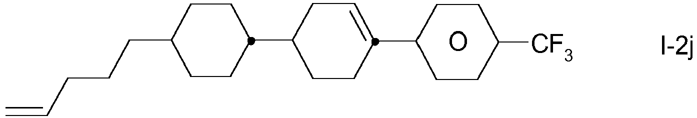

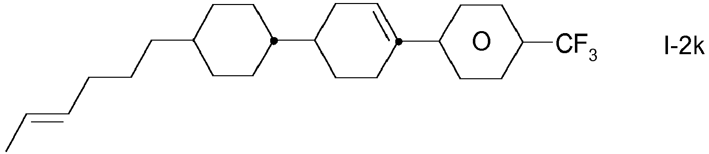

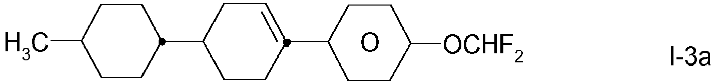

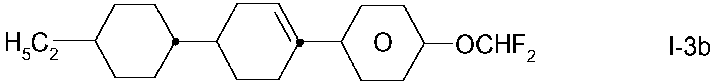

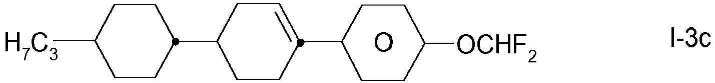

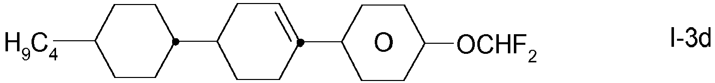

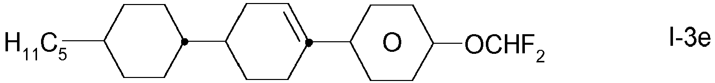

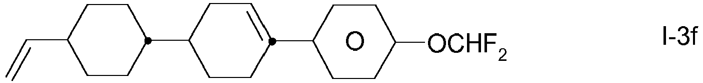

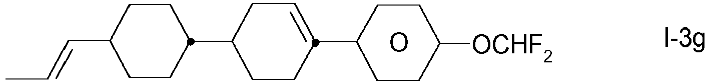

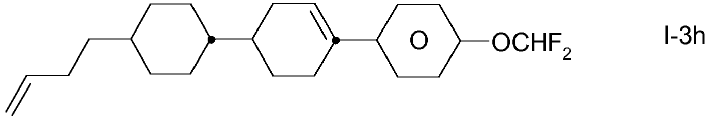

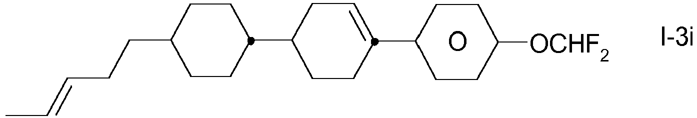

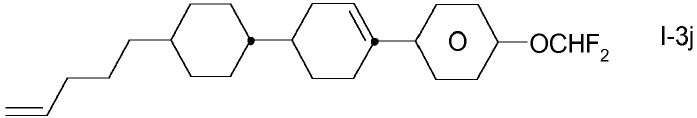

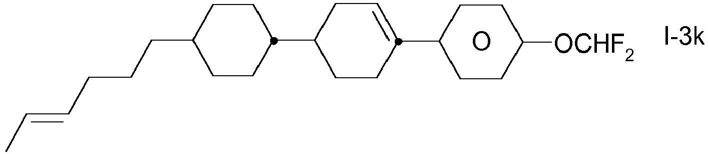

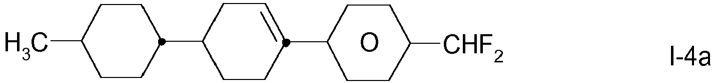

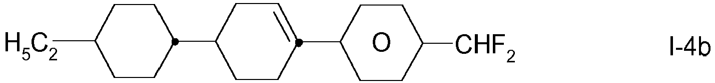

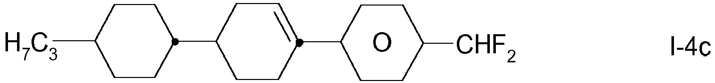

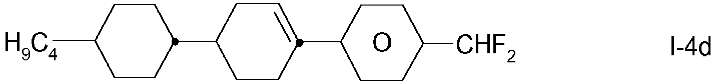

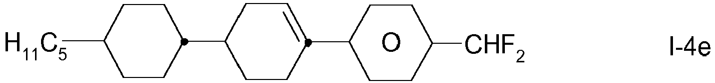

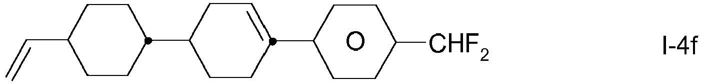

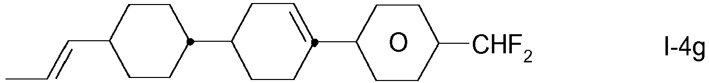

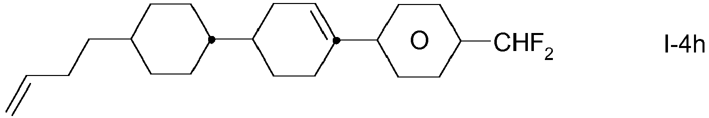

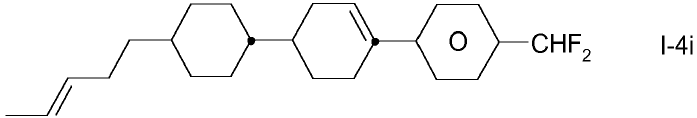

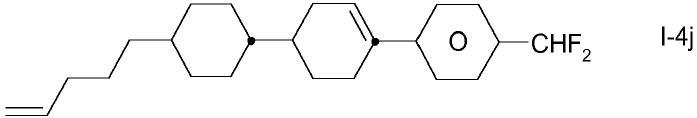

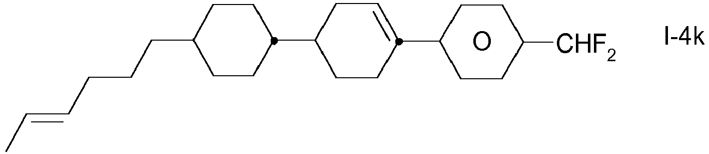

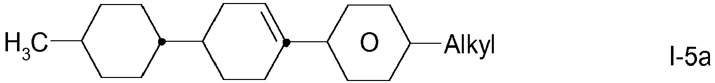

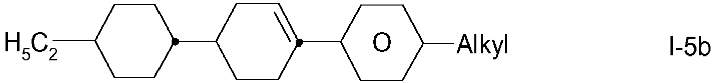

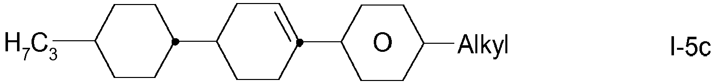

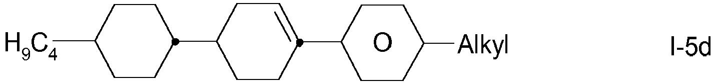

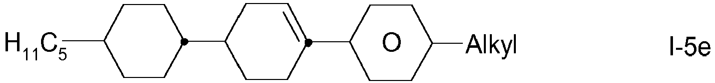

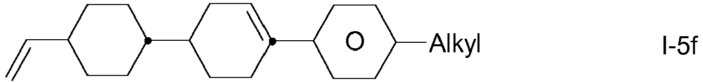

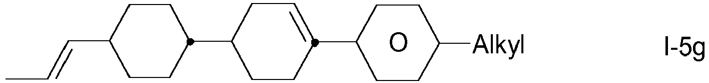

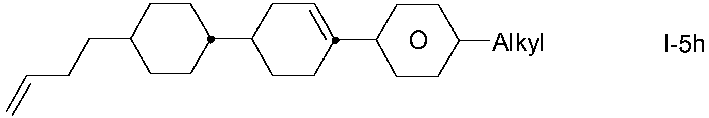

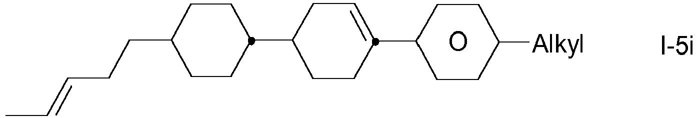

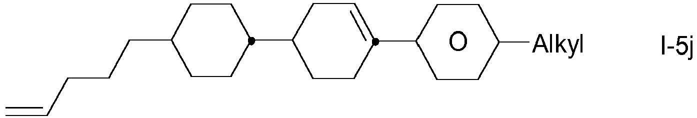

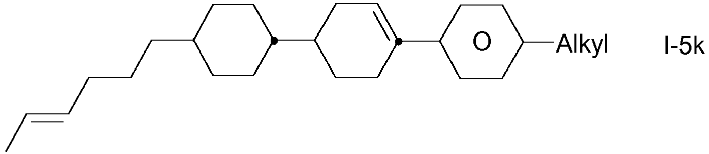

In den Verbindungen der Formeln I-5a bis I-5k bedeutet Alkyl insbesondere bevorzugt CH₃ oder C₂H₅., insbesondere CH₃.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Die Verbindungen der Formel I werden nach an sich bekannten Methoden dargestellt, wie sie in der Literatur (z.B. in den Standardwerken wie

Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen. Die Verbindungen der Formel I können beispielsweise wie folgt hergestellt werden:

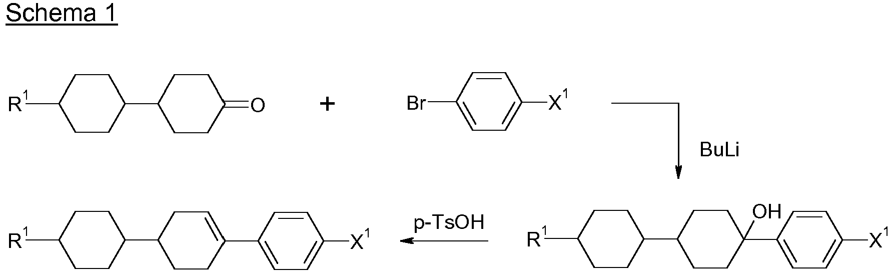

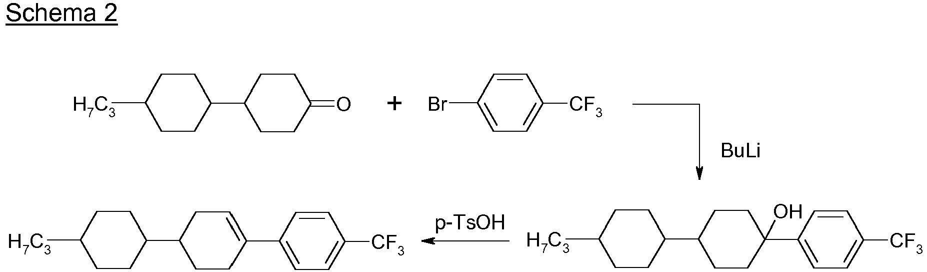

Falls in den oben- und untenstehenden Verbindungen der Formel I R¹ einen Alkylrest und/oder einen Alkoxyrest bedeutet, so kann dieser geradkettig oder verzweigt sein. Vorzugsweise ist er geradkettig, hat 2, 3, 4, 5, 6, oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy, oder Heptoxy, ferner Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxaheptyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1, 2-, 3-, 4-, 5-, 6-, 7-, 8- oder Dec-9-enyl. Diese Reste können auch ein- oder mehrfach halogeniert sein.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

X¹ bedeutet vorzugsweise einen Alkylrest, insbesondere CH₃, und CF₃, ferner OCF₃.
- Das Medium enthält zusätzlich zu den Verbindungen der Formel I eine oder mehrere neutrale Verbindungen der Formeln II und/oder III, 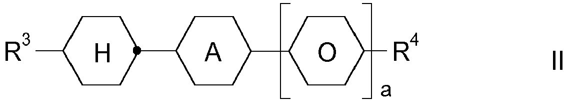 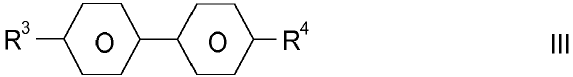
worin
- A: 1,4-Phenylen oder trans-1,4-Cyclohexylen bedeutet,
- a: 0 oder 1 ist, und
- R³: Alkenyl mit 2 bis 9 C-Atomen bedeutet,
und R⁴ die für R¹ in Formel I angegebene Bedeutung besitzt und vorzugsweise Alkyl mit 1 bis 12 C-Atomen oder Alkenyl mit 2 bis 9 C-Atomen bedeutet.

Bevorzugte Ausführungsformen für die erfindungsgemäßen Flüssigkristallmischungen sind im Folgenden angegeben:
- Die Verbindungen der Formel II sind vorzugsweise ausgewählt aus den Verbindungen der folgenden Formeln, 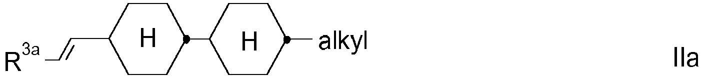 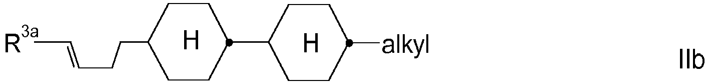 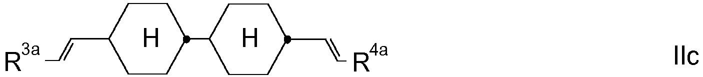 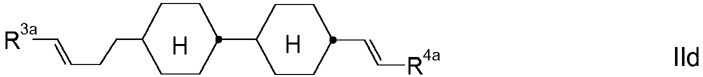 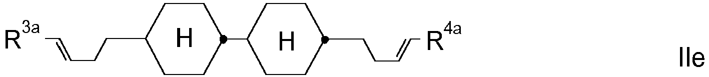 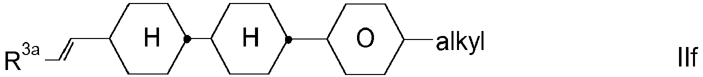 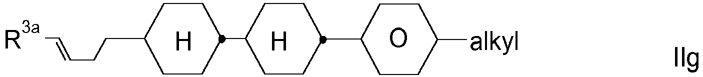 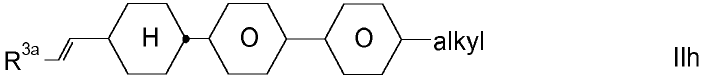 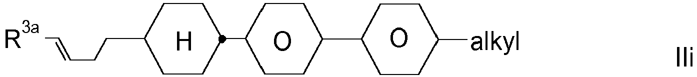
worin R^{3a} und R^{4a} jeweils unabhängig voneinander H, CH₃, C₂H₅ oder C₃H₇ bedeuten, und "alkyl" eine geradkettige Alkylgruppe mit 1 bis 8 C-Atomen bedeutet. Besonders bevorzugt sind Verbindungen der Formeln IIa und IIf, insbesondere worin R^{3a} H oder CH₃ bedeutet, und Verbindungen der Formel IIc, insbesondere worin R^{3a} und R^{4a} H, CH₃ oder C₂H₅ bedeuten.

Weiterhin sind Verbindungen der Formel II bevorzugt, die eine nichtendständige Doppelbindung in der Alkenylseitenkette aufweisen:

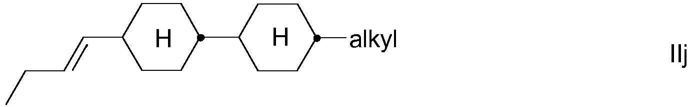

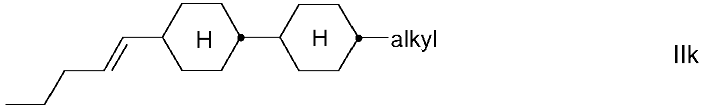

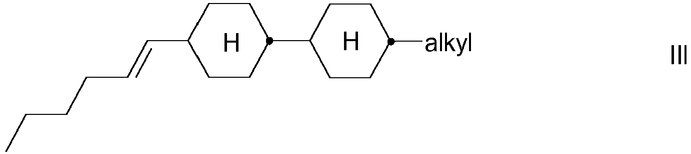

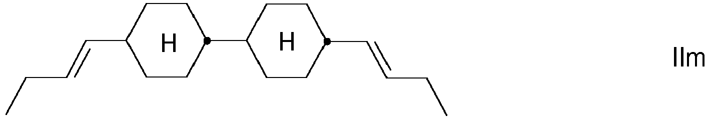

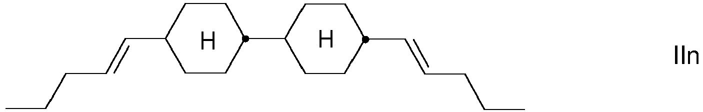

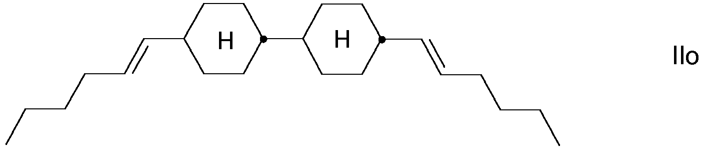

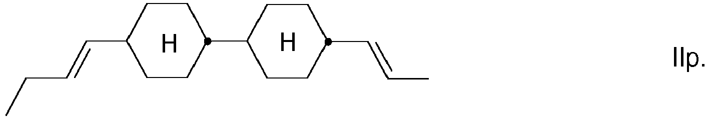

Ganz besonders bevorzugte Verbindungen der Formel IIa sind die Verbindungen der Formeln

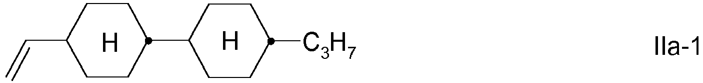

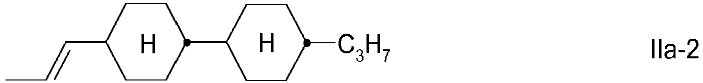

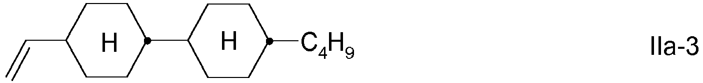

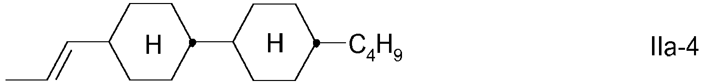

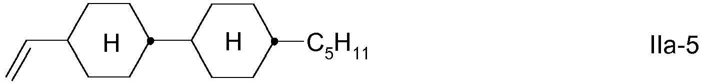

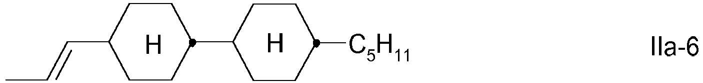

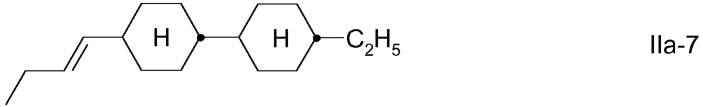

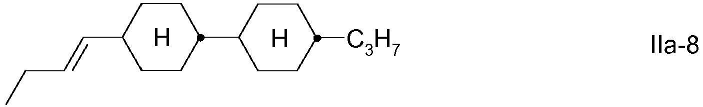

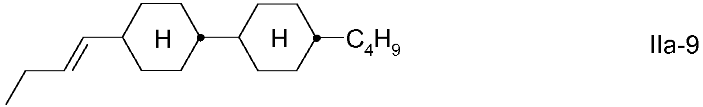

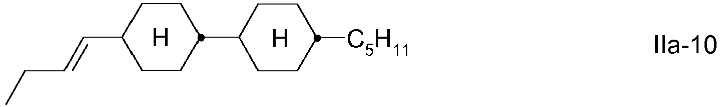

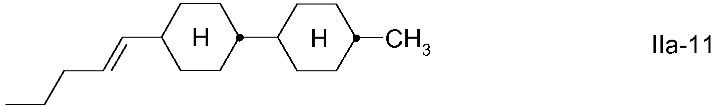

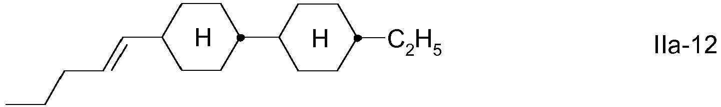

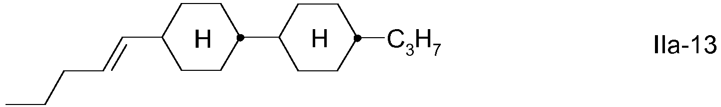

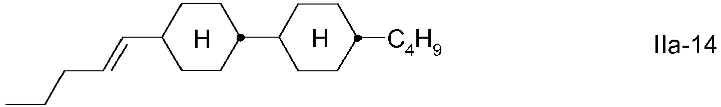

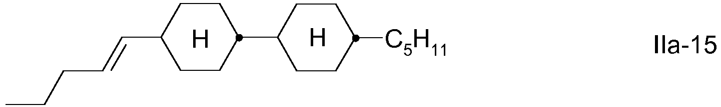

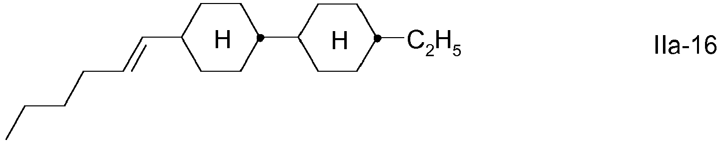

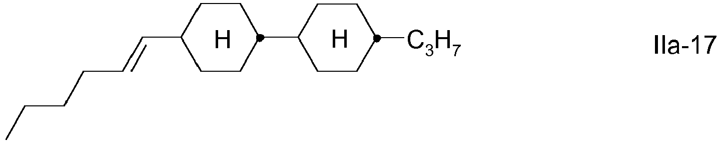

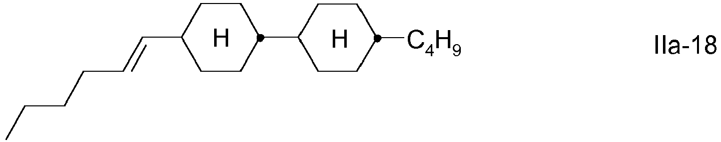

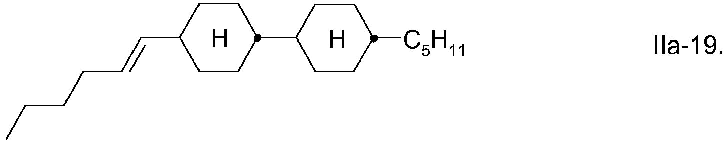

Von den Verbindungen der Formeln IIa-1 bis IIa-19 sind inbesondere die Verbindungen der Formeln IIa-1, IIa-2, IIa-3, IIa-5 und IIc-1 besonders bevorzugt.

Besonders bevorzugt enthalten die erfindungsgemäßen flüssigkristallinen Medien neben einer oder mehreren Verbindungen der Formel I zusätzlich die Verbindung der Formel (CC-3-V), 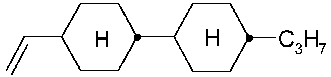 vorzugsweise in Konzentrationen von 5 - 70 Gew.%, insbesondere 10 - 65 Gew.% und ganz besonders bevorzugt 20 - 55 Gew.%, bezogen auf die Mischung.

Bevorzugte Verbindungen der Formel IIb sind die Verbindungen der Formeln

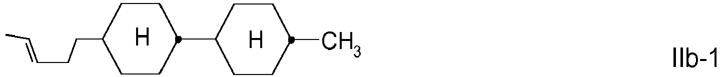

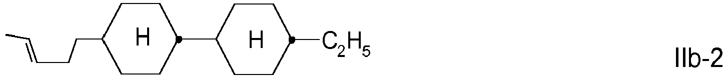

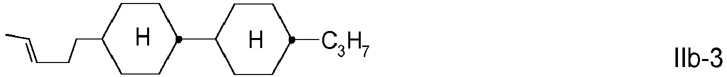

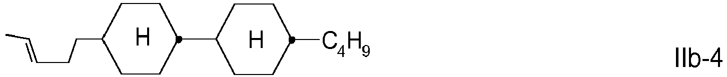

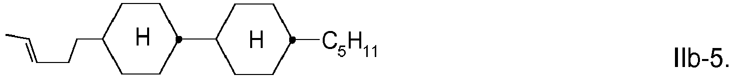

Bevorzugte Verbindungen der Formel IIc sind die Verbindungen der Formeln 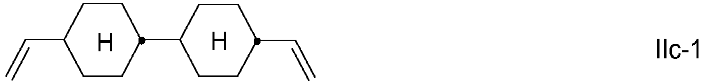 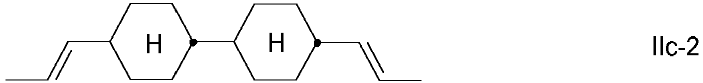 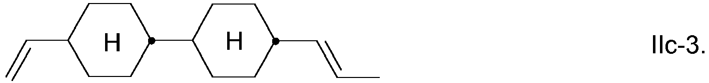
- Die Verbindungen der Formel III sind vorzugsweise ausgewählt aus den folgenden Formeln, 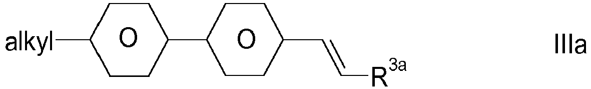 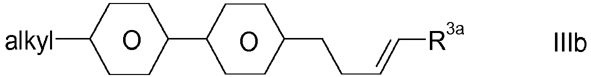 worin "Alkyl" und R^{3a} die oben angegebenen Bedeutungen haben und R^{3a} vorzugsweise H oder CH₃ bedeutet. Besonders bevorzugt sind Verbindungen der Formel Illb;
   Ganz besonders bevorzugt ist die Verbindung der Formel IIIb-1, 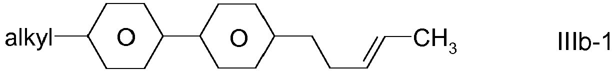 worin "alkyl" die oben angegebene Bedeutung hat und vorzugsweise CH₃, ferner C₂H₅ oder n-C₃H₇, bedeutet.
- Das Medium enthält vorzugsweise zusätzlich eine oder mehrere Verbindungen ausgewählt aus folgenden Formeln IV bis VIII, 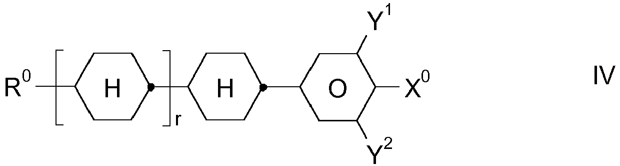 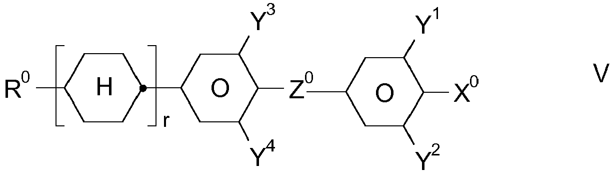 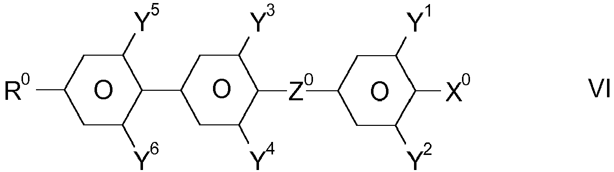 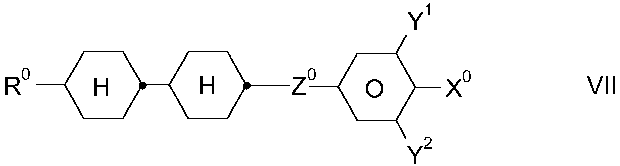 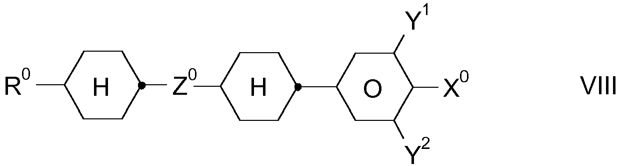
worin
- R⁰: die in Anspruch 6 angegebenen Bedeutungen besitzt,
- X⁰: F, Cl, ein- oder mehrfach fluorierter Alkyl- oder Alkoxyrest mit jeweils 1 bis 6 C-Atomen, ein- oder mehrfach fluorierter Alkenyl- oder Alkenyloxyrest mit jeweils 2 bis 6 C-Atomen,
- Y¹⁻⁶: jeweils unabhängig voneinander H oder F,
- Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in den Formeln V und VI auch eine Einfachbindung, und
- r: 0 oder 1
bedeuten.

In den obenstehenden Formeln ist X⁰ vorzugsweise F, Cl oder ein ein- oder mehrfach fluorierter Alkyl- oder Alkoxyrest mit 1, 2 oder 3 C-Atomen oder ein ein- oder mehrfach fluorierter Alkenylrest bzw. Alkenyloxyrest mit 2 oder 3 C-Atomen. X⁰ ist besonders bevorzugt F, Cl, CF₃, CHF₂, OCF₃, OCHF₂, OCHFCF₃, OCHFCHF₂, OCHFCH₂F, OCF₂CH₃, OCF₂CHF₂, OCF₂CH₂F, OCF₂CF₂CHF₂, OCF₂CF₂CH₂F, OCFHCF₂CF₃, OCFHCF₂CHF₂, OCH=CF₂, OCF=CF₂, OCF₂CHFCF₃, OCF₂CF₂CF₃, OCF₂CF₂CClF₂, OCClFCF₂CF₃, CF=CF₂, CF=CHF, OCH=CF₂, OCF=CF₂, oder CH=CF₂.

In den Verbindungen der Formel IV bis VIII bedeutet X⁰ vorzugsweise F oder OCF₃, ferner OCHF₂, CF₃, CF₂H, Cl, OCH=CF₂. R⁰ ist vorzugsweise geradkettiges Alkyl oder Alkenyl mit bis zu 6 C-Atomen.
- Die Verbindungen der Formel IV sind vorzugsweise ausgewählt aus den folgenden Formeln, 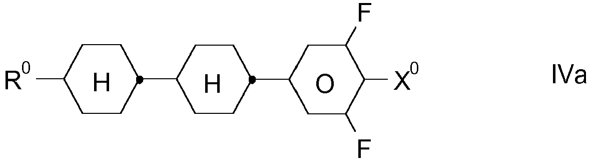 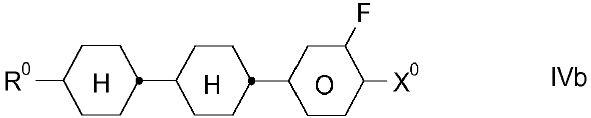 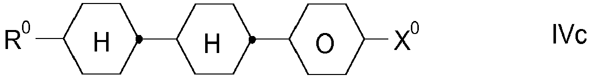 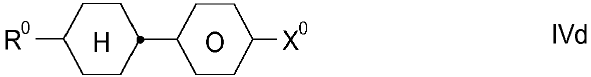 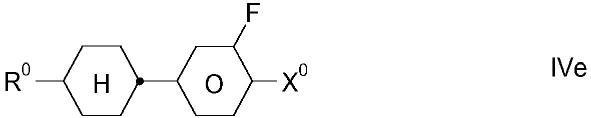 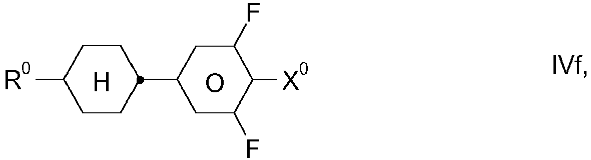 worin R⁰ und X⁰ die in Anspruch 6 angegebenen Bedeutungen haben.

Vorzugsweise bedeutet in Formel IV R⁰ Alkyl mit 1 bis 8 C-Atomen und X⁰ F, Cl, OCHF₂ oder OCF₃, ferner OCH=CF₂. In der Verbindung der Formel IVb bedeutet R⁰ vorzugsweise Alkyl oder Alkenyl. In der Verbindung der Formel IVd bedeutet X⁰ vorzugsweise Cl, ferner F.
- Die Verbindungen der Formel V sind vorzugsweise ausgewählt aus den Formeln Va bis Vj, 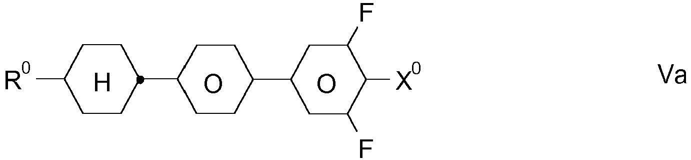 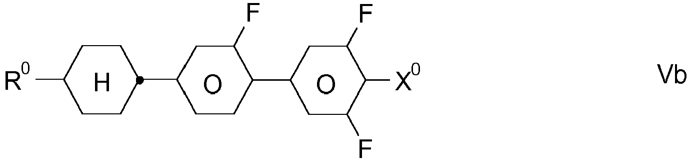 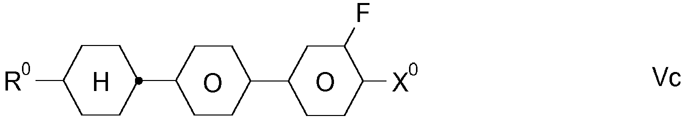 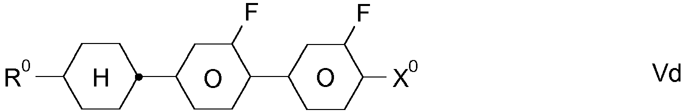 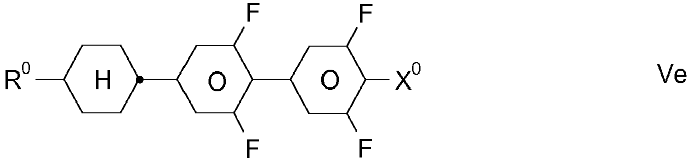 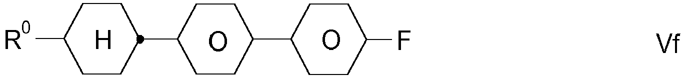 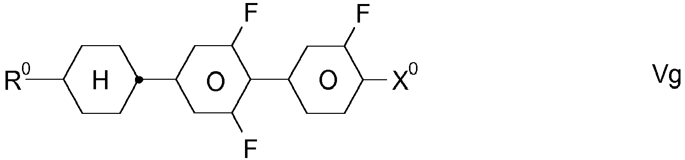 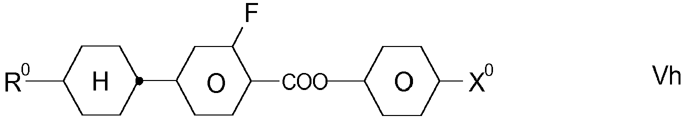 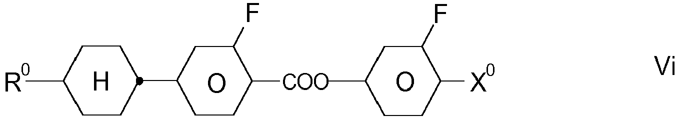 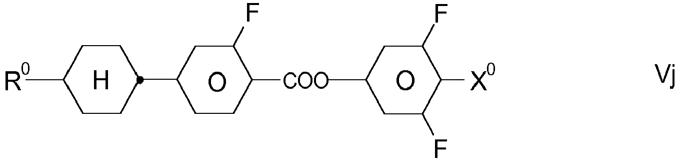 worin R⁰ und X⁰ die in Anspruch 6 angegebenen Bedeutungen haben. Vorzugsweise bedeutet in Formel V R⁰ Alkyl mit 1 bis 8 C-Atomen und X⁰ F, OCF₃ oder OCH=CF₂.
- Das Medium enthält eine oder mehrere Verbindungen der Formel VI-1, 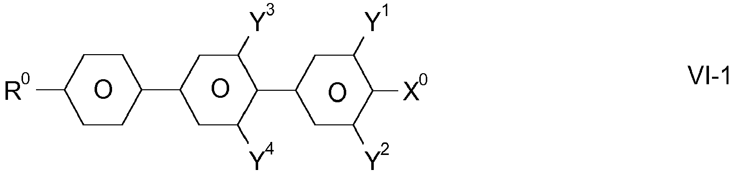 besonders bevorzugt solche ausgewählt aus den folgenden Formeln, 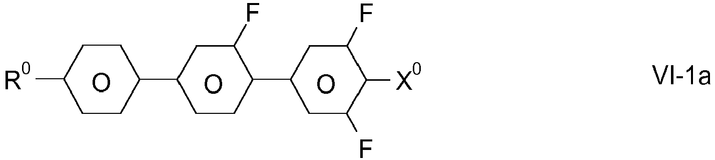 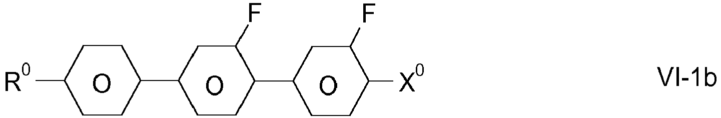 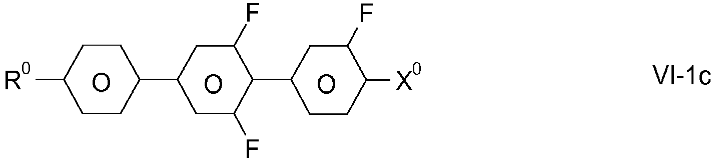 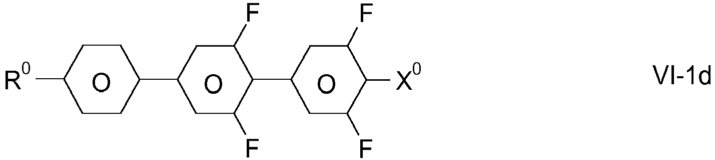 worin R⁰ und X⁰ die in Anspruch 6 angegebenen Bedeutungen haben. Vorzugsweise bedeutet in Formel VI R⁰ Alkyl mit 1 bis 8 C-Atomen und X⁰ F, ferner CF₃ und OCF₃.
- Das Medium enthält eine oder mehrere Verbindungen der Formel VI-2, 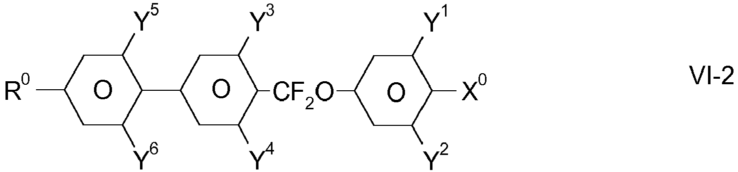 besonders bevorzugt solche ausgewählt aus den folgenden Formeln, 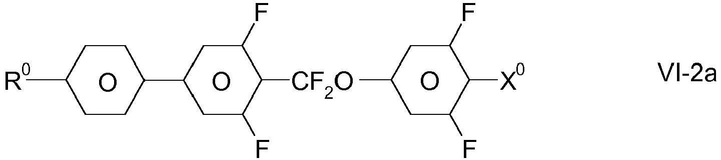 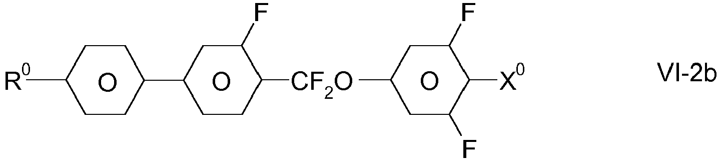 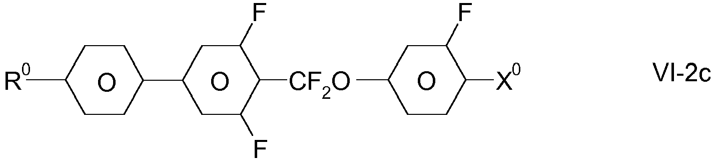 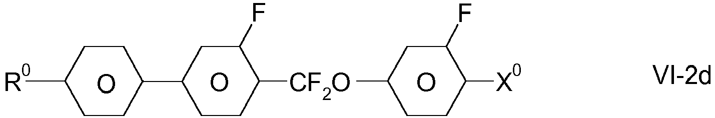 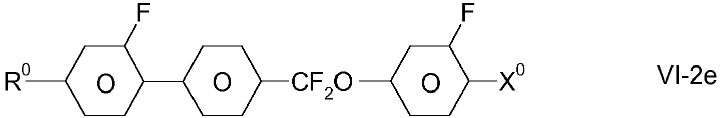 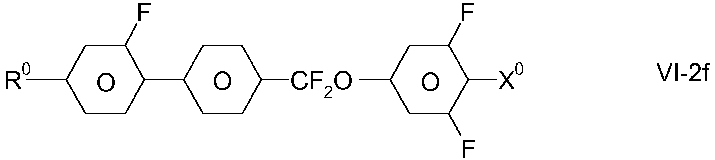 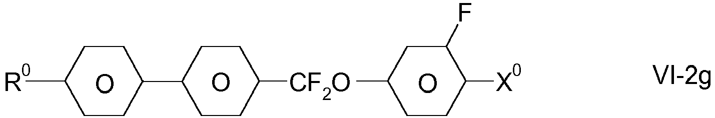 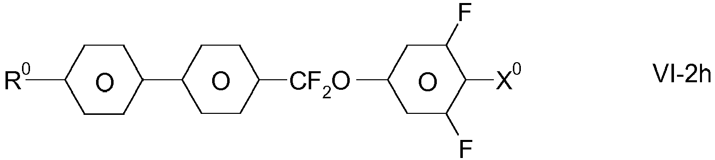 worin R⁰ und X⁰ die in Anspruch 6 angegebenen Bedeutungen haben. Vorzugsweise bedeutet in Formel VI R⁰ Alkyl mit 1 bis 8 C-Atomen und X⁰ F;
- Das Medium enthält vorzugsweise eine oder mehrere Verbindungen der Formel VII, worin Z⁰ -CF₂O-, -CH₂CH₂- oder -COO- bedeutet, besonders bevorzugt solche ausgewählt aus folgenden Formeln, 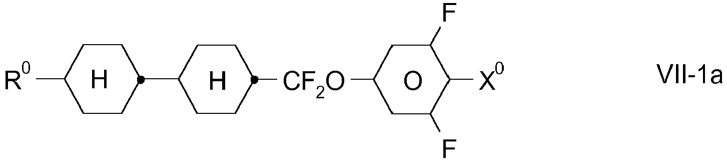 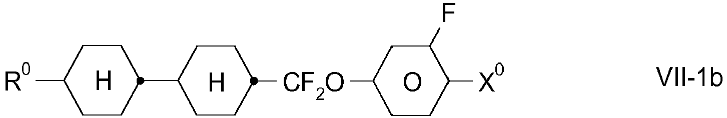 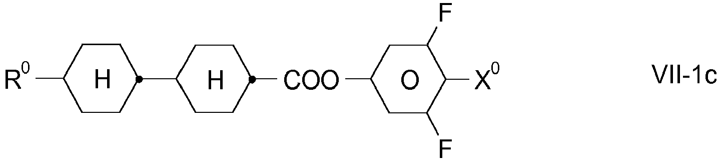 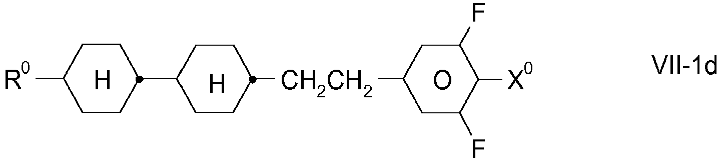
worin R⁰ und X⁰ die in Anspruch 6 angegebenen Bedeutungen haben. Vorzugsweise bedeutet in Formel VII R⁰ Alkyl mit 1 bis 8 C-Atomen und X⁰ F, ferner OCF₃ und CF₃.

Die Verbindungen der Formel VIII sind vorzugsweise ausgewählt aus den folgenden Formeln, 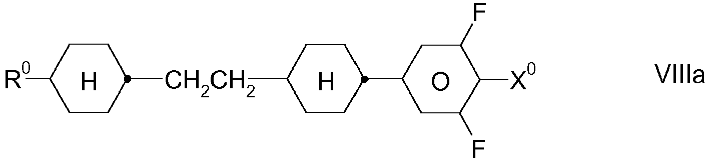 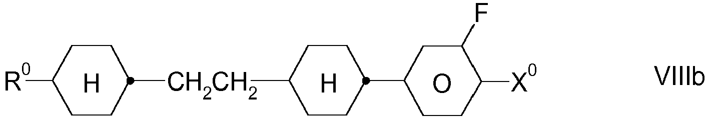 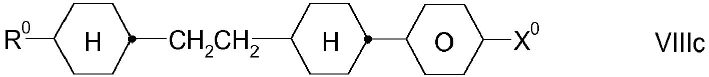 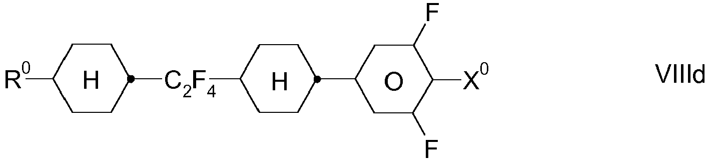 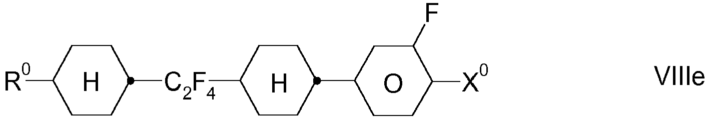 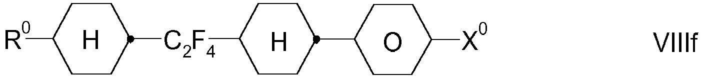 worin R⁰ und X⁰ die oben angegebenen Bedeutungen haben. Vorzugsweise bedeutet in Formel VIII R⁰ einen geradkettigen Alkylrest mit 1 bis 8 C-Atomen. X⁰ bedeutet vorzugsweise F.
- Das Medium enthält zusätzlich eine oder mehrere Verbindungen der folgenden Formel, 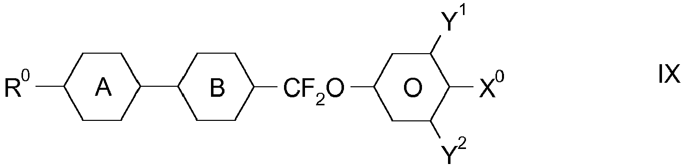 worin R⁰, X⁰, Y¹ und Y² die oben angegebene Bedeutung besitzen, und 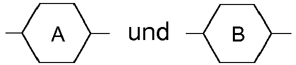 jeweils unabhängig voneinander bedeuten, 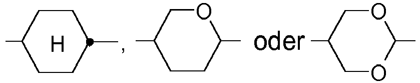 wobei die Ringe A und B nicht beide gleichzeitig 1,4-Cyclohexylen bedeuten;
- Die Verbindungen der Formel IX sind vorzugsweise ausgewählt aus folgenden Formeln, 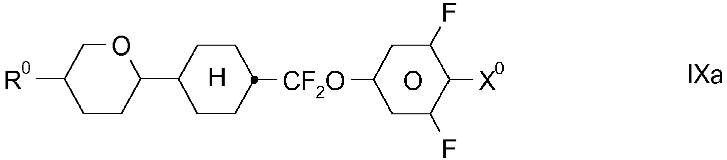 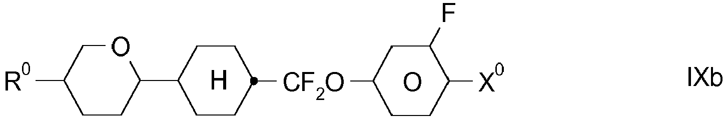 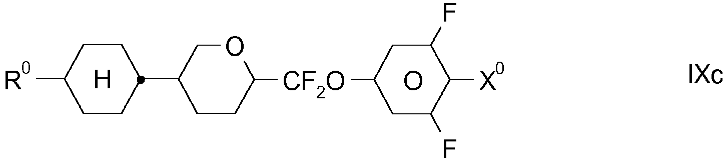 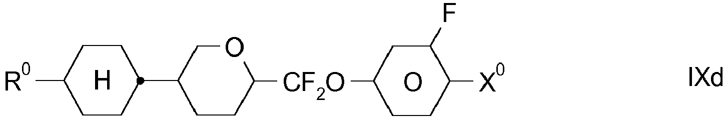 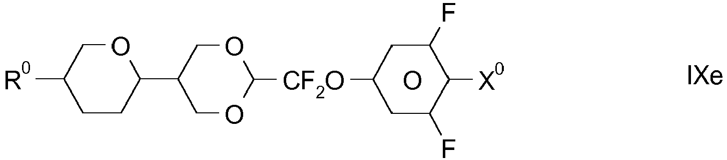 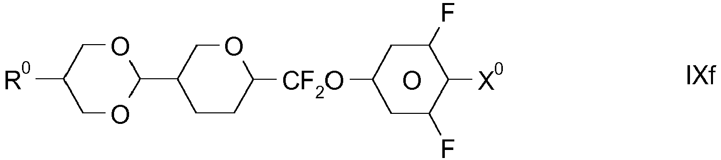 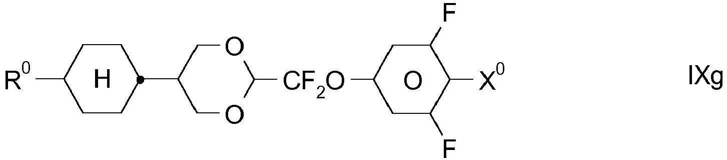 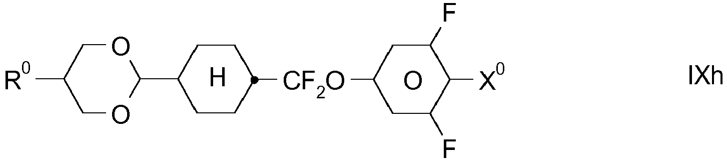 worin R⁰ und X⁰ die in Anspruch 6 angegebenen Bedeutungen haben. Vorzugsweise bedeutet in Formel IX R⁰ Alkyl mit 1 bis 8 C-Atomen und X⁰ F. Besonders bevorzugt sind Verbindungen der Formel IXa;
- Das Medium enthält zusätzlich eine oder mehrere Verbindungen ausgewählt aus den folgenden Formeln, 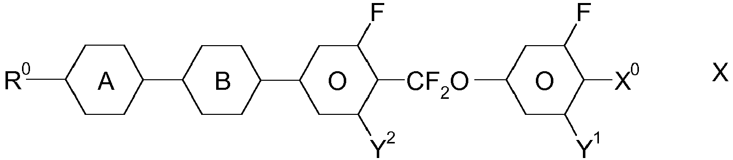 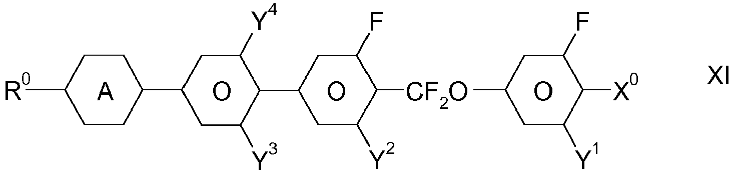 worin R⁰, X⁰ und Y¹⁻⁴ die in Anspruch 6 angegebenen Bedeutungen besitzen, und 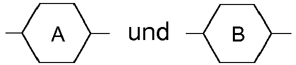 jeweils unabhängig voneinander 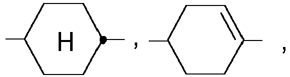 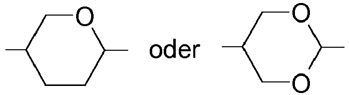 bedeuten;
- Die Verbindungen der Formeln X und XI sind vorzugsweise ausgewählt aus den folgenden Formeln, 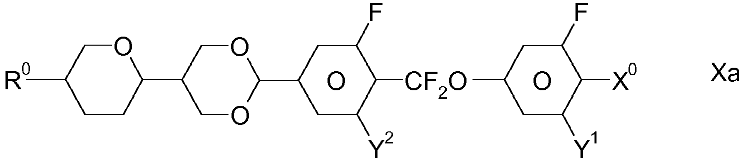 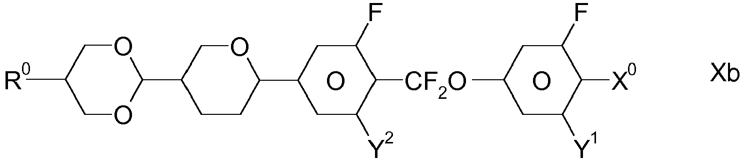 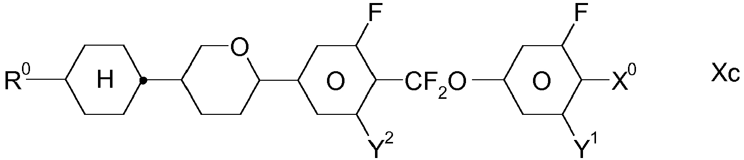 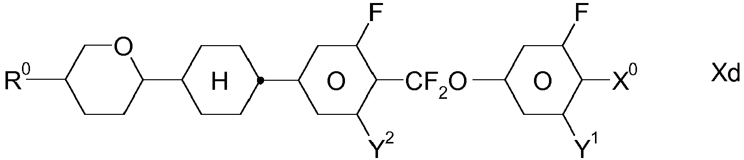 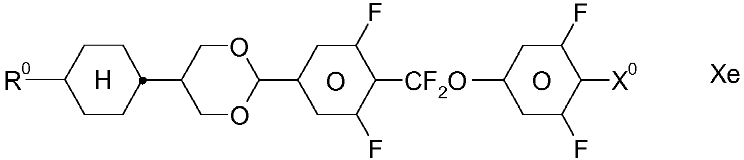 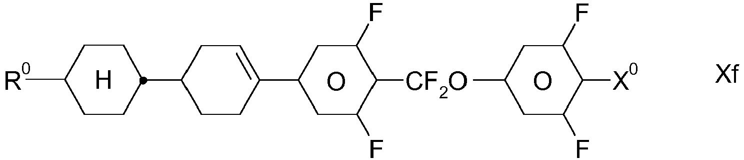 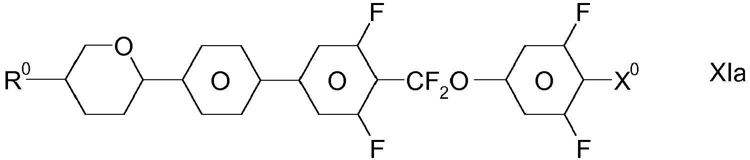 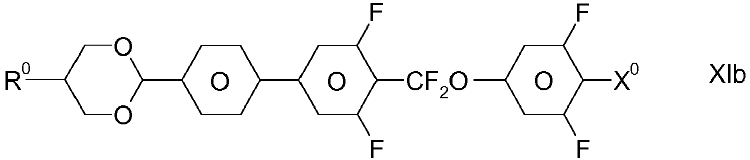 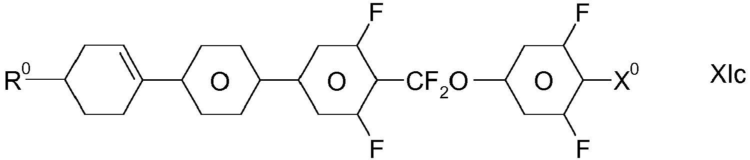 worin R⁰ und X⁰ die in Anspruch 6 angegebenen Bedeutungen haben. Vorzugsweise bedeutet R⁰ Alkyl mit 1 bis 8 C-Atomen, Cycloalkyl und X⁰ F. Besonders bevorzugte Verbindungen sind solche, worin Y¹ F und Y² H oder F, vorzugsweise F, bedeuten;
- Das Medium enthält zusätzlich eine oder mehrere Verbindungen der folgenden Formel XII, 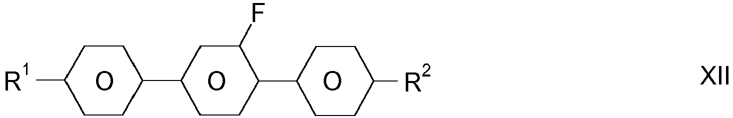 worin R¹ und R² jeweils unabhängig voneinander Alkyl, Alkenyl, Alkoxy, Oxaalkyl, Fluoralkyl oder Alkenyloxy mit jeweils bis zu 9 C-Atomen bedeuten, und vorzugsweise jeweils unabhängig voneinander Alkyl oder Alkenyl mit 1 bis 8 C-Atomen bzw. 2 bis 8 C-Atomen bedeuten.

Bevorzugte Verbindungen der Formel XII sind die Verbindungen der Formel, 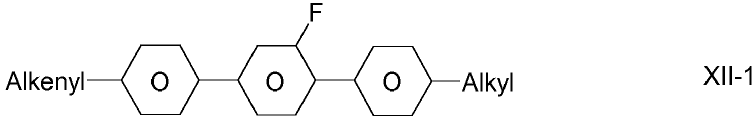 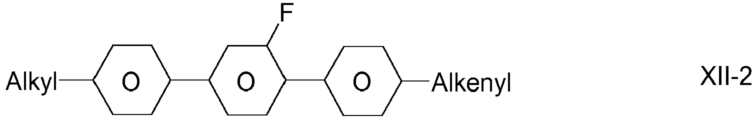 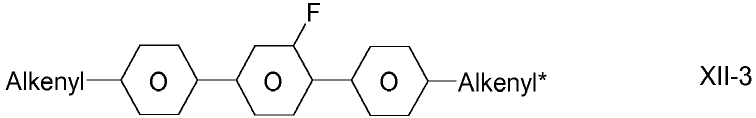 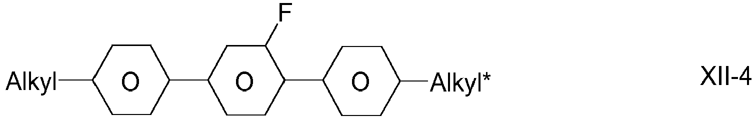 worin
- Alkyl und Alkyl*: jeweils unabhängig voneinander einen geradkettigen Alkylrest mit 1 bis 8 C-Atomen, und
- Alkenyl und Alkenyl*: jeweils unabhängig voneinander einen geradkettigen Alkenylrest mit 2 bis 8 C-Atomen
bedeuten.

Besonders bevorzugt sind die Verbindungen der Formeln XII-2 und XII-4.

Besonders bevorzugte Verbindungen der Formel XII-2 sind die Verbindungen der Formel XII-2a, XII-2b und XII-2c:

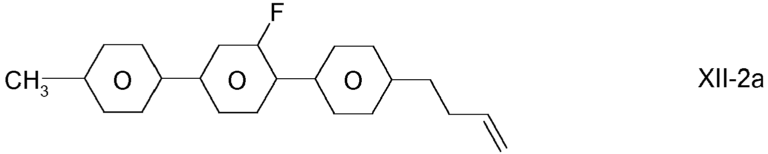

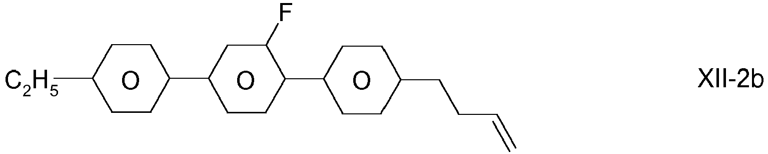

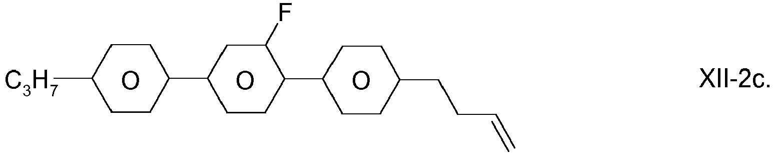

Besonders bevorzugte Verbindungen der Formel XII-4 sind die Verbindungen der Formel XII-4a, XII-4b und XII-4c:

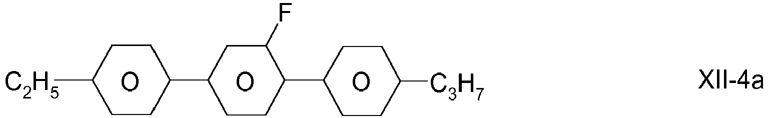

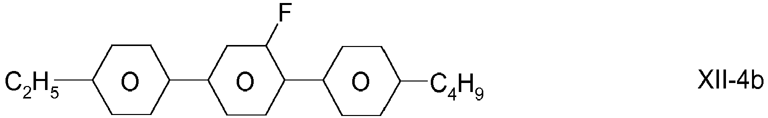

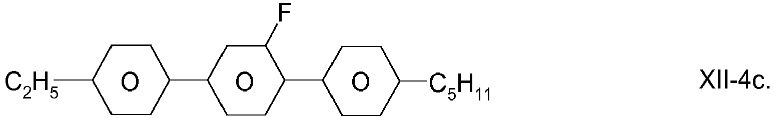

Die Verbindung(en) der Formel XII werden vorzugsweise in Mengen von 3 - 40 Gew.% eingesetzt.
- Das Medium enthält zusätzlich eine oder mehrere Verbindungen ausgewählt aus folgenden Formeln, 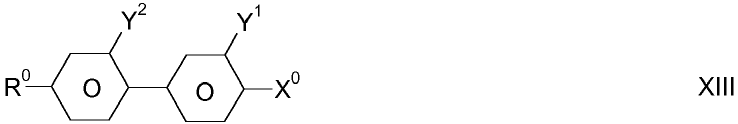 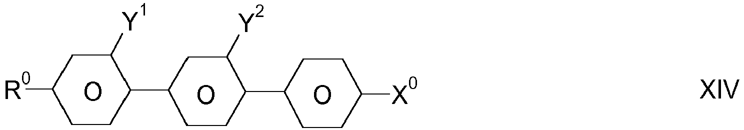 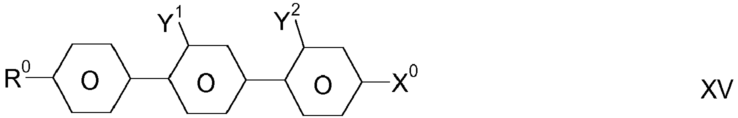 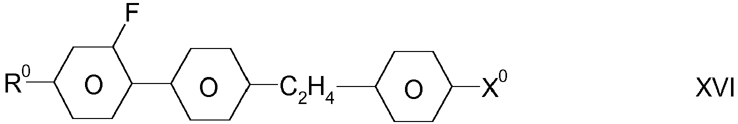 worin R⁰, X⁰, Y¹ und Y² die in Anspruch 6 angegebenen Bedeutungen haben. Vorzugsweise bedeutet R⁰ Alkyl mit 1 bis 8 C-Atomen und X⁰ F oder Cl;
- Die Verbindungen der Formeln XIII und XIV sind vorzugsweise ausgewählt aus den Verbindungen der Formeln, 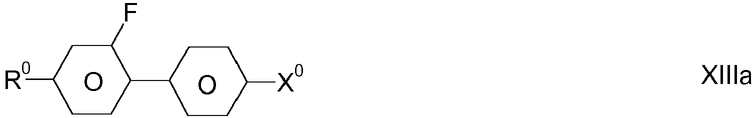 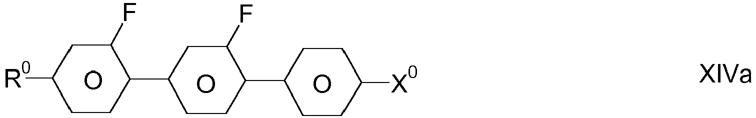 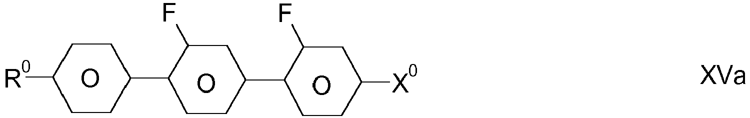 worin R⁰ und X⁰ die in Anspruch 6 angegebenen Bedeutungen haben. Vorzugsweise bedeutet R⁰ Alkyl mit 1 bis 8 C-Atomen. In den Verbindungen der Formel XIII bedeutet X⁰ vorzugsweise F oder Cl.
- Das Medium enthält zusätzlich eine oder mehrere Verbindungen der Formeln D1, D2, D3, D4 und/oder D5, 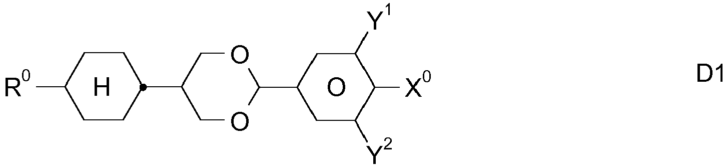 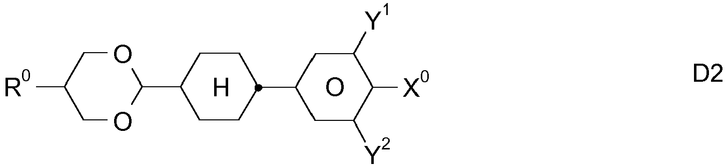 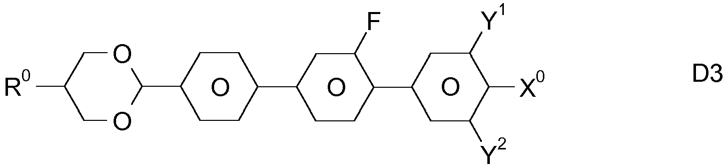 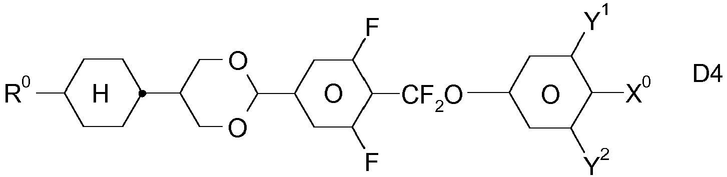 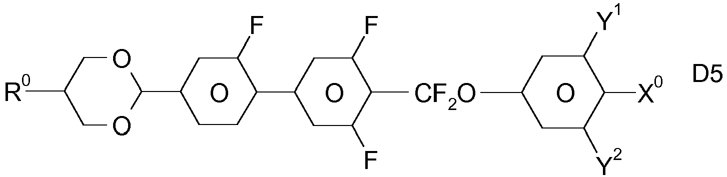 worin Y¹, Y², R⁰ und X⁰ die in Anspruch 6 angegebenen Bedeutungen besitzen. Vorzugsweise bedeutet R⁰ Alkyl mit 1 bis 8 C-Atomen und X⁰ F. Y¹ und Y² bedeuten vorzugsweise beide F.

Besonders bevorzugt sind Verbindungen der Formeln, 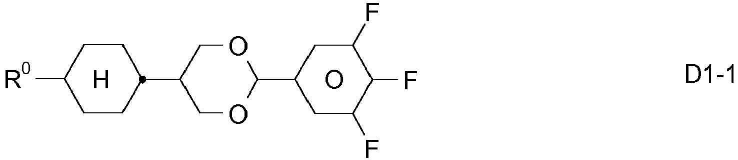 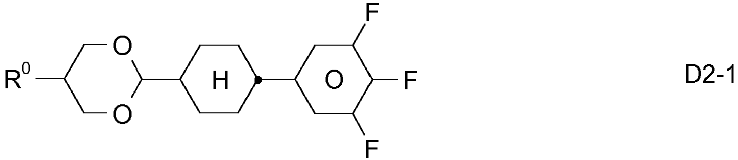 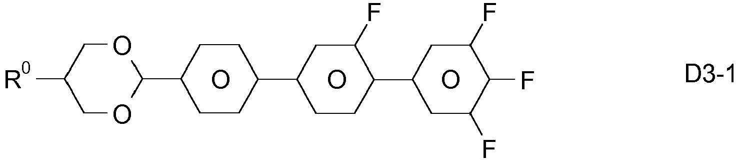 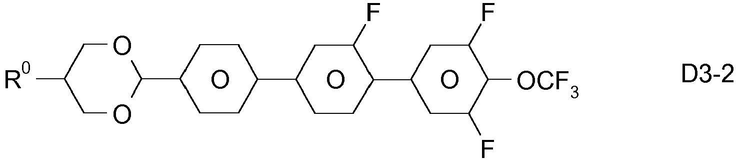 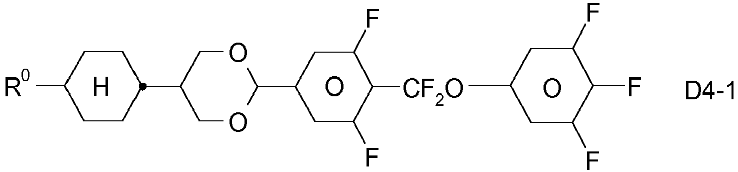 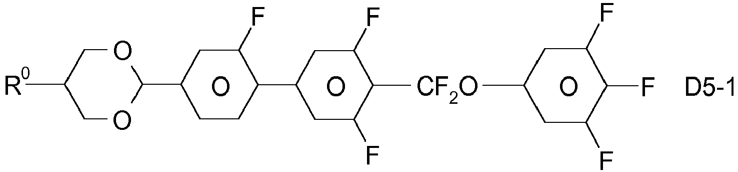 worin R⁰ die oben angegebenen Bedeutungen hat und vorzugsweise geradkettiges Alkyl mit 1 bis 6 C-Atomen, insbesondere C₂H₅, n-C₃H₇ oder n-C₅H₁₁ bedeutet.
- Das Medium enthält zusätzlich eine oder mehrere Verbindungen der folgenden Formel XVII, 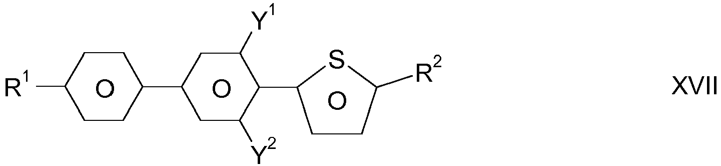 worin Y¹, R¹ und R² die oben angegebene Bedeutung besitzen. R¹ und R² bedeuten vorzugsweise jeweils unabhängig voneinander Alkyl oder Alkenyl mit 1 bzw. 2 bis 8 C-Atomen; Y¹ und Y² bedeuten vorzugsweise beide F. Die Verbindung(en) der Formel XVII werden vorzugsweise in Mengen von 3 - 30 Gew.% bezogen auf das Medium eingesetzt.
- Das Medium enthält zusätzlich eine oder mehrere Verbindungen der folgenden Formel, 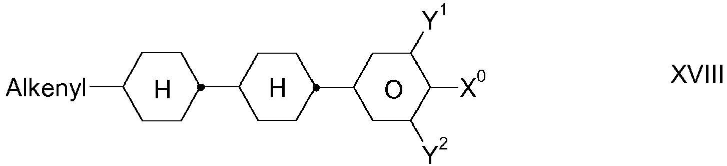 worin X⁰, Y¹ und Y² die in Anspruch 6 angegebenen Bedeutungen besitzen und "Alkenyl" C₂₋₇-Alkenyl bedeutet. Besonders bevorzugt sind Verbindungen der folgenden Formel, 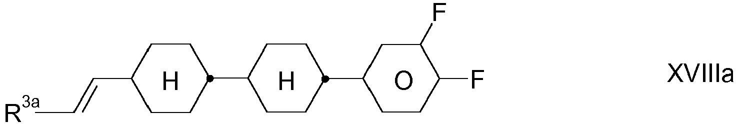 worin R^{3a} die oben angegebene Bedeutung hat und vorzugsweise H bedeutet;
- Das Medium enthält zusätzlich eine oder mehrere Vierkern-Verbindungen ausgewählt aus den Formeln XIX bis XXVIII, 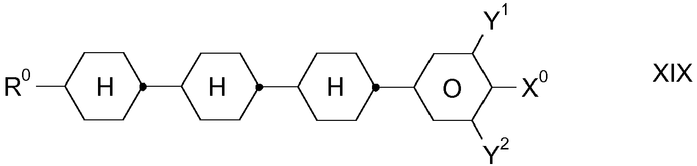 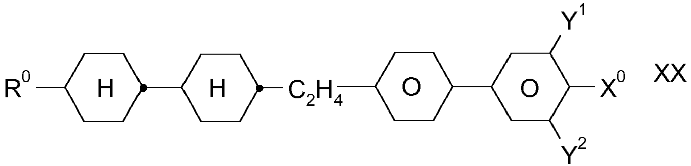 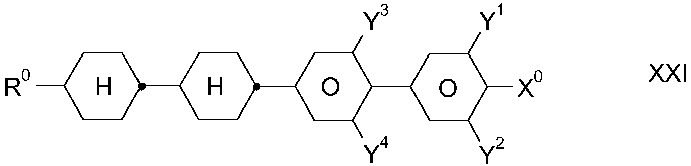 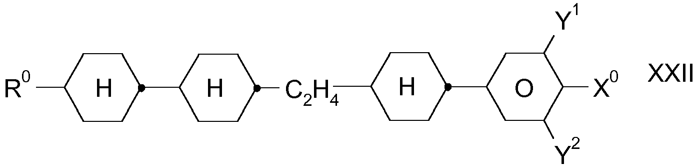 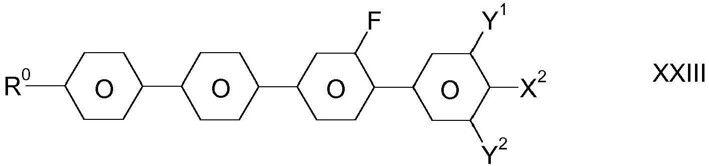 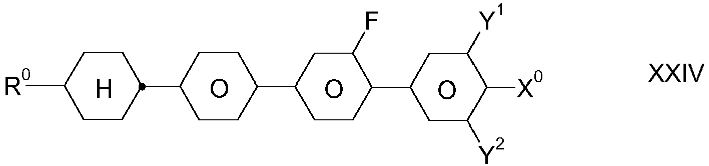 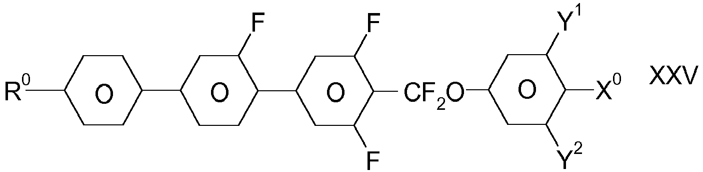 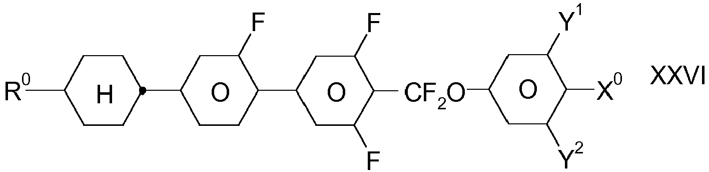 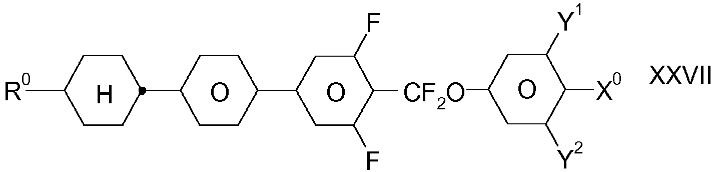 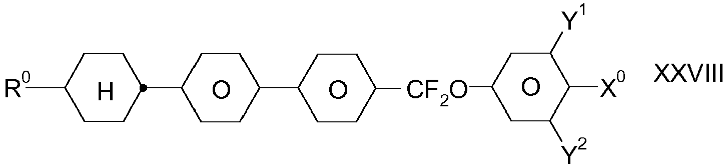 worin Y¹⁻⁴, R⁰ und X⁰ jeweils unabhängig voneinander eine der oben angegebenen Bedeutungen haben. X⁰ ist vorzugsweise F, Cl, CF₃, OCF₃ oder OCHF₂. R⁰ bedeutet vorzugsweise Alkyl, Alkoxy, Oxaalkyl, Fluoralkyl, Cycloalkyl oder Alkenyl mit jeweils bis zu 8 C-Atomen.

In den Verbindungen der Formeln XIX bis XXVIII bedeuten R⁰ vorzugsweise geradkettiges Alkyl. X⁰ ist vorzugsweise F oder OCF₃, ferner CF₃. Y¹ und Y² bedeuten vorzugsweise Y¹ = F und Y² = H oder Y¹=Y²=F.

Besonders bevorzugte Verbindungen der Formel XIX bis XXVIII sind die Verbindungen der Formeln XXV, worin X⁰ vorzugsweise F, ferner OCF₃ bedeutet.

Bevorzugte Mischungen enthalten mindestens eine Verbindung aus der Gruppe S-1, S-2, S-3 und S-4 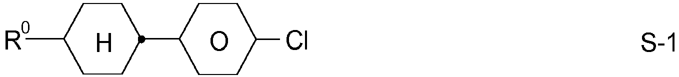 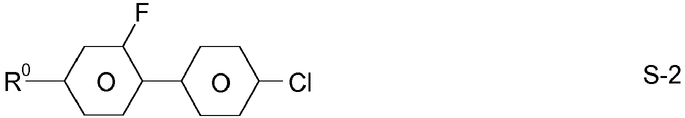 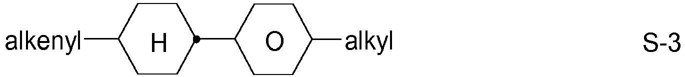 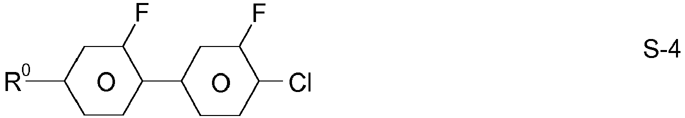 da diese Verbindungen u.a. die smektischen Phasen der Mischungen unterdrücken helfen.

Das Medium enthält vorzugsweise ein oder mehrere neutrale Verbindungen der allgemeinen Formel N, 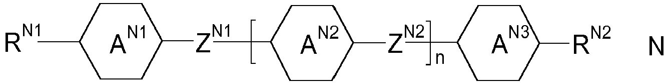 worin
- R^{N1} und R^{N2}: jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, 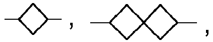 -O-, -CO-O-, -O-CO- so ersetzt sein können, dass O-Atome nicht direkt miteinander verknüpft sind, und worin auch ein oder mehrere H-Atome durch Halogen ersetzt sein können,
- Ringe A^{N1}, A^{N2} und A^{N3}: jeweils unabhängig voneinander 1,4-Phenylen, 2-Fluor-1,4-Phenylen, 3-Fluor-1,4-Phenylen, trans-1,4-Cyclohexylen, worin auch eine oder zwei CH₂-Gruppen durch -O- ersetzt sein können oder 1,4-Cyclohexenylen,
- Z^{N1} und Z^{N2}: jeweils unabhängig voneinander eine Einfachbindung, -CH₂CH₂-, -COO-, -OCO-, -C≡C-, -CH₂O-, -OCH₂-, -CF₂O-, -OCF₂- oder -CH=CH-,
- n: 0, 1 oder 2,
bedeuten, wobei die Verbindung der Formel N nicht identisch ist mit der Verbindung der Formel I.

Bevorzugte Verbindungen der Formel N werden nachfolgend genannt: 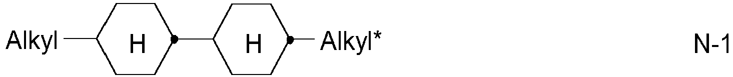 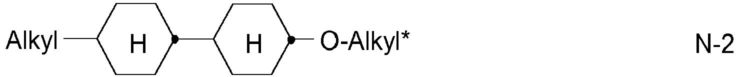 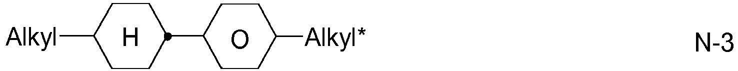 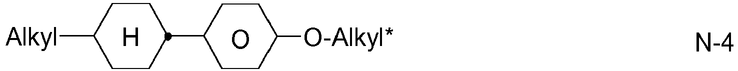 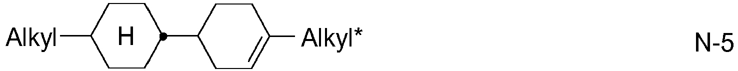 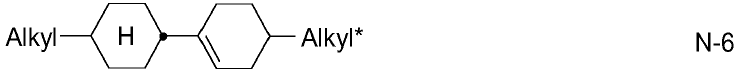 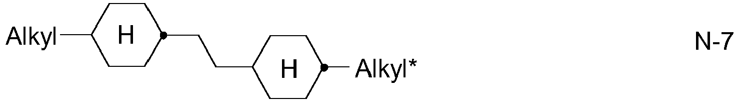 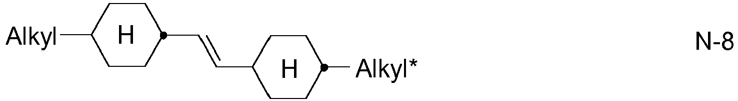 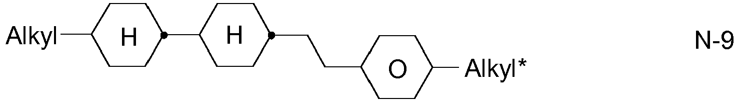 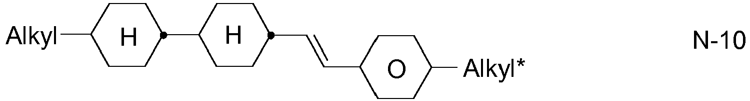 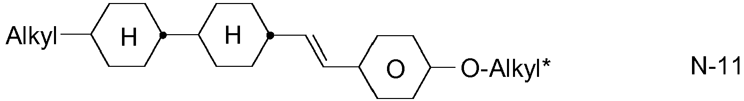 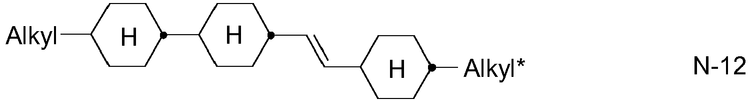 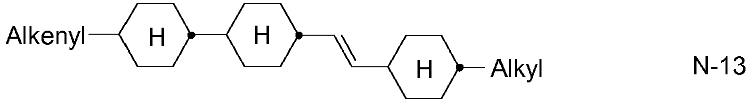 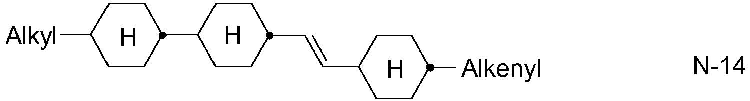 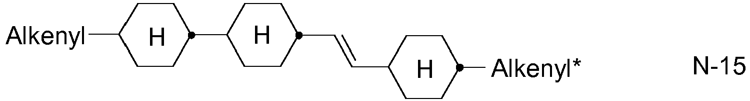 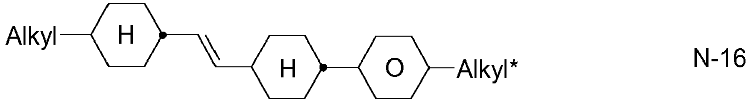 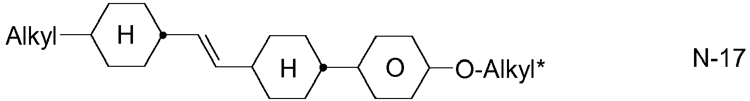 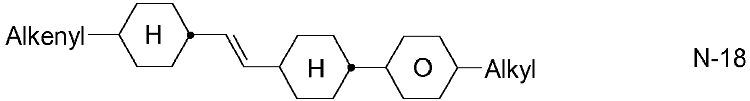 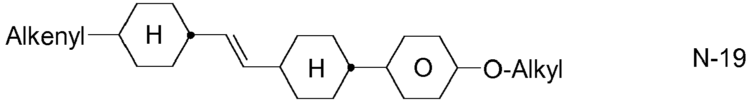 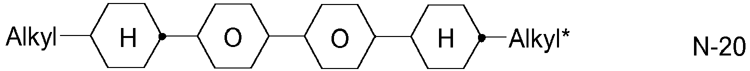 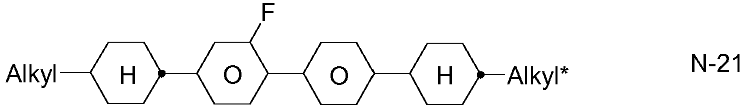 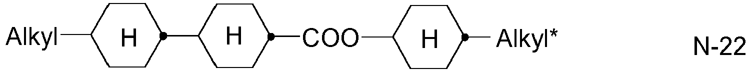 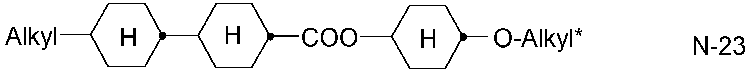 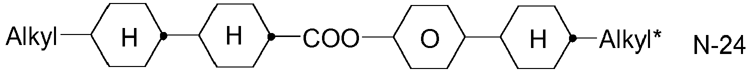 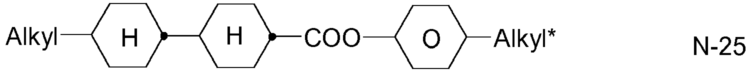 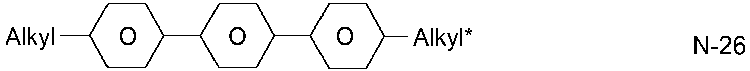 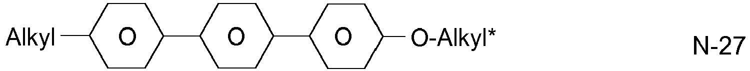 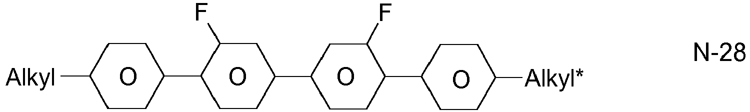 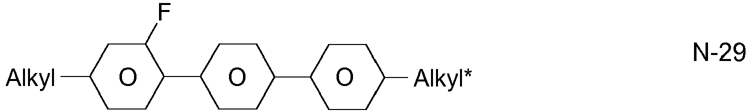 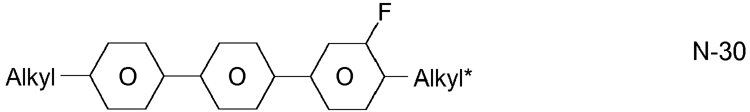 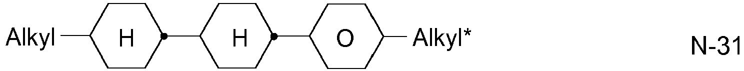 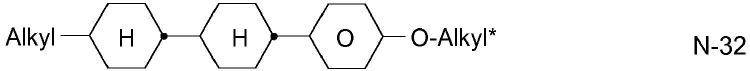 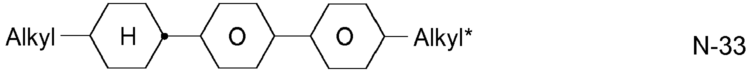 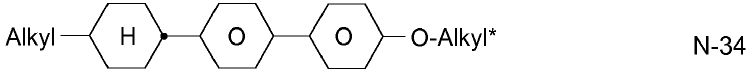 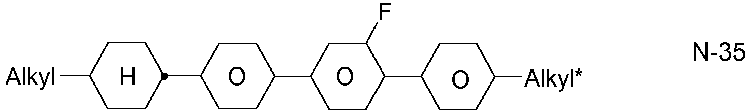 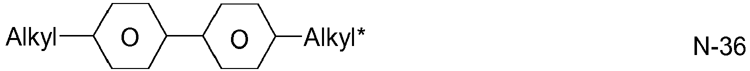 worin

Alkyl und Alkyl* jeweils unabhängig voneinander einen geradkettigen Alkylrest mit 1 bis 9 C-Atomen, vorzugsweise 2 bis 6 C-Atomen, bedeuten und Alkenyl und Alkenyl* jeweils unabhängig voneinander einen geradkettigen Alkenylrest mit 2-6 C-Atomen bedeuten.

Von den Verbindungen der Formel N sind insbesondere die Verbindungen der Formeln N-1, N-2, N-3, N-4, N-8, N-9, N-14, N-15, N-17, N-18, N-19, N-20, N-21, N-22, N-23, N-24, N-25, N-31, N-33 und N-36 bevorzugt.
- Das Medium enthält zusätzlich eine oder mehrere Verbindungen der Formeln St-1 bis St-3, 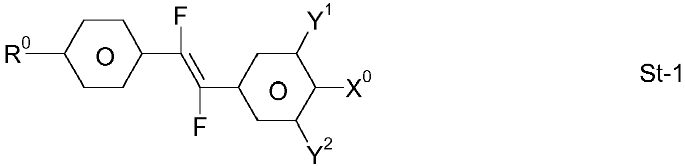 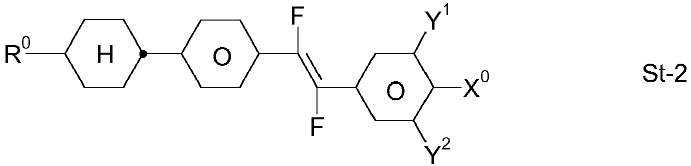 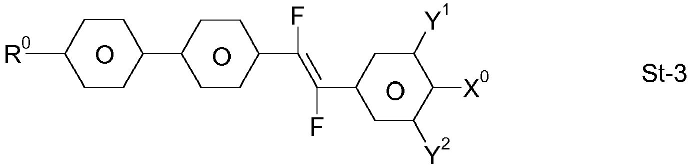 worin R⁰, Y¹, Y² und X⁰ die in Anspruch 6 angegebenen Bedeutungen haben. R⁰ bedeutet vorzugsweise geradkettiges Alkyl, vorzugsweise mit 1-6 C-Atomen. X⁰ ist vorzugsweise F, CF₃ oder OCF₃. Y¹ bedeutet vorzugsweise F. Y² bedeutet vorzugsweise F. Weiterhin sind Verbindungen bevorzugt, worin Y¹=F und Y²=H bedeuten. Vorzugsweise werden die Verbindungen der Formeln St-1 bis St-3 in Konzentration von 3 - 30 Gew.% in den erfindungsgemäßen Mischungen eingesetzt, insbesondere von 5 - 25 Gew.%.
- Das Medium enthält zusätzlich eine oder mehrere Pyrimidin- oder Pyridin-Verbindungen der Formeln Py-1 bis Py-5, 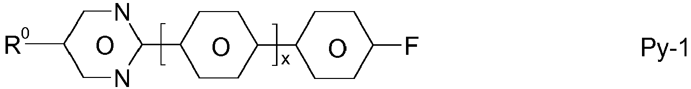 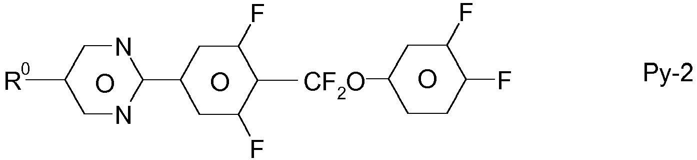 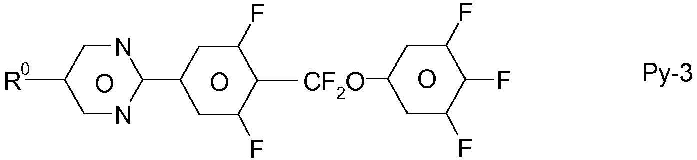 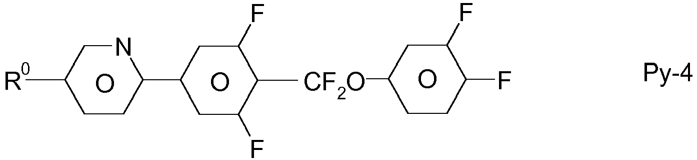 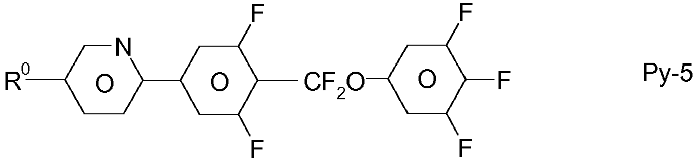 worin R⁰ vorzugsweise geradkettiges Alkyl mit 2-5 C-Atomen ist. x bedeutet 0 oder 1, vorzugsweise ist x=1. Bevorzugte Mischungen enthalten 3 - 30 Gew.%, insbesondere 5 - 20 Gew.% dieser Pyri(mi)d in-Verbindung(en).
- Das Medium enthält zusätzlich eine oder mehrere Verbindungen ausgewählt aus der Gruppe der Verbindungen der Formeln Y-1, Y-2, Y-3 und Y-4, 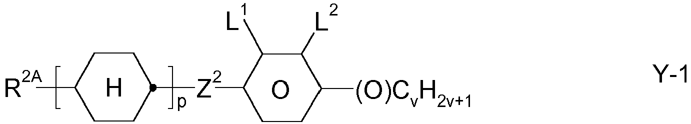 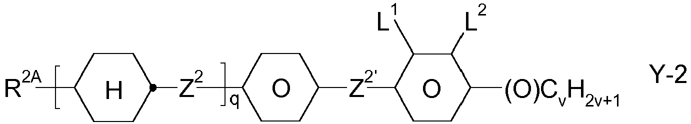 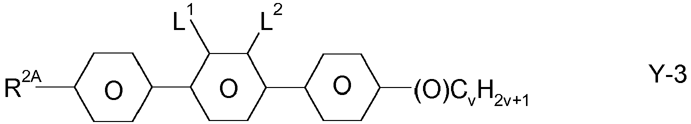 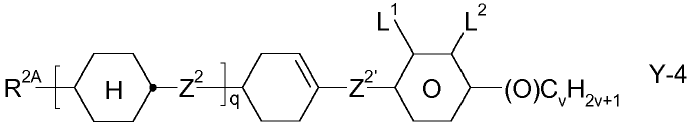 worin
   - R^{2A}: H, einen Alkyl- oder Alkoxyrest mit 1 bis 15 C-Atomen, wobei in diesen Resten auch eine oder mehrere CH₂-Gruppen jeweils unabhängig voneinander durch -CH=CH-, -C≡C-, -CF₂O-, -CH=CH-, 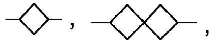 -O-, -CO-O-, -O-CO- so ersetzt sein können, dass O-Atome nicht direkt miteinander verknüpft sind, und worin auch ein oder mehrere H-Atome durch Halogen ersetzt sein können,
   - L¹ und L²: jeweils unabhängig voneinander F, Cl, CF₃ oder CHF₂, vorzugsweise jeweils F,
   - Z² und Z^{2'}: jeweils unabhängig voneinander eine Einfachbindung, -CH₂CH₂-, -CH=CH-, -CF₂O-, -OCF₂-,-CH₂O-, -OCH₂-, -COO-, -OCO-, -C₂F₄-, -CF=CF-, -CH= CHCH₂O-,
   - p: 0, 1 oder 2,
   - q: 0 oder 1,
   - (O)CᵥH₂ᵥ₊₁: OCᵥH₂ᵥ₊₁ oder CᵥH₂ᵥ₊₁, und
   - v: 1 bis 6.
   bedeuten.

Besonders bevorzugte Verbindungen der Formeln Y-1 bis Y-4 werden nachfolgend genannt: 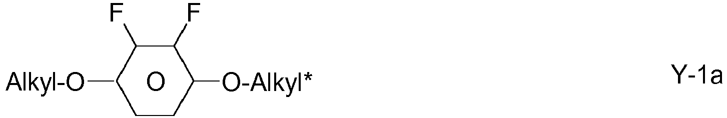 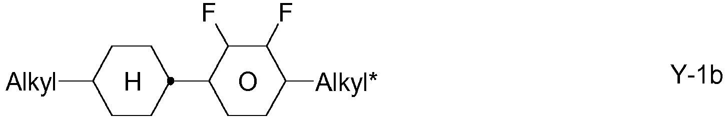 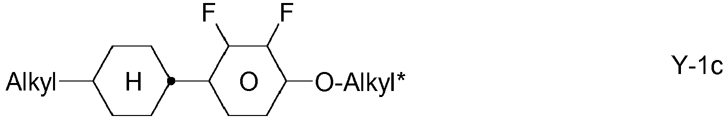 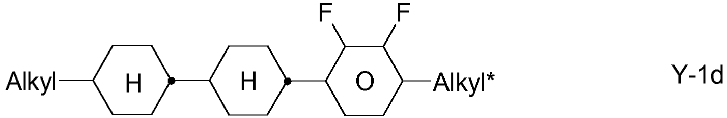 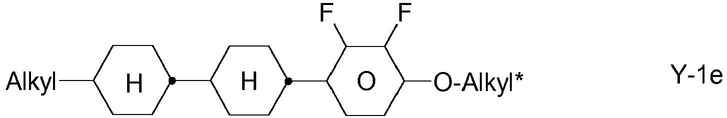 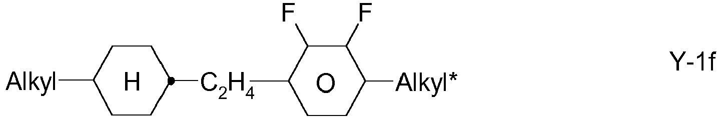 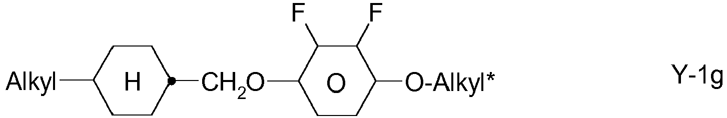 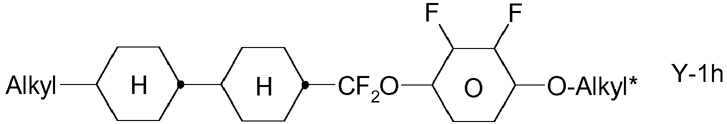 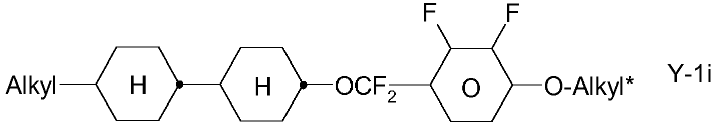 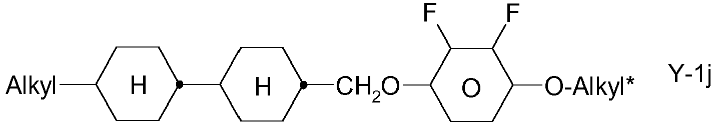 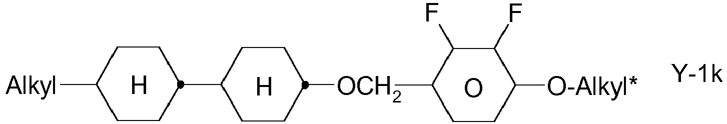 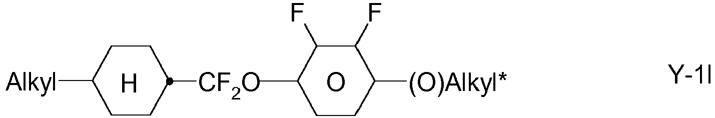 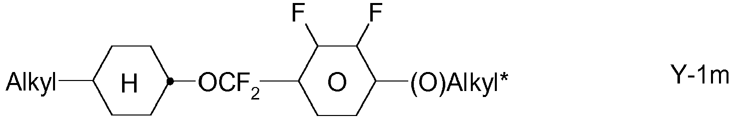 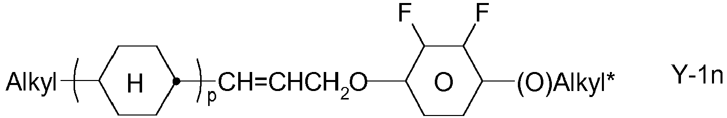 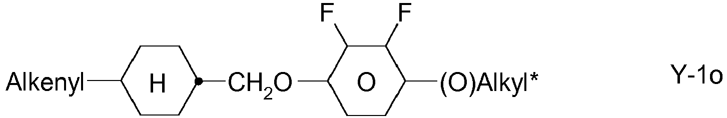 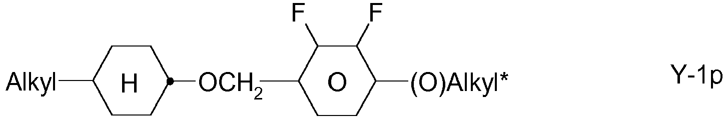 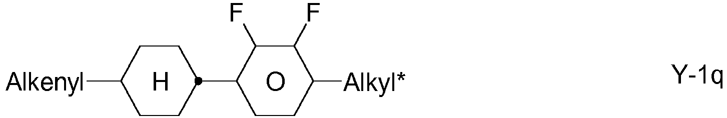 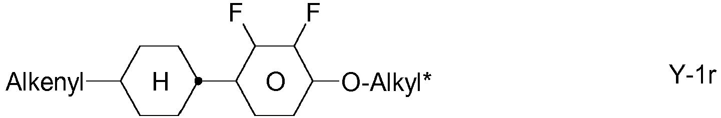 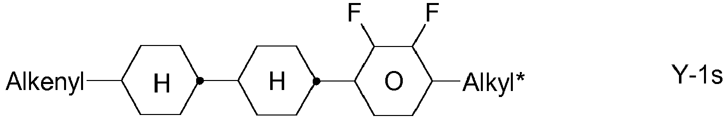 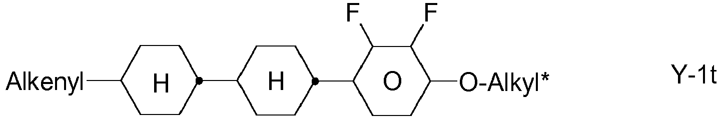 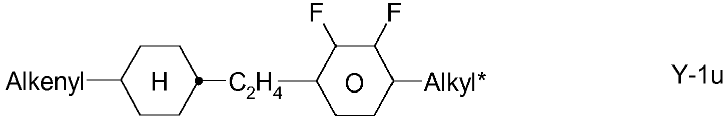 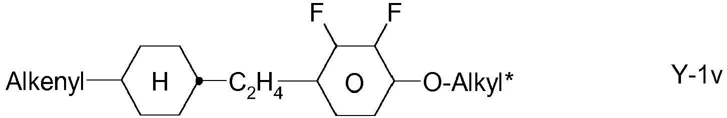 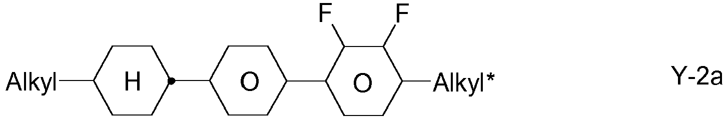 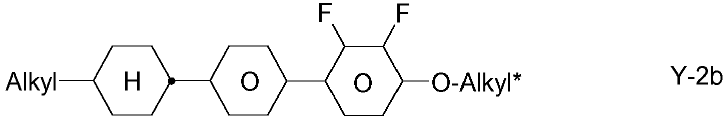 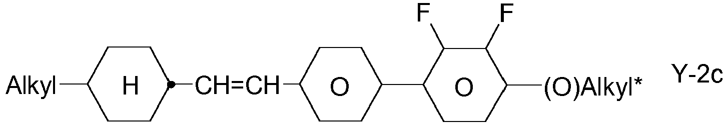 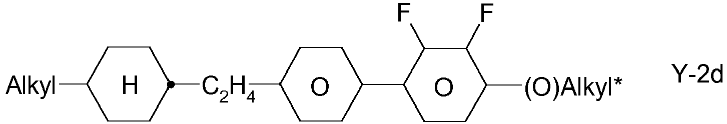 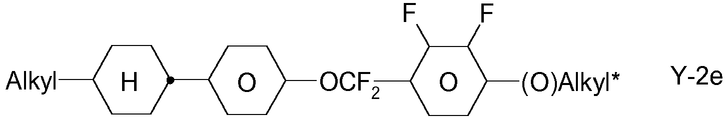 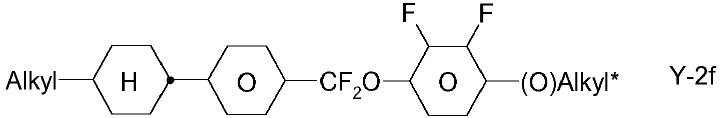 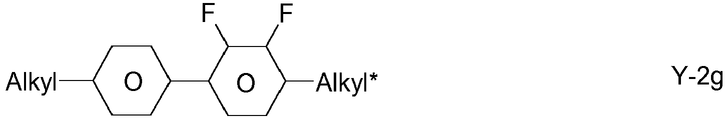 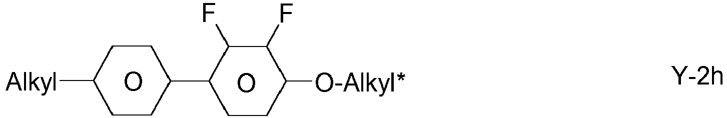 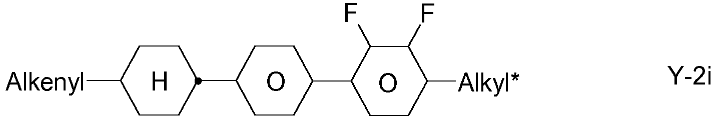 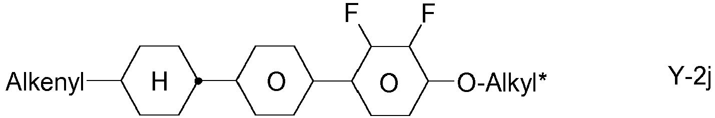 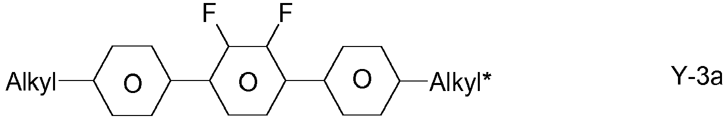 worin
- Alkyl und Alkyl*: jeweils unabhängig voneinander einen geradkettigen Alkylrest mit 1-6 C-Atomen und
- Alkenyl und Alkenyl*: jeweils unabhängig voneinander einen geradkettigen Alkenylrest mit 2-6 C-Atomen
bedeuten.

Von den genannten Verbindungen sind insbesondere die Verbindungen der Formel Y-1a, Y-1c, Y-1e, Y-1g, Y-1j, Y-1r, Y-1t, Y-2b, Y-2h, Y-2j und Y-3a bevorzugt.

Der Anteil der Verbindungen der Formeln Y-1 bis Y-3 in den erfindungsgemäßen Mischungen beträgt vorzugsweise 0-30 Gew.%.
- Das Medium enthält zusätzlich eine oder mehrere Verbindungen ausgewählt aus der Gruppe der Verbindungen der Formeln BC, CR, PH-1, PH-2, BF und BS, 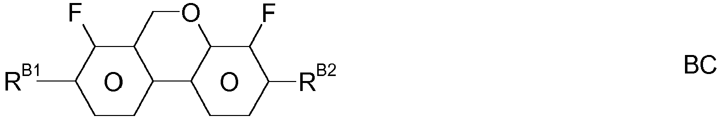 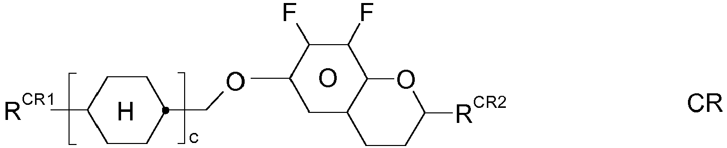 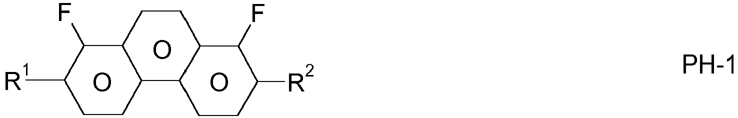 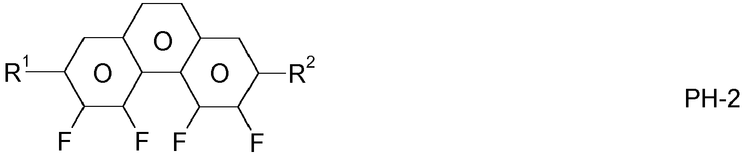 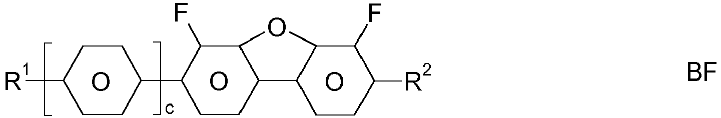 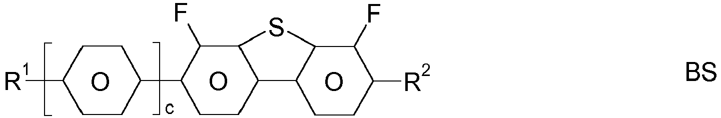 worin
   R^{B1}, R^{B2}, R^{CR1}, R^{CR2}, R¹, R² jeweils unabhängig voneinander die Bedeutung von R^{2A} aufweisen. c ist 0, 1 oder 2.

Die erfindungsgemäßen Mischungen enthalten die Verbindungen der Formeln BC, CR, PH-1, PH-2 und/oder BF vorzugsweise in Mengen von 0,5 bis 20 Gew.%, insbesondere in Mengen von 1 bis 15 Gew.%.

Besonders bevorzugte Verbindungen der Formeln BC, CR, BF und BS sind die Verbindungen BC-1 bis BC-7, CR-1 bis CR-5, BF-1 bis BF-3 und BS-1 bis BS-3, 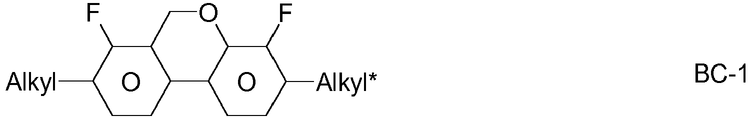 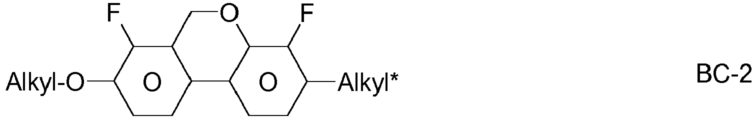 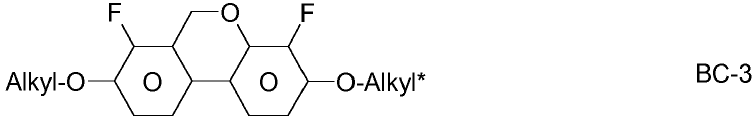 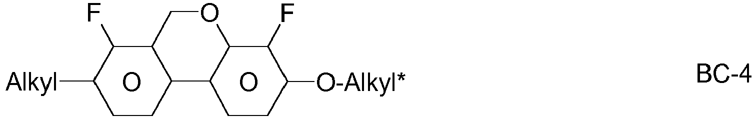 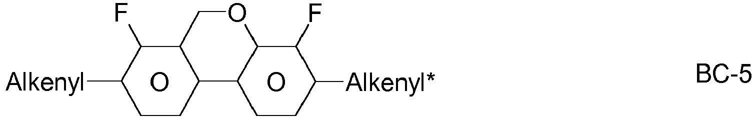 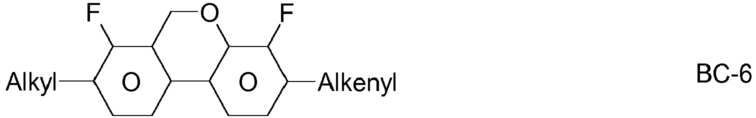 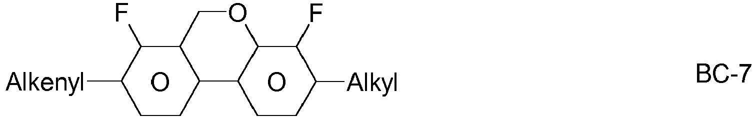 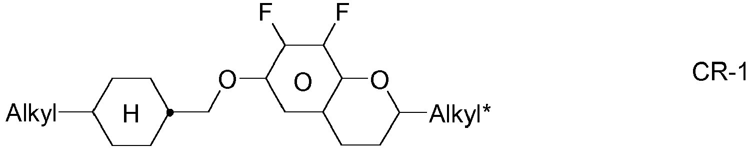 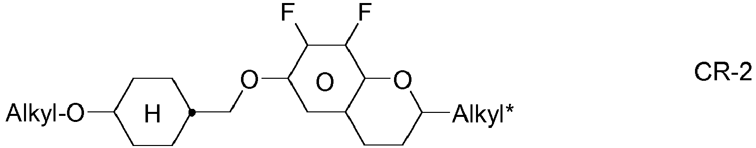 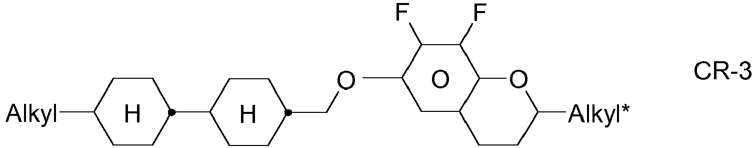 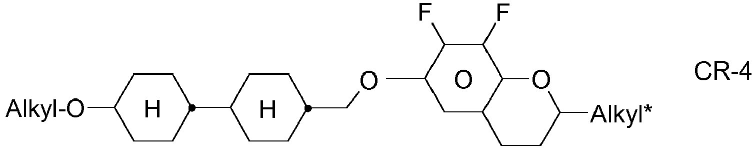 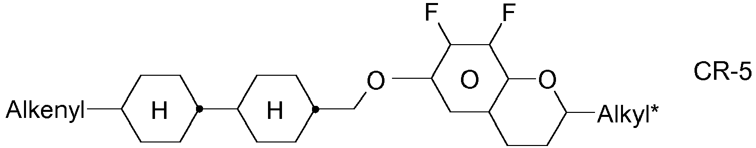 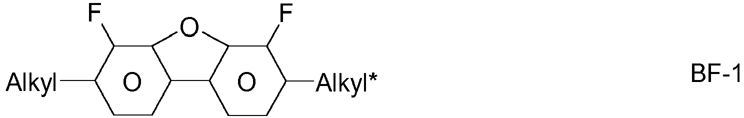 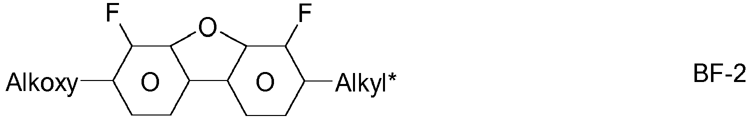 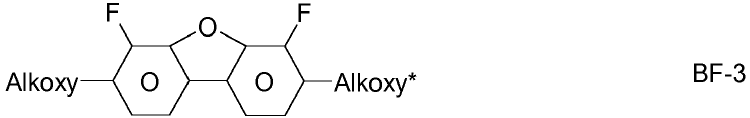 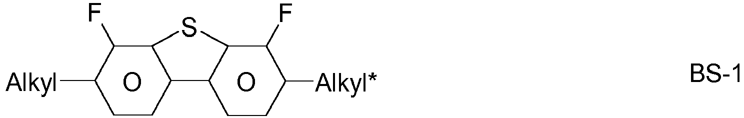 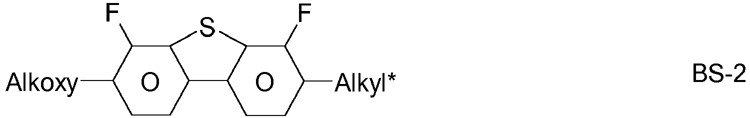 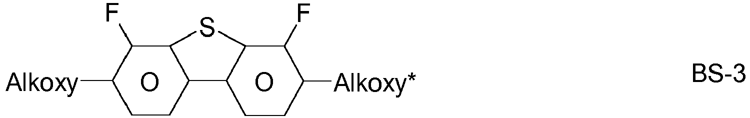 worin
- Alkyl und Alkyl*: jeweils unabhängig voneinander einen geradkettigen Alkylrest mit 1-6 C-Atomen,
- Alkenyl und Alkenyl*: jeweils unabhängig voneinander einen geradkettigen Alkenylrest mit 2-6 C-Atomen, und
- Alkoxy und Alkoxy*: jeweils unabhängig voneinander einen geradkettigen Alkoxyrest mit 1-6 C-Atomen,
bedeuten.

Ganz besonders bevorzugt sind Mischungen enthaltend eine, zwei oder drei Verbindungen der Formel BF-3 und BS-3.

In den vorstehend und nachfolgend genannten Formeln bedeutet 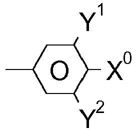 vorzugsweise 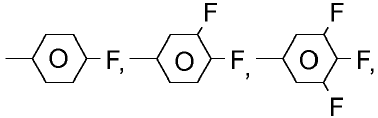 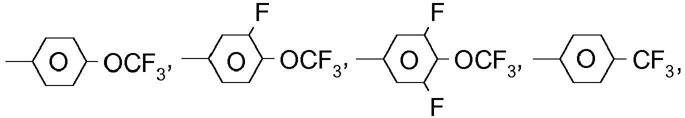 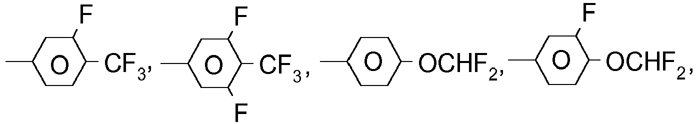 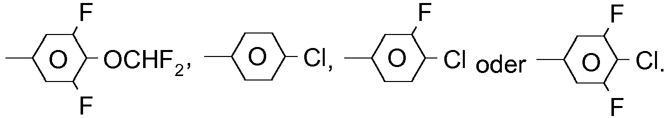
- R⁰ ist vorzugsweise geradkettiges Alkyl oder Alkenyl mit 2 bis 7 C-Atomen;
- X¹ in Formel I ist vorzugsweise CF₃, ferner OCF₃;
- Das Medium enthält vorzugsweise eine, zwei oder drei Verbindungen der Formel I;
- Das Medium enthält CLP-n-T und/oder CLP-n-OT, wobei n 2, 3, 4 oder 5 bedeutet;
- Das Medium enthält CLP-n-T, wobei n 2, 3, 4 oder 5 bedeutet, und/oder CLP-V-n, wobei n 1, 2 oder 3, vorzugsweise 1, bedeutet;
- Das Medium enthält CLP-V-T, CLP-nV-T oder CLP-Vn-T, wobei n 1 oder 2 bedeutet;
- Das Medium enthält CLP-V-OT, CLP-nV-OT oder CLP-Vn-OT, wobei n 1 oder 2 bedeutet;
- Das Medium enthält CLP-nV-m oder CLP-Vn-m, wobei m 1 oder 2 und n 1 oder 2 bedeutet;
- Das Medium enthält CLP-nV2-m, CLP-nV2-T oder CLP-nV2-OT, wobei m 1 oder 2 und n 1 oder 2 bedeutet;
- Das Medium enthält vorzugsweise eine oder mehrere Verbindungen ausgewählt aus der Gruppe der Verbindungen der Formeln I, II, III, V, VI-1, VI-2, XII, XIII, XIV, XVII, XXIII, XXV;
- Das Medium enthält vorzugsweise eine oder mehrere Verbindungen der Formel VI-1;
- Das Medium enthält vorzugsweise eine oder mehrere Verbindungen der Formel VI-2;
- Das Medium enthält vorzugsweise 1-30 Gew.%, bevorzugt 2-20 Gew.%, besonders bevorzugt 2-15 Gew.%, an Verbindungen der Formel I;
- Der Anteil an Verbindungen der Formeln II-XXVII im Gesamtgemisch beträgt vorzugsweise 20 bis 99 Gew.%;
- Das Medium enthält vorzugsweise 25-80 Gew.%, besonders bevorzugt 30-70 Gew.% an Verbindungen der Formel II und/oder III;
- Das Medium enthält vorzugsweise 0-70 Gew.%, besonders bevorzugt 20-60 Gew.% an Verbindungen der Formel IIa-1;
- Das Medium enthält vorzugsweise 0-25 Gew.%, besonders bevorzugt 5-25 Gew.% an Verbindungen der Formel IIa-2;
- Das Medium enthält vorzugsweise 0-30 Gew.%, besonders bevorzugt 5-25 Gew.% an Verbindungen der Formel IIa-3;
- Das Medium enthält vorzugsweise 0-25 Gew.%, besonders bevorzugt 5-25 Gew.% an Verbindungen der Formel IIa-5;
- Das Medium enthält vorzugsweise 5-40 Gew.%, besonders bevorzugt 10-30 Gew.% an Verbindungen der Formel V;
- Das Medium enthält vorzugsweise 3-30 Gew.%, besonders bevorzugt 6-25 Gew.% an Verbindungen der Formel VI-1;
- Das Medium enthält vorzugsweise 2-30 Gew.%, besonders bevorzugt 4-25 Gew.% an Verbindungen der Formel VI-2;
- Das Medium enthält 5-40 Gew.%, besonders bevorzugt 10-30 Gew.% an Verbindungen der Formel XII;
- Das Medium enthält vorzugsweise 1-25 Gew.%, besonders bevorzugt 2-15 Gew.%, an Verbindungen der Formel XIII;
- Das Medium enthält vorzugsweise 5-45 Gew.%, besonders bevorzugt 10-35 Gew.%, an Verbindungen der Formel XIV;
- Das Medium enthält vorzugsweise 1-20 Gew.%, besonders bevorzugt 2-15 Gew.% an Verbindungen der Formel XVI;
- Das Medium enthält vorzugsweise 5 - 30 Gew.%, besonders bevorzugt 8 - 22 Gew.%, an Verbindungen der Formel Va, worin X⁰ = OCH=CF₂ bedeutet;
- Das Medium enthält vorzugsweise die Verbindung der Formel CC-3-2V und die Verbindung der Formel CC-3-V und/oder CC-3-V1;
- Das Medium enthält vorzugsweise die Verbindung der Formel CC-3-2V und die Verbindung der Formel APUQU-2-F und/oder APUQU-3-F;
- Das Medium enthält vorzugsweise die Verbindung CC-3-2V und die Verbindung der Formel CC-3-2V1;
- Das Medium enthält vorzugsweise die Verbindung CC-3-2V und die Verbindung der Formel PP-1-2V1;
- Das Medium enthält vorzugsweise die Verbindung CC-3-2V und mindestens eine Verbindung der Formel PGUQU-n-F, wobei n = 3, 4 oder 5 bedeutet;
- Das Medium enthält vorzugsweise die Verbindung CC-3-2V und mindestens eine Verbindung der Formel DPGU-n-F, wobei n = 2, 3, 4, oder 5 bedeutet.

Es wurde gefunden, dass bereits ein relativ geringer Anteil an Verbindungen der Formel I im Gemisch mit üblichen Flüssigkristallmaterialien, insbesondere jedoch mit einer oder mehreren Verbindungen der Formeln II bis XXVII zu einer klaren Verringerung des Schalzeitparameters γ₁/K₁ führt. Weiterhin zeichnet sich das erfindungs-gemäße flüssigkristalline Medium durch seine relativ hohen Werte für die Doppelbrechung aus sowie durch seine Lichtstabilität. Gleichzeitig zeigen die Mischungen sehr gute Werte für die VHR nach UV-Belastung.

Der Ausdruck "Alkyl" bzw. "Alkyl*" umfasst in dieser Anmeldung geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-6 Kohlenstoffatomen sind im allgemeinen bevorzugt.

Der Ausdruck "OAlkyl" umfasst in dieser Anmeldung geradkettige und verzweigte Alkoxygruppen.

Der Ausdruck "Alkenyl" bzw. "Alkenyl*" umfasst in dieser Anmeldung geradkettige und verzweigte Alkenylgruppen mit 2-7 Kohlenstoffatomen,insbesondere die geradkettigen Gruppen. Bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele besonders bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst in dieser Anmeldung geradkettige Gruppen mit mindestens einem Fluoratom, vorzugsweise einem endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst in dieser Anmeldung geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Durch geeignete Wahl der Bedeutungen von R¹ und R² in Formel I können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Mischungen zeichnen sich insbesondere durch hohe K₁-Werte aus und besitzen somit deutlich schnellere Schaltzeilen als die Mischungen aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

In einer besonders bevorzugten Ausführungsform enthalten die erfindungsgemäßen Medien Verbindungen der Formel IV bis VIII, worin X⁰ F, OCF₃, OCHF₂, OCH=CF₂, OCF=CF₂ oder OCF₂-CF₂H bedeutet. Eine günstige synergistische Wirkung mit den Verbindungen der Formel I führt zu besonders vorteilhaften Eigenschaften. Insbesondere Mischungen enthaltend Verbindungen der Formeln I und VI, bzw. I und XI, bzw. I und VI und XI zeichnen sich durch ihre niedrigen Schwellenspannungen aus.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. TN-, STN-, TFT-, OCB-, IPS-, PS-IPS, FFS-, PS-FFS-, positive VA- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Weiterhin sind die erfindungsgemäßen Mischungen auch für positiv-VA-Anwendungen geeignet, auch HT-VA-Anwendungen genannt. Hierunter versteht man elektrooptische Anzeigen mit einer In-plane Ansteuerelektroden-Konfiguration und homeotroper Anordnung des Flüssigkristallmediums mit positiver dielektrischer Anisotropie.

Insbesondere bevorzugt sind die erfindungsgemäßen Mischungen für TN-TFT-Display-Anwendungen mit kleiner Operationsspannung, d.h. besonders bevorzugt für Notebook-Anwendungen.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Rotationsviskosität und elastischen Konstanten, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und high-Δn-TFT-Anwendungen wie z. B. PDAs, Notebooks, LCD-TV und Monitore geeignet.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥ 74 °C, gleichzeitig Rotationsviskositäten γ₁ von ≤ 120 mPa·s, besonders bevorzugt 60 mPa·s zu erreichen, wodurch hervorragende MFK-Anzeigen mit schnellen Schaltzeiten erzielt werden können.

Die dielektrische Anisotropie der erfindungsgemäßen Flüssigkristallmischungen Δε ist vorzugsweise ≥ +2, besonders bevorzugt ≥ +4.

Die Doppelbrechung Δn der erfindungsgemäßen Flüssigkristallmischungen ist vorzugsweise ≥ 0,08, insbesondere ≥ 0,10.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -20 °C bis +70 °C.

Sofern die erfindungsgemäßen Mischungen in IPS- oder FFS-Anwendungen zum Einsatz kommen, besitzen die Mischungen vorzugsweise einen Wert für die dielektrische Anisotropie von 2-30 und einen Wert für die optische Anisotropie von 0,07-0,13.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.
Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise, beispielsweise indem man eine oder mehrere Verbindungen der Formel I mit einer oder mehreren Verbindungen der Formeln II-XXVII oder mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin® der Fa. Ciba Chemicals, insbesondere Tinuvin® 770, Antioxidantien, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Zur Erhöhung der Ankerkraft können den erfindungsgemäßen Mischungen zusätzlich auch polymerisierbare Verbindungen, sogenannte "Reaktive Mesogene", zugesetzt werden. Bevorzugte polymerisierbare Verbindungen sind in der Tabelle E gelistet.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben.

In der gesamten Patentanmeldung werden 1,4-Cyclohexylenringe und 1,4-Phenylenringe wie folgt dargestellt:

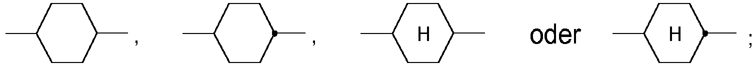

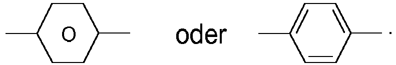

Bei den Cyclohexylenringen handelt es sich um trans-1,4-Cyclohexylenringe.

In der gesamten Patentanmeldung sowie in den Ausführungsbeispielen sind die Strukturen der Flüssigkristallverbindungen durch Acronyme angegeben. Sofern nicht anders angegeben, erfolgt die Transformation in chemische Formeln gemäß der Tabellen 1-3. Alle Reste CₙH₂ₙ₊₁, CₘH₂ₘ₊₁, und C_{m'}H_{2m'+1} bzw. CₙH₂ₙ und CₘH₂ₘ sind geradkettige Alkylreste bzw. Alkylenreste jeweils mit n, m, m' bzw. z C-Atomen.n, m, m', z bedeuten jeweils unabhängig voneinander 1, 2, 3, 4, 5, 6, 7, 8, 9, 10, 11, 12, vorzugsweise 1, 2, 3, 4, 5 oder 6. In Tabelle 1 sind die Ringelemente der jeweiligen Verbindung codiert, in Tabelle 2 sind die Brückenglieder aufgelistet und in Tabelle 3 sind die Bedeutungen der Symbole für die linken bzw. rechten Seitenketten der Verbindungen angegeben.

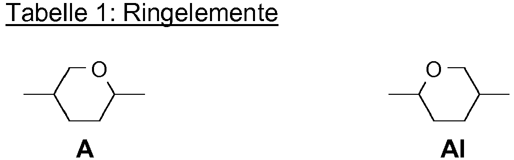

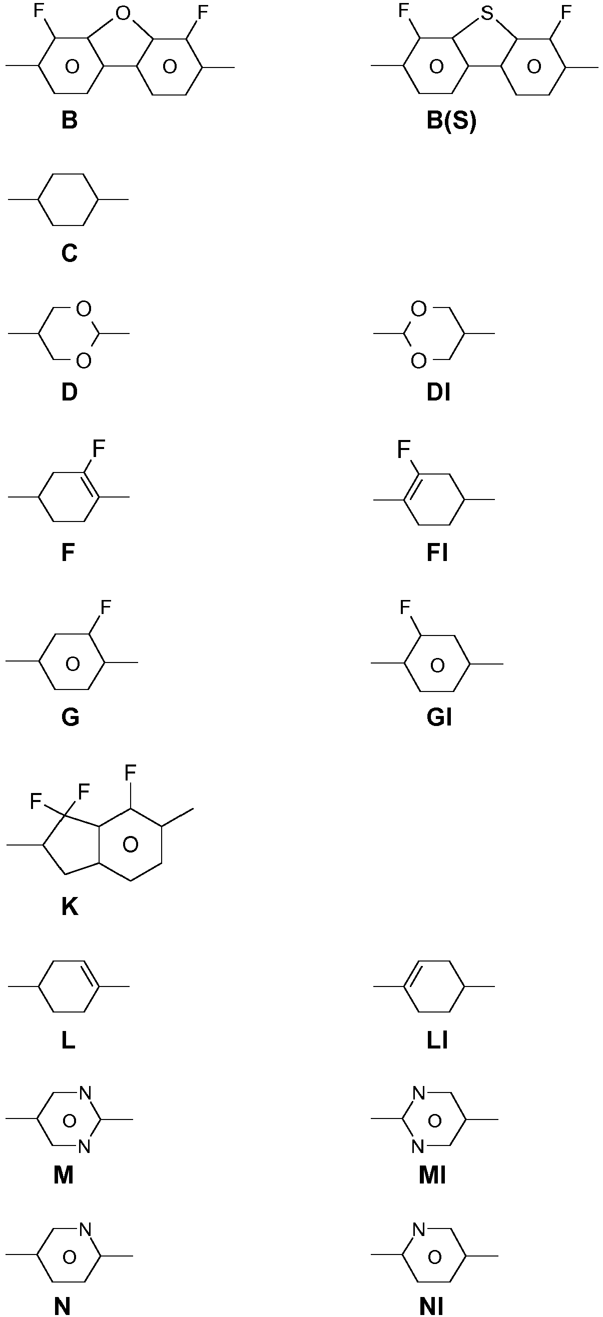

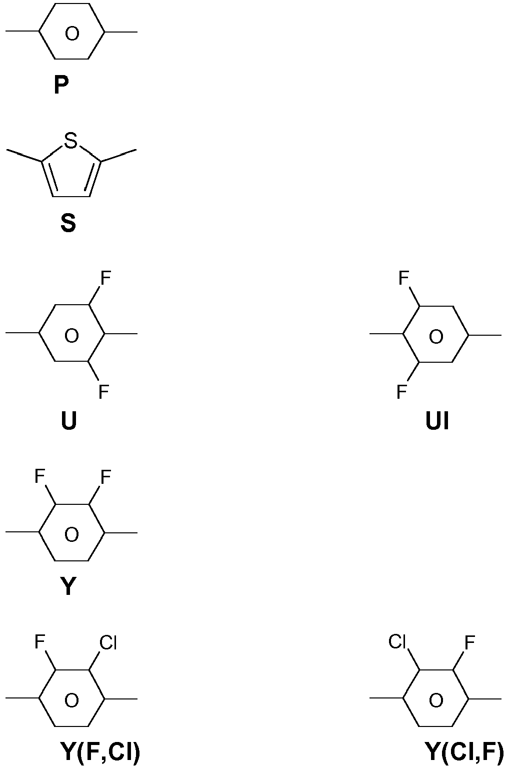

**Tabelle 2: Brückenglieder**

| | | | |
|---|---|---|---|
| **E** | -CH₂CH₂- | | |
| **V** | -CH=CH- | | |
| **T** | -C≡C- | | |
| **W** | -CF₂CF₂- | | |
| **Z** | -COO- | **ZI** | -OCO- |
| **O** | -CH₂O- | **OI** | -OCH₂- |
| **Q** | -CF₂O- | **QI** | -OCF₂- |

**Tabelle 3: Seitenketten**

| Seiten kette links | | Seiten kette rechts | |
|---|---|---|---|
| **n-** | CₙH₂ₙ₊₁- | **-n** | -CₙH₂ₙ₊₁ |
| **nO-** | CₙH₂ₙ₊₁-O- | **-On** | -O-CₙH₂ₙ₊₁ |
| **V-** | CH₂=CH- | **-V** | -CH=CH₂ |
| **nV-** | CₙH₂ₙ₊₁-CH=CH- | **-nV** | -CₙH₂ₙ-CH=CH₂ |
| **Vn-** | CH₂=CH-CₙH₂ₙ- | **-Vn** | -CH=CH-CₙH₂ₙ₊₁ |
| **nVm-** | CnH₂ₙ+₁-CH=CH-CmH₂ₘ- | **-nVm** | - CₙH₂ₙ-CH=CH-CₘH₂ₘ₊₁ |
| **N-** | N≡C- | **-N** | -C≡N |
| **F-** | F- | **-F** | -F |
| **Cl-** | Cl- | **-Cl** | -Cl |
| **M-** | CFH₂- | **-M** | -CFH₂ |
| **D-** | CF₂H- | **-D** | -CF₂H |
| **T-** | CF₃- | **-T** | -CF₃ |
| **MO-** | CFH₂O- | **-OM** | -OCFH₂ |
| **DO-** | CF₂HO- | **-OD** | -OCF₂H |
| **TO-** | CF₃O- | **-OT** | -OCF₃ |
| **T-** | CF₃- | **-T** | -CF₃ |
| **A-** | H-C≡C- | **-A** | -C≡C-H |
| **FXO-** | CF₂=CHO- | **-OXF** | -OCH=CF₂ |
| **C3-** | 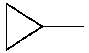 | **-C3** | 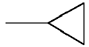 |
| **C4-** | 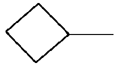 | **-C4** | 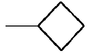 |
| **C5-** | 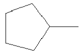 | **-C5** | 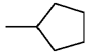 |

Bevorzugte Mischungskomponenten finden sich in den Tabellen A und B.

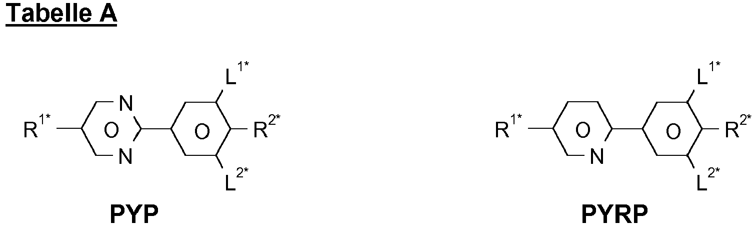

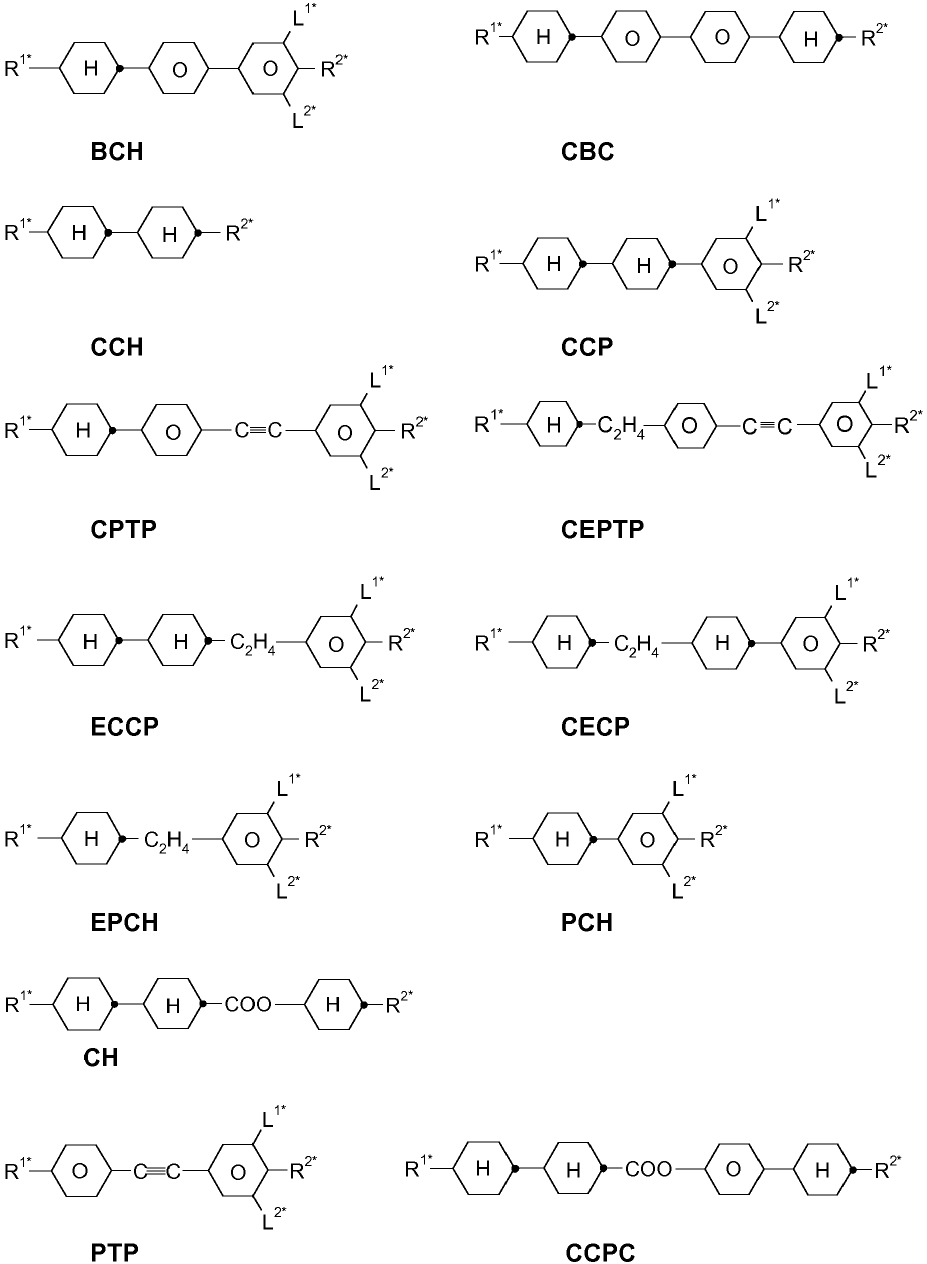

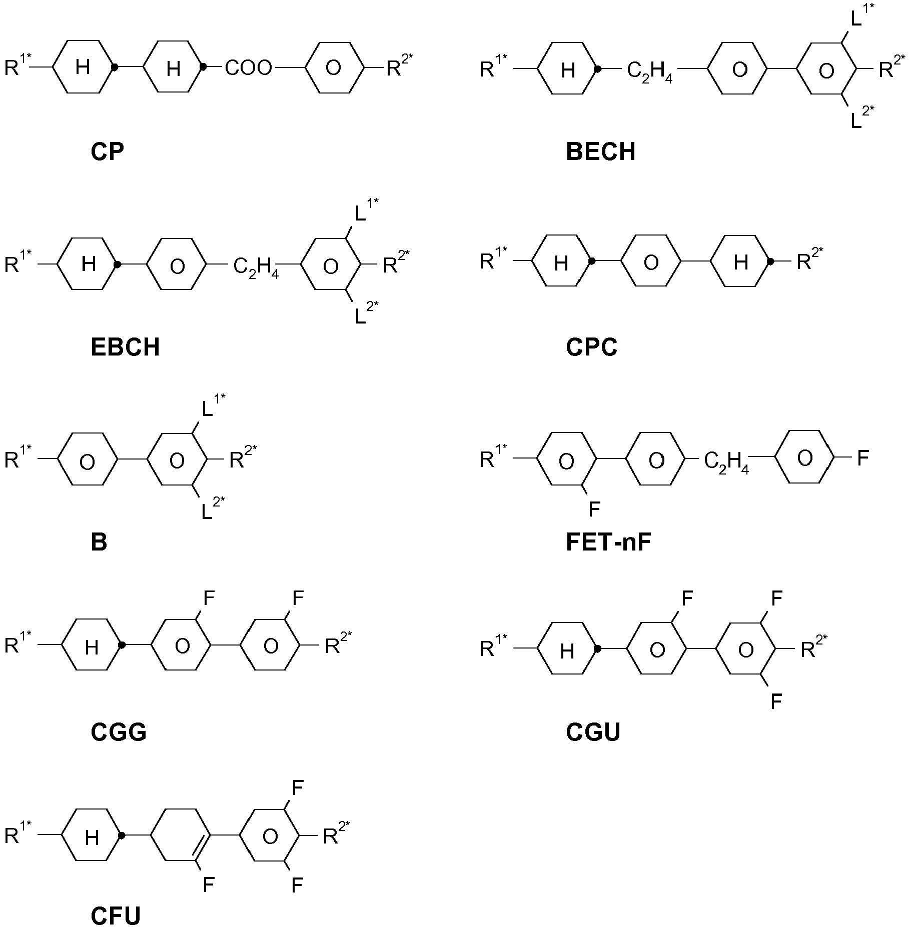

**Tabelle B**

| |
|---|
| (n = 1-15; (O)CₙH₂ₙ₊₁ bedeutet CₙH₂ₙ₊₁ oder OCₙH₂ₙ₊₁) |
| 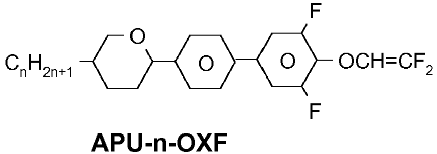 |
| 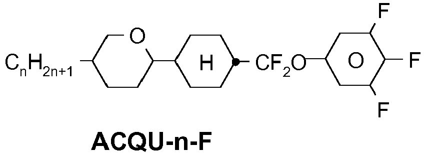 |
| 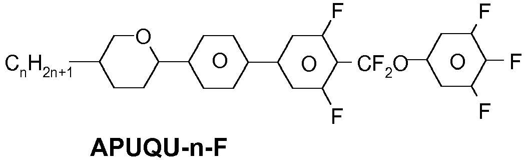 |
| 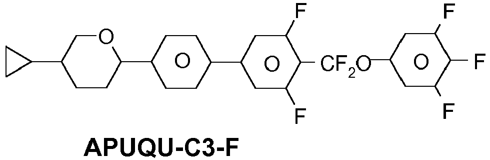 |
| 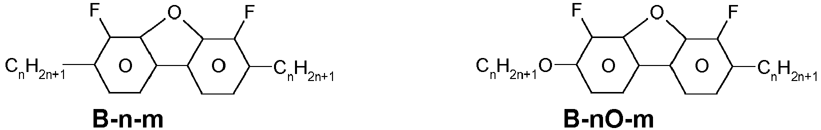 |
| 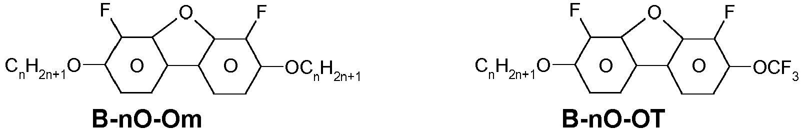 |
| 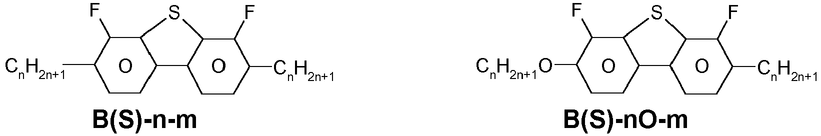 |
| 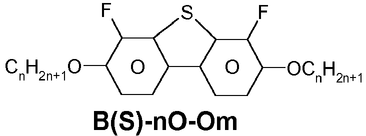 |
| 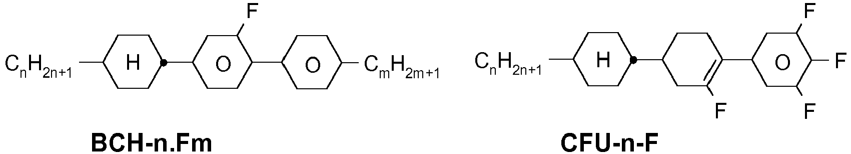 |
| 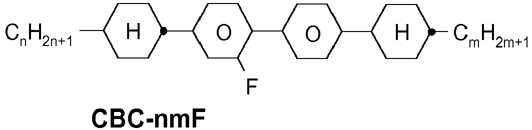 |
| 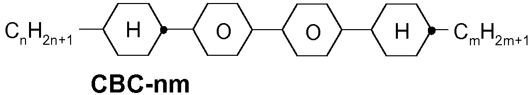 |
| 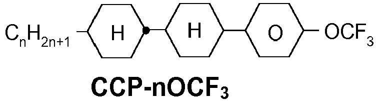 |
| 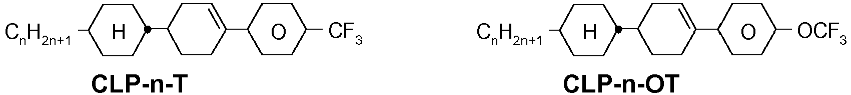 |
| 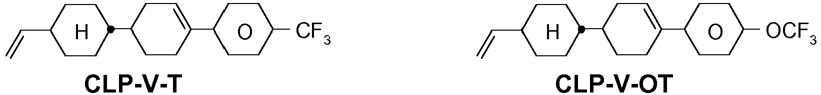 |
| 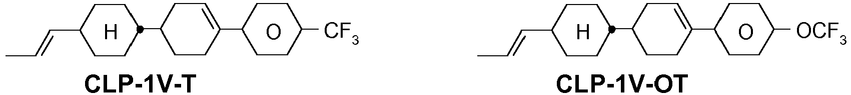 |
| 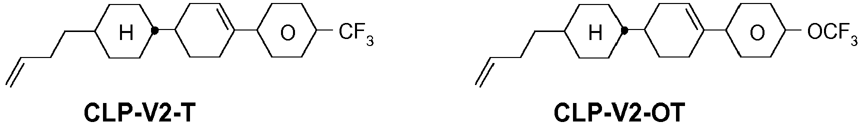 |
| 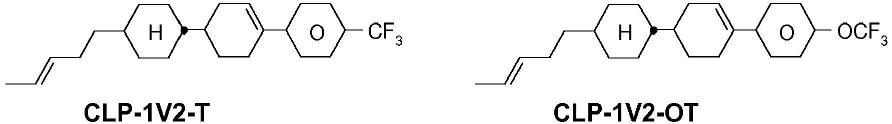 |
| 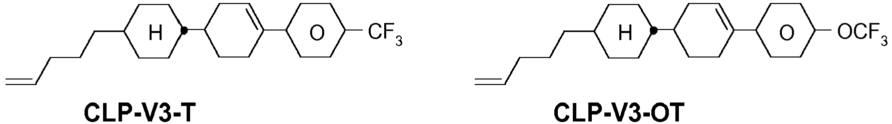 |
| 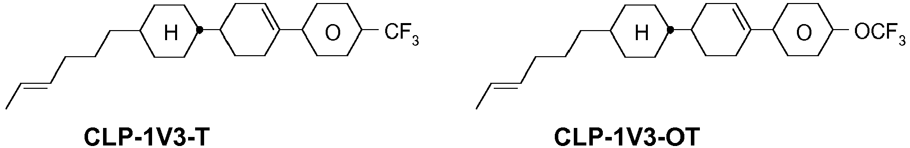 |
| 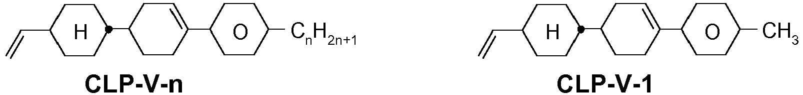 |
| 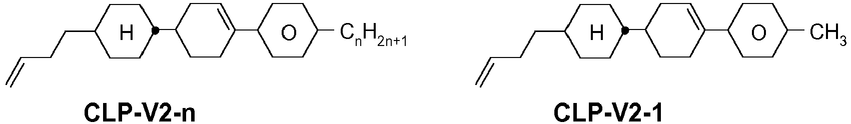 |
| 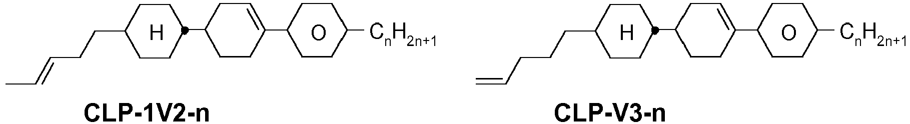 |
| 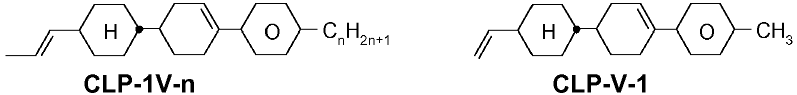 |
| 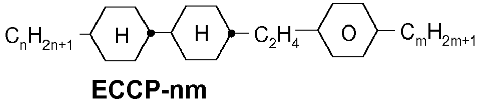 |
| 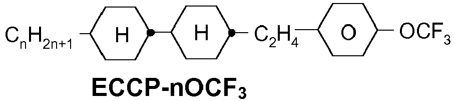 |
| 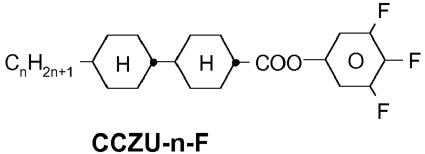 |
| 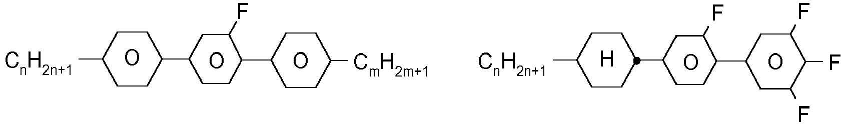 |
| 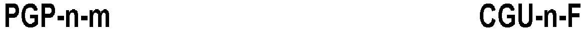 |
| 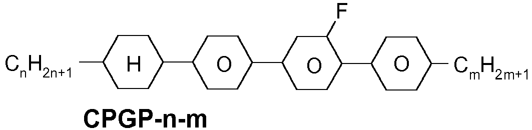 |
| 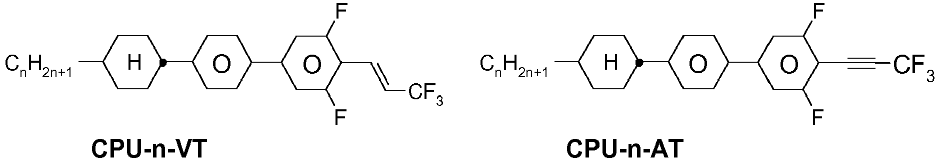 |
| 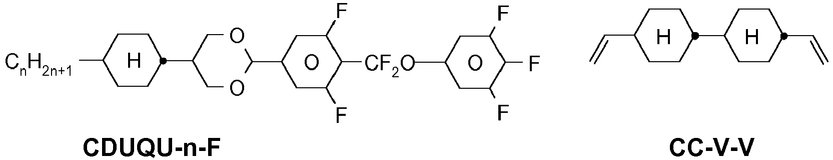 |
| 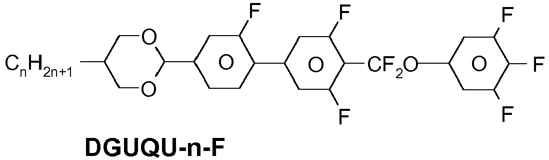 |
| 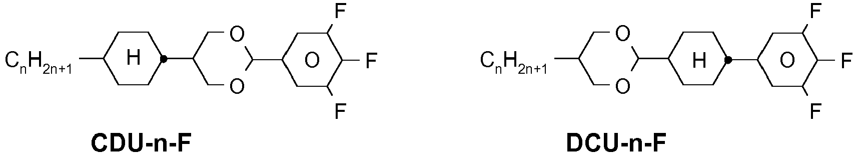 |
| 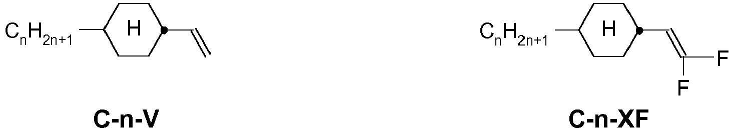 |
| 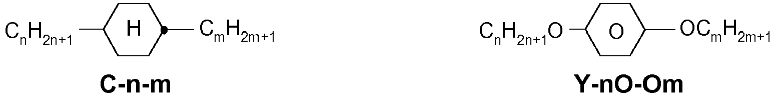 |
| 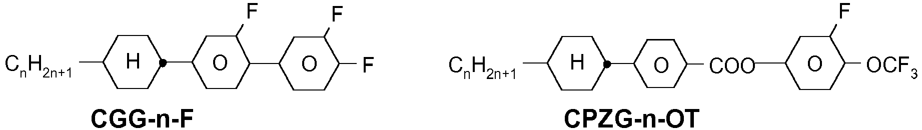 |
| 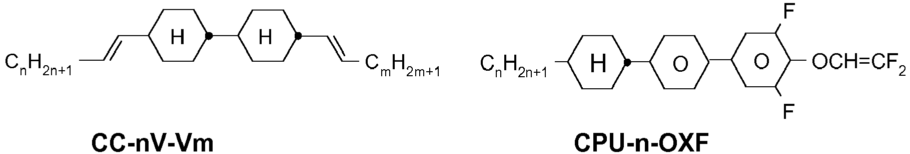 |
| 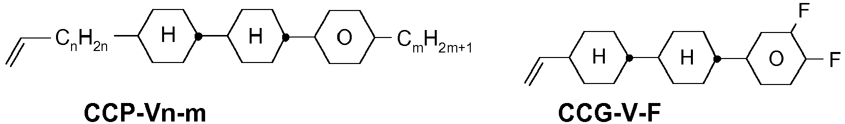 |
| 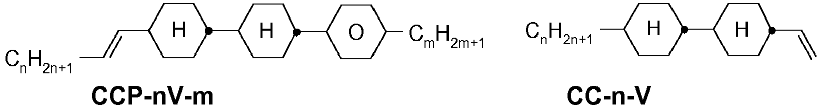 |
| 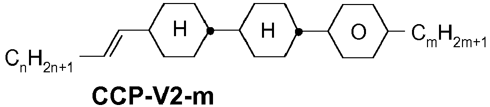 |
| 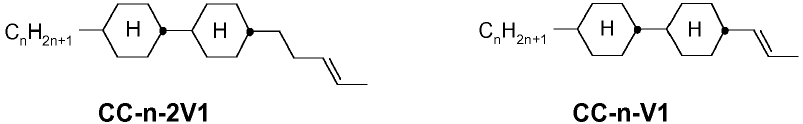 |
| 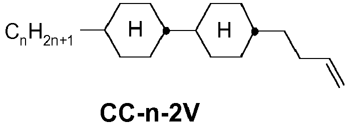 |
| 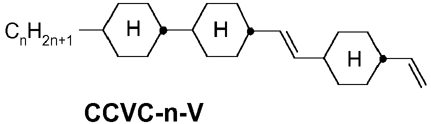 |
| 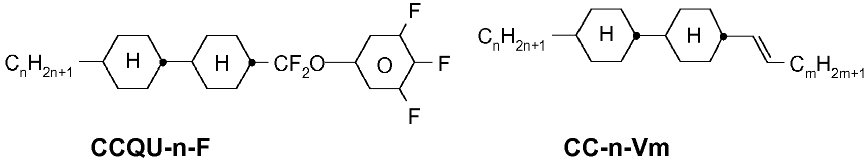 |
| 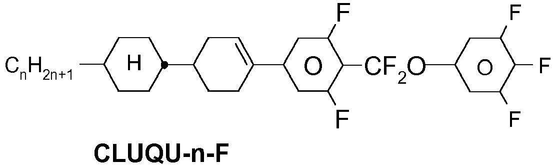 |
| 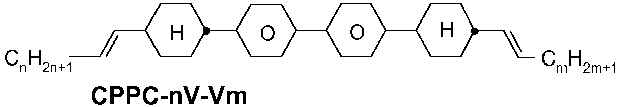 |
| 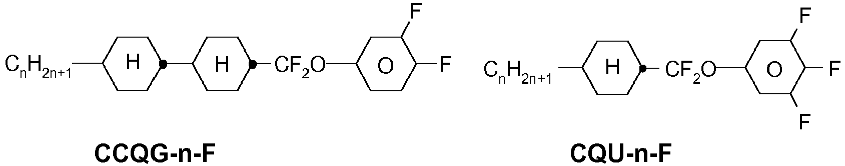 |
| 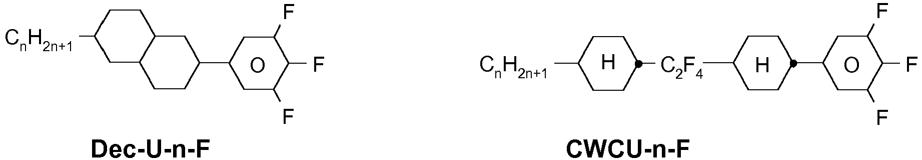 |
| 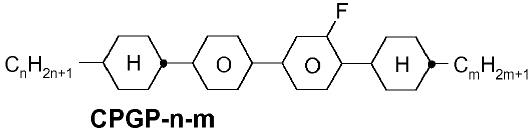 |
| 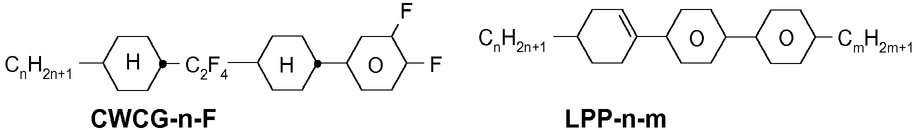 |
| 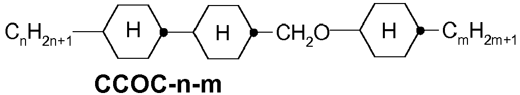 |
| 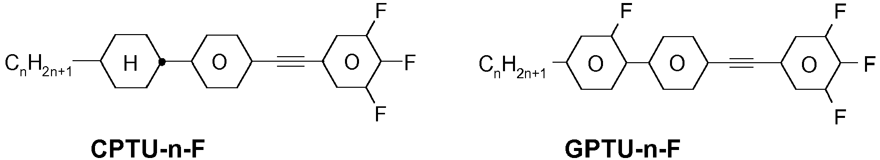 |
| 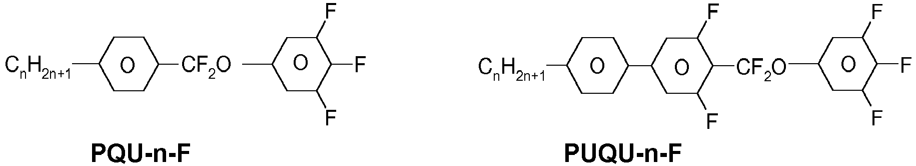 |
| 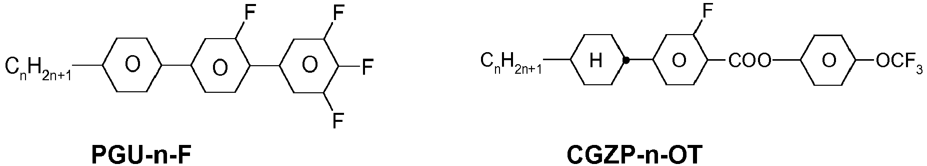 |
| 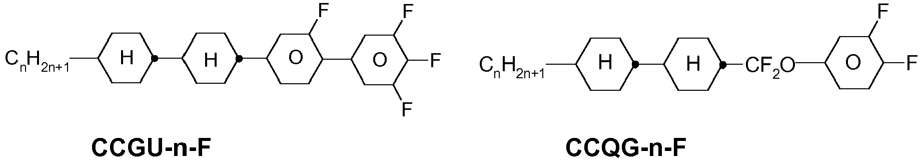 |
| 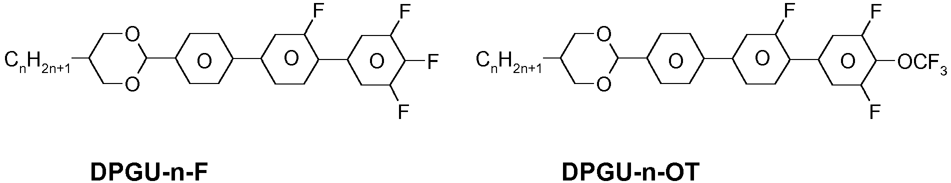 |
| 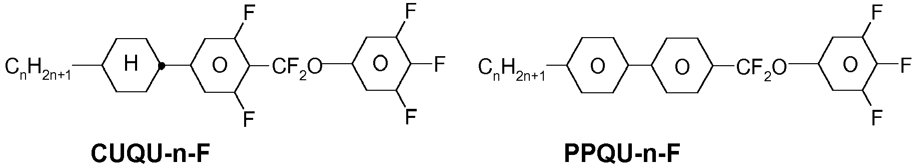 |
| 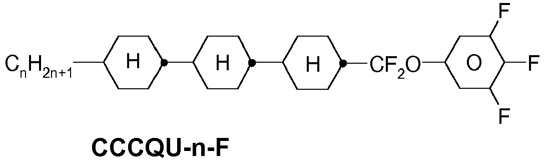 |
| 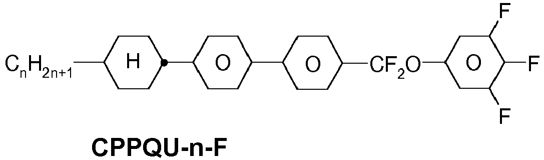 |
| 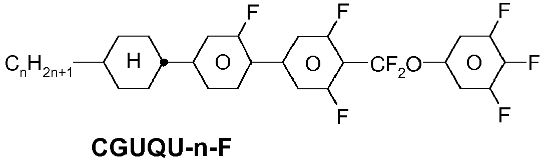 |
| 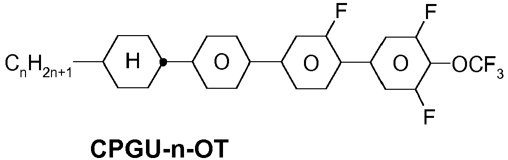 |
| 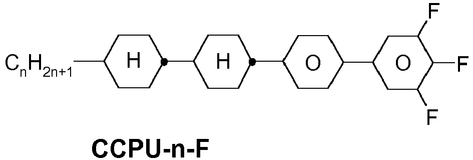 |
| 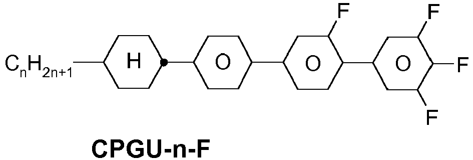 |
| 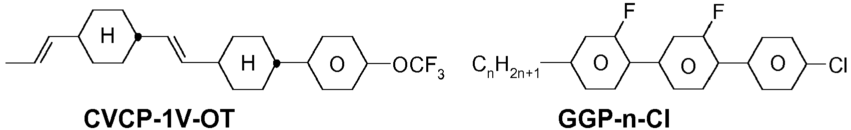 |
| 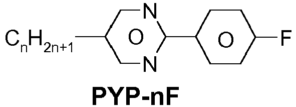 |
| 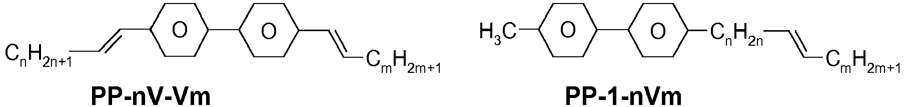 |
| 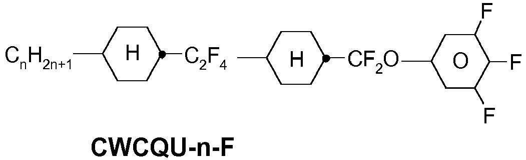 |
| 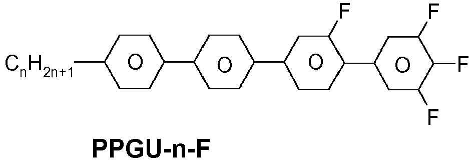 |
| 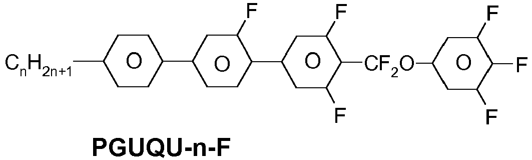 |
| 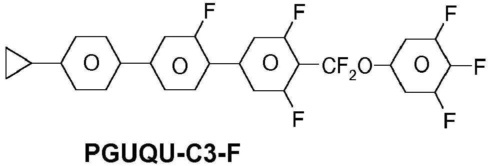 |
| 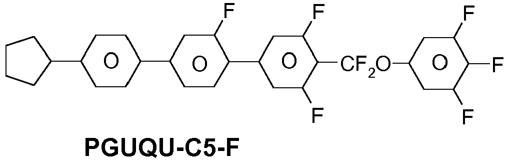 |
| 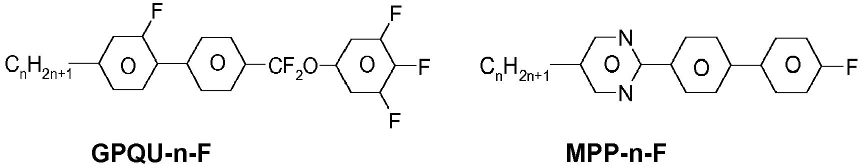 |
| 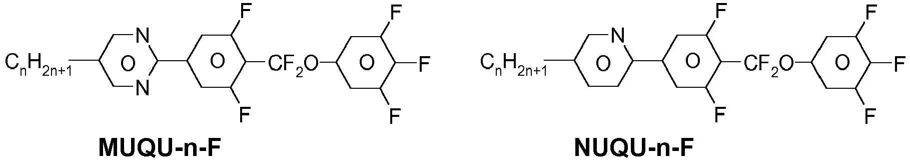 |
| 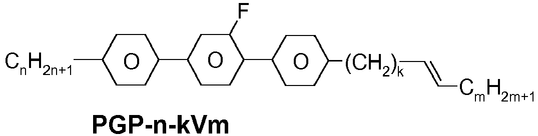 |
| 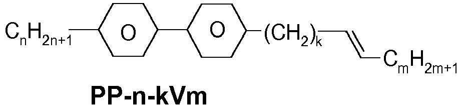 |
| 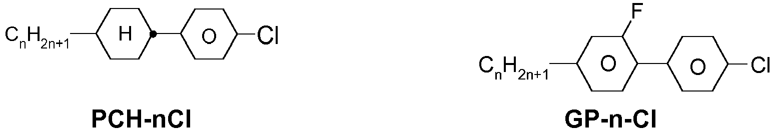 |
| 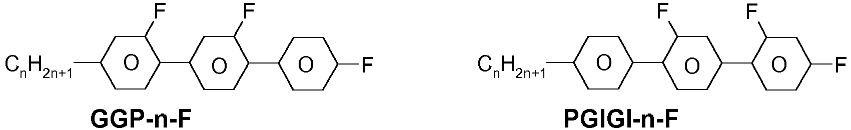 |
| 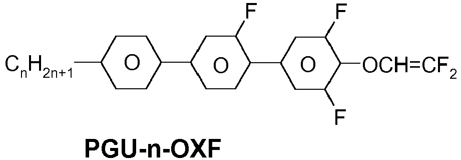 |
| 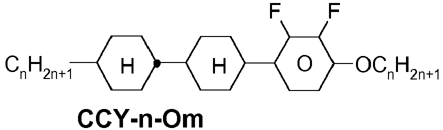 |
| 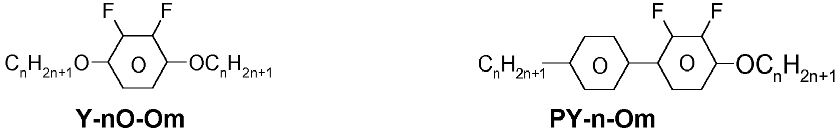 |

Besonders bevorzugt sind flüssigkristalline Mischungen, die neben den Verbindungen der Formeln I mindestens ein, zwei, drei, vier oder mehr Verbindungen aus der Tabelle B enthalten.

In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,001-5 Gew.% und besonders bevorzugt 0,001-3 Gew.% an Dotierstoffen.

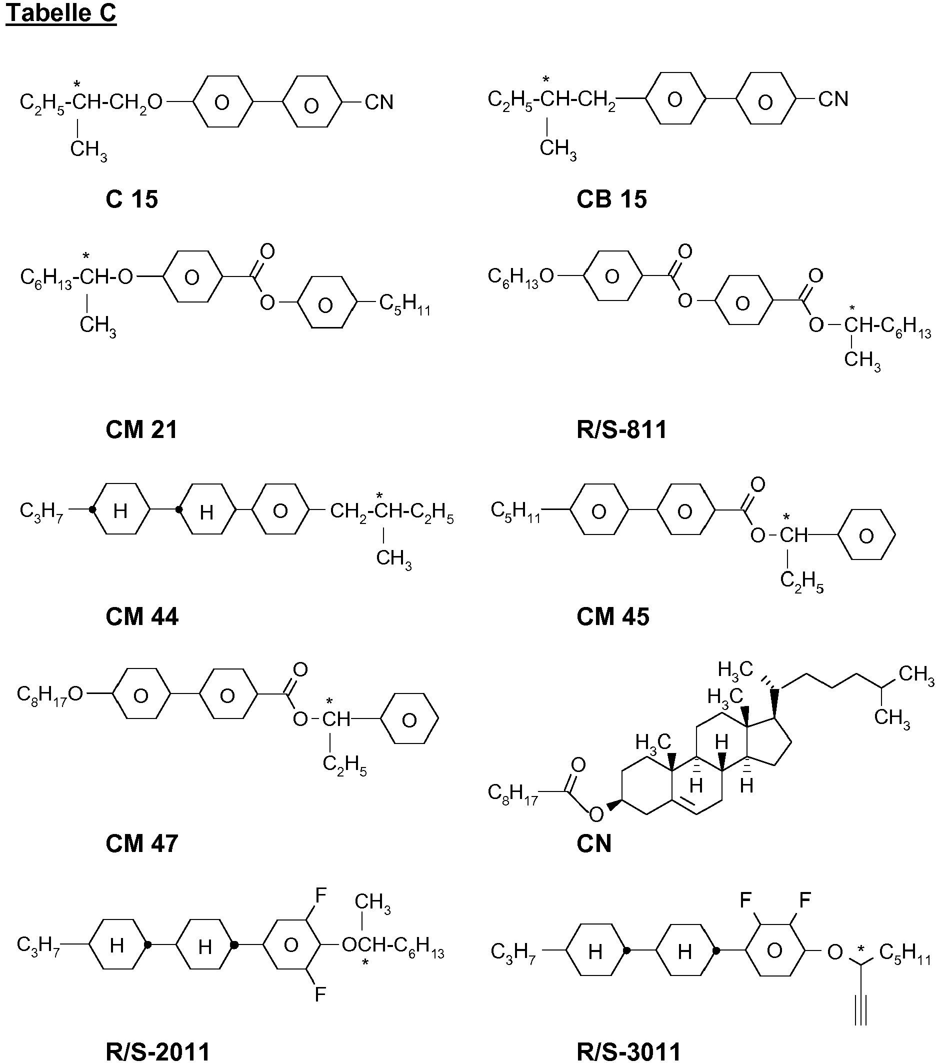

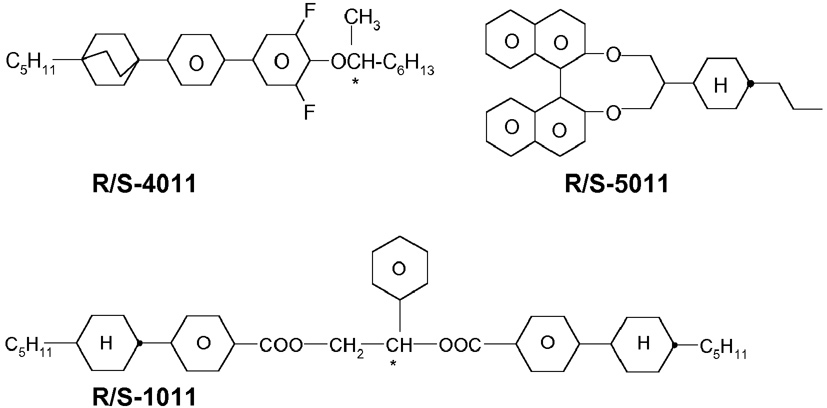

**Tabelle D**

| | |
|---|---|
| Stabilisatoren, die beispielsweise den erfindungsgemäßen Mischungen in Mengen von 0-10 Gew.% zugesetzt werden können, werden nachfolgend genannt. | |
| 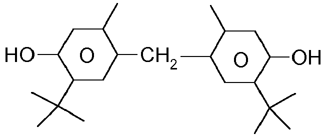 | 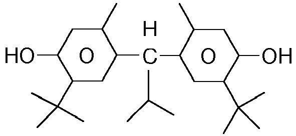 |
| 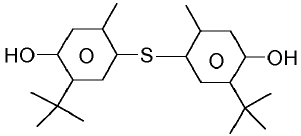 | 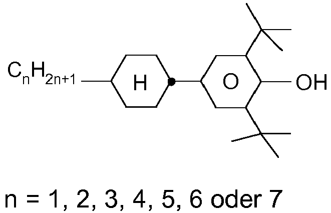 |
| 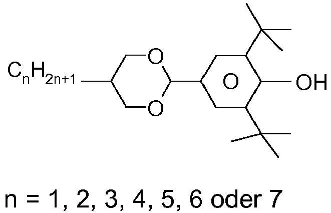 | 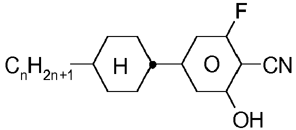 |
| 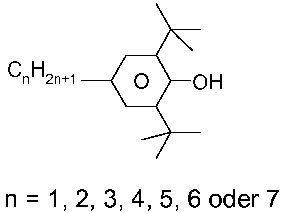 | 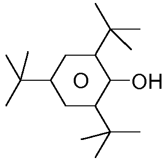 |
| 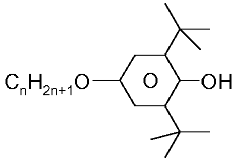 | 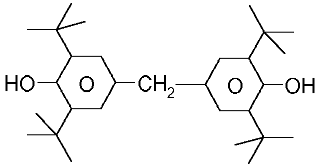 |
| 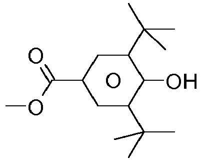 | 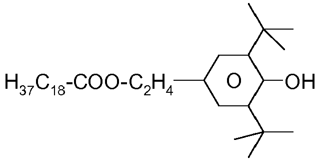 |
| 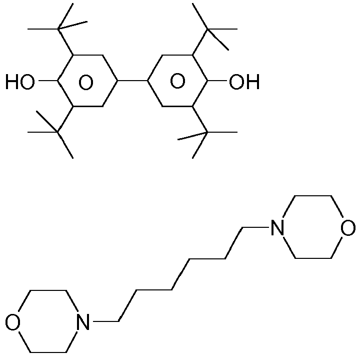 | 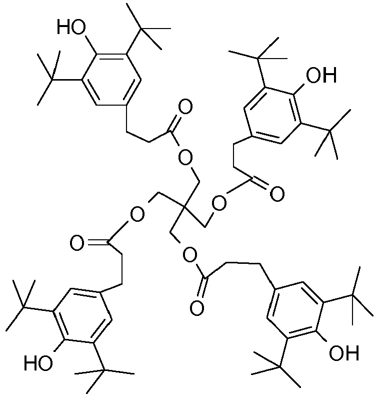 |
| 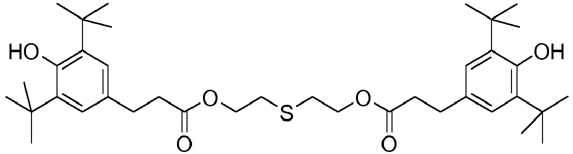 | |
| 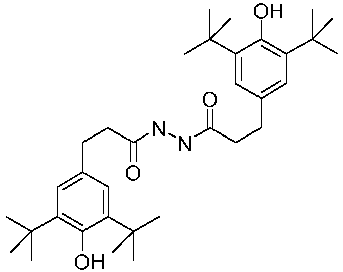 | 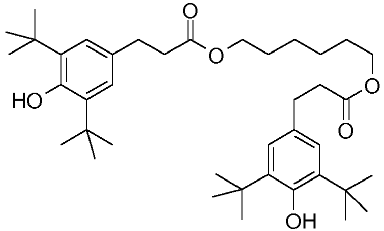 |
| 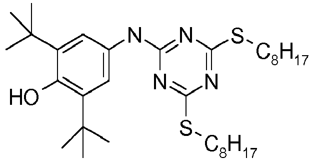 | 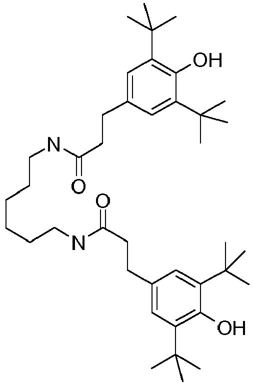 |
| 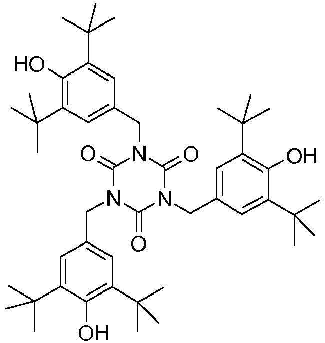 | 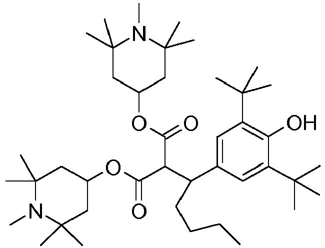 |
| 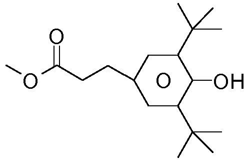 | 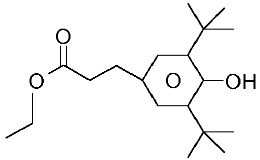 |
| 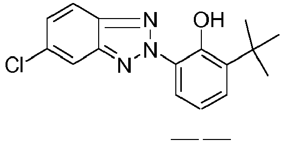 | 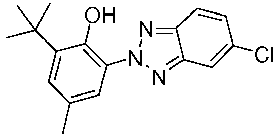 |
| 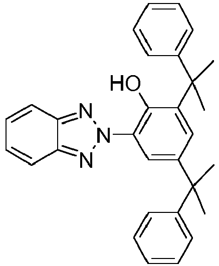 | 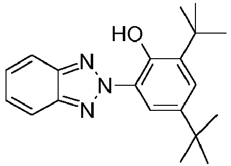 |
| 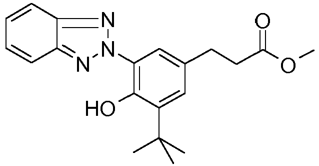 | 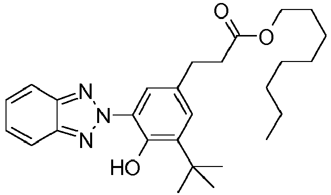 |
| 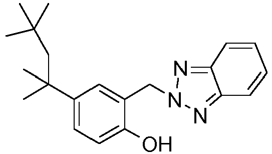 | 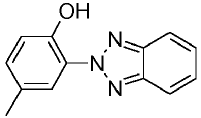 |
| 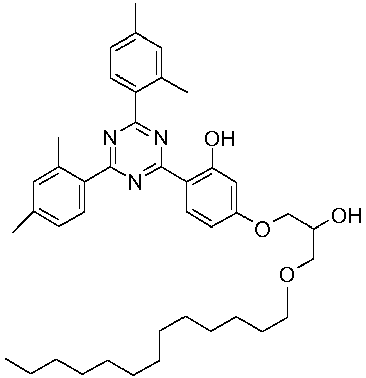 | 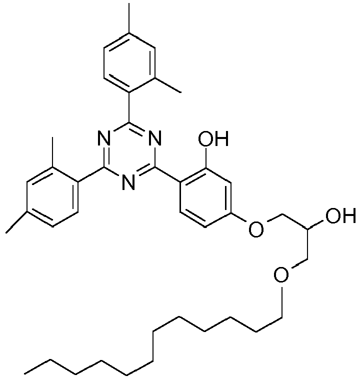 |
| 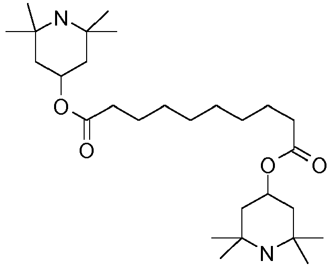 | 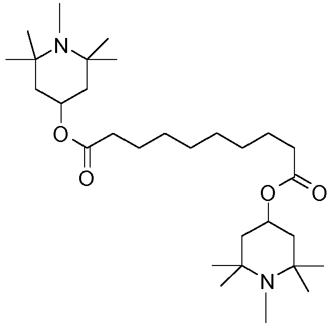 |
| 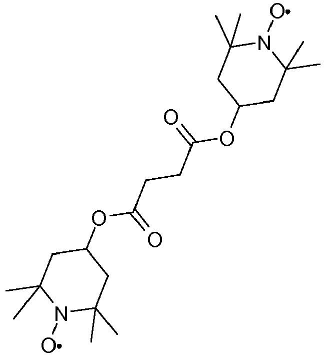 | 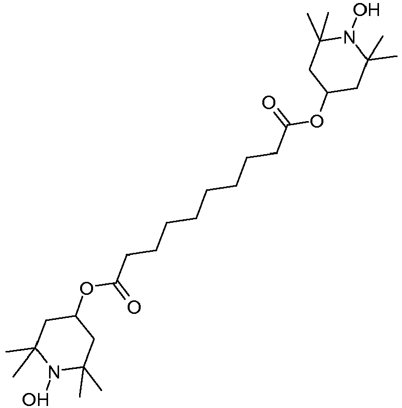 |
| 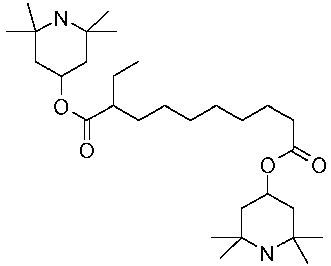 | 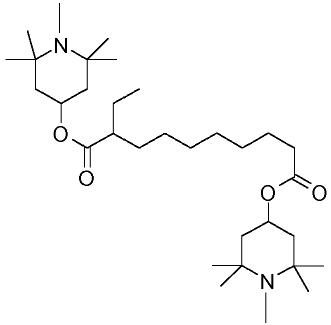 |
| 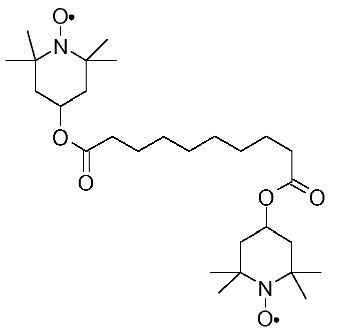 | 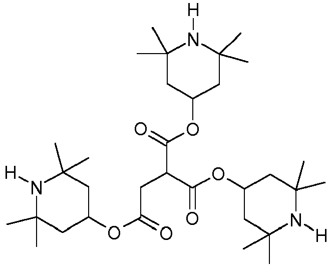 |
| 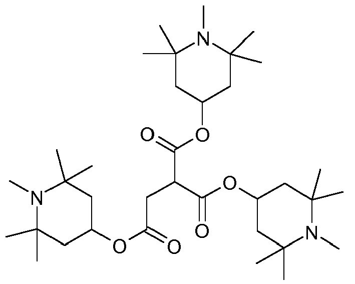 | 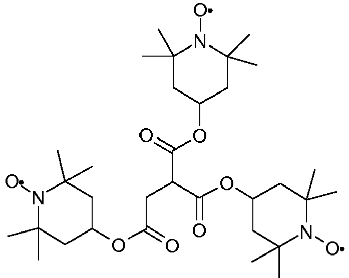 |
| 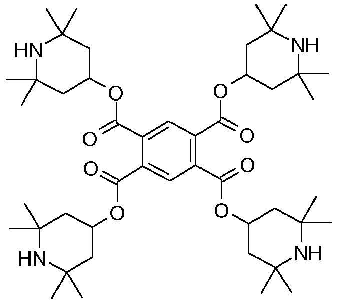 | 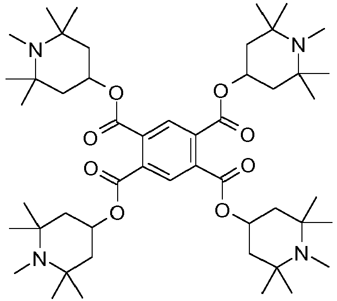 |
| 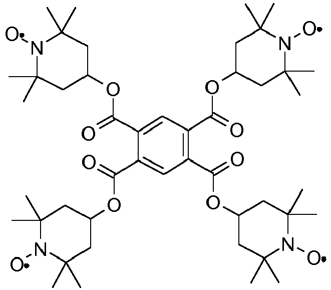 | 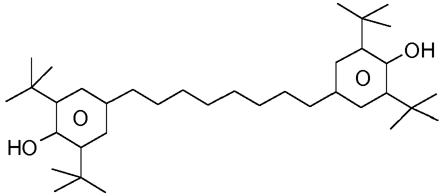 |

**Tabelle E**

| |
|---|
| In der Tabelle E sind Beispielverbindungen zusammengestellt, die in den FK-Medien gemäß der vorliegenden Erfindung vorzugsweise als reaktive mesogene Verbindungen verwendet werden können. Sofern die erfindungsgemäßen Mischungen ein oder mehrere reaktive Verbindungen enthalten, werden sie vorzugsweise in Mengen von 0,01-5 Gew.% eingesetzt. Gegebenenfalls muss für die Polymerisation noch ein Initiator oder ein Gemisch aus zwei oder mehr Initiatoren zugesetzt werden. Der Initiator oder das Initiatorgemisch wird vorzugsweise in Mengen von 0,001-2 Gew.% bezogen auf die Mischung zugesetzt. Ein geeigneter Initiator ist z. B. Irgacure (Fa. BASF) oder Irganox (Fa. BASF). |
| 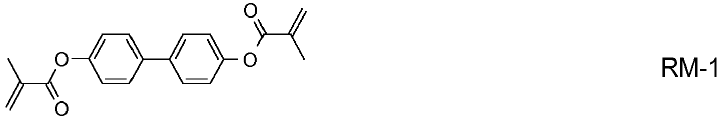 |
| 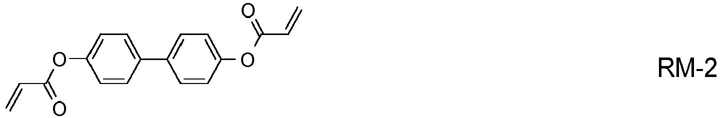 |
| 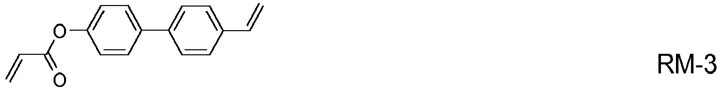 |
| 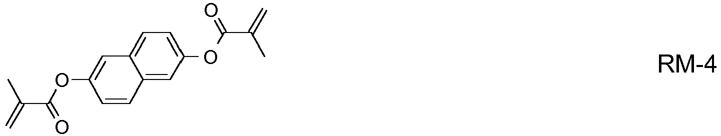 |
| 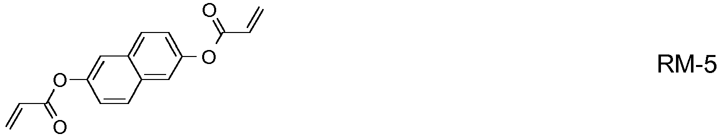 |
| 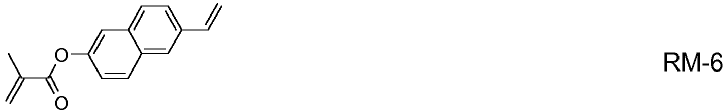 |
| 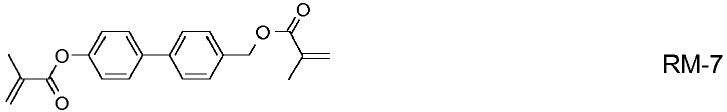 |
| 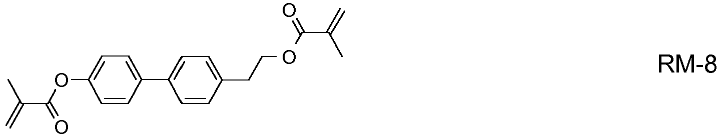 |
| 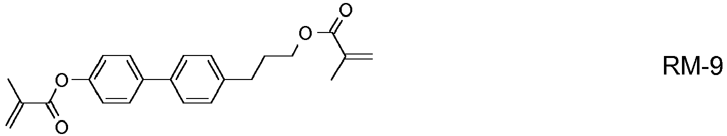 |
| 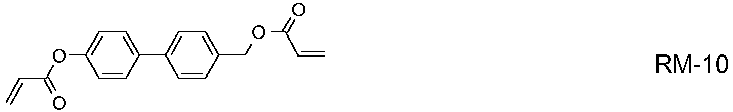 |
| 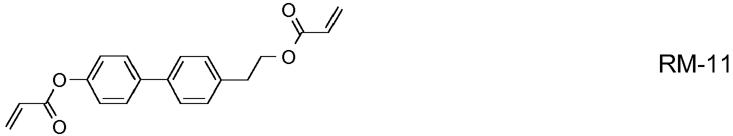 |
| 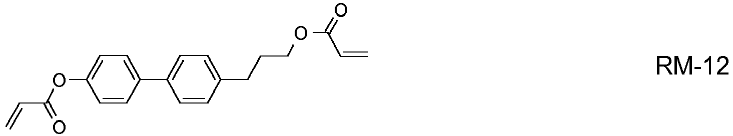 |
| 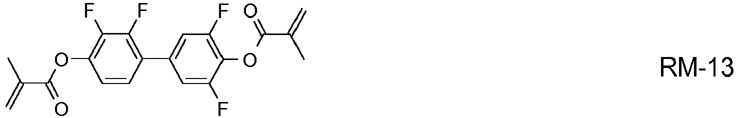 |
| 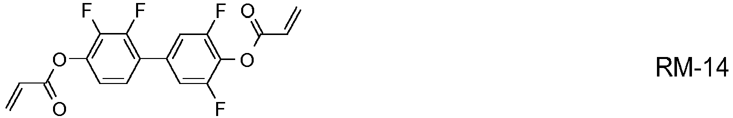 |
| 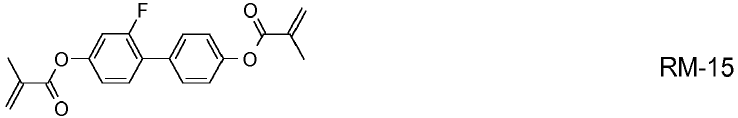 |
| 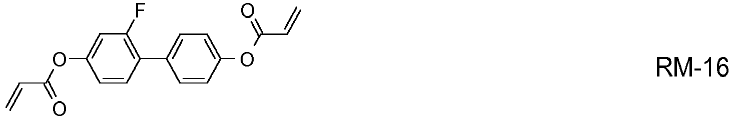 |
| 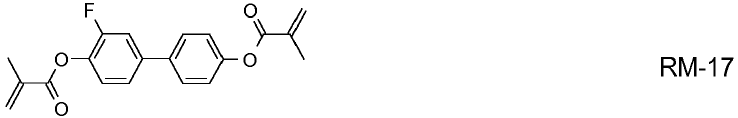 |
| 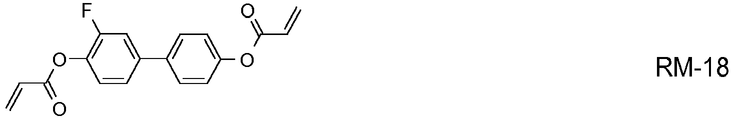 |
| 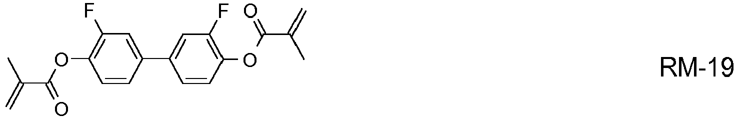 |
| 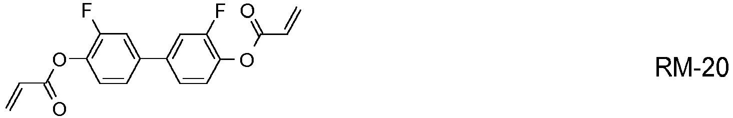 |
| 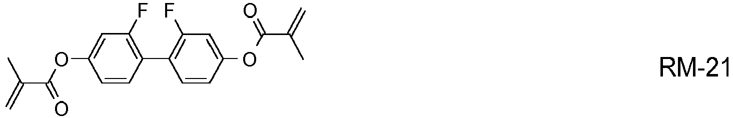 |
| 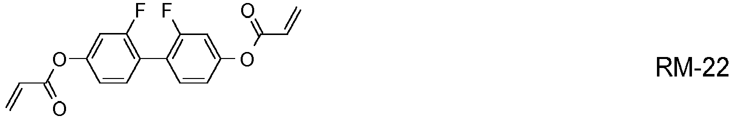 |
| 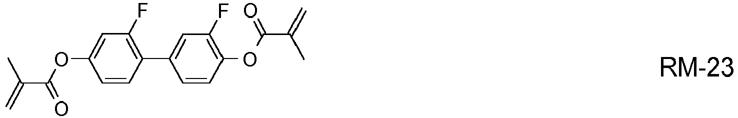 |
| 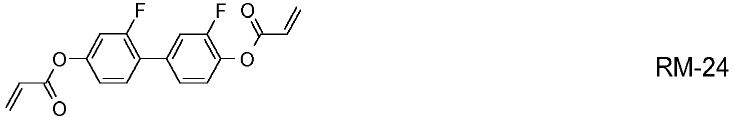 |
| 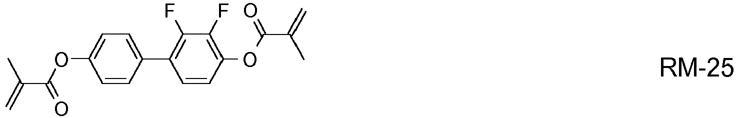 |
| 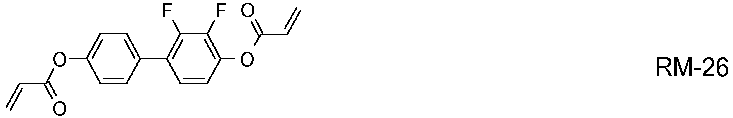 |
| 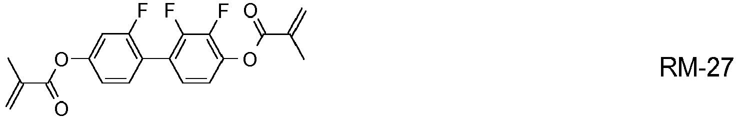 |
| 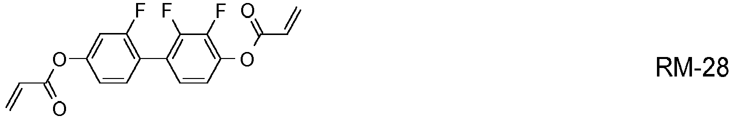 |
| 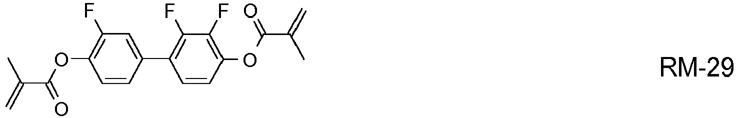 |
| 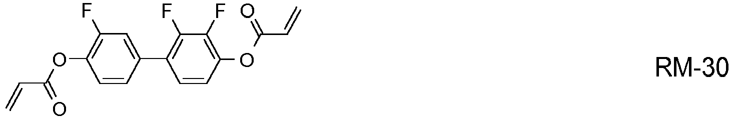 |
| 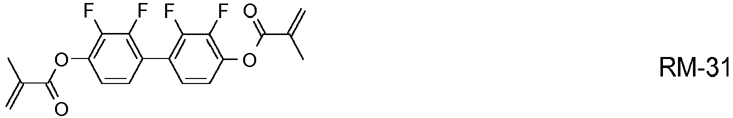 |
| 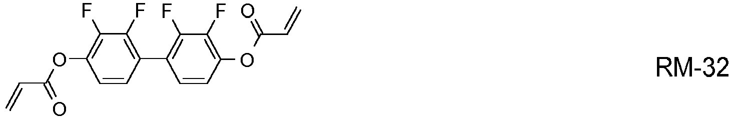 |
| 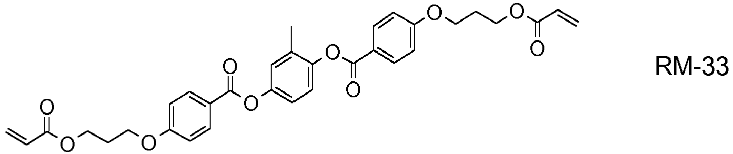 |
| 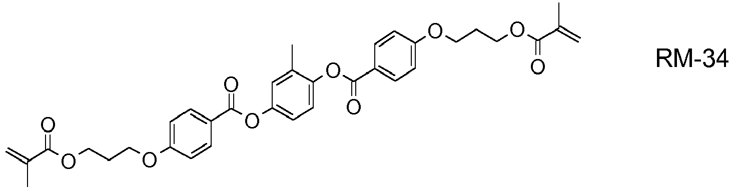 |
| 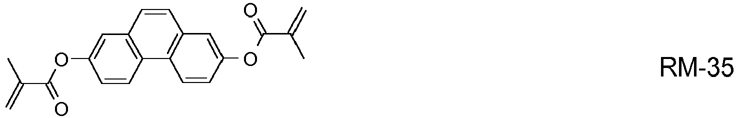 |
| 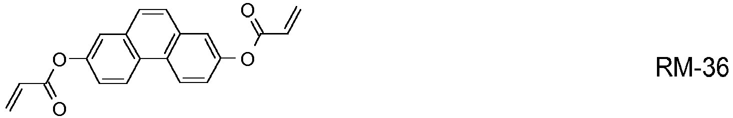 |
| 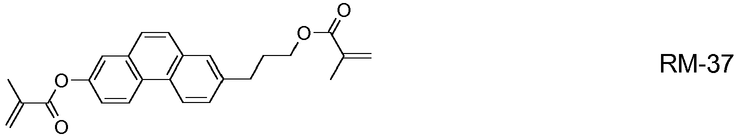 |
| 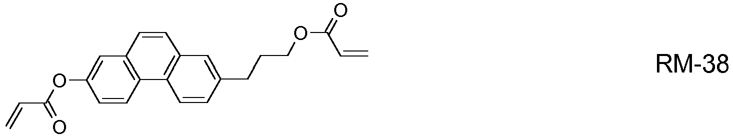 |
| 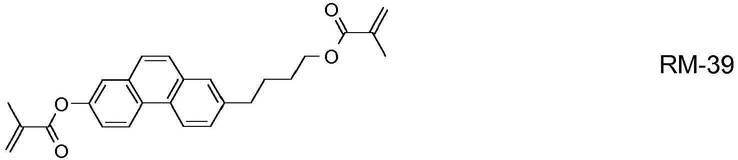 |
| 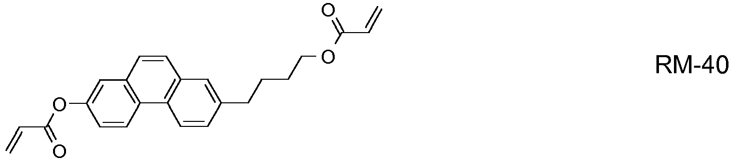 |
| 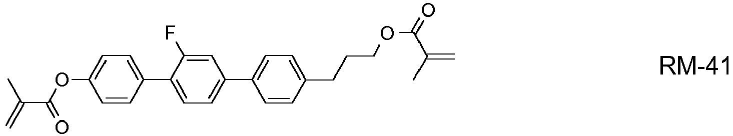 |
| 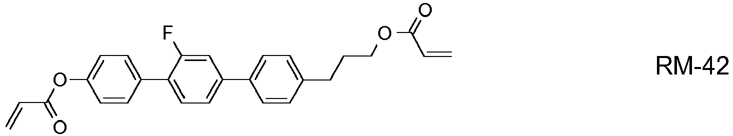 |
| 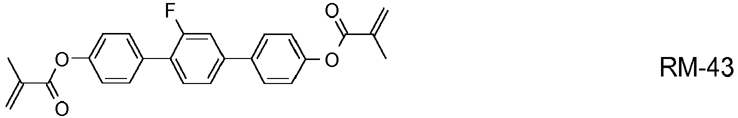 |
| 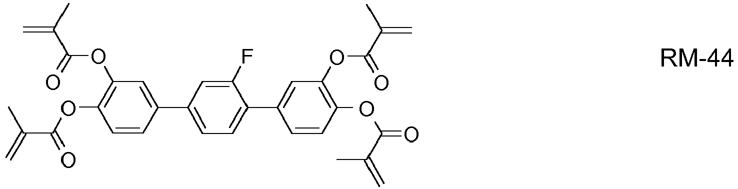 |
| 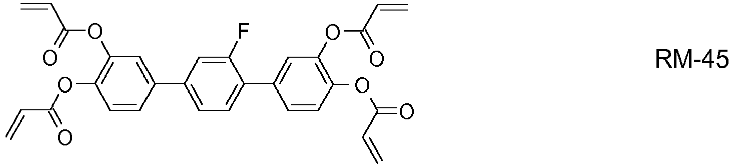 |
| 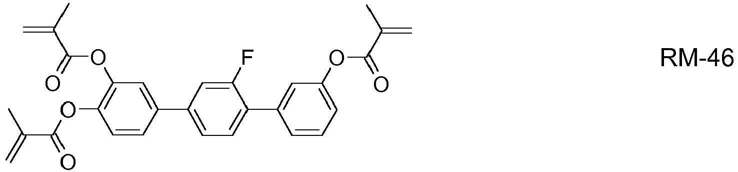 |
| 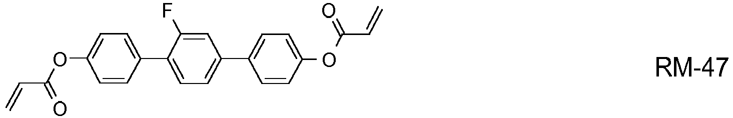 |
| 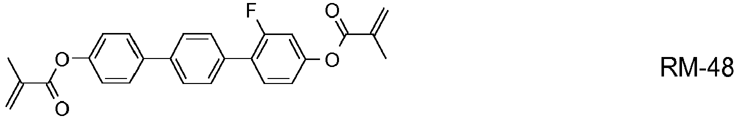 |
| 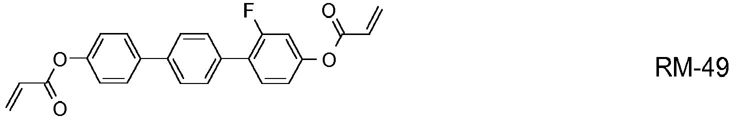 |
| 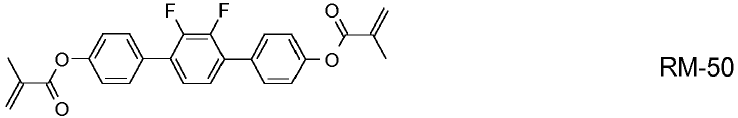 |
| 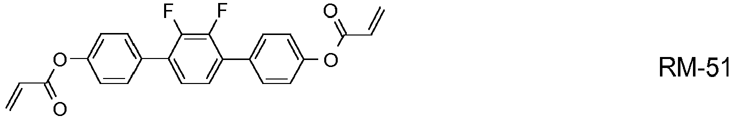 |
| 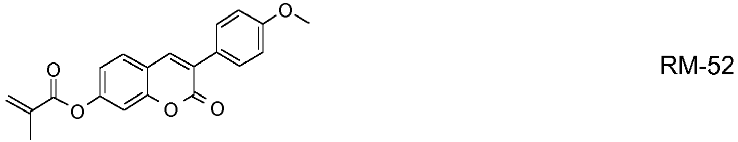 |
| 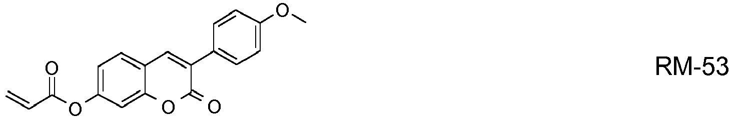 |
| 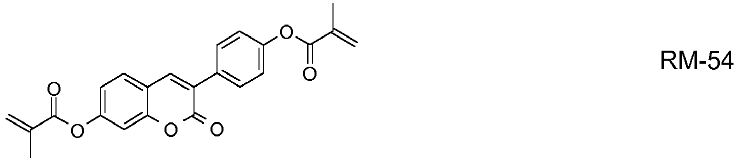 |
| 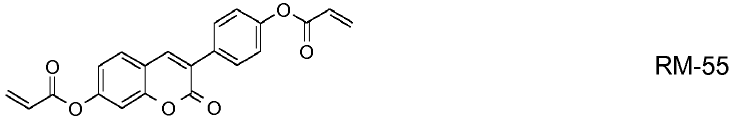 |
| 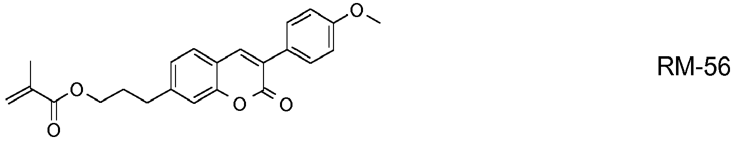 |
| 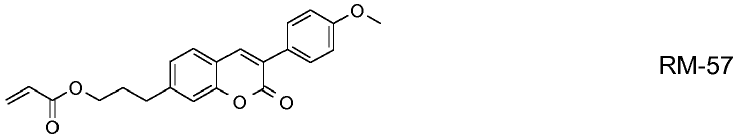 |
| 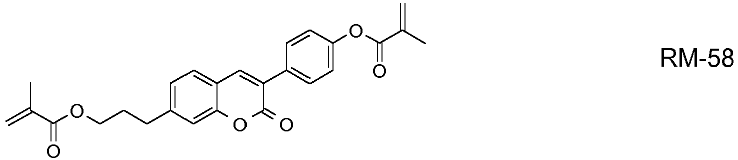 |
| 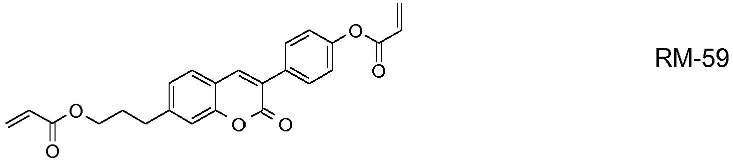 |
| 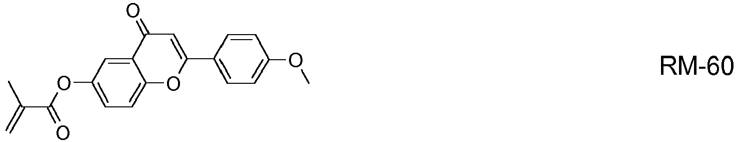 |
| 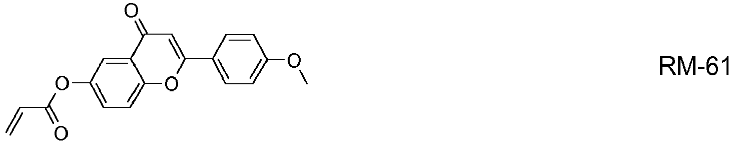 |
| 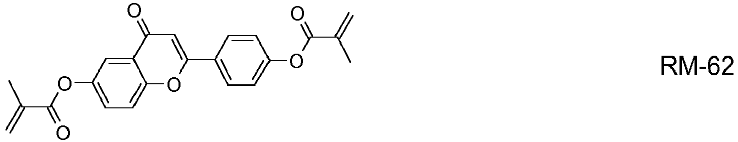 |
| 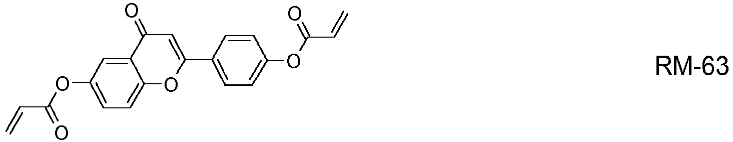 |
| 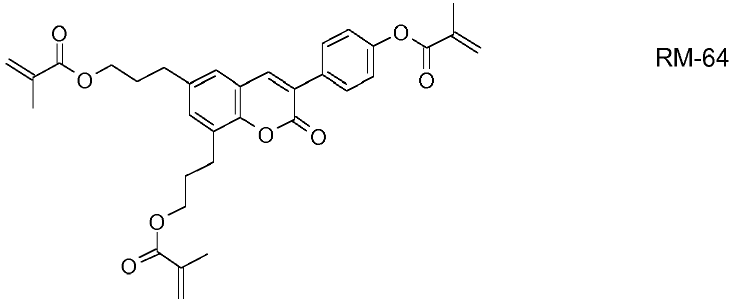 |
| 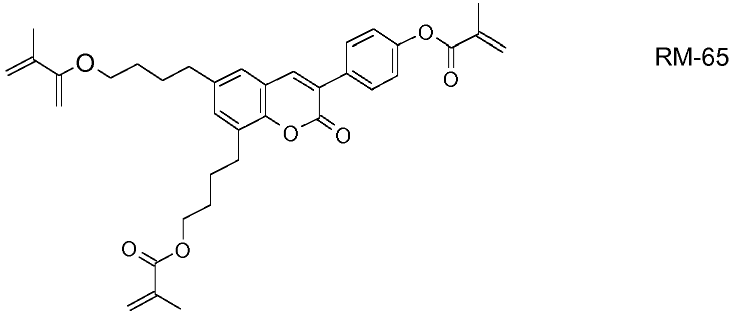 |
| 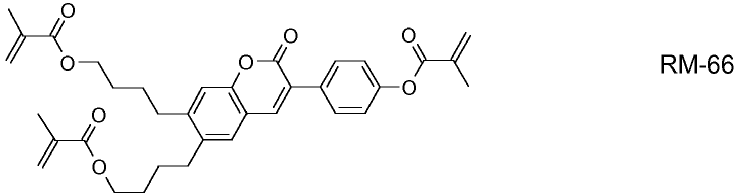 |
| 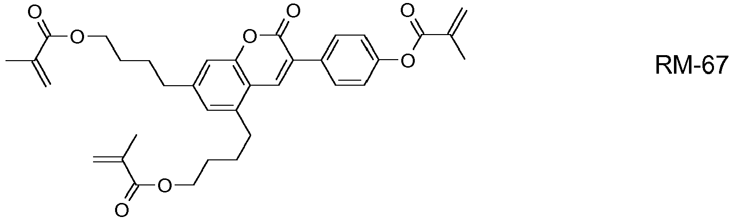 |
| 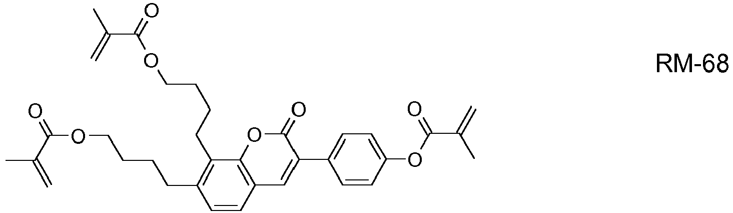 |
| 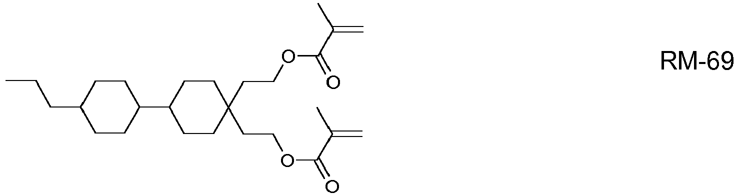 |
| 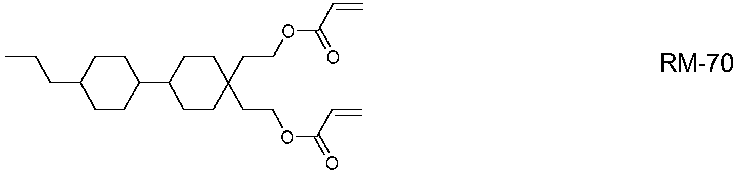 |
| 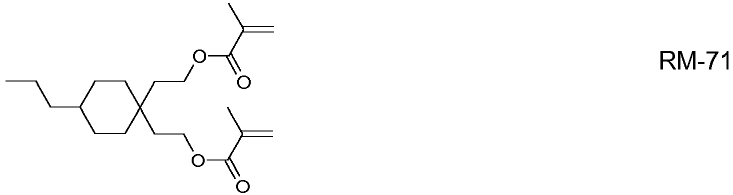 |
| 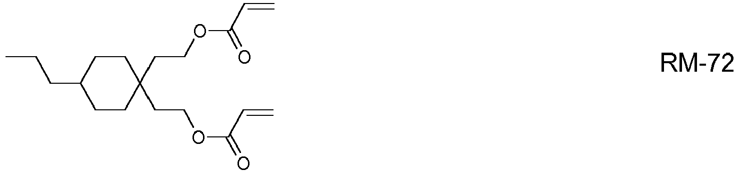 |
| 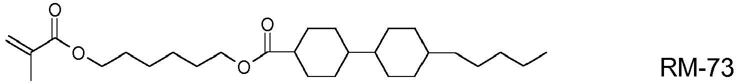 |
| 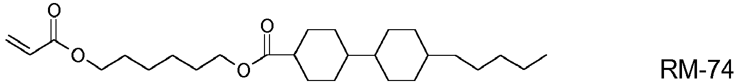 |
| 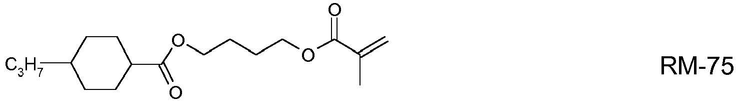 |
| 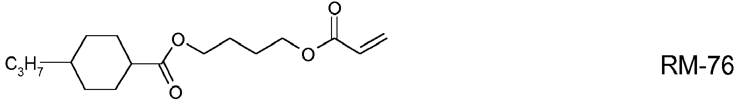 |
| 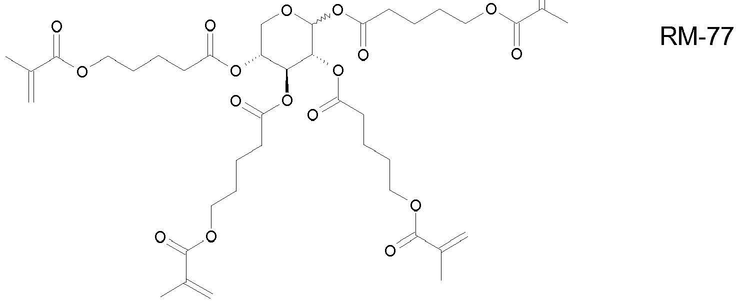 |
| 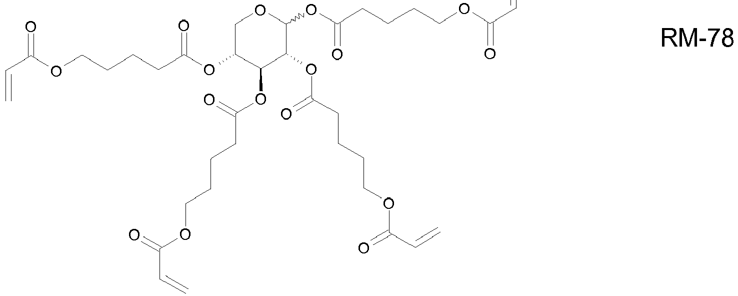 |
| 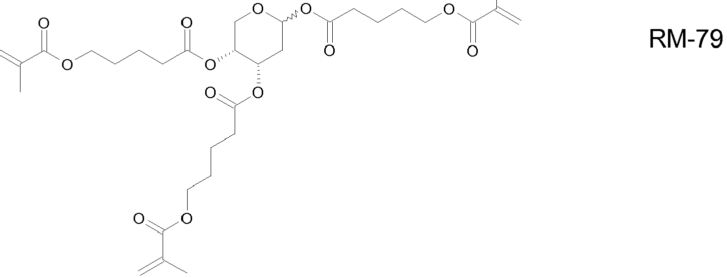 |
| 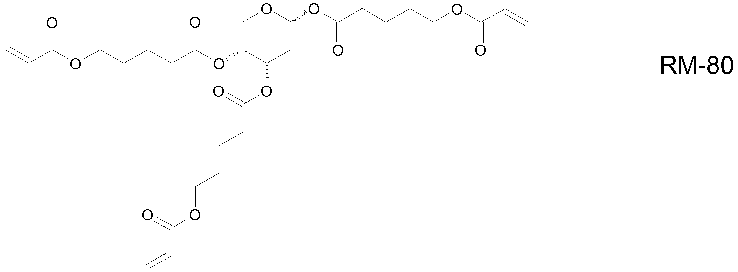 |
| 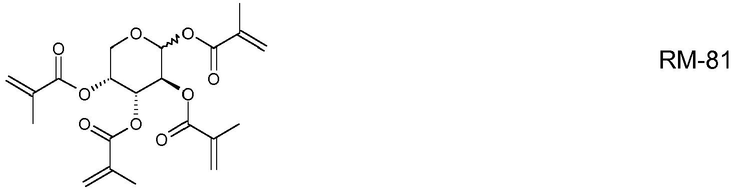 |
| 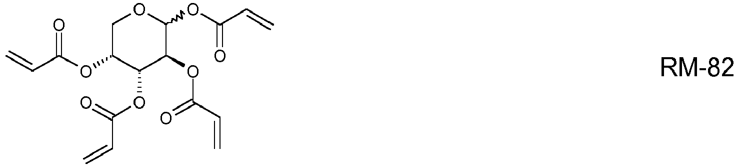 |
| 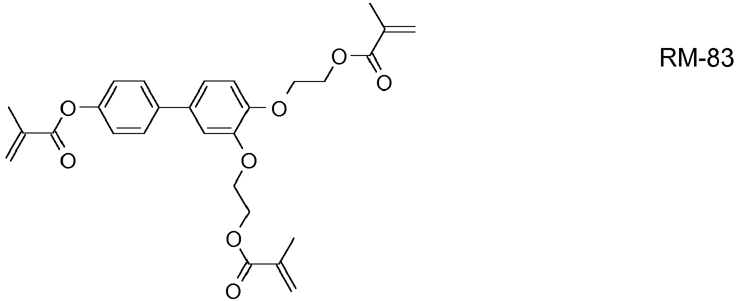 |
| 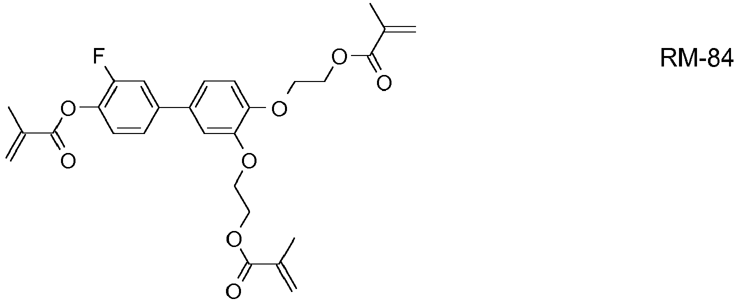 |
| 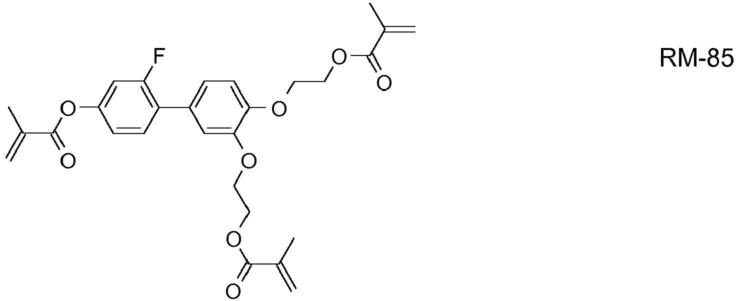 |
| 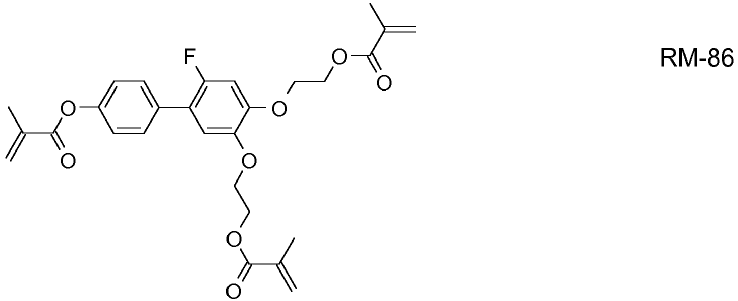 |
| 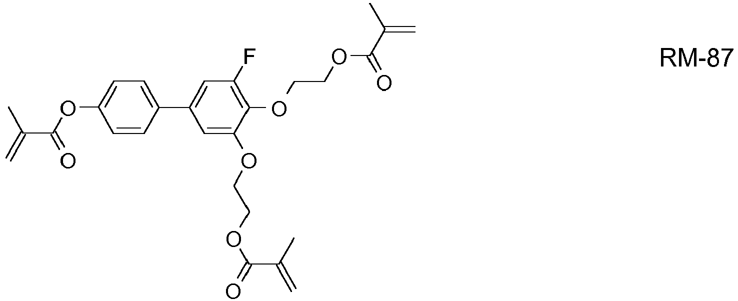 |
| 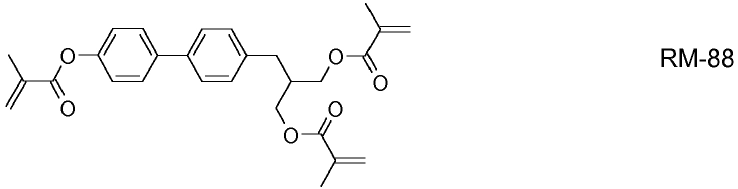 |
| 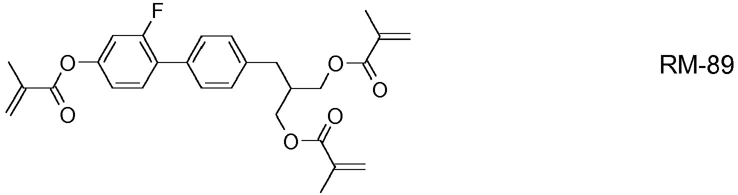 |
| 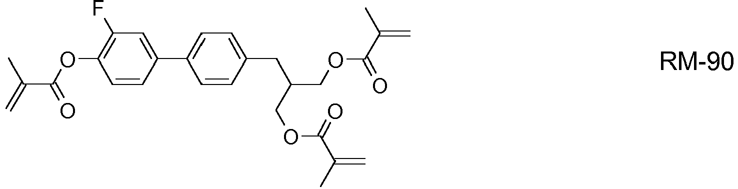 |
| 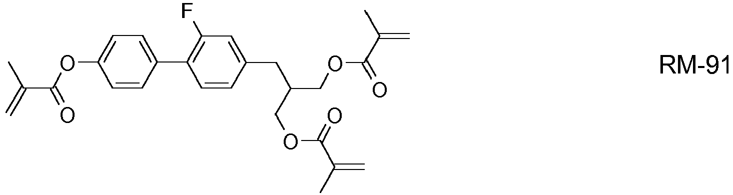 |
| 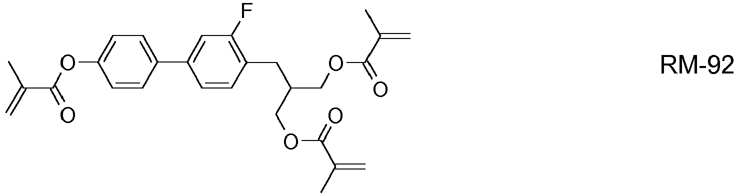 |
| 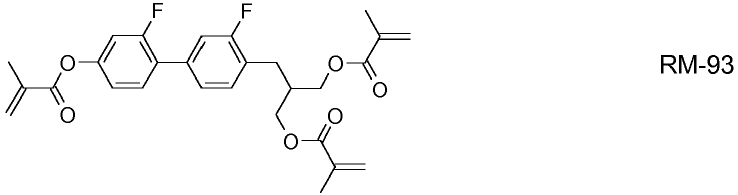 |
| 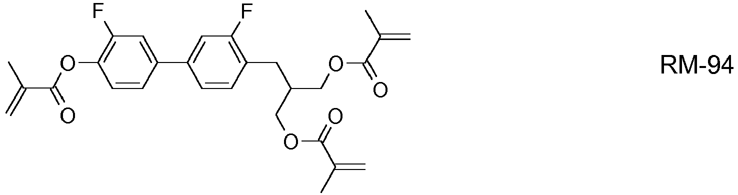 |
| 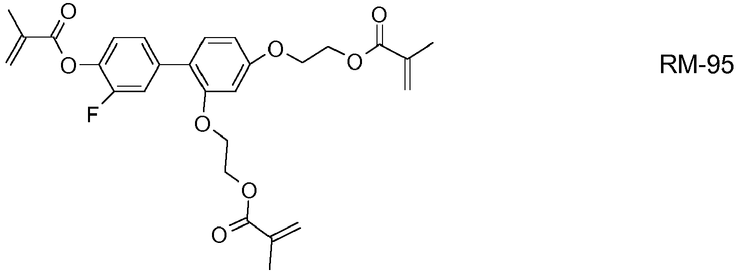 |
| 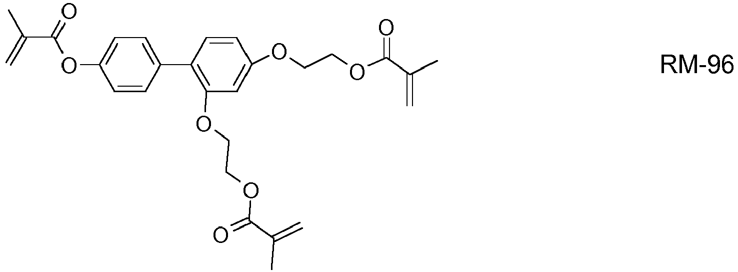 |
| 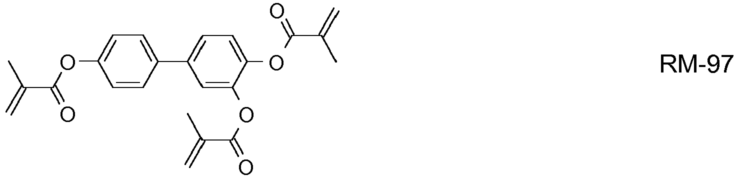 |
| 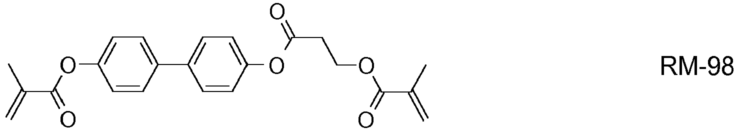 |
| 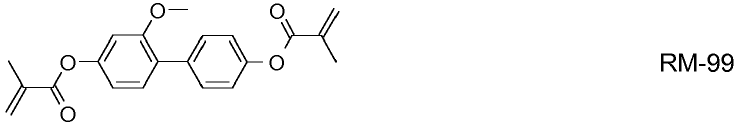 |
| 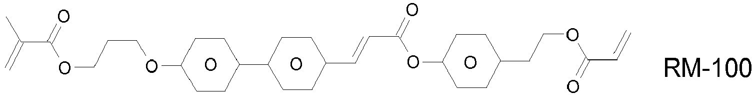 |
| 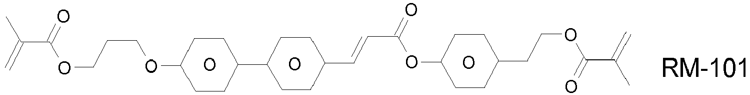 |

In einer bevorzugten Ausführungsform enthalten die erfindungsgemäßen Mischungen eine oder mehrere polymerisierbare Verbindungen, vorzugsweise ausgewählt aus den polymerisierbaren Verbindungen der Formeln RM-1 bis RM-94. Derartige Medien sind insbesondere für PS-FFS- und PS-IPS-Anwendungen geeignet. Von den in der Tabelle E genannten reaktiven Mesogenen sind die Verbindungen RM-1, RM-2, RM-3, RM-4, RM-5, RM-11, RM-17, RM-35, RM-41, RM-44, RM-62, RM-81 und RM-99 insbesondere bevorzugt.

### Beispiele

Die folgenden Ausführungsbeispiele sollen die Erfindung erläutern, ohne sie zu begrenzen.

Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Weiterhin bedeutet
- Vₒ: Schwellenspannung, kapazitiv [V] bei 20 °C
- Δn: die optische Anisotropie gemessen bei 20 °C und 589 nm
- Δε: die dielektrische Anisotropie bei 20 °C und 1 kHz
- cp.: Klärpunkt [°C]
- K₁: elastische Konstante, "Splay"-Deformation bei 20 °C, [pN]
- K₃: elastische Konstante, "Bend"-Deformation bei 20 °C, [pN]
- γ₁: Rotationsviskosität gemessen bei 20 °C [mPa·s], bestimmt nach dem Transientenstromverfahren in einem elektrischen Feld
- LTS: Tieftemperaturstabilität [Low temperature stability (nematische Phase)], bestimmt in Glasfläschchen.

### Mischungsbeispiele

Die elektro-optischen Daten werden in einer TN-Zelle im 1. Minimum (d.h. bei einem d · Δn-Wert von 0,5 µm) bei 20 °C gemessen, sofern nicht ausdrücklich etwas anderes angegeben wird. Die optischen Daten werden bei 20 °C gemessen, sofern nicht ausdrücklich etwas anderes angegeben wird. Alle physikalischen Eigenschaften werden nach "Merck Liquid Crystals, Physical Properties of Liquid Crystals" Status Nov. 1997, Merck KGaA, Deutschland bestimmt und gelten für eine Temperatur von 20 °C, sofern nicht explizit anders angegeben.

### Beispiel M1

| | | | |
|---|---|---|---|
| CC-3-V | 51,50 % | Klärpunkt [°C]: | 74 |
| CC-3-V1 | 5,00 % | Δn[589nm,20°C]: | 0,1175 |
| CCP-V-1 | 5,50 % | Δε [1 kHz, 20 °C]: | 2,2 |
| CLP-3-T | 4,50 % | ε_{∥} [1 kHz, 20°C]: | 4,9 |
| PGP-1-2V | 3,00 % | ε_{⊥} [1 kHz, 20°C]: | 2,7 |
| PGP-2-2V | 14,50 % | γ₁ [mPa·s, 20°C]: | 45 |
| PGP-3-2V | 2,00 % | K₁ [pN, 20°C]: | 14,1 |
| PGU-2-F | 4,00 % | K₃ [pN, 20 °C]: | 13,7 |
| PGUQU-3-F | 3,00 % | LTS [bulk, -20°C]: | > 1000 h |
| PP-1-2V1 | 6,00 % | | |
| PPGU-3-F | 1,00 % | | |

### Beispiel M2

| | | | |
|---|---|---|---|
| CC-3-V | 51,50 % | Klärpunkt [°C]: | 73,5 |
| CC-3-V1 | 5,00 % | Δn[589nm,20°C]: | 0,1174 |
| CCP-V-1 | 6,00 % | Δε [1 kHz, 20 °C]: | 2,2 |
| CLP-3-T | 3,50 % | ε_{∥} [1 kHz, 20°C]: | 4,9 |
| PGP-1-2V | 3,00 % | ε_{⊥} [1 kHz, 20°C]: | 2,7 |
| PGP-2-2V | 14,50 % | γ₁ [mPa·s, 20 °C]: | 44 |
| PGP-3-2V | 2,00 % | K₁ [pN, 20 °C]: | 13,9 |
| PGU-2-F | 4,50 % | K₃ [pN, 20 °C]: | 13,6 |
| PGUQU-3-F | 3,00 % | LTS [bulk, -20 °C]: | > 1000 h |
| PP-1-2V1 | 6,00 % | | |
| PPGU-3-F | 1,00 % | | |

### Beispiel M3

| | | | |
|---|---|---|---|
| CC-3-V | 53,00 % | Klärpunkt [°C]: | 73,5 |
| CC-3-V1 | 3,00 % | Δn [589 nm, 20 °C] : | 0,1170 |
| CCP-V-1 | 7,00 % | Δε [1 kHz, 20 °C]: | 2,2 |
| CLP-3-T | 3,00 % | ε_{∥} [1 kHz, 20°C]: | 4,9 |
| PGP-1-2V | 3,00 % | ε_{⊥} [1 kHz, 20°C]: | 2,7 |
| PGP-2-2V | 14,50 % | γ₁ [mPa·s, 20 °C]: | 43 |
| PGP-3-2V | 2,00 % | K₁ [pN, 20 °C]: | 13,7 |
| PGU-2-F | 5,00 % | K₃ [pN, 20 °C]: | 13,5 |
| PGUQU-3-F | 3,00 % | | |
| PP-1-2V1 | 5,50 % | | |
| PPGU-3-F | 1,00 % | | |

### Beispiel M4

| | | | |
|---|---|---|---|
| CC-3-V | 51,50 % | Klärpunkt [°C]: | 74 |
| CC-3-V1 | 5,00 % | Δn [589 nm, 20 °C] : | 0,1182 |
| CCP-V-1 | 5,00 % | Δε [1 kHz, 20 °C]: | 2,2 |
| CLP-3-T | 4,50 % | ε_{∥} [1 kHz, 20°C]: | 4,9 |
| PGP-1-2V | 3,00 % | ε_{⊥} [1 kHz, 20°C]: | 2,7 |
| PGP-2-2V | 15,00 % | γ₁ [mPa·s, 20 °C]: | 45 |
| PGP-3-2V | 2,00 % | K₁ [pN, 20 °C]: | 14,2 |
| PGU-2-F | 4,00 % | K₃ [pN, 20 °C]: | 13,7 |
| PGUQU-3-F | 3,00 % | | |
| PP-1-2V1 | 6,00 % | | |
| PPGU-3-F | 1,00 % | | |

### Beispiel M5

| | | | |
|---|---|---|---|
| CC-3-V | 26,00 % | Klärpunkt [°C]: | 105 |
| CC-3-V1 | 10,00 % | Δn [589 nm, 20 °C] : | 0,1192 |
| CC-3-2V1 | 9,00 % | Δε [1 kHz, 20 °C]: | 5,2 |
| CCP-V-1 | 11,00 % | ε_{∥} [1 kHz, 20°C]: | 8,0 |
| CCP-V2-1 | 8,00 % | ε_{⊥} [1 kHz, 20°C]: | 2,8 |
| CLP-3-T | 5,50 % | γ₁ [mPa·s, 20 °C]: | 86 |
| PGP-2-3 | 2,50 % | K₁ [pN, 20 °C]: | 19,6 |
| PGP-2-2V | 10,00 % | K₃ [pN, 20 °C]: | 19,2 |
| APUQU-3-F | 6,50 % | | |
| PGUQU-3-F | 2,50 % | | |
| PGUQU-4-F | 6,00 % | | |
| CPGU-3-OT | 3,00 % | | |

### Beispiel M6

| | | | |
|---|---|---|---|
| CC-3-V | 28,00 % | Klärpunkt [°C]: | 102 |
| CC-3-V1 | 9,00 % | Δn [589 nm, 20 °C] | : 0,1184 |
| CC-3-2V1 | 9,00 % | Δε [1 kHz, 20 °C]: | 5,2 |
| CCP-V-1 | 12,00 % | ε_{∥} [1 kHz, 20°C]: | 8,0 |
| CCP-V2-1 | 6,00 % | ε_{⊥} [1 kHz, 20°C]: | 2,8 |
| CLP-3-T | 6,00 % | γ₁ [mPa·s, 20 °C]: | 83 |
| PGP-2-3 | 2,50 % | K₁ [pN, 20 °C]: | 19,0 |
| PGP-2-2V | 10,00 % | K₃ [pN, 20 °C]: | 18,7 |
| APUQU-3-F | 6,00 % | LTS [bulk, -20 °C]: | > 1000 h |
| PGUQU-3-F | 3,50 % | | |
| PGUQU-4-F | 6,00 % | | |
| CPGU-3-OT | 2,00 % | | |

### Beispiel M7

| | | | |
|---|---|---|---|
| CC-3-V | 28,00 % | Klärpunkt [°C]: | 101 |
| CC-3-V1 | 9,50 % | Δn [589 nm, 20 °C] : | 0,1176 |
| CC-3-2V1 | 9,00 % | Δε [1 kHz, 20 °C]: | 5,2 |
| CCP-V-1 | 12,00 % | ε_{∥} [1 kHz, 20°C]: | 8,0 |
| CCP-V2-1 | 5,50 % | ε_{⊥} [1 kHz, 20°C]: | 2,8 |
| CLP-3-T | 6,50 % | γ₁ [mPa·s, 20 °C]: | 81 |
| PGP-2V | 12,50 % | K₁ [pN, 20 °C]: | 18,7 |
| APUQU-3-F | 5,50 % | K₃ [pN, 20 °C]: | 18,5 |
| PGUQU-3-F | 4,00 % | | |
| PGUQU-4-F | 6,50 % | | |
| CPGU-3-OT | 1,00 % | | |

### Beispiel M8

| | | | |
|---|---|---|---|
| CC-3-V | 28,00 % | Klärpunkt [°C]: | 100 |
| CC-3-V1 | 10,00 % | Δn [589 nm, 20 °C] : | 0,1181 |
| CC-3-2V1 | 8,50 % | Δε [1 kHz, 20 °C]: | 5,2 |
| CCP-V-1 | 12,00 % | ε_{∥} [1 kHz, 20°C]: | 8,0 |
| CCP-V2-1 | 5,50 % | ε_{⊥} [1 kHz, 20°C]: | 2,8 |
| CLP-3-T | 6,50 % | γ₁ [mPa·s, 20 °C]: | 80 |
| PGP-2-2V | 13,00 % | K₁ [pN, 20 °C]]: | 18,6 |
| APUQU-3-F | 5,50 % | K₃ [pN, 20 °C]: | 18,6 |
| PGUQU-3-F | 5,00 % | | |
| PGUQU-4-F | 6,00 % | | |

### Beispiel M9

| | | | |
|---|---|---|---|
| CC-3-V | 28,00 % | Klärpunkt [°C]: | 100,5 |
| CC-3-V1 | 10,00 % | Δn [589 nm, 20 °C] : | 0,1184 |
| CC-3-2V1 | 9,00 % | Δε [1 kHz, 20 °C]: | 5,2 |
| CCP-V-1 | 11,50 % | ε_{∥} [1 kHz, 20°C]: | 8,0 |
| CCP-V2-1 | 4,50 % | ε_{⊥} [1 kHz, 20°C]: | 2,8 |
| CLP-3-T | 7,50 % | γ₁ [mPa·s, 20 °C]: | 81 |
| PGP-2-2V | 13,00 % | K₁ [pN, 20 °C]: | 19,1 |
| APUQU-3-F | 5,50 % | K₃ [pN,20 °C]: | 18,7 |
| PGUQU-3-F | 4,00 % | | |
| PGUQU-4-F | 6,00 % | | |
| CPGU-3-OT | 1,00 % | | |

### Beispiel M 10

| | | | |
|---|---|---|---|
| APUQU-2-F | 6,00 % | Klärpunkt [°C]: | 80 |
| APUQU-3-F | 4,00 % | Δn[589nm,20°C]: | 0,1009 |
| CC-3-V | 41,00 % | Δε [1 kHz, 20 °C]: | 9,5 |
| CC-3-V1 | 10,50 % | ε_{∥} [1 kHz, 20°C]: | 12,7 |
| CLP-3-T | 7,00 % | ε_{⊥} [1 kHz, 20°C]: | 3,2 |
| CCP-3OCF₃ | 4,50 % | γ₁ [mPa·s, 20 °C]: | 69 |
| CCP-V-1 | 6,00 % | K₁ [pN, 20 °C]: | 13,5 |
| PGUQU-3-F | 6,00 % | K₃ [pN, 20 °C]: | 15,8 |
| PGUQU-4-F | 6,00 % | | |
| PGUQU-5-F | 6,00 % | | |
| PUQU-3-F | 3,00 % | | |

### Beispiel M11

| | | | |
|---|---|---|---|
| APUQU-2-F | 6,00 % | Klärpunkt [°C]: | 79,5 |
| APUQU-3-F | 4,00 % | Δn[589nm,20°C]: | 0,1006 |
| CC-3-V | 44,00 % | Δε [1 kHz, 20 °C]: | 9,4 |
| CC-3-V1 | 6,50 % | ε_{∥} [1 kHz, 20°C]: | 12,6 |
| CLP-3-T | 7,00 % | ε_{⊥} [1 kHz, 20°C]: | 3,2 |
| CCP-3OCF₃ | 4,50 % | γ₁ [mPa·s, 20 °C]: | 68 |
| CCP-V-1 | 7,00 % | K₁ [pN, 20 °C]: | 13,2 |
| PGUQU-3-F | 6,00 % | K₃ [pN, 20 °C]: | 15,4 |
| PGUQU-4-F | 6,00 % | | |
| PGUQU-5-F | 6,00 % | | |
| PUQU-3-F | 3,00 % | | |

### Beispiel M12

| | | | |
|---|---|---|---|
| APUQU-2-F | 7,00 % | Klärpunkt [°C]: | 83,5 |
| APUQU-3-F | 5,00 % | Δn[589nm,20°C]: | 0,1014 |
| CC-3-V | 42,00 % | Δε [1 kHz, 20 °C]: | 9,5 |
| CC-3-V1 | 9,00 % | ε_{∥} [1 kHz, 20°C]: | 12,7 |
| CLP-3-T | 6,00 % | ε_{⊥} [1 kHz, 20°C]: | 3,2 |
| CCP-3OCF₃ | 5,00 % | γ₁ [mPa·s, 20 °C]: | 72 |
| CCP-V-1 | 7,00 % | K₁ [pN, 20 °C]: | 13,8 |
| PGUQU-3-F | 7,00 % | K₃ [pN, 20 °C]: | 16,0 |
| PGUQU-4-F | 6,00 % | | |
| PGUQU-5-F | 6,00 % | | |

### Beispiel M13

| | | | |
|---|---|---|---|
| APUQU-2-F | 6,00 % | Klärpunkt [°C]: | 83,5 |
| APUQU-3-F | 5,00 % | Δn[589nm,20°C]: | 0,1014 |
| CC-3-V | 40,50 % | Δε [1 kHz, 20 °C]: | 9,5 |
| CC-3-V1 | 8,00 % | ε_{∥} [1 kHz, 20°C]: | 12,6 |
| CLP-3-T | 7,00 % | ε_{⊥} [1 kHz, 20°C]: | 3,2 |
| CCP-3OCF₃ | 5,00 % | γ₁ [mPa·s, 20 °C]: | 73 |
| CCP-V-1 | 9,00 % | K₁ [pN, 20 °C]: | 13,8 |
| PGUQU-3-F | 6,00 % | K₃ [pN, 20 °C]: | 16,0 |
| PGUQU-4-F | 6,00 % | | |
| PGUQU-5-F | 5,00 % | | |
| PUQU-3-F | 2,50 % | | |

### Beispiel M14

| | | | |
|---|---|---|---|
| APUQU-2-F | 7,00 % | Klärpunkt [°C]: | 83,5 |
| APUQU-3-F | 5,00 % | Δn[589nm,20°C]: | 0,1019 |
| CC-3-V | 42,00 % | Δε [1 kHz, 20 °C]: | 9,5 |
| CC-3-V1 | 9,00 % | ε_{∥} [1 kHz, 20°C]: | 12,7 |
| CLP-3-T | 7,00 % | ε_{⊥} [1 kHz, 20°C]: | 3,2 |
| CCP-3OCF3 | 5,00 % | γ₁ [mPa·s, 20 °C]: | 72 |
| CCP-V-1 | 6,50 % | K₁ [pN, 20 °C]: | 14,0 |
| PGUQU-3-F | 7,00 % | K₃ [pN, 20 °C]: | 15,7 |
| PGUQU-4-F | 6,00 % | | |
| PGUQU-5-F | 5,50 % | | |

### Beispiel M15

| | | | |
|---|---|---|---|
| CC-3-V | 28,50 % | Klärpunkt [°C]: | 94 |
| CC-3-V1 | 10,00 % | Δn[589nm,20°C]: | 0,1057 |
| CC-3-2V1 | 6,00 % | Δε [1 kHz, 20 °C]: | 17,6 |
| CCP-V-1 | 6,00 % | ε_{∥} [1 kHz, 20°C]: | 21,5 |
| CLP-3-T | 5,50 % | ε_{⊥} [1 kHz, 20°C]: | 3,9 |
| APUQU-2-F | 6,00 % | γ₁ [mPa·s, 20 °C]: | 111 |
| APUQU-3-F | 9,00 % | K₁ [pN, 20 °C]: | 16,0 |
| PGUQU-3-F | 3,50 % | K₃ [pN, 20 °C]: | 16,7 |
| CDUQU-3-F | 7,50 % | | |
| DPGU-4-F | 6,00 % | | |
| DGUQU-4-F | 8,00 % | | |
| DGUQU-2-F | 3,00 % | | |
| PGU-4-T | 1,00 % | | |

### Beispiel M16

| | | | |
|---|---|---|---|
| CC-3-V | 29,00 % | Klärpunkt [°C]: | 94,5 |
| CC-3-V1 | 10,00 % | Δn[589nm,20°C]: | 0,1040 |
| CC-3-2V1 | 6,00 % | Δε [1 kHz, 20 °C]: | 17,2 |
| CCP-V-1 | 6,00 % | ε_{∥} [1 kHz, 20°C]: | 21,1 |
| CLP-3-T | 5,50 % | ε_{⊥} [1 kHz, 20°C]: | 3,9 |
| APUQU-2-F | 6,00 % | γ₁ [mPa·s, 20 °C]: | 108 |
| APUQU-3-F | 9,00 % | K₁ [pN, 20 °C]: | 15,9 |
| PGUQU-3-F | 3,50 % | K₃ [pN,20 °C]: | 16,9 |
| CDUQU-3-F | 8,00 % | | |
| DPGU-4-F | 6,00 % | | |
| DGUQU-4-F | 8,00 % | | |
| DGUQU-2-F | 3,00 % | | |

### Beispiel M17

| | | | |
|---|---|---|---|
| CC-3-V | 29,00 % | Klärpunkt [°C]: | 93,5 |
| CC-3-V1 | 10,00 % | Δn[589nm,20°C]: | 0,1040 |
| CC-3-2V1 | 6,00 % | Δε [1 kHz, 20 °C]: | 17,1 |
| CCP-V-1 | 5,00 % | ε_{∥} [1 kHz, 20°C]: | 20,9 |
| CLP-3-T | 7,00 % | ε_{⊥} [1 kHz, 20°C]: | 3,8 |
| APUQU-2-F | 6,00 % | γ₁ [mPa·s, 20 °C]: | 110 |
| APUQU-3-F | 9,00 % | K₁ [pN, 20 °C]: | 16,2 |
| PGUQU-3-F | 4,00 % | K₃ [pN, 20 °C]: | 16,6 |
| CDUQU-3-F | 8,00 % | | |
| DPGU-4-F | 5,00 % | | |
| DGUQU-4-F | 8,00 % | | |
| DGUQU-2-F | 3,00 % | | |

### Beispiel M18

| | | | |
|---|---|---|---|
| CC-3-V | 50,00 % | Klärpunkt [°C]: | 77,5 |
| CC-3-V1 | 2,00 % | Δn [589 nm, 20 °C] : | 0,1172 |
| CLP-3-OT | 7,50 % | Δε [1 kHz, 20 °C]: | 2,3 |
| CLP-3-T | 11,00 % | ε_{∥} [1 kHz, 20°C]: | 4,8 |
| PGP-1-2V | 2,00 % | ε_{⊥} [1 kHz, 20°C]: | 2,6 |
| PGP-2-2V | 16,00 % | γ₁ [mPa·s, 20 °C]: | 48 |
| PGP-3-2V | 1,00 % | K₁ [pN, 20 °C]: | 17,3 |
| PGU-2-F | 2,50 % | K₃ [pN,20 °C]: | 14,4 |
| PP-1-2V1 | 8,00 % | | |

### Beispiel M19

| | | | |
|---|---|---|---|
| CC-3-V | 51,50 % | Klärpunkt [°C]: | 75 |
| CC-3-V1 | 4,00 % | Δn [589 nm, 20 °C] : | 0,1175 |
| CLP-3-OT | 6,00 % | Δε [1 kHz, 20 °C]: | 2,3 |
| CLP-3-T | 7,00 % | ε_{∥} [1 kHz, 20°C]: | 4,9 |
| PGP-1-2V | 2,00 % | ε_{⊥} [1 kHz, 20°C]: | 2,7 |
| PGP-2-2V | 17,00 % | γ₁ [mPa·s, 20 °C]: | 46 |
| PGP-3-2V | 2,00 % | K₁ [pN, 20 °C]: | 15,6 |
| PGU-2-F | 5,50 % | K₃ [pN, 20 °C]: | 13,6 |
| PP-1-2V1 | 5,00 % | | |

### Beispiel M20

| | | | |
|---|---|---|---|
| CC-3-V | 53,50 % | Klärpunkt [°C]: | 75 |
| CC-3-V1 | 3,50 % | Δn [589 nm, 20 °C] : | 0,1188 |
| CLP-3-OT | 7,00 % | Δε [1 kHz, 20 °C]: | 2,3 |
| CLP-3-T | 3,00 % | ε_{∥} [1 kHz, 20°C]: | 4,9 |
| PGP-1-2V | 2,50 % | ε_{⊥} [1 kHz, 20°C]: | 2,7 |
| PGP-2-2V | 14,00 % | γ₁ [mPa·s, 20 °C]: | 44 |
| PGP-3-2V | 2,00 % | K₁ [pN, 20 °C]: | 14,5 |
| PGU-2-F | 5,00 % | K₃ [pN, 20 °C]: | 13,8 |
| PGUQU-3-F | 3,00 % | | |
| PP-1-2V1 | 5,50 % | | |
| PPGU-3-F | 1,00 % | | |

### Beispiel M21

| | | | |
|---|---|---|---|
| CC-3-V | 28,50 % | Klärpunkt [°C]: | 98,5 |
| CC-3-V1 | 10,00 % | Δn [589 nm, 20 °C] : | 0,1183 |
| CC-3-2V1 | 10,00 % | Δε [1 kHz, 20 °C]: | 5,2 |
| CLP-V-1 | 11,50 % | ε_{∥} [1 kHz, 20°C]: | 7,9 |
| CCP-V-1 | 7,50 % | ε_{⊥} [1 kHz, 20°C]: | 2,7 |
| CLP-3-T | 7,00 % | γ₁ [mPa·s, 20 °C]: | 77 |
| PGP-2-2V | 8,00 % | K₁ [pN, 20 °C]: | 19,6 |
| PGU-2-F | 4,00 % | K₃ [pN, 20 °C]: | 18,2 |
| PGUQU-3-F | 6,00 % | | |
| PGUQU-4-F | 6,00 % | | |
| CPGU-3-OT | 1,50 % | | |

### Beispiel M22

| | | | |
|---|---|---|---|
| CC-3-V | 28,50 % | Klärpunkt [°C]: | 98 |
| CC-3-V1 | 11,00 % | Δn [589 nm, 20 °C] : | 0,1179 |
| CC-3-2V1 | 9,00 % | Δε [1 kHz, 20 °C]: | 5,1 |
| CLP-V-1 | 11,50 % | ε_{∥} [1 kHz, 20°C]: | 7,9 |
| CCP-V-1 | 8,00 % | ε_{⊥} [1 kHz, 20°C]: | 2,8 |
| CLP-3-T | 7,00 % | γ₁ [mPa·s, 20 °C]: | 76 |
| PGP-2-2V | 7,50 % | K₁ [pN, 20 °C]: | 19,3 |
| PGU-2-F | 4,50 % | K₃ [pN, 20 °C]: | 18,4 |
| PGUQU-3-F | 6,00 % | | |
| PGUQU-4-F | 6,00 % | | |
| CPGU-3-OT | 1,00 % | | |

### Beispiel M23

| | | | |
|---|---|---|---|
| CC-3-V | 33,00 % | Klärpunkt [°C]: | 92 |
| CC-3-V1 | 7,00 % | Δn[589nm,20°C]: | 0,1042 |
| CC-3-2V1 | 4,00 % | Δε [1 kHz, 20 °C]: | 16,7 |
| CLP-V-1 | 8,00 % | ε_{∥} [1 kHz, 20°C]: | 20,5 |
| CLP-3-T | 5,50 % | ε_{⊥} [1 kHz, 20°C]: | 3,8 |
| APUQU-2-F | 9,00 % | γ₁ [mPa·s, 20 °C]: | 105 |
| APUQU-3-F | 9,00 % | K₁ [pN, 20 °C]: | 15,8 |
| PGUQU-3-F | 3,50 % | K₃ [pN, 20 °C]: | 16,4 |
| CDUQU-3-F | 8,00 % | | |
| DPGU-4-F | 2,00 % | | |
| DGUQU-4-F | 8,00 % | | |
| DGUQU-2-F | 3,00 % | | |

### Beispiel M24

| | | | |
|---|---|---|---|
| APUQU-2-F | 9,50 % | Klärpunkt [°C]: | 85 |
| APUQU-3-F | 7,50 % | Δn[589nm,20°C]: | 0,1035 |
| CC-3-V | 46,00 % | Δε [1 kHz, 20 °C]: | 9,6 |
| CC-3-V1 | 6,00 % | ε_{∥} [1 kHz, 20°C]: | 12,8 |
| CLP-3-T | 3,00 % | ε_{⊥} [1 kHz, 20°C]: | 3,2 |
| CLP-V-1 | 10,00 % | γ₁ [mPa·s, 20 °C]: | 70 |
| CCP-V-1 | 3,00 % | K₁ [pN, 20 °C]: | 14,0 |
| PGUQU-3-F | 8,00 % | K₃ [pN, 20 °C]: | 15,6 |
| PGUQU-4-F | 7,00 % | | |

### Beispiel M25

| | | | |
|---|---|---|---|
| APUQU-2-F | 9,00 % | Klärpunkt [°C]: | 84 |
| APUQU-3-F | 7,50 % | Δn[589nm,20°C]: | 0,1013 |
| CC-3-V | 42,50 % | Δε [1 kHz, 20 °C]: | 9,4 |
| CC-3-V1 | 11,00 % | ε_{∥} [1 kHz, 20°C]: | 12,6 |
| CCP-V-1 | 6,50 % | ε_{⊥} [1 kHz, 20°C]: | 3,2 |
| CLP-3-T | 4,00 % | γ₁ [mPa·s, 20 °C]: | 70 |
| CLP-V-1 | 4,50 % | K₁ [pN, 20 °C]: | 13,9 |
| PGUQU-3-F | 8,00 % | K₃ [pN, 20 °C]: | 16,0 |
| PGUQU-4-F | 5,00 % | | |
| PGUQU-5-F | 2,00 % | | |

### Beispiel M26

| | | | |
|---|---|---|---|
| CC-3-V | 51,50 % | Klärpunkt [°C]: | 74 |
| CC-3-V1 | 5,00 % | Δn[589nm,20°C]: | 0,1174 |
| CCP-V-1 | 5,00 % | Δε [1 kHz, 20 °C]: | 2,2 |
| CLP-3-OT | 4,50 % | ε_{∥} [1 kHz, 20°C]: | 4,9 |
| PGP-1-2V | 3,00 % | ε_{⊥} [1 kHz, 20°C]: | 2,7 |
| PGP-2-2V | 14,50 % | γ₁ [mPa·s, 20 °C]: | 44 |
| PGP-3-2V | 2,00 % | K₁ [pN, 20 °C]: | 13,7 |
| PGU-2-F | 4,00 % | K₃ [pN 20 °C]: | 13,6 |
| PGUQU-3-F | 3,50 % | LTS [bulk, -20 °C]: | > 1000 h |
| PP-1-2V1 | 6,00 % | | |
| PPGU-3-F | 1,00 % | | |

### Beispiel M26a

Die flüssigkristalline Mischung M26 wird zusätzlich mit 300 ppm der Verbindung der Formel ST-2 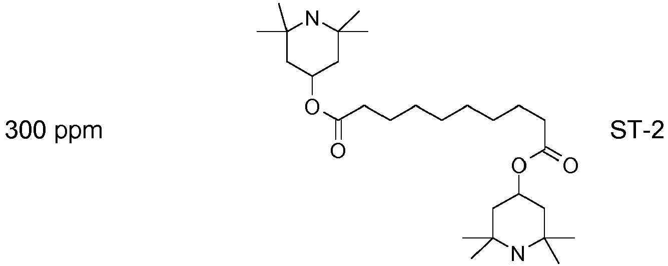 stabilisiert.

### Beispiel M27

| | | | |
|---|---|---|---|
| CC-3-V | 51,50 % | Klärpunkt [°C]: | 73,5 |
| CC-3-V1 | 5,00 % | Δn[589nm,20°C]: | 0,1174 |
| CCP-V-1 | 6,00 % | Δε [1 kHz, 20 °C]: | 2,2 |
| CLP-3-T | 3,50 % | ε_{∥} [1 kHz, 20°C]: | 4,9 |
| PGP-1-2V | 3,00 % | ε_{⊥} [1 kHz, 20°C]: | 2,7 |
| PGP-2-2V | 14,50 % | γ₁ [mPa·s, 20 °C]: | 44 |
| PGP-3-2V | 2,00 % | K₁ [pN, 20 °C]: | 13,9 |
| PGU-2-F | 4,50 % | K₃ [pN, 20 °C]: | 13,6 |
| PGUQU-3-F | 3,00 % | LTS [bulk, -20 °C]: | > 1000 h |
| PP-1-2V1 | 6,00 % | | |
| PPGU-3-F | 1,00 % | | |

### Beispiel M27a

Die flüssigkristalline Mischung M27 wird zusätzlich mit 300 ppm der Verbindung der Formel ST-2 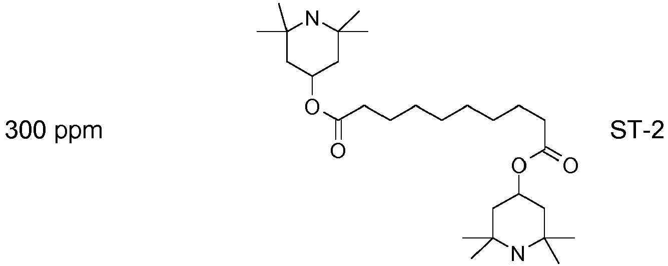 stabilisiert.

### Beispiel M28

| | | | |
|---|---|---|---|
| CC-3-V | 48,00 % | Klärpunkt [°C]: | 88 |
| CCP-V-1 | 12,00 % | Δn[589nm,20°C]: | 0,1154 |
| CCP-V2-1 | 5,00 % | Δε [1 kHz, 20 °C]: | 3,5 |
| PP-1-2V1 | 1,00 % | ε_{∥} [1 kHz, 20°C]: | 6,3 |
| PGP-1-2V | 5,00 % | ε_{⊥} [1 kHz, 20°C]: | 2,8 |
| PGP-2-2V | 7,50 % | γ₁ [mPa·s, 20 °C]: | 59 |
| PGP-3-2V | 4,00 % | K₁ [pN, 20 °C]: | 15,0 |
| CLP-3-T | 5,00 % | K₃ [pN, 20 °C]: | 15,7 |
| PGUQU-4-F | 5,50 % | LTS [bulk, -20 °C]: | > 1000 h |
| APUQU-3-F | 4,00 % | | |
| PGU-2-F | 3,00 % | | |

### Beispiel M28a

Die flüssigkristalline Mischung M28 wird zusätzlich mit 400 ppm der Verbindung der Formel ST-1 und mit 1000 ppm der der Verbindung der Formel ST-2 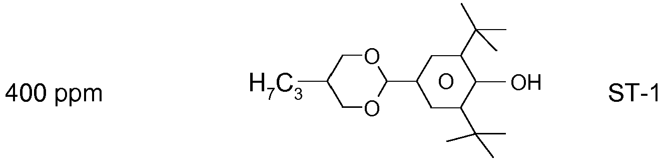 und 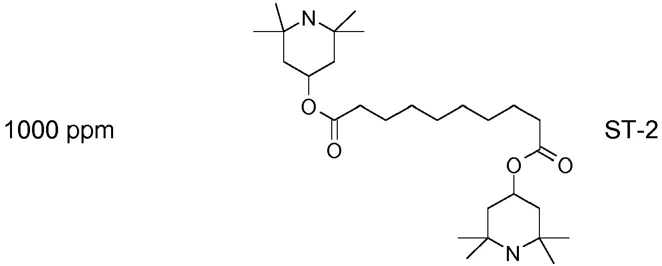 stabilisiert.

### Beispiel M29

| | | | |
|---|---|---|---|
| CC-3-V | 49,50 % | Klärpunkt [°C]: | 89,5 |
| CCP-V-1 | 5,00 % | Δn[589nm,20°C]: | 0,1172 |
| CLP-V-1 | 8,50 % | Δε [1 kHz, 20 °C]: | 3,5 |
| PP-1-2V1 | 1,00 % | ε_{∥} [1 kHz, 20°C]: | 6,3 |
| PGP-1-2V | 5,00 % | ε_{⊥} [1 kHz, 20°C]: | 2,8 |
| PGP-2-2V | 8,00 % | γ₁ [mPa·s, 20 °C]: | 57 |
| PGP-3-2V | 4,50 % | K₁ [pN, 20 °C]: | 15,2 |
| CCP-3OCF₃ | 6,50 % | K₃ [pN, 20 °C]: | 15,8 |
| PGUQU-4-F | 5,00 % | | |
| PGUQU-5-F | 2,50 % | | |
| APUQU-3-F | 4,50 % | | |

### Beispiel M29a

Die flüssigkristalline Mischung M29 wird zusätzlich mit 400 ppm der Verbindung der Formel ST-1 und mit 1000 ppm der Verbindung der Formel ST-2 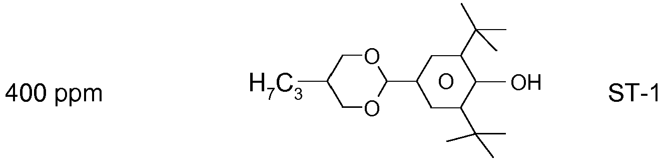 und 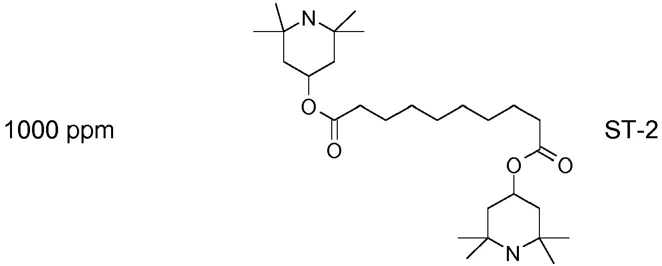 stabilisiert.

### Beispiel M30

| | | | |
|---|---|---|---|
| CC-3-V | 52,00 % | Klärpunkt [°C]: | 85,5 |
| CLP-V-1 | 11,50 % | Δn [589 nm, 20 °C] : | 0,1187 |
| PP-1-2V1 | 3,00 % | Δε [1 kHz, 20 °C]: | 3,5 |
| PGP-1-2V | 5,00 % | ε_{∥} [1 kHz, 20°C]: | 6,2 |
| PGP-2-2V | 8,00 % | ε_{⊥} [1 kHz, 20°C]: | 2,7 |
| PGP-3-2V | 3,00 % | γ₁ [mPa·s, 20 °C]: | 56 |
| CLP-3-T | 7,00 % | K₁ [pN, 20 °C]: | 16,2 |
| PGUQU-4-F | 5,00 % | K₃ [pN, 20 °C]: | 15,4 |
| PGUQU-5-F | 2,50 % | LTS [bulk, -20 °C]: > | > 1000 h |
| APUQU-3-F | 3,00 % | | |

### Beispiel M30a

Die flüssigkristalline Mischung M30 wird zusätzlich mit 400 ppm der Verbindung der Formel ST-1 und mit 1000 ppm der Verbindung der Formel ST-2 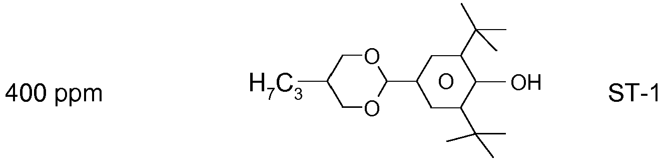 und 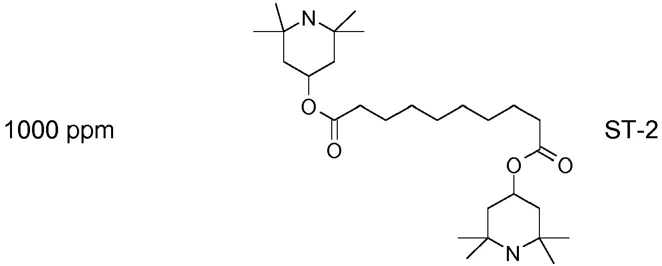 stabilisiert.

### Beispiel M31

| | | | |
|---|---|---|---|
| CC-3-V | 51,50 % | Klärpunkt [°C]: | 85,5 |
| CLP-V-1 | 11,50 % | Δn [589 nm, 20 °C] : | 0,1175 |
| PP-1-2V1 | 3,00 % | Δε [1 kHz, 20 °C]: | 4,1 |
| PGP-1-2V | 5,00 % | ε_{∥} [1 kHz, 20°C]: | 6,8 |
| PGP-2-2V | 6,00 % | ε_{⊥} [1 kHz, 20°C]: | 2,8 |
| PGP-3-2V | 3,00 % | γ₁ [mPa·s, 20 °C]: | 58 |
| CLP-3-T | 7,00 % | K₁ [pN, 20 °C]: | 16,0 |
| PGUQU-4-F | 5,00 % | K₃ [pN, 20 °C]: | 15,5 |
| PGUQU-5-F | 5,00 % | LTS [bulk, -20 °C]: | > 1000 h |
| APUQU-3-F | 3,00 % | | |

### Beispiel M31a

Die flüssigkristalline Mischung M31 wird zusätzlich mit 400 ppm der Verbindung der Formel ST-1 und mit 1000 ppm der Verbindung der Formel ST-2 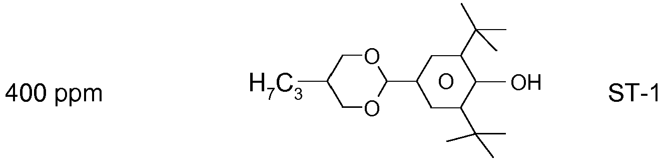 und 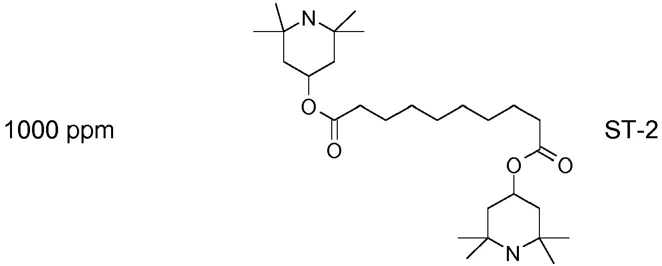 stabilisiert.

### Beispiel M32

| | | | |
|---|---|---|---|
| CC-3-V | 41,00 % | Klärpunkt [°C]: | 81 |
| B-5O-OT | 4,50 % | Δn [589 nm, 20 °C] : | 0,1060 |
| B-2O-O5 | 6,00 % | Δε [1 kHz, 20 °C]: | 4,6 |
| CCP-V-1 | 15,50 % | ε_{∥} [1 kHz, 20°C]: | 9,0 |
| CLP-V-1 | 6,00 % | ε_{⊥} [1 kHz, 20°C]: | 4,4 |
| PGP-2-2V | 2,00 % | γ₁ [mPa·s, 20 °C]: | 64 |
| CCP-30CF₃ | 7,50 % | K₁ [pN, 20 °C]: | 14,5 |
| PUQU-3-F | 3,00 % | K₃ [pN, 20 °C]: | 13,9 |
| PGU-3-F | 5,00 % | V₀ [V, 20°C]: | 1,88 |
| DGUQU-4-F | 5,00 % | | |
| DPGU-4-F | 4,50 % | | |

### Beispiel M33

| | | | |
|---|---|---|---|
| CC-3-V | 42,00 % | Klärpunkt [°C]: | 80,5 |
| B-5O-OT | 4,50 % | Δn [589 nm, 20 °C] : | 0,1047 |
| B-2O-O5 | 6,00 % | Δε [1 kHz, 20 °C]: | 4,5 |
| CCP-V-1 | 15,50 % | ε_{∥} [1 kHz, 20°C]: | 8,9 |
| CCP-V2-1 | 8,00 % | ε_{⊥} [1 kHz, 20°C]: | 4,4 |
| PGP-2-2V | 2,00 % | γ₁ [mPa·s, 20 °C]: | 66 |
| CLP-3-T | 4,50 % | K₁ [pN, 20 °C]: | 14,4 |
| PUQU-3-F | 3,00 % | K₃ [pN,20 °C]: | 14,0 |
| PGU-3-F | 5,00 % | V₀ [V, 20°C]: | 1,89 |
| DGUQU-4-F | 5,00 % | | |
| DPGU-4-F | 4,50 % | | |

### Beispiel M34

| | |
|---|---|
| CC-3-V | 33,00 % |
| CC-3-V1 | 5,00 % |
| B-2O-O5 | 4,00 % |
| B(S)-2O-O4 | 3,00 % |
| B(S)-2O-O5 | 4,00 % |
| CLP-3-T | 3,00 % |
| CCP-3OCF₃ | 3,00 % |
| CCP-V-1 | 13,00 % |
| CCP-V2-1 | 6,00 % |
| CCVC-3-V | 5,00 % |
| PUQU-3-F | 6,00 % |
| CPGP-5-2 | 4,00 % |
| APUQU-2-F | 2,50 % |
| APUQU-3-F | 2,00 % |
| CDUQU-3-F | 2,50 % |
| DGUQU-4-F | 2,00 % |
| DPGU-4-F | 1,50 % |
| PPGU-3-F | 0,50 % |

### Beispiel M35

| | |
|---|---|
| CC-3-V | 30,00 % |
| CC-3-V1 | 5,00 % |
| B-2O-O5 | 4,00 % |
| B(S)-2O-O4 | 6,00 % |
| B(S)-2O-O5 | 6,00 % |
| CLP-3-T | 3,00 % |
| CCP-30CF₃ | 5,00 % |
| CCP-V-1 | 13,00 % |
| CCP-V2-1 | 1,50 % |
| CCVC-3-V | 5,00 % |
| PUQU-3-F | 6,00 % |
| CPGP-5-2 | 4,00 % |
| APUQU-2-F | 2,50 % |
| APUQU-3-F | 2,50 % |
| CDUQU-3-F | 2,50 % |
| DGUQU-4-F | 2,00 % |
| DPGU-4-F | 1,50 % |
| PPGU-3-F | 0,50 % |

### Beispiel M36

| | |
|---|---|
| CC-3-V | 34,00 % |
| CC-3-V1 | 7,50 % |
| B-2O-O5 | 4,00 % |
| B(S)-2O-O4 | 3,00 % |
| B(S)-2O-O5 | 4,00 % |
| CCP-3OCF₃ | 5,00 % |
| CLP-3-T | 5,00 % |
| CCP-V-1 | 13,00 % |
| PUQU-3-F | 4,00 % |
| APUQU-2-F | 5,00 % |
| APUQU-3-F | 4,00 % |
| CDUQU-3-F | 3,50 % |
| DGUQU-4-F | 3,00 % |
| DPGU-4-F | 2,00 % |
| PPGU-3-F | 0,50 % |
| CPGP-5-2 | 2,50 % |

### Beispiel M37

| | |
|---|---|
| CC-3-V | 31,50 % |
| CC-3-V1 | 7,50 % |
| B-2O-O5 | 4,00 % |
| B(S)-2O-O4 | 6,00 % |
| B(S)-2O-O5 | 6,00 % |
| CCP-3OCF₃ | 5,00 % |
| CLP-3-T | 5,00 % |
| CCP-V-1 | 9,50 % |
| PUQU-3-F | 4,00 % |
| APUQU-2-F | 4,00 % |
| APUQU-3-F | 4,50 % |
| CDUQU-3-F | 5,00 % |
| DGUQU-4-F | 3,00 % |
| DPGU-4-F | 2,00 % |
| PPGU-3-F | 0,50 % |
| CPGP-5-2 | 2,50 % |

### Beispiel M38

| | |
|---|---|
| Y-4O-O4 | 10,50 % |
| CC-3-V | 25,00 % |
| CCP-3OCF₃ | 5,00 % |
| CLP-3-T | 5,00 % |
| CCP-V-1 | 14,00 % |
| CCP-V2-1 | 4,50 % |
| PGP-2-2V | 6,50 % |
| DGUQU-4-F | 5,00 % |
| DPGU-4-F | 5,00 % |
| APUQU-2-F | 7,00 % |
| APUQU-3-F | 8,00 % |
| PGUQU-4-F | 4,00 % |
| PPGU-3-F | 0,50 % |

### Beispiel M39

| | |
|---|---|
| Y-4O-O4 | 10,50 % |
| CC-3-V | 22,00 % |
| B(S)-2O-O4 | 3,00 % |
| B(S)-2O-O5 | 4,00 % |
| CCP-3OCF₃ | 5,00 % |
| CLP-3-T | 5,00 % |
| CCP-V-1 | 13,00 % |
| CCP-V2-1 | 6,00 % |
| DGUQU-4-F | 5,00 % |
| DPGU-4-F | 5,00 % |
| APUQU-2-F | 7,00 % |
| APUQU-3-F | 7,00 % |
| CDUQU-3-F | 3,00 % |
| PGUQU-4-F | 4,00 % |
| PPGU-3-F | 0,50 % |

### Beispiel M40

| | | | |
|---|---|---|---|
| CC-3-V | 49,00 % | Klärpunkt [°C]: | 80,5 |
| CC-3-V1 | 12,00 % | Δn [589 nm, 20 °C] : | 0,0930 |
| CCP-V-1 | 10,00 % | Δε [1 kHz, 20 °C]: | 2,7 |
| CLP-V-1 | 7,00 % | ε_{∥} [1 kHz, 20°C]: | 5,3 |
| PGP-2-2V | 5,50 % | ε_{⊥} [1 kHz, 20°C]: | 2,6 |
| CLP-3-T | 4,00 % | γ₁ [mPa·s, 20 °C]: | 48 |
| PGUQU-3-F | 3,00 % | K₁ [pN, 20 °C]: | 14,9 |
| APUQU-2-F | 6,00 % | K₃ [pN, 20 °C]: | 16,0 |
| PP-1-2V1 | 3,00 % | | |
| PPGU-3-F | 0,50 % | | |

Die flüssigkristalline Mischung M40 wird zusätzlich mit 400 ppm der Formel ST-1 und verschiedenen Konzentrationen der Verbindungen der Formel ST-2 oder ST-3 stabilisiert:

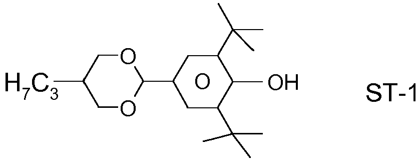

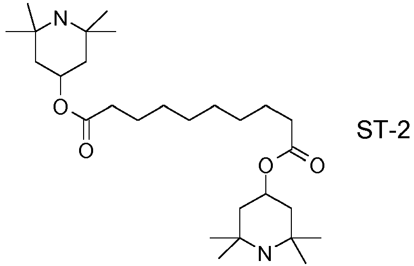

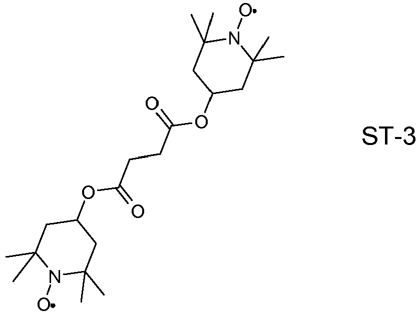

| Mischung | ST-1 | ST-2 | ST-3 |
|---|---|---|---|
| Beispiel M40a | 400 ppm | - | - |
| Beispiel M40b | 400 ppm | 100 ppm | - |
| Beispiel M40c | 400 ppm | 500 ppm | - |
| Beispiel M40d | 400 ppm | 1000 ppm | - |
| Beispiel M40e | 400 ppm | - | 100 ppm |
| Beispiel M40f | 400 ppm | - | 500 ppm |
| Beispiel M40g | 400 ppm | - | 1000 ppm |

### Beispiel M41

| | |
|---|---|
| CC-3-V | 42,50 % |
| B-2O-O5 | 3,50 % |
| B(S)-2O-O4 | 4,00 % |
| B(S)-2O-O5 | 4,00 % |
| CCP-3OCF₃ | 5,00 % |
| CLP-3-T | 5,00 % |
| CCP-V-1 | 1,50 % |
| DGUQU-4-F | 5,00 % |
| DPGU-4-F | 4,00 % |
| APUQU-2-F | 7,00 % |
| APUQU-3-F | 7,00 % |
| CDUQU-3-F | 2,00 % |
| PGUQU-3-F | 4,00 % |
| PGUQU-4-F | 5,00 % |
| PPGU-3-F | 0,50 % |

### Beispiel M42

| | | | |
|---|---|---|---|
| CC-3-V | 43,00 % | Klärpunkt [°C]: | 80 |
| B-5O-OT | 5,00 % | Δn [589 nm, 20 °C] : | 0,1048 |
| B-2O-O5 | 5,50 % | Δε [1 kHz, 20 °C]: | 4,6 |
| CCP-V-1 | 15,00 % | ε_{∥} [1 kHz, 20°C]: | 9,0 |
| CCP-V2-1 | 6,00 % | ε_{⊥} [1 kHz, 20°C]: | 4,4 |
| PGP-2-2V | 2,50 % | K₁ [pN, 20 °C]: | 14,3 |
| CLP-3-T | 6,00 % | K₃ [pN, 20 °C]: | 13,9 |
| PGU-3-F | 7,00 % | V₀ [V, 20 °C]: | 1,88 |
| DGUQU-4-F | 5,50 % | | |
| DPGU-4-F | 4,50 % | | |

### Beispiel M43

| | |
|---|---|
| APUQU-2-F | 4,00 % |
| APUQU-3-F | 5,50 % |
| B-2O-O5 | 4,00 % |
| CC-3-V | 22,00 % |
| CC-3-V1 | 4,00 % |
| CCP-3-1 | 4,00 % |
| CCP-30CF₃ | 8,00 % |
| CCP-V-1 | 6,50 % |
| CDUQU-3-F | 4,50 % |
| DGUQU-4-F | 5,00 % |
| DPGU-4-F | 3,00 % |
| PGUQU-3-F | 3,00 % |
| PPGU-3-F | 0,50 % |
| PY-3-O2 | 9,50 % |
| Y-4O-O4 | 5,00 % |
| CCY-3-O2 | 4,50 % |
| CLP-3-T | 5,00 % |
| PGP-2-2V | 2,00 % |

### Beispiel M44

| | |
|---|---|
| APUQU-2-F | 4,00 % |
| APUQU-3-F | 5,00 % |
| B-2O-O5 | 3,50 % |
| CC-3-V | 33,00 % |
| PP-2-2V1 | 2,00 % |
| B(S)-2O-O4 | 4,00 % |
| CC-3-V1 | 4,00 % |
| CLP-3-T | 5,00 % |
| B(S)-2O-O5 | 4,00 % |
| CCP-30CF₃ | 8,00 % |
| CCP-V-1 | 5,00 % |
| CDUQU-3-F | 4,00 % |
| DGUQU-4-F | 5,00 % |
| DPGU-4-F | 3,00 % |
| PGUQU-3-F | 3,00 % |
| PGUQU-4-F | 3,00 % |
| PPGU-3-F | 1,50 % |
| Y-4O-O4 | 3,00 % |

### Beispiel M45

| | |
|---|---|
| APUQU-2-F | 4,00 % |
| APUQU-3-F | 5,00 % |
| B-2O-O5 | 3,50 % |
| CC-3-V | 33,00 % |
| PP-1-2V1 | 2,00 % |
| B(S)-2O-O4 | 4,00 % |
| CC-3-V1 | 4,00 % |
| CLP-3-T | 5,00 % |
| B(S)-2O-O5 | 4,00 % |
| CCP-30CF₃ | 8,00 % |
| CCP-V-1 | 5,00 % |
| CDUQU-3-F | 4,00 % |
| DGUQU-4-F | 5,00 % |
| DPGU-4-F | 3,00 % |
| PGUQU-3-F | 3,00 % |
| PGUQU-4-F | 3,00 % |
| PPGU-3-F | 1,50 % |
| Y-4O-O4 | 3,00 % |

### Beispiel M46

| | | | |
|---|---|---|---|
| Y-4O-O4 | 10,50 % | Klärpunkt [°C]: | 81 |
| CC-3-V | 25,00 % | Δn [589 nm, 20 °C] : | 0,1141 |
| CCP-30CF₃ | 6,00 % | Δε [1 kHz, 20 °C]: | 10,6 |
| CLP-3-T | 3,50 % | ε_{∥} [1 kHz, 20°C]: | 15,7 |
| CCP-V-1 | 14,00 % | ε_{⊥} [1 kHz, 20°C]: | 5,1 |
| CCP-V2-1 | 4,50 % | γ₁ [mPa·s, 20 °C]: | 87 |
| PGP-2-2V | 6,00 % | K₁ [pN, 20 °C]: | 13,3 |
| DGUQU-4-F | 6,00 % | K₃ [pN, 20 °C]: | 12,8 |
| DPGU-4-F | 5,00 % | V₀ [V, 20 °C]: | 1,17 |
| APUQU-2-F | 7,00 % | | |
| APUQU-3-F | 8,00 % | | |
| PGUQU-4-F | 4,00 % | | |
| PPGU-3-F | 0,50 % | | |

### Beispiel M47

| | | | |
|---|---|---|---|
| Y-4O-O4 | 5,00 % | Klärpunkt [°C]: | 81 |
| CC-3-V | 21,50 % | Δn [589 nm, 20 °C] : | 0,1120 |
| CC-3-V1 | 4,00 % | Δε [1 kHz, 20 °C]: | 7,9 |
| B-2O-O5 | 4,00 % | ε_{∥} [1 kHz, 20°C]: | 14,0 |
| PY-3-O2 | 9,50 % | ε_{⊥} [1 kHz, 20°C]: | 6,2 |
| CCP-30CF₃ | 6,00 % | γ₁ [mPa·s, 20 °C]: | 99 |
| CCP-V-1 | 14,00 % | K₁ [pN, 20 °C]: | 14,3 |
| CCP-V2-1 | 1,50 % | K₃ [pN, 20 °C]: | 13,9 |
| CLP-3-T | 4,00 % | V₀ [V, 20°C]: | 1,42 |
| CCY-3-O2 | 4,50 % | | |
| APUQU-2-F | 4,00 % | | |
| APUQU-3-F | 5,00 % | | |
| CDUQU-3-F | 4,00 % | | |
| DGUQU-4-F | 5,00 % | | |
| DPGU-4-F | 3,50 % | | |
| PGUQU-3-F | 4,00 % | | |
| PPGU-3-F | 0,50 % | | |

### Beispiel M48

| | |
|---|---|
| Y-4O-O4 | 7,00 % |
| CC-3-V | 28,00 % |
| B-2O-O5 | 4,00 % |
| B(S)-2O-O4 | 3,50 % |
| B(S)-2O-O5 | 4,00 % |
| CCP-30CF₃ | 6,50 % |
| CCP-V-1 | 15,00 % |
| PGP-2-2V | 4,00 % |
| CLP-3-T | 4,00 % |
| APUQU-2-F | 5,00 % |
| APUQU-3-F | 6,50 % |
| CDUQU-3-F | 4,50 % |
| DGUQU-4-F | 5,00 % |
| DPGU-4-F | 3,50 % |
| PPGU-3-F | 0,50 % |

### Beispiel M49

| | |
|---|---|
| Y-4O-O4 | 10,50 % |
| CC-3-V | 24,00 % |
| CCP-30CF₃ | 6,00 % |
| CLP-3-T | 4,00 % |
| CCP-V-1 | 13,00 % |
| CCP-V2-1 | 5,00 % |
| PGP-2-2V | 7,00 % |
| DGUQU-4-F | 5,00 % |
| DPGU-4-F | 3,50 % |
| APUQU-2-F | 6,50 % |
| APUQU-3-F | 7,50 % |
| CDUQU-3-F | 3,50 % |
| PGUQU-4-F | 4,00 % |
| PPGU-3-F | 0,50 % |

### Beispiel M50

| | | | |
|---|---|---|---|
| CC-3-V | 48,00 % | Klärpunkt [°C]: | 79,5 |
| CC-3-V1 | 10,50 % | Δn [589 nm, 20 °C] : | 0,0930 |
| CCP-V-1 | 11,00 % | Δε [1 kHz, 20 °C]: | 2,7 |
| CCP-V2-1 | 6,00 % | ε_{∥} [1 kHz, 20°C]: | 5,3 |
| CLP-3-T | 5,50 % | ε_{⊥} [1 kHz, 20°C]: | 2,6 |
| PGP-2-2V | 5,00 % | γ₁ [mPa·s, 20 °C]: | 49 |
| PGUQU-3-F | 4,00 % | K₁ [pN, 20 °C]: | 14,7 |
| APUQU-2-F | 4,50 % | K₃ [pN, 20 °C]: | 16,3 |
| PP-1-2V1 | 5,00 % | | |
| PPGU-3-F | 0,50 % | | |

Die flüssigkristalline Mischung M50 wird zusätzlich mit 400 ppm der Formel ST-1 und verschiedenen Konzentrationen der Verbindungen der Formel ST-2 oder ST-3 stabilisiert:

| Mischung | ST-1 | ST-2 | ST-3 |
|---|---|---|---|
| Beispiel M50a | 400 ppm | - | - |
| Beispiel M50b | 400 ppm | 100 ppm | - |
| Beispiel M50c | 400 ppm | 500 ppm | - |
| Beispiel M50d | 400 ppm | 1000 ppm | - |
| Beispiel M50e | 400 ppm | - | 100 ppm |
| Beispiel M50f | 400 ppm | - | 500 ppm |
| Beispiel M50g | 400 ppm | - | 1000 ppm |

### Beispiel M51

| | | | |
|---|---|---|---|
| CC-3-V | 48,00 % | Klärpunkt [°C]: | 80,5 |
| CC-3-V1 | 12,00 % | Δn [589 nm, 20 °C] : | 0,0937 |
| CCP-V-1 | 11,50 % | Δε [1 kHz, 20 °C]: | 2,7 |
| CLP-V-1 | 9,00 % | ε_{∥} [1 kHz, 20°C]: | 5,3 |
| PGP-2-2V | 4,50 % | ε_{⊥} [1 kHz, 20°C]: | 2,6 |
| PGUQU-3-F | 3,00 % | γ₁ [mPa·s, 20 °C]: | 49 |
| APUQU-2-F | 7,50 % | K₁ [pN, 20 °C]: | 14,4 |
| PP-1-2V1 | 4,00 % | K₃ [pN, 20 °C]: | 16,0 |
| PPGU-3-F | 0,50 % | | |

Die flüssigkristalline Mischung M51 wird zusätzlich mit 400 ppm der Formel ST-1 und verschiedenen Konzentrationen der Verbindungen der Formel ST-2 oder ST-3 stabilisiert:

| Mischung | ST-1 | ST-2 | ST-3 |
|---|---|---|---|
| Beispiel M51a | 400 ppm | - | - |
| Beispiel M51 b | 400 ppm | 100 ppm | - |
| Beispiel M51c | 400 ppm | 500 ppm | - |
| Beispiel M51d | 400 ppm | 1000 ppm | - |
| Beispiel M51e | 400 ppm | - | 100 ppm |
| Beispiel M51f | 400 ppm | - | 500 ppm |
| Beispiel M51g | 400 ppm | - | 1000 ppm |

### Beispiel M52

| | | | | |
|---|---|---|---|---|
| CC-3-V | 48,50 | % | Klärpunkt [°C]: | 79,0 |
| CC-3-V1 | 12,00 | % | Δn [589 nm, 20 °C] : | 0,0939 |
| CCP-V-1 | 10,50 | % | Δε [1 kHz, 20 °C]: | 2,9 |
| PGP-2-2V | 3,50 | % | ε_{∥} [1 kHz, 20°C]: | 5,4 |
| PGUQU-3-F | 5,00 | % | ε_{⊥} [1 kHz, 20°C]: | 2,6 |
| APUQU-2-F | 6,00 | % | γ₁ [mPa·s, 20 °C]: | 48 |
| PP-1-2V1 | 5,00 | % | K₁ [pN, 20 °C]: | 14,1 |
| PPGU-3-F | 0,50 | % | K₃ [pN, 20 °C]: | 15,8 |
| CLP-V-1 | 9,00 | % | | |

### Beispiel M53

| | | | | |
|---|---|---|---|---|
| CC-3-V | 48,00 | % | Klärpunkt [°C]: | 80,6 |
| CC-3-V1 | 12,00 | % | Δn [589 nm, 20 °C] : | 0,0930 |
| CCP-V-1 | 11,50 | % | Δε [1 kHz, 20 °C]: | 2,7 |
| PGP-2-2V | 4,50 | % | ε_{∥} [1 kHz, 20°C]: | 6,3 |
| PGUQU-3-F | 3,00 | % | ε_{⊥} [1 kHz, 20°C]: | 2,6 |
| APUQU-2-F | 6,00 | % | γ₁ [mPa·s, 20 °C]: | 49 |
| PP-1-2V1 | 4,00 | % | K₁ [pN, 20 °C]: | 15,0 |
| PPGU-3-F | 0,50 | % | K₃ [pN, 20 °C]: | 17,1 |
| CLP-3-T | 4,00 | % | | |
| CLP-V-1 | 6,50 | | | |

### Beispiel M54

| | | | | |
|---|---|---|---|---|
| CC-3-V | 48,00 | % | Klärpunkt [°C]: | 80,0 |
| CC-3-V1 | 10,50 | % | Δn [589 nm, 20 °C] : | 0,0956 |
| CCP-V-1 | 11,00 | % | Δε [1 kHz, 20 °C]: | 2,7 |
| PGP-2-2V | 5,00 | % | ε_{∥} [1 kHz, 20°C]: | 5,3 |
| PGUQU-3-F | 4,00 | % | ε_{⊥} [1 kHz, 20°C]: | 2,6 |
| APUQU-2-F | 4,50 | % | γ₁ [mPa·s, 20 °C]: | 49 |
| PP-1-2V1 | 5,00 | % | K₁ [pN, 20 °C]: | 15,0 |
| PPGU-3-F | 0,50 | % | K₃ [pN, 20 °C]: | 16,3 |
| CLP-3-T | 5,50 | % | | |
| CLP-V-1 | 6,00 | | | |

### Beispiel M55

| | | | |
|---|---|---|---|
| PGUQU-3-F | 3,00 % | Klärpunkt [°C]: | 70,2 |
| PPGU-3-F | 0,50 % | Δn [589 nm, 20 °C] : | 0,1345 |
| PGP-1-2V | 7,00 % | Δε [1 kHz, 20 °C]: | 5,3 |
| PGP-2-2V | 11,00 % | ε_{∥} [1 kHz, 20°C]: | 8,3 |
| PGU-3-F | 6,50 % | ε_{⊥} [1 kHz, 20°C]: | 3,0 |
| PUQU-3-F | 10,00 % | γ₁ [mPa·s, 20 °C]: | 55 |
| CC-3-V | 34,00 % | K₁ [pN, 20 °C]: | 15,7 |
| PP-1-2V1 | 10,00 % | K₃ [pN, 20 °C]: | 13,3 |
| CLP-3-T | 6,00 % | V₀ [V, 20°C]: | 1,81 |
| CCP-V-1 | 2,00 % | | |
| CC-3-V1 | 6,00 % | | |
| CC-3-2V1 | 4,00 % | | |

Die flüssigkristalline Mischung M55 wird zusätzlich mit 0,05 % der Verbindung der Formel ST-1 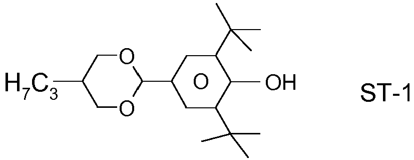 versetzt.

### Beispiel M56

| | | | |
|---|---|---|---|
| PGUQU-3-F | 1,50 % | Klärpunkt [°C]: | 63,1 |
| PPGU-3-F | 0,50 % | Δn [589 nm, 20 °C] : | 0,1337 |
| PGP-1-2V | 6,50 % | Δε [1 kHz, 20 °C]: | 5,1 |
| PGP-2-2V | 10,50 % | ε_{∥} [1 kHz, 20°C]: | 8,1 |
| PGU-3-F | 7,50 % | ε_{⊥} [1 kHz, 20°C]: | 3,0 |
| PUQU-3-F | 10,00 % | γ₁ [mPa·s, 20 °C]: | 49 |
| CC-3-V | 42,00 % | K₁ [pN, 20 °C]: | 14,6 |
| PP-1-2V1 | 14,00 % | K₃ [pN, 20 °C]: | 11,8 |
| CLP-3-T | 7,50 % | V₀ [V, 20°C]: | 1,78 |

Die flüssigkristalline Mischung M56 wird zusätzlich mit 0,05 % der Verbindung der Formel ST-1 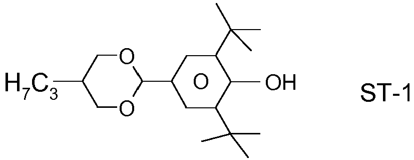 versetzt.

### Beispiel M57

| | | | |
|---|---|---|---|
| APUQU-2-F | 2,00 % | Klärpunkt [°C]: | 104 |
| DGUQU-4-F | 4,00 % | Δn [589 nm, 20 °C] : | 0,0949 |
| DPGU-4-F | 2,00 % | Δε [1 kHz, 20 °C]: | 4,5 |
| CCG-V-F | 17,00 % | ε_{∥} [1 kHz, 20°C]: | 7,4 |
| CCP-30CF3 | 4,00 % | ε_{⊥} [1 kHz, 20°C]: | 2,9 |
| CCP-50CF3 | 3,00 % | γ₁ [mPa·s, 20 °C]: | 84 |
| CCP-V-1 | 10,00 % | K₁ [pN, 20 °C]: | 16,0 |
| CCP-V2-1 | 4,00 % | K₃ [pN, 20 °C]: | 20,2 |
| CCQU-3-F | 5,50 % | V₀ [V, 20°C]: | 1,98 |
| CCVC-3-V | 5,00 % | | |
| CLP-3-T | 3,00 % | | |
| PGP-2-2V | 3,00 % | | |
| CC-3-2V1 | 4,00 % | | |
| CC-3-V | 23,50 % | | |
| CC-3-V1 | 5,50 % | | |
| PP-1-2V1 | 4,50 % | | |

### Beispiel M58

| | | | |
|---|---|---|---|
| DGUQU-4-F | 1,50 % | Klärpunkt [°C]: | 79,5 |
| CCG-V-F | 6,00 % | Δn [589 nm, 20 °C] : | 0,1051 |
| CCQU-2-F | 3,50 % | Δε [1 kHz, 20 °C]: | 4,5 |
| CCVC-3-V | 6,50 % | ε_{∥} [1 kHz, 20°C]: | 7,9 |
| CDU-2-F | 13,00 % | ε_{⊥} [1 kHz, 20°C]: | 3,4 |
| CLP-3-T | 4,00 % | γ₁ [mPa·s, 20 °C]: | 61 |
| PGP-1-2V | 12,00 % | K₁ [pN, 20 °C]: | 13,4 |
| PGP-2-2V | 4,00 % | K₃ [pN, 20 °C]: | 13,3 |
| PGU-2-F | 4,00 % | V₀ [V, 20°C]: | 1,80 |
| CC-3-V | 31,50 % | | |
| CCH-34 | 4,00 % | | |
| PCH-302 | 10,00 % | | |

### Beispiel M59

| | | | |
|---|---|---|---|
| CC-3-2V1 | 3,50 % | Klärpunkt [°C]: | 74,8 |
| CC-3-V | 49,00 % | Δn [589 nm, 20 °C] : | 0,1189 |
| CC-3-V1 | 4,00 % | Δε [1 kHz, 20 °C]: | 3,1 |
| CLP-3-T | 6,50 % | ε_{∥} [1 kHz, 20°C]: | 5,9 |
| PGP-1-2V | 6,00 % | ε_{⊥} [1 kHz, 20°C]: | 2,8 |
| PGP-2-2V | 12,00 % | γ₁ [mPa·s, 20 °C]: | 45 |
| PGP-3-2V | 4,00 % | K₁ [pN, 20 °C]: | 13,6 |
| PGU-2-F | 5,00 % | K₃ [pN, 20 °C]: | 13,1 |
| PGUQU-3-F | 3,00 % | V₀ [V, 20°C]: | 2,20 |
| PP-1-2V1 | 3,00 % | | |
| PPGU-3-F | 1,00 % | | |
| PUQU-3-F | 3,00 % | | |

Die flüssigkristalline Mischung M59 wird zusätzlich mit 0,04 % der Verbindung der Formel ST-1 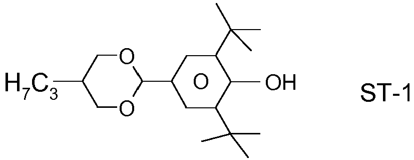 versetzt.

### Beispiel M60

| | | | |
|---|---|---|---|
| APUQU-2-F | 6,00 % | Klärpunkt [°C]: | 90,6 |
| APUQU-3-F | 7,00 % | Δn [589 nm, 20 °C] : | 0,1155 |
| CC-3-V | 27,50 % | Δε [1 kHz, 20 °C]: | 10,4 |
| CC-3-V1 | 8,00 % | ε_{∥} [1 kHz, 20°C]: | 13,9 |
| CCP-V-1 | 9,00 % | ε_{⊥} [1 kHz, 20°C]: | 3,5 |
| CCP-V2-1 | 6,50 % | γ₁ [mPa·s, 20 °C]: | 73 |
| CDUQU-3-F | 8,50 % | K₁ [pN, 20 °C]: | 13,9 |
| CLP-3-T | 3,00 % | K₃ [pN, 20 °C]: | 15,5 |
| PGP-1-2V | 5,00 % | V₀ [V, 20°C]: | 1,22 |
| PGP-2-2V | 5,00 % | | |
| PGUQU-3-F | 1,00 % | | |
| PPGU-3-F | 1,00 % | | |
| PUQU-3-F | 12,50 % | | |

Die flüssigkristalline Mischung M60 wird zusätzlich mit 0,04 % der Verbindung der Formel ST-1 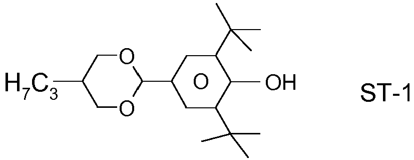 versetzt.

### Beispiel M61

| | | | |
|---|---|---|---|
| APUQU-2-F | 6,00 % | Klärpunkt [°C]: | 90,4 |
| APUQU-3-F | 7,00 % | Δn [589 nm, 20 °C] : | 0,1179 |
| CC-3-V | 28,00 % | Δε [1 kHz, 20 °C]: | 10,9 |
| CC-3-V1 | 8,00 % | ε_{∥} [1 kHz, 20°C]: | |
| CCP-V-1 | 9,00 % | ε_{⊥} [1 kHz, 20°C]: | |
| CCP-V2-1 | 5,50 % | γ₁ [mPa·s, 20 °C]: | 91 |
| CDUQU-3-F | 8,00 % | K₁ [pN, 20 °C]: | 14,7 |
| CLP-3-T | 4,00 % | K₃ [pN, 20 °C]: | 16,4 |
| PGP-1-2V | 5,00 % | V₀ [V, 20°C]: | 1,23 |
| PGP-2-2V | 5,00 % | | |
| PQUQU-3-F | 1,50 % | | |
| PPGU-3-F | 1,00 % | | |
| PUQU-3-F | 12,00 % | | |

Die flüssigkristalline Mischung M61 wird zusätzlich mit 0,04 % der Verbindung der Formel ST-1 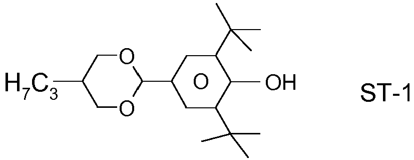 versetzt.

### Beispiel M62

| | | | |
|---|---|---|---|
| CC-3-V | 47,50 % | Klärpunkt [°C]: | 74,8 |
| CC-3-V1 | 7,50 % | Δn [589 nm, 20 °C] : | 0,1192 |
| CLP-3-T | 7,00 % | Δε [1 kHz, 20 °C]: | 4,7 |
| CPGU-3-OT | 2,00 % | ε_{∥} [1 kHz, 20°C]: | 7,6 |
| PGP-2-2V | 16,00 % | ε_{⊥} [1 kHz, 20°C]: | 2,9 |
| PGU-2-F | 11,00 % | γ₁ [mPa·s, 20 °C]: | 45 |
| PGUQU-3-F | 6,00 % | K₁ [pN, 20 °C]: | 13,3 |
| PP-1-2V1 | 2,00 % | K₃ [pN, 20 °C]: | 12,6 |
| PPGU-3-F | 1,00 % | V₀ [V, 20°C]: | 1,78 |

Die flüssigkristalline Mischung M62 wird zusätzlich mit 0,04 % der Verbindung der Formel ST-1 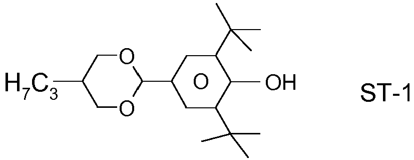 versetzt.

### Beispiel M63

| | | | |
|---|---|---|---|
| CC-3-2V1 | 7,00 % | Klärpunkt [°C]: | 70,0 |
| CC-3-V | 46,00 % | Δn [589 nm, 20 °C] : | 0,1185 |
| CC-3-V1 | 3,00 % | Δε [1 kHz, 20 °C]: | 3,0 |
| CLP-3-T | 5,00 % | ε_{∥} [1 kHz, 20°C]: | 5,7 |
| PGP-1-2V | 7,00 % | ε_{⊥} [1 kHz, 20°C]: | 2,8 |
| PGP-2-2V | 11,00 % | γ₁ [mPa·s, 20 °C]: | 45 |
| PGP-3-2V | 2,50 % | K₁ [pN, 20 °C]: | 13,2 |
| PGU-2-F | 5,50 % | K₃ [pN, 20 °C]: | 12,8 |
| PP-1-2V1 | 6,00 % | V₀ [V, 20°C]: | 2,21 |
| PPGU-3-F | 1,00 % | | |
| PUQU-3-F | 6,00 % | | |

Die flüssigkristalline Mischung M63 wird zusätzlich mit 0,04 % der Verbindung der Formel ST-1 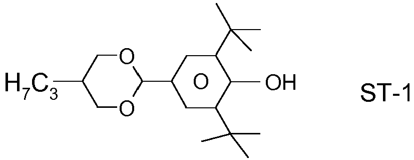 versetzt.

### Beispiel M64

| | | | |
|---|---|---|---|
| APUQU-2-F | 6,00 % | Klärpunkt [°C]: | 80,0 |
| APUQU-3-F | 5,00 % | Δn [589 nm, 20 °C] : | 0,1129 |
| PGUQU-3-F | 2,00 % | Δε [1 kHz, 20 °C]: | 3,9 |
| PPGU-3-F | 0,50 % | ε_{∥} [1 kHz, 20°C]: | 6,7 |
| CCP-V-1 | 2,50 % | ε_{⊥} [1 kHz, 20°C]: | 2,8 |
| PGP-1-2V | 4,00 % | γ₁ [mPa·s, 20 °C]: | 52 |
| PGP-2-2V | 16,50 % | K₁ [pN, 20 °C]: | 14,1 |
| CC-3-V | 52,00 % | K₃ [pN, 20 °C]: | 14,1 |
| CC-3-V1 | 6,50 % | V₀ [V, 20°C]: | 2,03 |
| CLP-3-T | 5,00 % | | |

### Beispiel M65

| | | | |
|---|---|---|---|
| APUQU-2-F | 5,00 % | Klärpunkt [°C]: | 80,0 |
| PGUQU-3-F | 6,00 % | Δn [589 nm, 20 °C] : | 0,1131 |
| PPGU-3-F | 0,50 % | Δε [1 kHz, 20 °C]: | 3,5 |
| CCP-V-1 | 8,50 % | ε_{∥} [1 kHz, 20°C]: | 6,2 |
| PGP-2-2V | 16,50 % | ε_{⊥} [1 kHz, 20°C]: | 2,7 |
| PP-1-2V1 | 4,00 % | γ₁ [mPa·s, 20 °C]: | 51 |
| CC-3-V | 48,00 % | K₁ [pN, 20 °C]: | 14,6 |
| CC-3-V1 | 6,50 % | K₃ [pN, 20 °C]: | 14,7 |
| CLP-3-T | 5,00 % | V₀ [V, 20°C]: | 2,17 |

### Beispiel M66

| | | | |
|---|---|---|---|
| APUQU-2-F | 4,00 % | Klärpunkt [°C]: | 76,0 |
| PGUQU-3-F | 3,00 % | Δn [589 nm, 20 °C] : | 0,1176 |
| PPGU-3-F | 0,50 % | Δε [1 kHz, 20 °C]: | 2,4 |
| CCP-V-1 | 4,00 % | ε_{∥} [1 kHz, 20°C]: | 5,1 |
| PGP-1-2V | 6,00 % | ε_{⊥} [1 kHz, 20°C]: | 2,7 |
| PGP-2-2V | 15,50 % | γ₁ [mPa·s, 20 °C]: | 48 |
| PP-1-2V1 | 6,00 % | K₁ [pN, 20 °C]: | 14,3 |
| CC-3-V | 52,00 % | K₃ [pN, 20 °C]: | 14,6 |
| CC-3-V1 | 4,50 % | | |
| CLP-3-T | 4,50 % | | |

### Beispiel M67

| | | | |
|---|---|---|---|
| APUQU-2-F | 7,00 % | Klärpunkt [°C]: | 76,0 |
| PGUQU-3-F | 6,00 % | Δn [589 nm, 20 °C] : | 0,1173 |
| PPGU-3-F | 0,50 % | Δε [1 kHz, 20 °C]: | 4,0 |
| CCP-V-1 | 1,00 % | ε_{∥} [1 kHz, 20°C]: | 6,8 |
| PGP-1-2V | 4,00 % | ε_{⊥} [1 kHz, 20°C]: | 2,8 |
| PGP-2-2V | 15,00 % | γ₁ [mPa·s, 20 °C]: | 51 |
| PP-1-2V1 | 4,50 % | K₁ [pN, 20 °C]: | 14,5 |
| CC-3-V | 50,00 % | K₃ [pN, 20 °C]: | 14,6 |
| CC-3-V1 | 5,00 % | | |
| CC-3-2V1 | 2,00 % | | |
| CLP-3-T | 5,00 % | | |

### Beispiel M68

| | | | |
|---|---|---|---|
| APUQU-2-F | 3,00 % | Klärpunkt [°C]: | 76,5 |
| PGUQU-3-F | 2,00 % | Δn [589 nm, 20 °C] : | 0,1177 |
| PPGU-3-F | 0,50 % | Δε [1 kHz, 20 °C]: | 2,5 |
| CLP-V-1 | 7,00 % | ε_{∥} [1 kHz, 20°C]: | 5,1 |
| PGP-1-2V | 2,50 % | ε_{⊥} [1 kHz, 20°C]: | 2,6 |
| PGP-2-2V | 14,00 % | γ₁ [mPa·s, 20 °C]: | 51 |
| PP-1-2V1 | 7,00 % | K₁ [pN, 20 °C]: | 15,4 |
| CC-3-V | 49,00 % | K₃ [pN, 20 °C]: | 14,4 |
| CC-3-V1 | 6,00 % | | |
| CLP-3-T | 4,50 % | | |
| PUQU-3-F | 2,00 % | | |
| PGP-3-2V | 2,50 % | | |

### Beispiel M69

| | | | |
|---|---|---|---|
| CC-3-2V1 | 1,00 % | Klärpunkt [°C]: | 77,5 |
| CC-3-V | 50,00 % | Δn [589 nm, 20 °C] : | 0,1169 |
| CC-3-V1 | 2,00 % | Δε [1 kHz, 20 °C]: | 4,2 |
| CCP-V-1 | 2,00 % | ε_{∥} [1 kHz, 20°C]: | 7,0 |
| CLP-3-T | 6,00 % | ε_{⊥} [1 kHz, 20°C]: | 2,8 |
| CLP-V-1 | 6,50 % | γ₁ [mPa·s, 20 °C]: | 51 |
| PGP-2-2V | 12,50 % | K₁ [pN, 20 °C]: | 15,0 |
| PP-1-2V1 | 6,00 % | K₃ [pN, 20 °C]: | 13,9 |
| PGU-4-T | 3,50 % | | |
| PGUQU-3-F | 5,00 % | | |
| PGUQU-4-F | 5,00 % | | |
| PPGU-3-F | 0,50 % | | |

### Beispiel M70

| | | | |
|---|---|---|---|
| APUQU-2-F | 3,00 % | Klärpunkt [°C]: | 75,0 |
| CC-3-V | 49,50 % | Δn [589 nm, 20 °C] : | 0,1186 |
| CC-3-V1 | 6,00 % | Δε [1 kHz, 20 °C]: | 2,3 |
| CLP-1V2-1 | 5,50 % | ε_{∥} [1 kHz, 20°C]: | 5,0 |
| CLP-3-T | 4,00 % | ε_{⊥} [1 kHz, 20°C]: | 2,6 |
| PGP-1-2V | 3,00 % | γ₁ [mPa·s, 20 °C]: | 50 |
| PGP-2-2V | 13,00 % | K₁ [pN, 20 °C]: | 15,2 |
| PGP-3-2V | 2,50 % | K₃ [pN, 20 °C]: | 15,1 |
| PGUQU-3-F | 2,00 % | | |
| PP-1-2V1 | 9,00 % | | |
| PPGU-3-F | 0,50 % | | |
| PUQU-3-F | 2,00 % | | |

### Beispiel M71

| | | | |
|---|---|---|---|
| APUQU-2-F | 3,00 % | Klärpunkt [°C]: | 76,5 |
| CC-3-V | 49,50 % | Δn [589 nm, 20 °C] : | 0,1178 |
| CC-3-V1 | 6,00 % | Δε [1 kHz, 20 °C]: | 2,4 |
| CLP-1V-1 | 6,00 % | ε_{∥} [1 kHz, 20°C]: | 5,0 |
| CLP-3-T | 4,50 % | ε_{⊥} [1 kHz, 20°C]: | 2,7 |
| PGP-1-2V | 3,00 % | γ₁ [mPa·s, 20 °C]: | 50 |
| PGP-2-2V | 13,00 % | K₁ [pN, 20 °C]: | 15,2 |
| PGP-3-2V | 2,50 % | K₃ [pN, 20 °C]: | 15,1 |
| PGUQU-3-F | 2,00 % | | |
| PP-1-2V1 | 8,00 % | | |
| PPGU-3-F | 0,50 % | | |
| PUQU-3-F | 2,00 % | | |

### Beispiel M72

| | |
|---|---|
| APUQU-2-F | 3,00 % |
| CC-3-V | 49,50 % |
| CC-3-V1 | 6,00 % |
| CLP-V2-1 | 6,00 % |
| CLP-3-T | 4,50 % |
| PGP-1-2V | 3,00 % |
| PGP-2-2V | 13,00 % |
| PGP-3-2V | 2,50 % |
| PGUQU-3-F | 2,00 % |
| PP-1-2V1 | 8,00 % |
| PPGU-3-F | 0,50 % |
| PUQU-3-F | 2,00 % |

### Beispiel M73

| | | | |
|---|---|---|---|
| CC-3-V | 46,00 % | Klärpunkt [°C]: | 76,0 |
| CC-3-V1 | 6,00 % | Δn [589 nm, 20 °C] : | 0,1180 |
| CCP-V-1 | 1,50 % | Δε [1 kHz, 20 °C]: | 2,9 |
| PGP-2-2V | 12,00 % | ε_{∥} [1 kHz, 20°C]: | 6,8 |
| PGP-3-2V | 2,50 % | ε_{⊥} [1 kHz, 20°C]: | 3,9 |
| PGU-2-F | 6,50 % | γ₁ [mPa·s, 20 °C]: | 54 |
| PGUQU-3-F | 5,00 % | K₁ [pN, 20 °C]: | 15,0 |
| CLP-3-T | 5,00 % | K₃ [pN, 20 °C]: | 13,4 |
| B(S)-2O-O4 | 3,50 % | | |
| B(S)-2O-O5 | 3,50 % | | |
| CC-3-2V1 | 1,00 % | | |
| DGUQU-4-F | 1,50 % | | |
| CY-3-O2 | 2,50 % | | |
| CLP-V-1 | 3,50 % | | |

### Beispiel M74

| | | | |
|---|---|---|---|
| CC-3-V | 44,00 % | Klärpunkt [°C]: | 85,00 |
| CC-3-V1 | 6,00 % | Δn [589 nm, 20 °C] : | 0,1178 |
| CC-3-2V1 | 4,00 % | Δε [1 kHz, 20 °C]: | 3,2 |
| CCP-V-1 | 1,50 % | ε_{∥} [1 kHz, 20°C]: | 6,9 |
| PGP-2-2V | 10,50 % | ε_{⊥} [1 kHz, 20°C]: | 3,7 |
| PGP-3-2V | 5,00 % | γ₁ [mPa·s, 20 °C]: | 62 |
| DGUQU-4-F | 4,50 % | K₁ [pN, 20 °C]: | 16,7 |
| PGUQU-3-F | 6,50 % | K₃ [pN, 20 °C]: | 15,5 |
| CLP-3-T | 5,00 % | | |
| B(S)-2O-O4 | 4,00 % | | |
| B(S)-2O-O5 | 3,50 % | | |
| CLP-V-1 | 5,50 % | | |

### Beispiel M75

| | | | |
|---|---|---|---|
| CC-3-V | 44,00 % | Klärpunkt [°C]: | 80,0 |
| CC-3-V1 | 6,00 % | Δn [589 nm, 20 °C] : | 0,1186 |
| CC-3-2V1 | 5,00 % | Δε [1 kHz, 20 °C]: | 3,0 |
| PGP-2-2V | 10,50 % | ε_{∥} [1 kHz, 20°C]: | 6,9 |
| PGP-3-2V | 4,00 % | ε_{⊥} [1 kHz, 20°C]: | 3,8 |
| DGUQU-4-F | 2,50 % | γ₁ [mPa·s, 20 °C]: | 60 |
| PGUQU-3-F | 6,50 % | K₁ [pN, 20 °C]: | 16,2 |
| CLP-3-T | 5,00 % | K₃ [pN, 20 °C]: | 14,9 |
| B(S)-2O-O4 | 4,00 % | | |
| B(S)-2O-O5 | 3,50 % | | |
| CLP-V-1 | 4,50 % | | |
| PGU-2-F | 3,00 % | | |
| PY-3-O2 | 1,50 % | | |

### Beispiel M76

| | |
|---|---|
| Y-4O-O4 | 7,00 % |
| CC-3-V | 28,00 % |
| B-2O-O5 | 4,00 % |
| B(S)-2O-O4 | 3,50 % |
| B(S)-2O-O5 | 4,00 % |
| CCP-30CF₃ | 6,50 % |
| CCP-V-1 | 15,00 % |
| PGP-2-2V | 4,00 % |
| CLP-1V2-T | 4,00 % |
| APUQU-2-F | 5,00 % |
| APUQU-3-F | 6,50 % |
| CDUQU-3-F | 4,50 % |
| DGUQU-4-F | 5,00 % |
| DPGU-4-F | 3,50 % |
| PPGU-3-F | 0,50 % |

### Beispiel M77

| | |
|---|---|
| Y-4O-O4 | 10,50 % |
| CC-3-V | 24,00 % |
| CCP-30CF₃ | 6,00 % |
| CLP-V2-T | 4,00 % |
| CCP-V-1 | 13,00 % |
| CCP-V2-1 | 5,00 % |
| PGP-2-2V | 7,00 % |
| DGUQU-4-F | 5,00 % |
| DPGU-4-F | 3,50 % |
| APUQU-2-F | 6,50 % |
| APUQU-3-F | 7,50 % |
| CDUQU-3-F | 3,50 % |
| PGUQU-4-F | 4,00 % |
| PPGU-3-F | 0,50 % |

### Beispiel M78

| | |
|---|---|
| CC-3-V | 49,50 % |
| CCP-V-1 | 5,00 % |
| CLP-1V2-1 | 8,50 % |
| PP-1-2V1 | 1,00 % |
| PGP-1-2V | 5,00 % |
| PGP-2-2V | 8,00 % |
| PGP-3-2V | 4,50 % |
| CCP-30CF₃ | 6,50 % |
| PGUQU-C4-F | 5,00 % |
| PGUQU-C5-F | 2,50 % |
| APUQU-C3-F | 4,50 % |

### Beispiel M79

| | |
|---|---|
| Y-4O-O4 | 10,50 % |
| CC-3-V | 24,00 % |
| CCP-30CF₃ | 6,00 % |
| CLP-V2-1 | 4,00 % |
| CCP-V-1 | 13,00 % |
| CCP-V2-1 | 5,00 % |
| PGP-2-2V | 7,00 % |
| DGUQU-4-F | 5,00 % |
| DPGU-4-F | 3,50 % |
| APUQU-2-F | 6,50 % |
| APUQU-3-F | 7,50 % |
| CDUQU-3-F | 3,50 % |
| PGUQU-4-F | 4,00 % |
| PPGU-3-F | 0,50 % |

### Beispiel M80

| | | | |
|---|---|---|---|
| CC-3-V | 48,00 % | Klärpunkt [°C]: | 79,0 |
| CC-3-V1 | 10,50 % | Δn [589 nm, 20 °C] : | 0,0932 |
| CCP-V-1 | 11,00 % | Δε [1 kHz, 20 °C]: | 2,6 |
| CCP-V2-1 | 6,00 % | ε_{∥} [1 kHz, 20°C]: | 5,2 |
| PGP-2-2V | 5,00 % | ε_{⊥} [1 kHz, 20°C]: | 2,6 |
| PGUQU-3-F | 4,00 % | γ₁ [mPa·s, 20 °C]: | 49 |
| APUQU-2-F | 4,50 % | K₁ [pN, 20 °C]: | 14,3 |
| PP-1-2V1 | 5,00 % | K₃ [pN, 20 °C]: | 15,9 |
| PPGU-3-F | 0,50 % | | |
| CLP-V-T | 5,50 % | | |

### Beispiel M81

| | | | |
|---|---|---|---|
| CC-3-V | 48,00 % | Klärpunkt [°C]: | 79,0 |
| CC-3-V1 | 10,50 % | Δn [589 nm, 20 °C] : | 0,0930 |
| CCP-V-1 | 11,00 % | Δε [1 kHz, 20 °C]: | 2,5 |
| CCP-V2-1 | 6,00 % | ε_{∥} [1 kHz, 20°C]: | 5,0 |
| PGP-2-2V | 5,00 % | ε_{⊥} [1 kHz, 20°C]: | 2,5 |
| PGUQU-3-F | 4,00 % | γ₁ [mPa·s, 20 °C]: | 48 |
| APUQU-2-F | 4,50 % | K₁ [pN, 20 °C]: | 13,9 |
| PP-1-2V1 | 5,00 % | K₃ [pN, 20 °C]: | 15,9 |
| PPGU-3-F | 0,50 % | | |
| CLP-V-OT | 5,50 % | | |

### Beispiel M82

| | | | |
|---|---|---|---|
| APUQU-2-F | 6,00 % | Klärpunkt [°C]: | 80,0 |
| DGUQU-4-F | 5,50 % | Δn [589 nm, 20 °C] : | 0,0983 |
| DPGU-4-F | 4,00 % | Δε [1 kHz, 20 °C]: | 5,9 |
| PGUQU-3-F | 3,50 % | ε_{∥} [1 kHz, 20°C]: | 8,9 |
| PPGU-3-F | 0,50 % | ε_{⊥} [1 kHz, 20°C]: | 3,0 |
| CC-3-2V1 | 9,00 % | γ₁ [mPa·s, 20 °C]: | 58 |
| CC-3-V | 48,50 % | K₁ [pN, 20 °C]: | 14,7 |
| CC-3-V1 | 7,00 % | K₃ [pN, 20 °C]: | 15,2 |
| CLP-3-T | 4,00 % | | |
| CLP-1V2-OT | 3,00 % | | |
| PGP-2-2V | 9,00 % | | |

### Beispiel M83

| | | | |
|---|---|---|---|
| APUQU-2-F | 6,00 % | Klärpunkt [°C]: | 79,0 |
| DGUQU-4-F | 5,50 % | Δn [589 nm, 20 °C] : | 0,0987 |
| DPGU-4-F | 4,00 % | Δε [1 kHz, 20 °C]: | 5,9 |
| PGUQU-3-F | 4,00 % | ε_{∥} [1 kHz, 20°C]: | 8,8 |
| PPGU-3-F | 0,50 % | ε_{⊥} [1 kHz, 20°C]: | 2,9 |
| CC-3-2V1 | 6,00 % | γ₁ [mPa·s, 20 °C]: | 58 |
| CC-3-V | 49,00 % | K₁ [pN, 20 °C]: | 14,1 |
| CC-3-V1 | 9,00 % | K₃ [pN, 20 °C]: | 15,3 |
| CLP-1V2-OT | 7,00 % | LTS bulk [-20 °C]: | > 1000 h |
| PGP-2-2V | 8,00 % | | |
| PP-1-2V1 | 1,00 % | | |

### Beispiel M84

| | | | |
|---|---|---|---|
| APUQU-3-F | 1,00 % | Klärpunkt [°C]: | 75,5 |
| PPGU-3-F | 0,50 % | Δn [589 nm, 20 °C] : | 0,0987 |
| CCP-V-1 | 12,00 % | Δε [1 kHz, 20 °C]: | 2,3 |
| PGP-2-3 | 6,00 % | ε_{∥} [1 kHz, 20°C]: | 4,9 |
| PGP-2-4 | 7,00 % | ε_{⊥} [1 kHz, 20°C]: | 2,6 |
| PGP-2-5 | 2,00 % | γ₁ [mPa·s, 20 °C]: | 52 |
| PUQU-3-F | 7,00 % | K₁ [pN, 20 °C]: | 14,2 |
| CC-3-2V1 | 3,00 % | K₃ [pN, 20 °C]: | 14,8 |
| CC-3-V | 46,50 % | | |
| CC-3-V1 | 10,00 % | | |
| CLP-1V2-T | 5,00 % | | |

### Beispiel M85

| | | | |
|---|---|---|---|
| CC-3-V | 48,00 % | Klärpunkt [°C]: | 80,5 |
| CC-3-V1 | 10,50 % | Δn [589 nm, 20 °C] : | 0,0938 |
| CCP-V-1 | 11,00 % | Δε [1 kHz, 20 °C]: | 2,7 |
| CCP-V2-1 | 6,00 % | ε_{∥} [1 kHz, 20°C]: | 5,2 |
| CLP-1V2-T | 5,50 % | ε_{⊥} [1 kHz, 20°C]: | 2,6 |
| PGP-2-2V | 5,00 % | γ₁ [mPa·s, 20 °C]: | 54 |
| PGUQU-3-F | 4,00 % | K₁ [pN, 20 °C]: | 15,0 |
| APUQU-2-F | 4,50 % | K₃ [pN, 20 °C]: | 17,0 |
| PP-1-2V1 | 5,00 % | LTS bulk [-20 °C]: | > 1000 h |
| PPGU-3-F | 0,50 % | | |

### Beispiel M86

| | |
|---|---|
| CC-3-V | 48,00 % |
| CC-3-V1 | 10,50 % |
| CCP-V-1 | 11,00 % |
| CLP-V-1 | 6,00 % |
| CLP-1V2-T | 5,50 % |
| PGP-2-2V | 5,00 % |
| PGUQU-3-F | 4,00 % |
| APUQU-2-F | 4,50 % |
| PP-1-2V1 | 5,00 % |
| PPGU-3-F | 0,50 % |

### Beispiel M87

| | | | |
|---|---|---|---|
| CC-3-V | 48,00 % | Klärpunkt [°C]: | 80,5 |
| CC-3-V1 | 10,50 % | Δn [589 nm, 20 °C] : | 0,0941 |
| CCP-V-1 | 11,00 % | Δε [1 kHz, 20 °C]: | 2,7 |
| CCP-V2-1 | 6,00 % | ε_{∥} [1 kHz, 20°C]: | 5,3 |
| CLP-1V-T | 5,50 % | ε_{⊥} [1 kHz, 20°C]: | 2,6 |
| PGP-2-2V | 5,00 % | γ₁ [mPa·s, 20 °C]: | 50 |
| PGUQU-3-F | 4,00 % | K₁ [pN, 20 °C]: | 14,7 |
| APUQU-2-F | 4,50 % | K₃ [pN, 20 °C]: | 16,8 |
| PP-1-2V1 | 5,00 % | | |
| PPGU-3-F | 0,50 % | | |

### Beispiel M88

| | | | |
|---|---|---|---|
| APUQU-2-F | 4,00 % | Klärpunkt [°C]: | 76,0 |
| PGUQU-3-F | 3,00 % | Δn [589 nm, 20 °C] : | 0,1180 |
| PPGU-3-F | 0,50 % | Δε [1 kHz, 20 °C]: | 2,4 |
| CCP-V-1 | 4,00 % | ε_{∥} [1 kHz, 20°C]: | 5,1 |
| PGP-1-2V | 6,00 % | ε_{⊥} [1 kHz, 20°C]: | 2,7 |
| PGP-2-2V | 15,50 % | γ₁ [mPa·s, 20 °C]: | 48 |
| PP-1-2V1 | 6,00 % | K₁ [pN, 20 °C]: | 14,4 |
| CC-3-V | 52,00 % | K₃ [pN, 20 °C]: | 14,4 |
| CC-3-V1 | 4,50 % | | |
| CLP-1V-T | 4,50 % | | |

### Beispiel M89

| | | | |
|---|---|---|---|
| APUQU-2-F | 7,00 % | Klärpunkt [°C]: | 76,5 |
| PGUQU-3-F | 6,00 % | Δn [589 nm, 20 °C] : | 0,1184 |
| PPGU-3-F | 0,50 % | Δε [1 kHz, 20 °C]: | 4,0 |
| CCP-V-1 | 1,00 % | ε_{∥} [1 kHz, 20°C]: | 6,8 |
| PGP-1-2V | 4,00 % | ε_{⊥} [1 kHz, 20°C]: | 2,8 |
| PGP-2-2V | 15,00 % | γ₁ [mPa·s, 20 °C]: | 52 |
| PP-1-2V1 | 4,50 % | K₁ [pN, 20 °C]: | 14,5 |
| CC-3-V | 50,00 % | K₃ [pN, 20 °C]: | 14,3 |
| CC-3-V1 | 5,00 % | | |
| CC-3-2V1 | 2,00 % | | |
| CLP-1V-T | 5,00 % | | |

### Beispiel M90

| | | | |
|---|---|---|---|
| CC-3-V | 49,00 % | Klärpunkt [°C]: | 79,5 |
| CC-3-V1 | 12,00 % | Δn [589 nm, 20 °C] : | 0,0938 |
| CCP-V-1 | 11,00 % | Δε [1 kHz, 20 °C]: | 2,7 |
| CLP-1V-1 | 8,00 % | ε_{∥} [1 kHz, 20°C]: | 5,3 |
| PGP-2-2V | 3,00 % | ε_{⊥} [1 kHz, 20°C]: | 2,6 |
| PGUQU-3-F | 3,50 % | γ₁ [mPa·s, 20 °C]: | 49 |
| APUQU-2-F | 7,00 % | K₁ [pN, 20 °C]: | 14,6 |
| PP-1-2V1 | 6,00 % | K₃ [pN, 20 °C]: | 16,9 |
| PPGU-3-F | 0,50 % | | |

### Beispiel M91

| | | | |
|---|---|---|---|
| CC-3-V | 49,00 % | Klärpunkt [°C]: | 81,0 |
| CC-3-V1 | 10,00 % | Δε [1 kHz, 20 °C]: | 2,7 |
| CCP-V-1 | 8,00 % | ε_{∥} [1 kHz, 20°C]: | 5,3 |
| CLP-V-1 | 7,50 % | ε_{⊥} [1 kHz, 20°C]: | 2,5 |
| CLP-1V-1 | 3,50 % | γ₁ [mPa·s, 20 °C]: | 50 |
| CLP-3-T | 5,50 % | K₁ [pN, 20 °C]: | 15,6 |
| PGP-2-2V | 2,00 % | K₃ [pN, 20 °C]: | 17,1 |
| PGUQU-3-F | 4,00 % | | |
| APUQU-2-F | 4,50 % | | |
| PP-1-2V1 | 5,50 % | | |
| PPGU-3-F | 0,50 % | | |

### Beispiel M92

| | | | |
|---|---|---|---|
| CC-3-V | 49,00 % | Klärpunkt [°C]: | 81,0 |
| CC-3-V1 | 10,50 % | Δε [1 kHz, 20 °C]: | 2,7 |
| CCP-V-1 | 10,00 % | ε_{∥} [1 kHz, 20°C]: | 5,3 |
| CLP-V2-1 | 5,00 % | ε_{⊥} [1 kHz, 20°C]: | 2,6 |
| CLP-1V-1 | 3,50 % | γ₁ [mPa·s, 20 °C]: | 50 |
| CLP-3-T | 5,50 % | K₁ [pN, 20 °C]: | 15,5 |
| PGP-2-2V | 2,00 % | K₃ [pN, 20 °C]: | 17,2 |
| PGUQU-3-F | 4,00 % | | |
| APUQU-2-F | 4,50 % | | |
| PP-1-2V1 | 5,50 % | | |
| PPGU-3-F | 0,50 % | | |

### Beispiel M93

| | | | |
|---|---|---|---|
| APUQU-3-F | 1,00 % | Klärpunkt [°C]: | 76,5 |
| PPGU-3-F | 0,50 % | Δn [589 nm, 20 °C] : | 0,0995 |
| CCP-V-1 | 5,00 % | Δε [1 kHz, 20 °C]: | 2,3 |
| CLP-1V-1 | 9,00 % | ε_{∥} [1 kHz, 20°C]: | 4,9 |
| PGP-2-3 | 6,00 % | ε_{⊥} [1 kHz, 20°C]: | 2,6 |
| PGP-2-4 | 7,00 % | γ₁ [mPa·s, 20 °C]: | 53 |
| PUQU-3-F | 9,00 % | K₁ [pN, 20 °C]: | 14,6 |
| CC-3-2V1 | 6,50 % | K₃ [pN, 20 °C]: | 15,4 |
| CC-3-V | 46,00 % | | |
| CC-3-V1 | 10,00 % | | |

### Beispiel M94

| | |
|---|---|
| APUQU-3-F | 1,50 % |
| PPGU-3-F | 0,50 % |
| CLP-V-1 | 10,00 % |
| CLP-1V-1 | 5,00 % |
| PGP-2-3 | 5,00 % |
| PGP-2-4 | 7,00 % |
| PUQU-3-F | 8,50 % |
| CC-3-2V1 | 6,50 % |
| CC-3-V | 47,00 % |
| CC-3-V1 | 9,00 % |

### Beispiel M95

| | |
|---|---|
| CC-3-V | 49,50 % |
| CC-3-V1 | 10,00 % |
| CCP-V-1 | 10,00 % |
| CLP-1V-1 | 6,00 % |
| CLP-1V2-T | 5,50 % |
| PGP-2-2V | 4,00 % |
| PGUQU-3-F | 4,00 % |
| APUQU-2-F | 4,50 % |
| PP-1-2V1 | 6,00 % |
| PPGU-3-F | 0,50 % |

### Beispiel M96

| | | | |
|---|---|---|---|
| APUQU-2-F | 3,00 % | Klärpunkt [°C]: | 75,5 |
| CC-3-V | 49,50 % | Δn [589 nm, 20 °C] : | 0,1132 |
| CC-3-V1 | 8,50 % | Δε [1 kHz, 20 °C]: | 2,5 |
| CLP-1V-1 | 6,00 % | ε_{∥} [1 kHz, 20°C]: | 5,1 |
| CLP-3-T | 4,50 % | ε_{⊥} [1 kHz, 20°C]: | 2,6 |
| PGP-2-2V | 14,00 % | γ₁ [mPa·s, 20 °C]: | 50 |
| PGP-3-2V | 2,50 % | K₁ [pN, 20 °C]: | 15,1 |
| PGUQU-3-F | 2,50 % | K₃ [pN, 20 °C]: | 15,0 |
| PP-1-2V1 | 7,00 % | | |
| PPGU-3-F | 0,50 % | | |
| PUQU-3-F | 2,00 % | | |

### Beispiel M97

| | | | |
|---|---|---|---|
| APUQU-2-F | 7,50 % | Klärpunkt [°C]: | 76,0 |
| CC-3-V | 50,00 % | Δn [589 nm, 20 °C] : | 0,1091 |
| CC-3-V1 | 9,50 % | Δε [1 kHz, 20 °C]: | 2,5 |
| CLP-1V-1 | 6,00 % | ε_{∥} [1 kHz, 20°C]: | 5,1 |
| CLP-3-T | 4,50 % | ε_{⊥} [1 kHz, 20°C]: | 2,6 |
| PGP-2-2V | 15,00 % | γ₁ [mPa·s, 20 °C]: | 50 |
| PP-1-2V1 | 7,00 % | K₁ [pN, 20 °C]: | 15,2 |
| PPGU-3-F | 0,50 % | K₃ [pN, 20 °C]: | 15,0 |

### Beispiel M98

| | | | |
|---|---|---|---|
| APUQU-2-F | 7,50 % | Klärpunkt [°C]: | 76,5 |
| CC-3-V | 52,00 % | Δn [589 nm, 20 °C] : | 0,1062 |
| CC-3-V1 | 7,50 % | Δε [1 kHz, 20 °C]: | 2,5 |
| CCP-V-1 | 2,00 % | ε_{∥} [1 kHz, 20°C]: | 5,1 |
| CLP-1V-1 | 6,00 % | ε_{⊥} [1 kHz, 20°C]: | 2,6 |
| CLP-3-T | 4,50 % | γ₁ [mPa·s, 20 °C]: | 50 |
| PGP-2-2V | 14,00 % | | |
| PP-1-2V1 | 6,00 % | | |
| PPGU-3-F | 0,50 % | | |

### Beispiel M99

| | |
|---|---|
| CC-3-V | 46,00 % |
| CC-3-V1 | 7,00 % |
| CLP-1V-1 | 8,00 % |
| CLP-3-T | 7,00 % |
| PGP-1-2V | 2,00 % |
| PGP-2-2V | 14,50 % |
| PP-1-2V1 | 9,00 % |
| PPGU-3-F | 0,50 % |
| PUQU-3-F | 4,50 % |
| PGUQU-3-F | 1,50 % |

### Beispiel M100

| | | | |
|---|---|---|---|
| APUQU-2-F | 7,50 % | Klärpunkt [°C]: | 81,5 |
| DGUQU-4-F | 5,00 % | Δn [589 nm, 20 °C] : | 0,0989 |
| DPGU-4-F | 4,50 % | Δε [1 kHz, 20 °C]: | 6,1 |
| PGUQU-3-F | 3,00 % | ε_{∥} [1 kHz, 20°C]: | 9,1 |
| PPGU-3-F | 0,50 % | ε_{⊥} [1 kHz, 20°C]: | 3,0 |
| CLP-1V-1 | 3,50 % | γ₁ [mPa·s, 20 °C]: | 58 |
| CC-3-2V1 | 5,00 % | K₁ [pN, 20 °C]: | 14,8 |
| CC-3-V | 50,00 % | K₃ [pN, 20 °C]: | 15,2 |
| CC-3-V1 | 8,00 % | | |
| CLP-3-T | 5,00 % | | |
| PGP-2-2V | 8,00 % | | |

### Beispiel M101

| | |
|---|---|
| CC-3-V | 48,00 % |
| CC-3-V1 | 10,50 % |
| CCP-V-1 | 11,00 % |
| CLP-V-1 | 6,00 % |
| CLP-1V2-T | 2,50 % |
| CLP-3-T | 3,00 % |
| PGP-2-2V | 5,00 % |
| PGUQU-3-F | 4,00 % |
| APUQU-2-F | 4,50 % |
| PP-1-2V1 | 5,00 % |
| PPGU-3-F | 0,50 % |

### Beispiel M102

| | |
|---|---|
| CC-3-V | 49,50 % |
| CC-3-V1 | 10,00 % |
| CCP-V-1 | 10,00 % |
| CLP-1V-1 | 6,00 % |
| CLP-1V2-T | 5,50 % |
| PGP-2-2V | 4,00 % |
| PGUQU-3-F | 4,00 % |
| APUQU-2-F | 4,50 % |
| PP-1-2V1 | 6,00 % |
| PPGU-3-F | 0,50 % |

### Beispiel M103

| | | | |
|---|---|---|---|
| APUQU-2-F | 4,00 % | Klärpunkt [°C]: | 76,5 |
| PGUQU-3-F | 3,00 % | Δn [589 nm, 20 °C] : | 0,1182 |
| PPGU-3-F | 0,50 % | Δε [1 kHz, 20 °C]: | 2,4 |
| CCP-V-1 | 4,00 % | ε_{∥} [1 kHz, 20°C]: | 5,1 |
| PGP-1-2V | 6,00 % | ε_{⊥} [1 kHz, 20°C]: | 2,7 |
| PGP-2-2V | 15,50 % | γ₁ [mPa·s, 20 °C]: | 50 |
| PP-1-2V1 | 6,00 % | K₁ [pN, 20 °C]: | 14,4 |
| CC-3-V | 52,00 % | K₃ [pN, 20 °C]: | 14,6 |
| CC-3-V1 | 4,50 % | | |
| CLP-1V2-T | 4,50 % | | |

### Beispiel M104

| | | | |
|---|---|---|---|
| APUQU-2-F | 7,00 % | Klärpunkt [°C]: | 77 |
| PGUQU-3-F | 6,00 % | Δn [589 nm, 20 °C] : | 0,1179 |
| PPGU-3-F | 0,50 % | Δε [1 kHz, 20 °C]: | 4,0 |
| CCP-V-1 | 1,00 % | ε_{∥} [1 kHz, 20°C]: | 6,8 |
| PGP-1-2V | 4,00 % | ε_{⊥} [1 kHz, 20°C]: | 2,8 |
| PGP-2-2V | 15,00 % | γ₁ [mPa·s, 20 °C]: | 53 |
| PP-1-2V1 | 4,50 % | K₁ [pN, 20 °C]: | 14,4 |
| CC-3-V | 50,00 % | K₃ [pN, 20 °C]: | 14,8 |
| CC-3-V1 | 5,00 % | | |
| CC-3-2V1 | 2,00 % | | |
| CLP-1V2-T | 5,00 % | | |

### Beispiel M105

| | | | |
|---|---|---|---|
| APUQU-2-F | 5,50 % | Klärpunkt [°C]: | 81 |
| DGUQU-4-F | 5,50 % | Δn [589 nm, 20 °C]: | 0,0982 |
| DPGU-4-F | 5,00 % | Δε [1 kHz, 20 °C]: | 6,1 |
| PGUQU-3-F | 3,00 % | ε_{∥} [1 kHz, 20°C]: | 9,0 |
| PPGU-3-F | 0,50 % | ε_{⊥} [1 kHz, 20°C]: | 3,0 |
| CCP-V-1 | 1,00 % | γ₁ [mPa·s, 20 °C]: | 60 |
| CC-3-2V1 | 9,00 % | K₁ [pN, 20 °C]: | 15,3 |
| CC-3-V | 48,00 % | K₃ [pN, 20 °C]: | 15,5 |
| CC-3-V1 | 7,00 % | | |
| CLP-3-T | 4,00 % | | |
| CLP-1V-T | 3,00 % | | |
| PGP-2-2V | 8,50 % | | |

### Beispiel M106

| | | | |
|---|---|---|---|
| CC-3-V | 50,00 % | Klärpunkt [°C]: | 83 |
| CC-3-V1 | 11,50 % | Δn [589 nm, 20 °C]: | 0,0943 |
| CLP-V-1 | 11,00 % | Δε [1 kHz, 20 °C]: | 2,7 |
| CLP-1-V1 | 6,00 % | ε_{∥} [1 kHz, 20°C]: | 5,2 |
| CLP-3-T | 6,00 % | ε_{⊥} [1 kHz, 20°C]: | 2,5 |
| PGP-2-2V | 3,00 % | γ₁ [mPa·s, 20 °C]: | 53 |
| PGUQU-3-F | 3,00 % | K₁ [pN, 20 °C]: | 16,6 |
| APUQU-3-F | 5,00 % | K₃ [pN, 20 °C]: | 17,3 |
| PP-1-2V1 | 4,00 % | | |
| PPGU-3-F | 0,50 % | | |

### Beispiel M107

| | | | |
|---|---|---|---|
| CC-3-V | 51,50 % | Klärpunkt [°C]: | 82 |
| CC-3-V1 | 11,50 % | Δn [589 nm, 20 °C] : | 0,0923 |
| CLP-1V-1 | 9,00 % | Δε [1 kHz, 20 °C]: | 2,7 |
| CLP-V2-1 | 6,50 % | ε_{∥} [1 kHz, 20°C]: | 5,2 |
| CLP-3-T | 6,00 % | ε_{⊥} [1 kHz, 20°C]: | 2,5 |
| PGP-2-2V | 2,00 % | γ₁ [mPa·s, 20 °C]: | 54 |
| PGUQU-3-F | 3,00 % | K₁ [pN, 20 °C]: | 16,5 |
| APUQU-2-F | 5,00 % | K₃ [pN, 20 °C]: | 18,0 |
| PP-1-2V1 | 5,00 % | | |
| PPGU-3-F | 0,50 % | | |

### Beispiel M108

| | | | |
|---|---|---|---|
| CC-3-V | 50,00 % | Klärpunkt [°C]: | 81 |
| CC-3-V1 | 11,50 % | Δn [589 nm, 20 °C] : | 0,0956 |
| CLP-1V-1 | 11,00 % | Δε [1 kHz, 20 °C]: | 2,7 |
| CLP-V2-1 | 6,00 % | ε_{∥} [1 kHz, 20°C]: | 5,2 |
| CLP-3-T | 6,00 % | ε_{⊥} [1 kHz, 20°C]: | 2,5 |
| PGP-2-2V | 1,00 % | γ₁ [mPa·s, 20 °C]: | 51 |
| PGUQU-3-F | 3,00 % | K₁ [pN, 20 °C]: | 16,4 |
| APUQU-2-F | 5,00 % | K₃ [pN, 20 °C]: | 17,3 |
| PP-1-2V1 | 6,00 % | | |
| PPGU-3-F | 0,50 % | | |

### Beispiel M109

| | |
|---|---|
| CC-3-V | 51,50 % |
| CC-3-V1 | 11,50 % |
| CLP-1V-1 | 8,00 % |
| CLP-V2-1 | 6,00 % |
| CLP-3-T | 6,50 % |
| PGP-2-2V | 2,00 % |
| PGUQU-3-F | 3,00 % |
| APUQU-2-F | 5,00 % |
| PP-1-2V1 | 6,00 % |
| PPGU-3-F | 0,50 % |

### Beispiel M110

| | | | |
|---|---|---|---|
| CC-3-2V1 | 1,00 % | Klärpunkt [°C]: | 77,5 |
| CC-3-V | 50,00 % | Δn [589 nm, 20 °C] : | 0,1175 |
| CC-3-V1 | 2,00 % | Δε [1 kHz, 20 °C]: | 4,2 |
| CCP-V-1 | 2,00 % | ε_{∥} [1 kHz, 20°C]: | 7,0 |
| CLP-3-T | 6,00 % | ε_{⊥} [1 kHz, 20°C]: | 2,7 |
| CLP-1V-1 | 6,50 % | γ₁ [mPa·s, 20 °C]: | 54 |
| PGP-2-2V | 11,50 % | K₁ [pN, 20 °C]: | 16,6 |
| PP-1-2V1 | 7,00 % | K₃ [pN, 20 °C]: | 14,9 |
| PGU-4-T | 3,50 % | | |
| PGUQU-3-F | 5,00 % | | |
| PGUQU-4-F | 5,00 % | | |
| PPGU-3-F | 0,50 % | | |

### Beispiel M111

| | | | |
|---|---|---|---|
| APUQU-2-F | 3,00 % | Klärpunkt [°C]: | 76,5 |
| CC-3-V | 49,50 % | Δn [589 nm, 20 °C] : | 0,1178 |
| CC-3-V1 | 6,00 % | Δε [1 kHz, 20 °C]: | 2,4 |
| CLP-1V-1 | 6,00 % | ε_{∥} [1 kHz, 20°C]: | 5,0 |
| CLP-3-T | 4,50 % | ε_{⊥} [1 kHz, 20°C]: | 2,7 |
| PGP-1-2V | 3,00 % | γ₁ [mPa·s, 20 °C]: | 50 |
| PGP-2-2V | 13,00 % | K₁ [pN, 20 °C]: | 15,2 |
| PGP-3-2V | 2,50 % | K₃ [pN, 20 °C]: | 15,1 |
| PGUQU-3-F | 2,00 % | | |
| PP-1-2V1 | 8,00 % | | |
| PPGU-3-F | 0,50 % | | |
| PUQU-3-F | 2,00 % | | |

### Beispiel M112

| | | | |
|---|---|---|---|
| APUQU-2-F | 2,00 % | Klärpunkt [°C]: | 75 |
| PGUQU-3-F | 5,00 % | Δn [589 nm, 20 °C] : | 0,1171 |
| PPGU-3-F | 0,50 % | Δε [1 kHz, 20 °C]: | 2,4 |
| CCP-V-1 | 2,50 % | ε_{∥} [1 kHz, 20°C]: | 5,0 |
| LPP-3-2 | 3,50 % | ε_{⊥} [1 kHz, 20°C]: | 2,7 |
| PGP-2-2V | 15,00 % | γ₁ [mPa·s, 20 °C]: | 46 |
| PP-1-2V1 | 9,00 % | K₁ [pN, 20 °C]: | 15,3 |
| CC-3-V | 51,00 % | K₃ [pN, 20 °C]: | 14,3 |
| CC-3-V1 | 5,00 % | | |
| CLP-3-T | 4,50 % | | |
| CLP-1V-1 | 2,00 % | | |

### Beispiel M113

| | | | |
|---|---|---|---|
| CC-3-V | 46,00 % | Klärpunkt [°C]: | 77,5 |
| CC-3-V1 | 6,00 % | Δn [589 nm, 20 °C] : | 0,1196 |
| CLP-1V-1 | 1,50 % | Δε [1 kHz, 20 °C]: | 2,9 |
| PGP-2-2V | 11,00 % | ε_{∥} [1 kHz, 20°C]: | 6,8 |
| PGP-3-2V | 2,00 % | ε_{⊥} [1 kHz, 20°C]: | 3,9 |
| PGU-2-F | 6,50 % | γ₁ [mPa·s, 20 °C]: | 57 |
| PGUQU-3-F | 5,00 % | K₁ [pN, 20 °C]: | 15,6 |
| CLP-3-T | 5,00 % | K₃ [pN, 20 °C]: | 14,2 |
| B(S)-2O-O4 | 3,50 % | | |
| B(S)-2O-O5 | 3,50 % | | |
| CC-3-2V1 | 1,00 % | | |
| DGUQU-4-F | 1,50 % | | |
| PY-3-O2 | 2,50 % | | |
| CLP-1-V1 | 5,00 % | | |

### Beispiel M114

| | |
|---|---|
| APUQU-2-F | 4,50 % |
| PGUQU-3-F | 4,00 % |
| PPGU-3-F | 0,50 % |
| CLP-1V-1 | 6,00 % |
| PGP-1-2V | 2,50 % |
| PGP-2-2V | 16,50 % |
| PP-1-2V1 | 7,50 % |
| CC-3-V | 52,50 % |
| CC-3-V1 | 6,00 % |

### Beispiel M115

| | | | |
|---|---|---|---|
| CC-3-V | 47,50 % | Klärpunkt [°C]: | 79,5 |
| CC-3-V1 | 5,50 % | Δn [589 nm, 20 °C] : | 0,1178 |
| PGP-2-2V | 12,00 % | Δε [1 kHz, 20 °C]: | 2,9 |
| PGP-3-2V | 2,50 % | ε_{∥} [1 kHz, 20°C]: | 6,6 |
| PGU-2-F | 5,50 % | ε_{⊥} [1 kHz, 20°C]: | 3,8 |
| PGUQU-3-F | 2,00 % | γ₁ [mPa·s, 20 °C]: | 58 |
| CLP-3-T | 5,00 % | K₁ [pN, 20 °C]: | 15,8 |
| B(S)-2O-O4 | 3,50 % | K₃ [pN, 20 °C]: | 14,7 |
| B(S)-2O-O5 | 3,50 % | | |
| CC-3-2V1 | 2,50 % | | |
| DGUQU-4-F | 3,00 % | | |
| CLP-1V-1 | 4,50 % | | |
| PGIY-2-O4 | 1,50 % | | |
| APUQU-2-F | 1,50 % | | |

### Beispiel M116

| | | | |
|---|---|---|---|
| CC-3-V | 47,50 % | Klärpunkt [°C]: | 81,5 |
| CC-3-V1 | 2,00 % | Δn [589 nm, 20 °C] : | 0,1195 |
| PGP-2-2V | 12,00 % | Δε [1 kHz, 20 °C]: | 2,9 |
| PGP-3-2V | 1,00 % | ε_{∥} [1 kHz, 20°C]: | 6,7 |
| PGU-2-F | 7,50 % | ε_{⊥} [1 kHz, 20°C]: | 3,8 |
| PGUQU-3-F | 1,00 % | | |
| CLP-3-T | 5,00 % | | |
| B(S)-2O-O4 | 3,50 % | | |
| B(S)-2O-O5 | 3,50 % | | |
| CC-3-2V1 | 3,00 % | | |
| DGUQU-4-F | 3,00 % | | |
| CLP-1V-1 | 4,00 % | | |
| PGIY-2-O4 | 1,00 % | | |
| APUQU-2-F | 1,00 % | | |
| CLP-V-1 | 5,00 % | | |

### Beispiel M117

| | | | |
|---|---|---|---|
| APUQU-2-F | 6,00 % | Klärpunkt [°C]: | 80 |
| APUQU-3-F | 5,00 % | Δn [589 nm, 20 °C] : | 0,1129 |
| PGUQU-3-F | 2,00 % | Δε [1 kHz, 20 °C]: | 3,9 |
| PPGU-3-F | 0,50 % | ε_{∥} [1 kHz, 20°C]: | 6,7 |
| CCP-V-1 | 2,50 % | ε_{⊥} [1 kHz, 20°C]: | 2,8 |
| PGP-1-2V | 4,00 % | γ₁ [mPa·s, 20 °C]: | 52 |
| PGP-2-2V | 16,50 % | K₁ [pN, 20 °C]: | 14,1 |
| CC-3-V | 52,00 % | K₃ [pN, 20 °C]: | 14,1 |
| CC-3-V1 | 6,50 % | V₀ [V, 20 °C]: | 2,03 |
| CLP-3-T | 5,00 % | | |

Die flüssigkristalline Mischung M117 wird zusätzlich mit 0,05 % der Verbindung der Formel ST-1 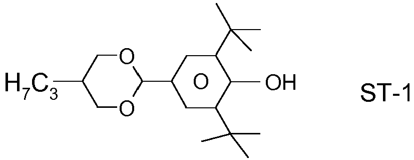 versetzt.

### Beispiel M118

| | | | |
|---|---|---|---|
| APUQU-2-F | 5,00 % | Klärpunkt [°C]: | 80 |
| PGUQU-3-F | 6,00 % | Δn [589 nm, 20 °C] : | 0,1131 |
| PPGU-3-F | 0,50 % | Δε [1 kHz, 20 °C]: | 3,5 |
| CCP-V-1 | 8,50 % | ε_{∥} [1 kHz, 20°C]: | 6,2 |
| PGP-2-2V | 16,50 % | ε_{⊥} [1 kHz, 20°C]: | 2,7 |
| PP-1-2V1 | 4,00 % | γ₁ [mPa·s, 20 °C]: | 51 |
| CC-3-V | 48,00 % | K₁ [pN, 20 °C]: | 14,6 |
| CC-3-V1 | 6,50 % | K₃ [pN, 20 °C]: | 14,7 |
| CLP-3-T | 5,00 % | V₀ [V, 20 °C]: | 2,17 |

Die flüssigkristalline Mischung M118 wird zusätzlich mit 0,05 % der Verbindung der Formel ST-1 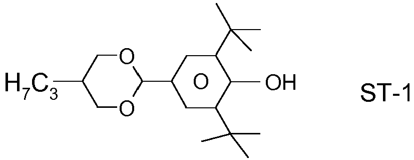 stabilisiert.

### Beispiel M119

| | | | |
|---|---|---|---|
| APUQU-2-F | 2,50 % | Klärpunkt [°C]: | 85 |
| CC-3-2V1 | 5,00 % | Δn [589 nm, 20 °C]: | 0,1093 |
| CC-3-V | 24,50 % | Δε [1 kHz, 20 °C]: | 8,6 |
| CC-3-V1 | 5,00 % | ε_{∥} [1 kHz, 20°C]: | 10,6 |
| CCP-3OCF₃ | 3,00 % | ε_{⊥} [1 kHz, 20°C]: | 2,0 |
| CCP-V-1 | 9,00 % | γ₁ [mPa·s, 20 °C]: | 94 |
| CCP-V2-1 | 8,00 % | K₁ [pN, 20 °C]: | 14,9 |
| CLP-3-T | 7,00 % | K₃ [pN, 20 °C]: | 14,6 |
| CPGP-4-3 | 2,00 % | V₀ [V, 20 °C]: | 1,39 |
| DGUQU-4-F | 5,00 % | | |
| DPGU-4-F | 5,50 % | | |
| PGP-2-2V | 2,00 % | | |
| PGUQU-3-F | 6,00 % | | |
| PGUQU-4-F | 6,00 % | | |
| PPGU-3-F | 0,50 % | | |
| Y-4O-O4 | 9,00 % | | |

Die flüssigkristalline Mischung M119 wird zusätzlich mit 0,04 % der Verbindung der Formel ST-1 und mit 0,02 % der Verbindung der Formel ST-3 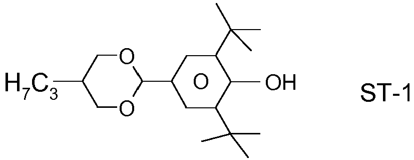 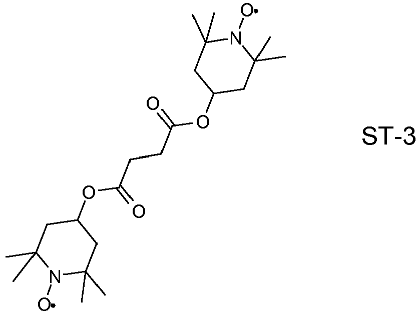 stabilisiert.

## Patentansprüche

1. Flüssigkristallines Medium, **dadurch gekennzeichnet, dass** es eine oder mehrere Verbindungen der Formel I, 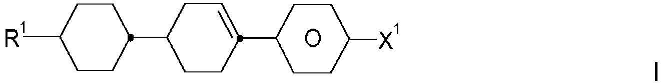 worin
R¹ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 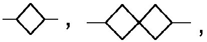 -O-, -CO-O-, -O-CO- so ersetzt sein können, dass O-Atome nicht direkt miteinander verknüpft sind, und worin auch ein oder mehrere H-Atome durch Halogen ersetzt sein können, und
X¹ einen Alkylrest mit 1 bis 5 C-Atomen, OCF₃, CF₃, CHF₂, OCHF₂, OCF₂CF₃, CCF₂CHFCF₃, OCF=CF₂, OCH=CF₂, oder F
bedeuten,
enthält,
und zusätzlich eine oder mehrere Verbindungen der Formeln II und/oder III, 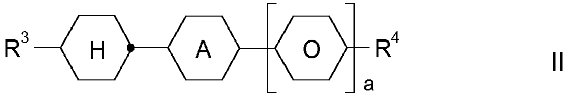 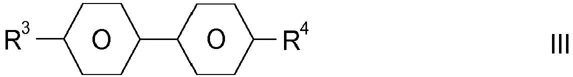 worin
A 1,4-Phenylen oder trans-1,4-Cyclohexylen,
a 0 oder 1,
R³ Alkenyl mit 2 bis 9 C-Atomen
bedeuten,
und
R⁴ die für R¹ in Anspruch 1 angegebenen Bedeutungen besitzt,
enthält.

2. Flüssigkristallines Medium nach Anspruch 1, **dadurch gekennzeichnet, dass** R¹ in Formel I ein geradkettiger Alkylrest bedeutet, worin auch eine oder mehrere CH₂-Gruppen durch -CH=CH- ersetzt sein können.

3. Flüssigkristallines Medium nach Anspruch 1 oder 2, **dadurch gekennzeichnet, dass** es mindestens eine Verbindung aus der Gruppe der Verbindungen der Formeln I-1 bis I-5 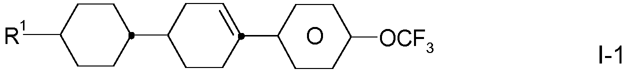 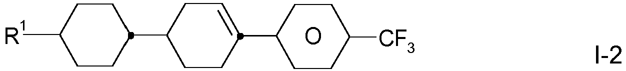 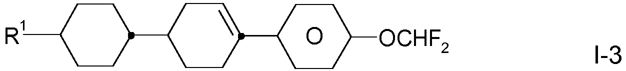 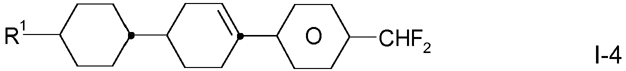 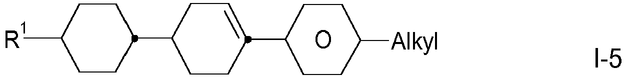 worin R¹ die in Anspruch 1 angegebenen Bedeutungen hat, enthält.

4. Flüssigkristallines Medium nach einem oder mehreren der Ansprüche 1 bis 3, **dadurch gekennzeichnet, dass** die Verbindungen ausgewählt sind aus den Formeln I-1 a bis I-5k, 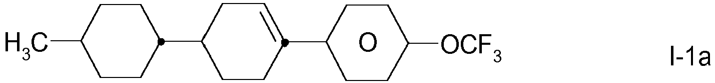 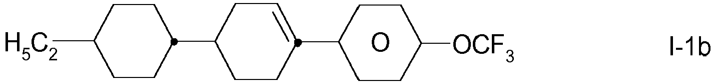 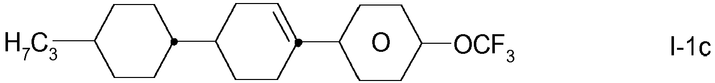 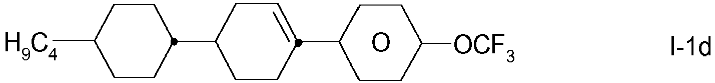 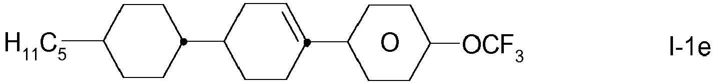 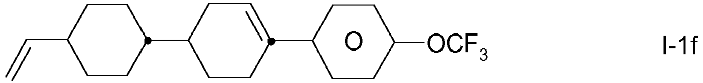 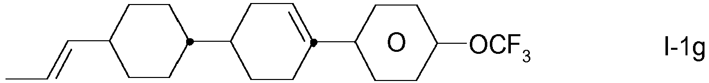 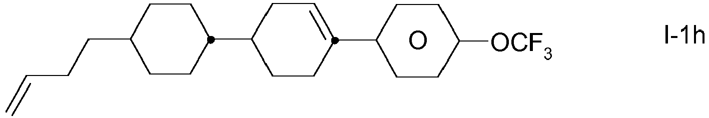 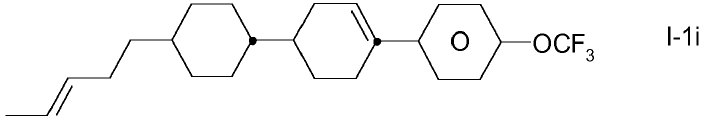 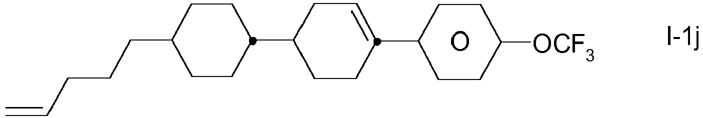 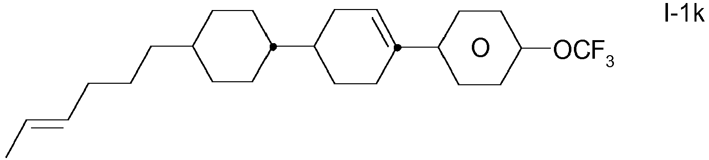 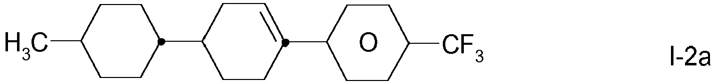 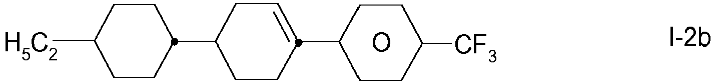 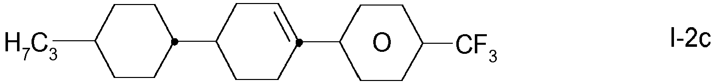 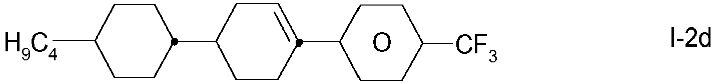 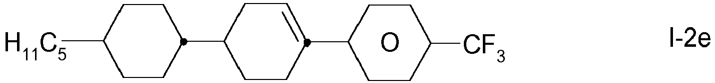 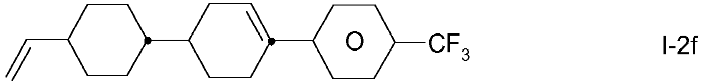 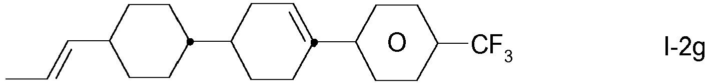 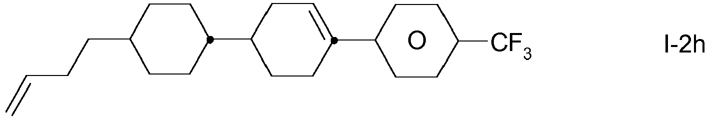 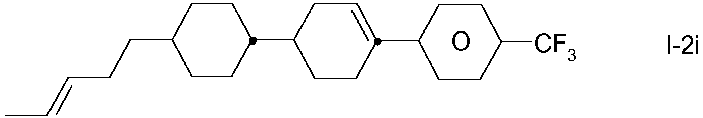 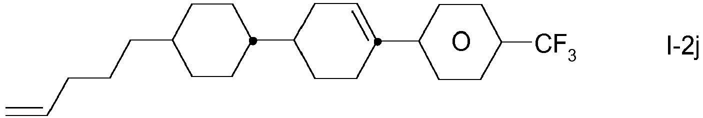 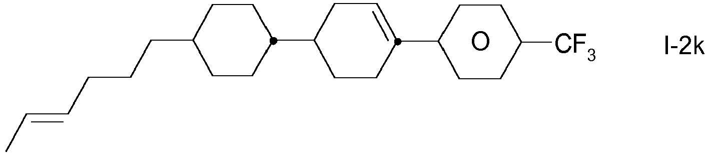 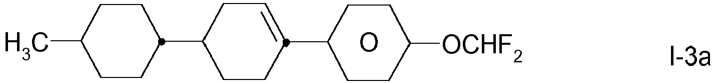 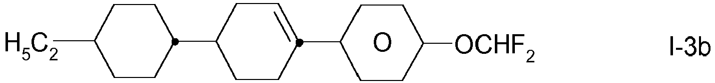 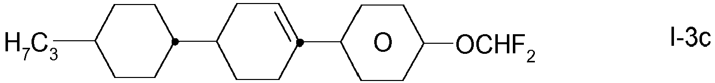 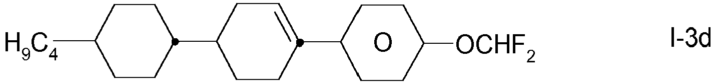 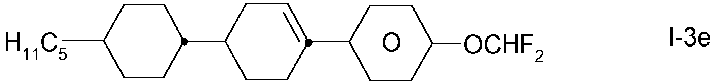 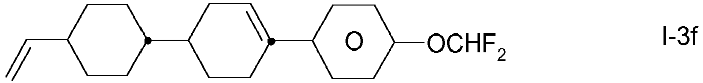 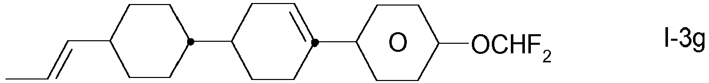 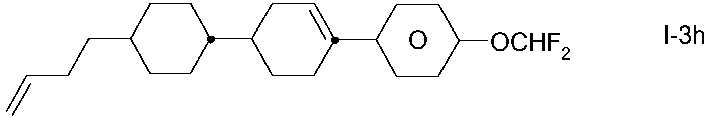 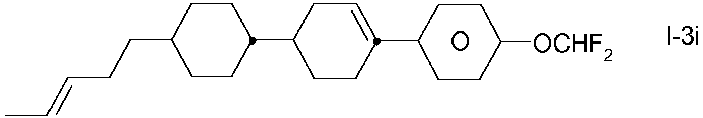 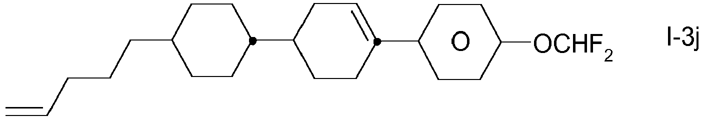 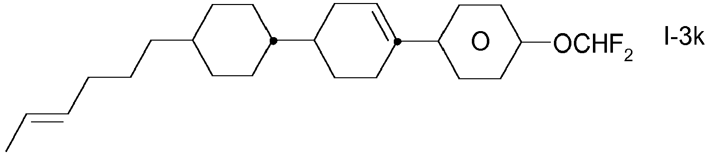 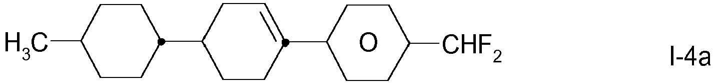 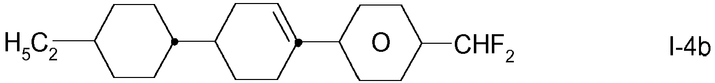 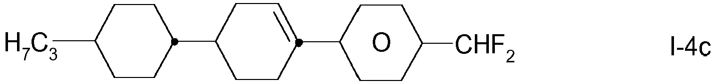 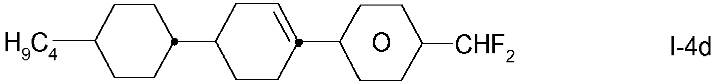 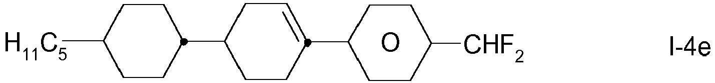 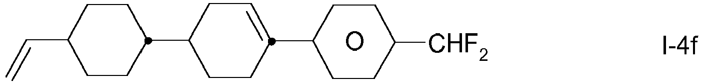 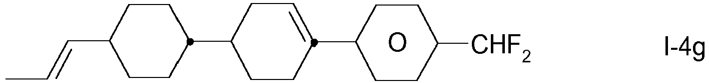 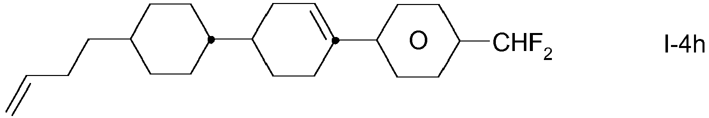 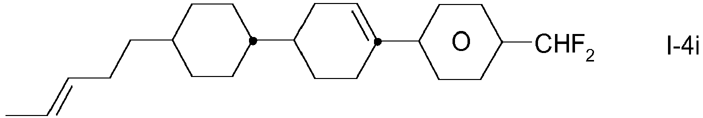 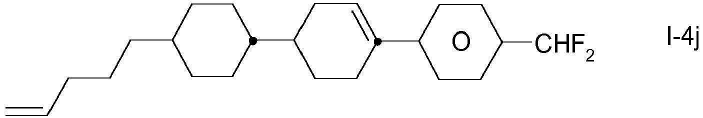 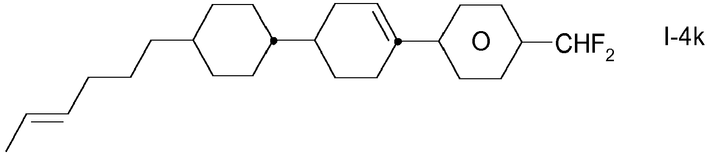 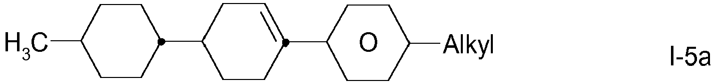 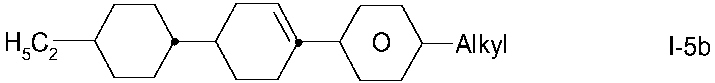 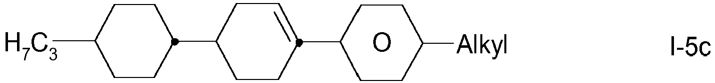 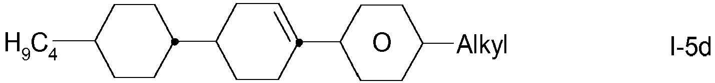 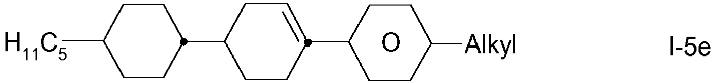 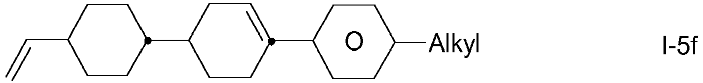 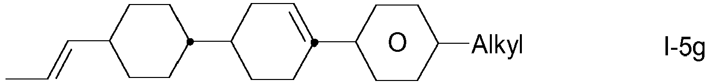 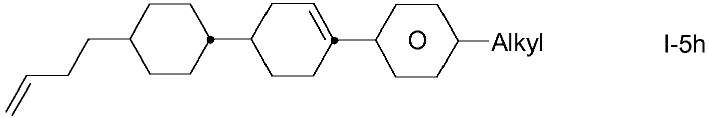 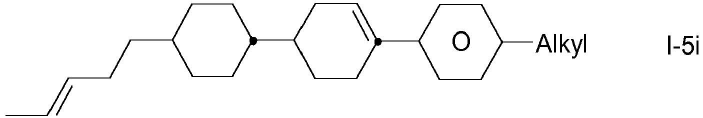 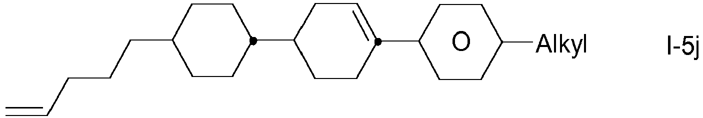 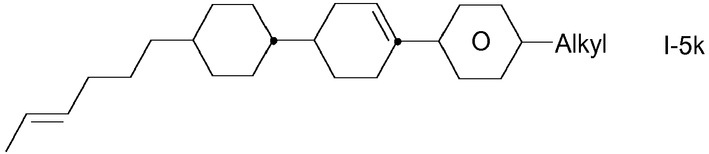 wobei Alkyl CH₃ oder C₂H₅ bedeutet.

5. Flüssigkristallines Medium nach einem oder mehreren der Ansprüche 1 bis 4, **dadurch gekennzeichnet, dass** es zusätzlich eine oder mehrere Verbindungen ausgewählt aus den Verbindungen der Formeln, 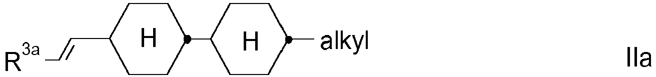 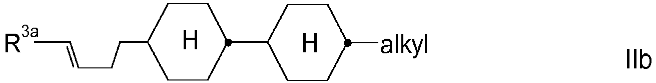 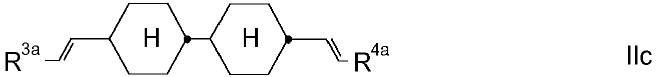 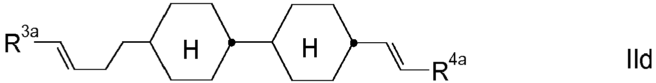 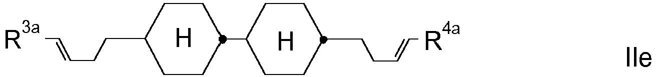 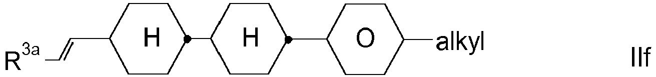 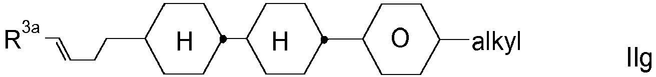 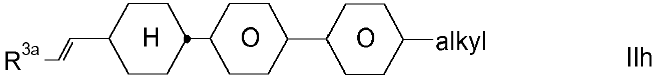 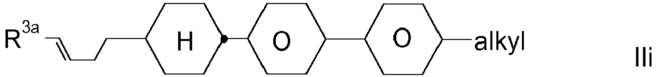 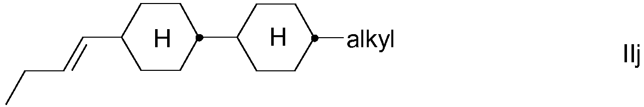 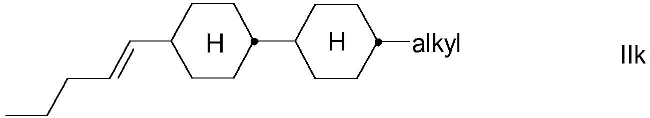 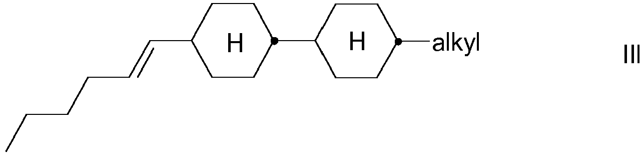 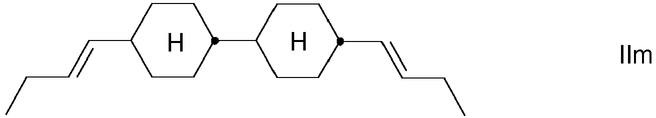 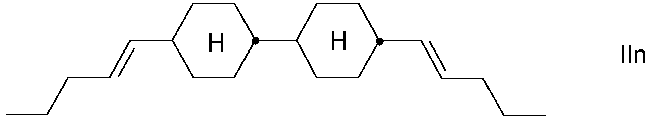 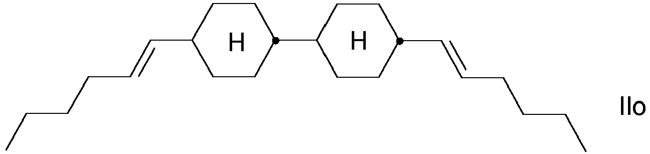 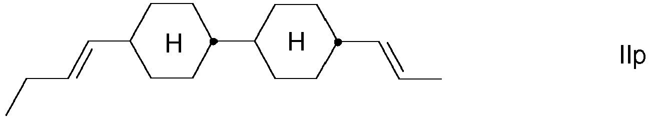 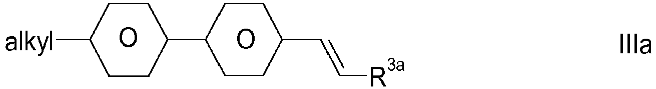 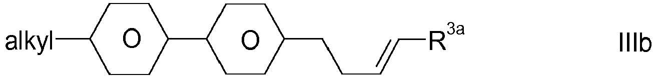 worin R^{3a} und R^{4a} jeweils unabhängig voneinander H, CH₃, C₂H₅ oder C₃H₇ bedeuten, und "alkyl" eine geradkettige Alkylgruppe mit 1 bis 8 C-Atomen bedeuten,
enthält.

6. Flüssigkristallines Medium nach einem oder mehreren der Ansprüche 1 bis 5, **dadurch gekennzeichnet, dass** es eine zusätzlich eine oder mehrere Verbindungen ausgewählt aus den Verbindungen der Formeln IV bis VIII, 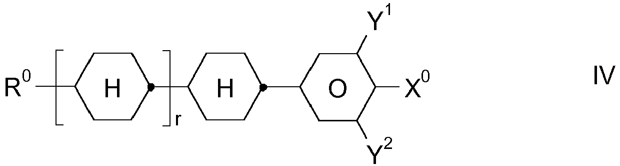 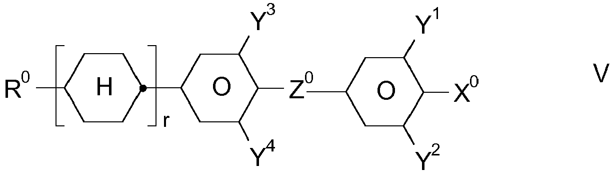 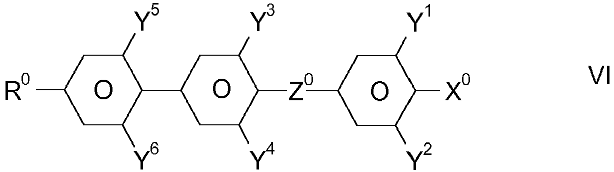 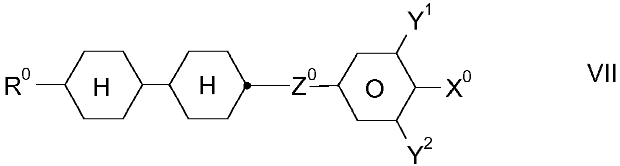 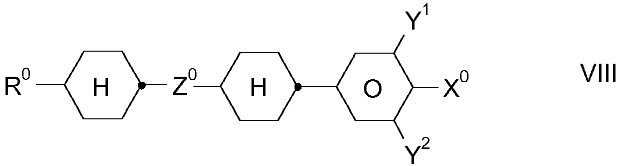 worin
R⁰ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 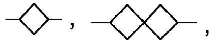 -O-, -CO-O- oder -O-CO- so ersetzt sein können, dass O-Atome nicht direkt miteinander verknüpft sind, und worin auch ein oder mehrere H-Atome durch Halogen ersetzt sein können, Cycloalkyl mit 3 bis 6 C-Atomen,
X⁰ F, Cl, ein- oder mehrfach fluorierter Alkyl- oder Alkoxyrest mit 1 bis 6 C-Atomen, ein oder mehrfach fluorierter Alkenyl- oder Alkenyloxyrest mit 2 bis 6 C-Atomen
Y¹⁻⁶ jeweils unabhängig voneinander H oder F,
Z⁰ -C₂H₄-, -(CH₂)₄-, -CH=CH-, -CF=CF-, -C₂F₄-, -CH₂CF₂-, -CF₂CH₂-, -CH₂O-, -OCH₂-, -COO-,-CF₂O- oder -OCF₂-, in den Formeln V und VI auch eine Einfachbindung, und
r 0 oder 1
bedeuten,
enthält.

7. Flüssigkristallines Medium nach einem oder mehreren der Ansprüche 1 bis 6, **dadurch gekennzeichnet, dass** es zusätzlich eine oder mehrere Verbindungen ausgewählt aus den Verbindungen der Formeln Va bis Vj, 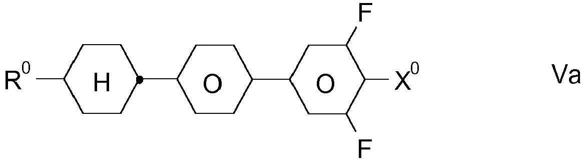 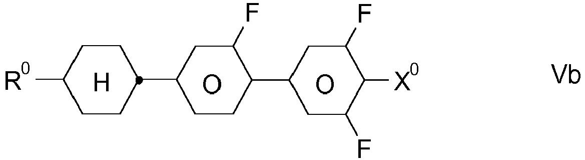 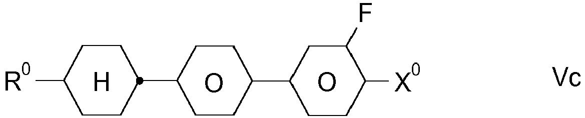 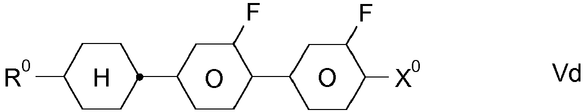 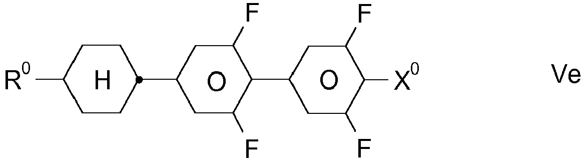 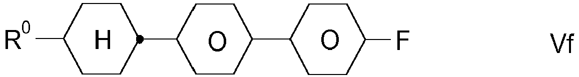 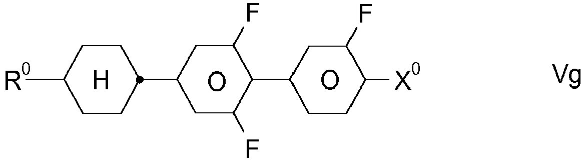 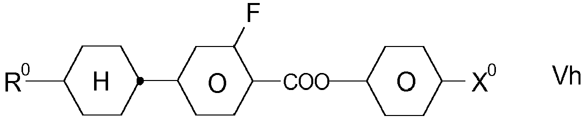 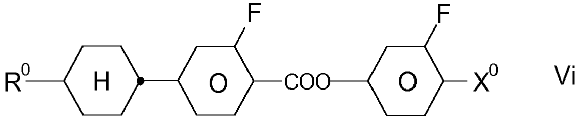 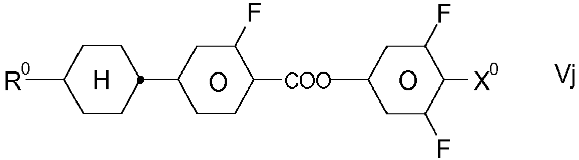 worin R⁰ und X⁰ die in Anspruch 6 angegebenen Bedeutungen haben,
enthält.

8. Flüssigkristallines Medium nach einem oder mehreren der Ansprüche 1 bis 7, **dadurch gekennzeichnet, dass** es zusätzlich eine oder mehrere Verbindungen ausgewählt aus den Verbindungen der Formeln VI-1a bis VI-1d, 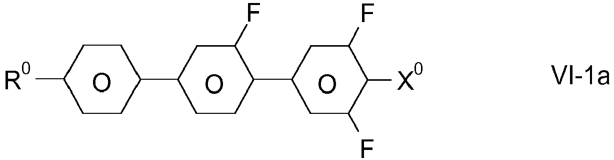 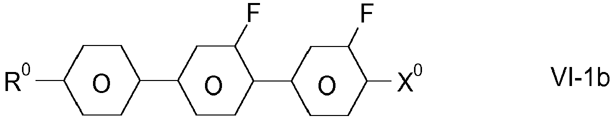 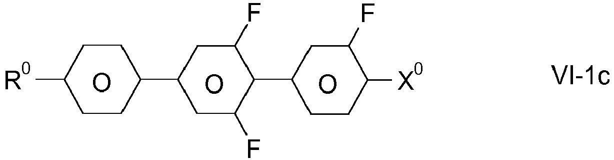 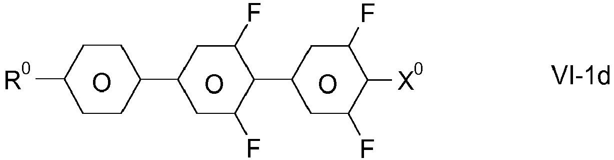 worin R⁰ und X⁰ die in Anspruch 6 angegebenen Bedeutungen haben,
enthält.

9. Flüssigkristallines Medium nach einem oder mehreren der Ansprüche 1 bis 8, **dadurch gekennzeichnet, dass** es zusätzlich eine oder mehrere Verbindungen ausgewählt aus den Verbindungen der Formeln VI-2a bis VI-2f, 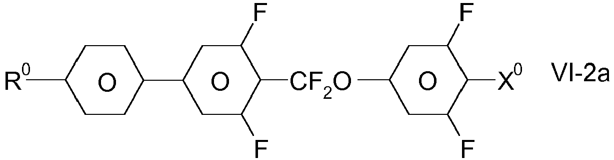 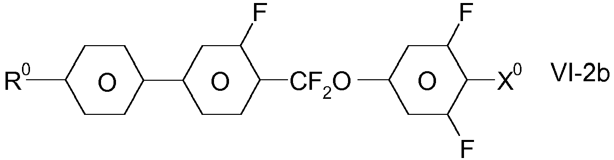 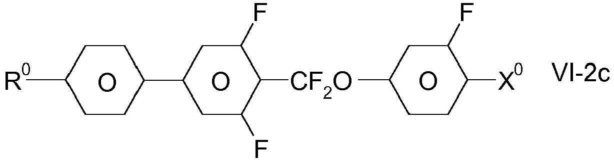 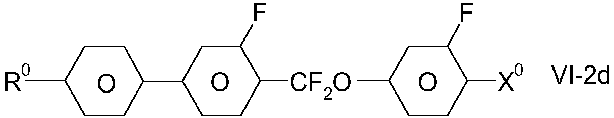 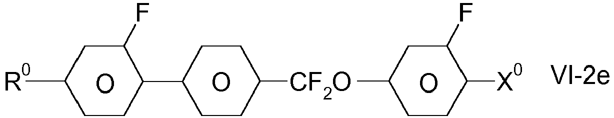 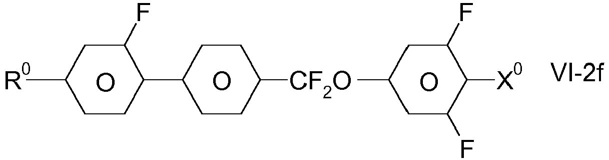 worin R⁰ und X⁰ die in Anspruch 6 angegebenen Bedeutungen haben,
enthält.

10. Flüssigkristallines Medium nach einem oder mehreren der Ansprüche 1 bis 9, **dadurch gekennzeichnet, dass** es zusätzlich eine oder mehrere Verbindungen ausgewählt aus den Verbindungen der Formeln X und/oder XI, 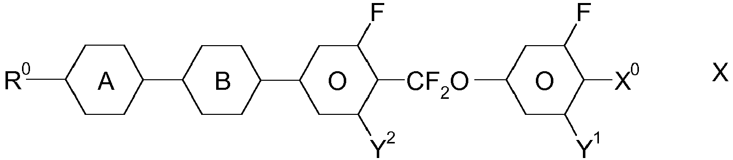 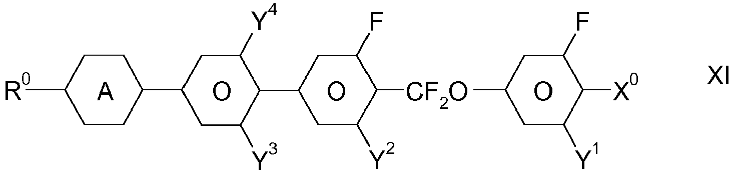 worin
R⁰ und X⁰ die in Anspruch 6 angegebenen Bedeutungen besitzen,
Y¹⁻⁴ jeweils unabhängig voneinander H oder F, und 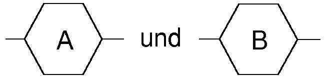 jeweils unabhängig voneinander 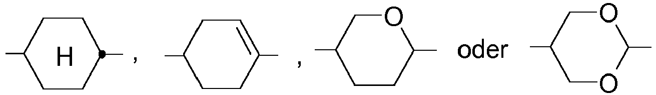 bedeuten,
enthält.

11. Flüssigkristallines Medium nach einem oder mehreren der Ansprüche 1 bis 10, **dadurch gekennzeichnet, dass** es zusätzlich eine oder mehrere Verbindungen ausgewählt aus den Verbindungen der Formel XII, 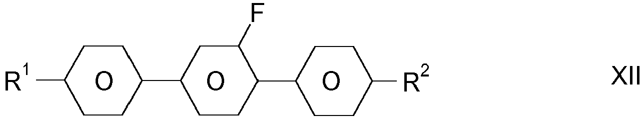 worin
R¹ und R² jeweils unabhängig voneinander Alkyl, Alkenyl, Alkoxy, Oxaalkyl, Fluoralkyl oder Alkenyloxy mit jeweils bis zu 9 C-Atomen und Y¹ H oder F,
bedeuten,
enthält.

12. Flüssigkristallines Medium nach einem oder mehreren der Ansprüche 1 bis 11, **dadurch gekennzeichnet, dass** es zusätzlich eine oder mehrere Verbindungen ausgewählt aus den Verbindungen der Formeln XIII bis XVI, 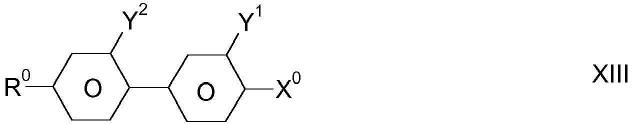 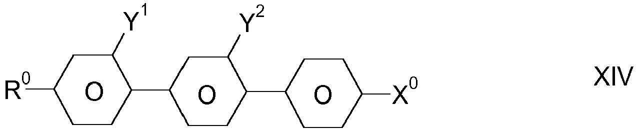 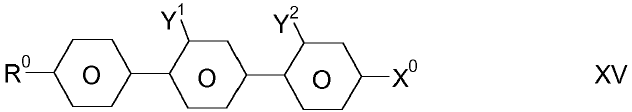 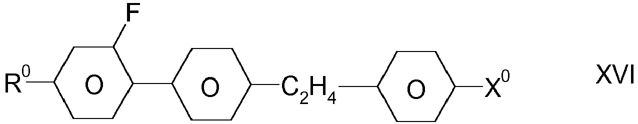 worin R⁰, X⁰, Y¹ und Y² die in Anspruch 6 angegebenen Bedeutungen haben,
enthält.

13. Flüssigkristallines Medium nach einem oder mehreren der Ansprüche 1 bis 12, **dadurch gekennzeichnet, dass** es zusätzlich eine oder mehrere Verbindungen ausgewählt aus der Gruppe der Verbindungen der Formeln 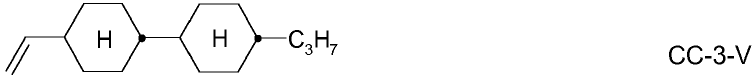 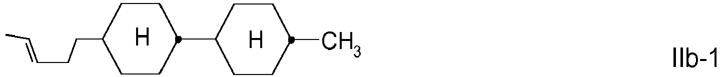 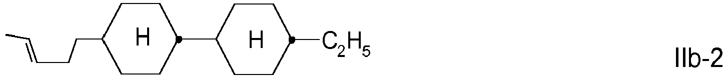 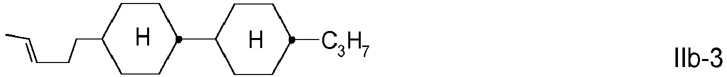 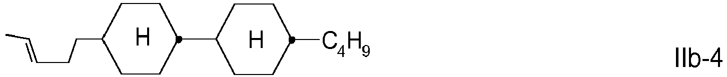 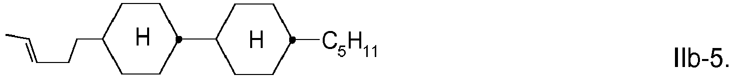 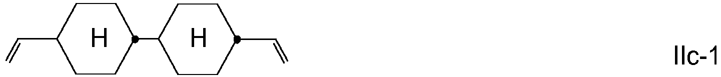 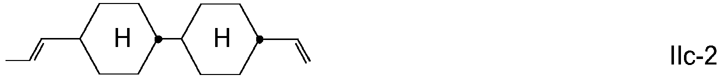 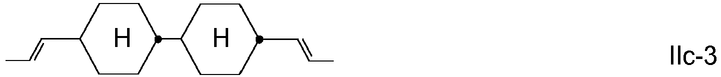 enthält.

14. Flüssigkristallines Medium nach einem oder mehreren der Ansprüche 1 bis 13, **dadurch gekennzeichnet, dass** es zusätzlich eine oder mehrere Verbindungen ausgewählt aus der Gruppe der Verbindungen der Formeln D1, D2, D3, D4 und D5, 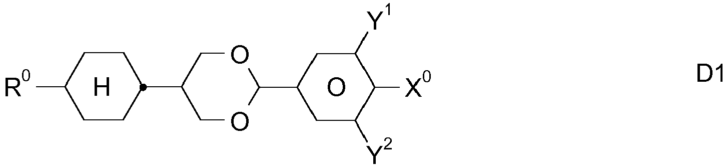 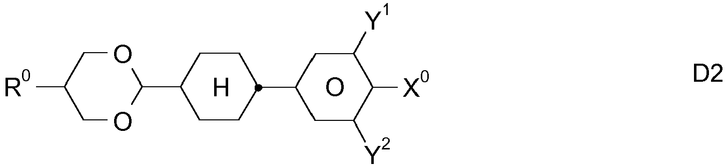 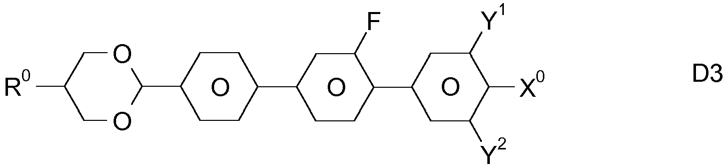 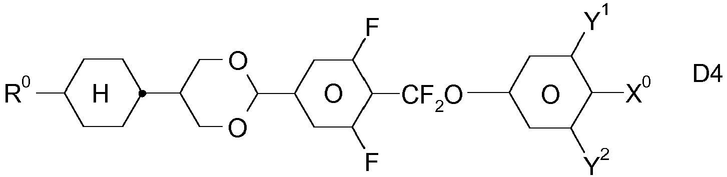 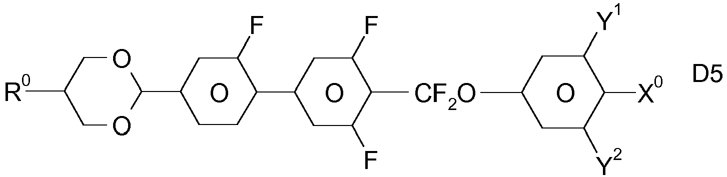 worin Y¹, Y², R⁰ und X⁰ die in Anspruch 6 angegebenen Bedeutungen besitzen,
enthält.

15. Flüssigkristallines Medium nach einem oder mehreren der Ansprüche 1 bis 14, **dadurch gekennzeichnet, dass** es zusätzlich eine oder mehrere Verbindungen ausgewählt aus der Gruppe der Verbindungen der Formeln Y-1, Y-2, Y-3 und Y-4, 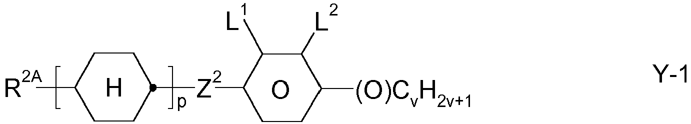 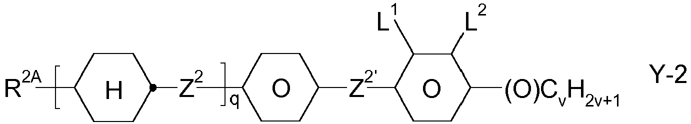 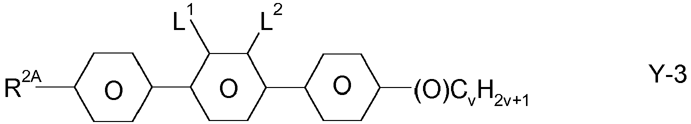 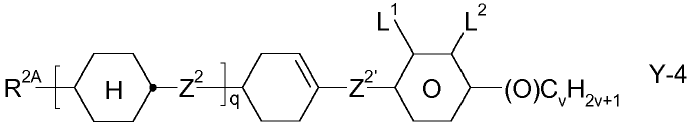 worin
R^{2A} H, einen Alkyl- oder Alkoxyrest mit 1 bis 15 C-Atomen, wobei in diesen Resten auch eine oder mehrere CH₂-Gruppen jeweils unabhängig voneinander durch -CH=CH-, -C≡C-, -CF₂O-, -CH=CH-, 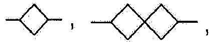 -O-, -CO-O-, -O-CO- so ersetzt sein können, dass O-Atome nicht direkt miteinander verknüpft sind, und worin auch ein oder mehrere H-Atome durch Halogen ersetzt sein können,
L¹ und L² jeweils unabhängig voneinander F, Cl, CF₃ oder CHF₂, vorzugsweise jeweils F,
Z² und Z^{2'} jeweils unabhängig voneinander eine Einfachbindung, -CH₂CH₂-, -CH=CH-, -CF₂O-, -OCF₂-,-CH₂O-, -OCH₂-, -COO-, -OCO-, -C₂F₄-, -CF=CF-, -CH= CHCH₂O-,
p 0, 1 oder 2,
q 0 oder 1,
(O)CᵥH₂ᵥ₊₁ OCᵥH₂ᵥ₊₁ oder CᵥH₂ᵥ₊₁, und
v 1 bis 6.
bedeuten.

16. Flüssigkristallines Medium nach einem oder mehreren der Ansprüche 1 bis 15, **dadurch gekennzeichnet, dass** es zusätzlich eine oder mehrere Verbindungen ausgewählt aus der Gruppe der Verbindungen der Formeln BC, CR, PH-1, PH-2, BF und BS, 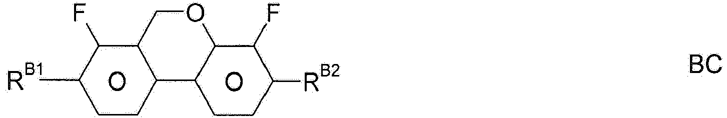 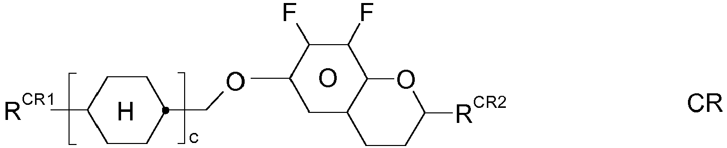 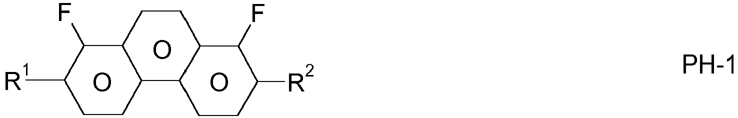 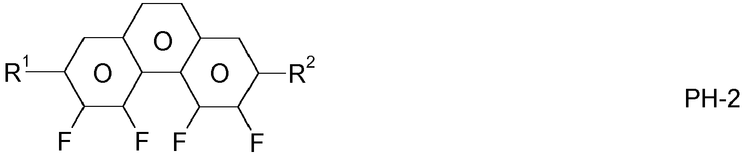 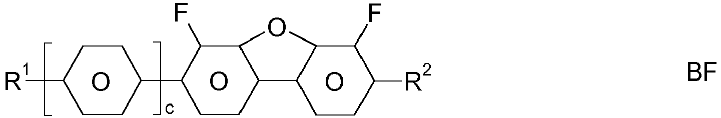 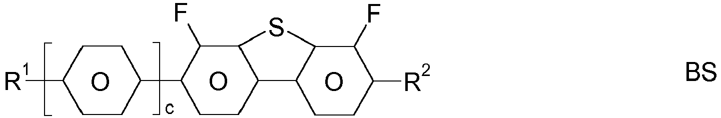 worin
R^{B1}, R^{B2}, R^{CR1}, R^{CR2}, R¹, R² jeweils unabhängig voneinander die Bedeutung von R^{2A} aufweisen und
c 0, 1 oder 2 bedeutet.

17. Flüssigkristallines Medium nach einem oder mehreren der Ansprüche 1 bis 16, **dadurch gekennzeichnet, dass** es 1-30 Gew.% an Verbindungen der Formel I enthält.

18. Flüssigkristallines Medium nach einem oder mehreren der Ansprüche 1 bis 17, **dadurch gekennzeichnet, dass** es zusätzlich einen oder mehrere UV-Stabilisatoren und/oder Antioxidantien enthält.

19. Flüssigkristallines Medium nach einem oder mehreren der Ansprüche 1 bis 18, **dadurch gekennzeichnet, dass** es zusätzlich eine oder mehrere polymerisierbare Verbindungen enthält.

20. Verfahren zur Herstellung eines flüssigkristallinen Mediums nach einem oder mehreren der Ansprüche 1 bis 19, **dadurch gekennzeichnet, dass** man eine oder mehrere Verbindungen der Formel I mit mindestens einer weiteren mesogenen Verbindung und optional mit einem oder mehreren Additiv(en) und/oder einer oder mehreren polymerisierbaren Verbindungen mischt.

21. Verwendung eines flüssigkristallinen Mediums nach einem oder mehreren der Ansprüche 1 bis 19 für elektrooptische Zwecke.

22. Verwendung des flüssigkristallinen Mediums nach Anspruch 21 in TN-, STN-, TN-TFT-, OCB-, IPS-, PS-IPS, FFS-, HB-FFS-, PS-FFS-Anzeigen, Shutter-Brillen für 3D-Effekte, LC-Linsen und positiven VA-Anzeigen.

23. Elektrooptische Flüssigkristallanzeige enthaltend ein flüssigkristallines Medium nach einem oder mehreren der Ansprüche 1 bis 19.

## Claims

1. Liquid-crystalline medium, **characterised in that** it comprises one or more compounds of the formula I, 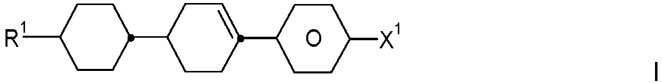 in which
R¹ denotes an alkyl or alkoxy radical having 1 to 15 C atoms, where, in addition, one or more CH₂ groups in these radicals may in each case be replaced, independently of one another, by -C≡C-, -CF₂O-, -CH=CH-, 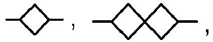 -O-, -CO-O- or -O-CO- in such a way that O atoms are not linked directly to one another, and in which, in addition, one or more H atoms may be replaced by halogen, and
X¹ denotes an alkyl radical having 1 to 5 C atoms, OCF₃, CF₃, CHF₂, OCHF₂, OCF₂CF₃, CCF₂CHFCF₃, OCF=CF₂, OCH=CF₂ or F,
and additionally comprises one or more compounds of the formulae II and/or III, 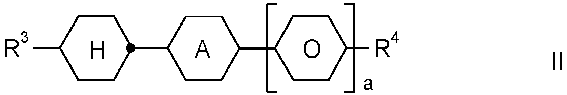 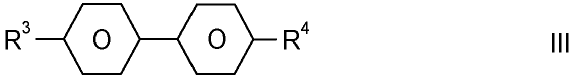 in which
A denotes 1,4-phenylene or trans-1,4-cyclohexylene,
a denotes 0 or 1,
R³ denotes alkenyl having 2 to 9 C atoms,
and
R⁴ has the meanings indicated for R¹ in Claim 1.

2. Liquid-crystalline medium according to Claim 1, **characterised in that** R¹ in formula I denotes a straight-chain alkyl radical, in which, in addition, one or more CH₂ groups may be replaced by -CH=CH-.

3. Liquid-crystalline medium according to Claim 1 or 2, **characterised in that** it comprises at least one compound from the group of the compounds of the formulae I-1 to I-5 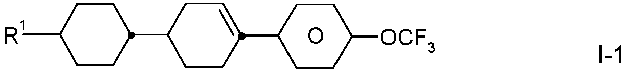 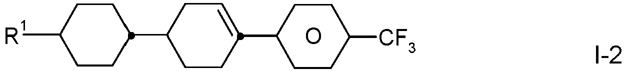 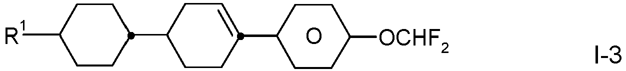 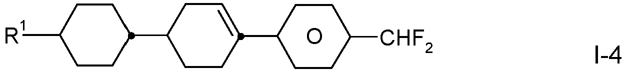 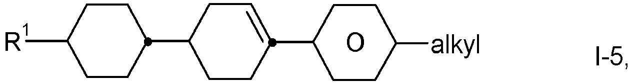 in which R¹ has the meanings indicated in Claim 1.

4. Liquid-crystalline medium according to one or more of Claims 1 to 3, **characterised in that** the compounds are selected from the formulae I-1 a to I-5k, 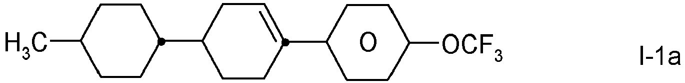 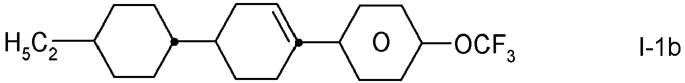 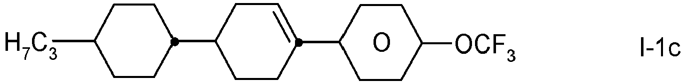 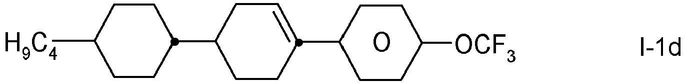 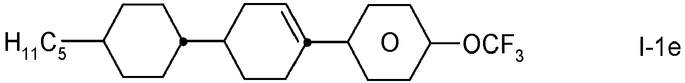 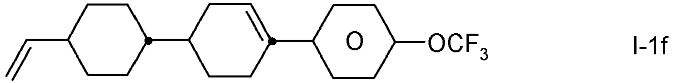 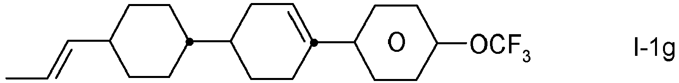 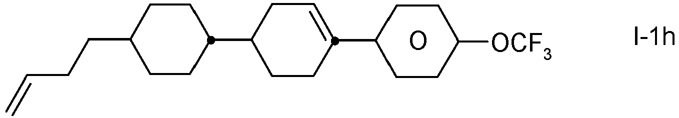 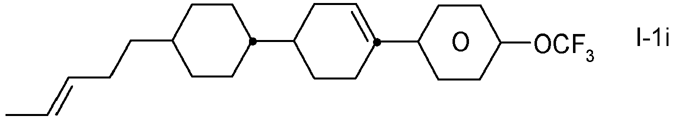 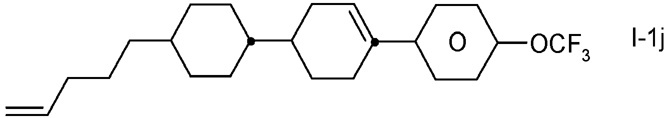 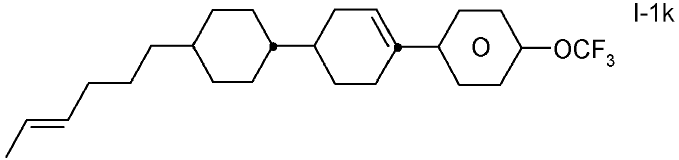 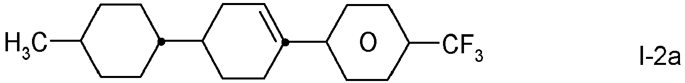 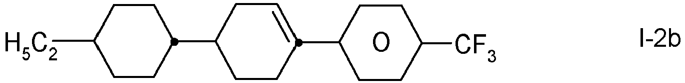 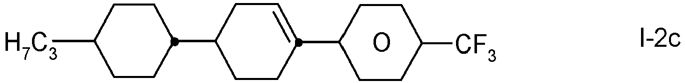 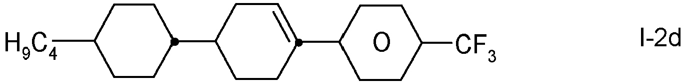 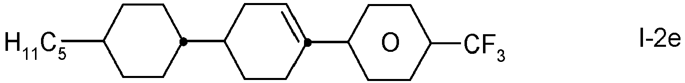 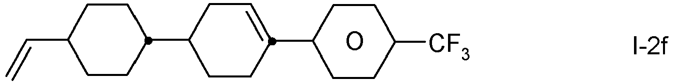 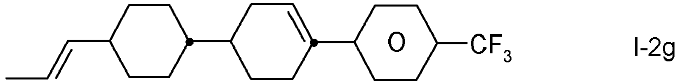 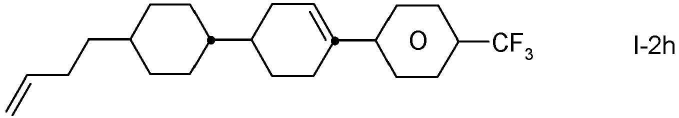 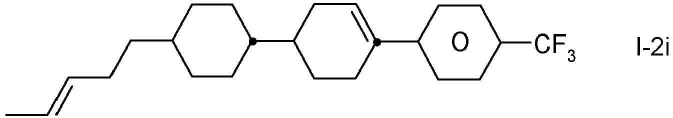 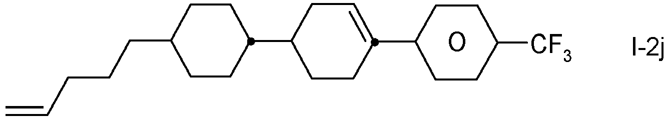 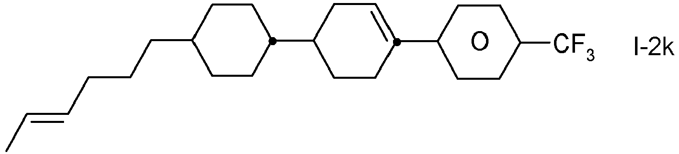 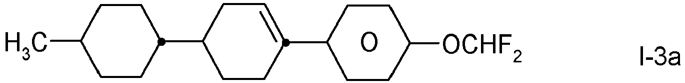 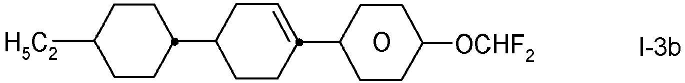 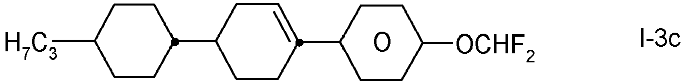 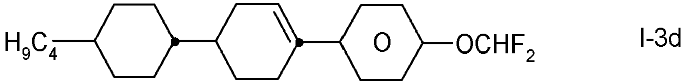 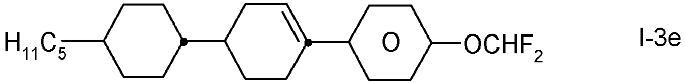 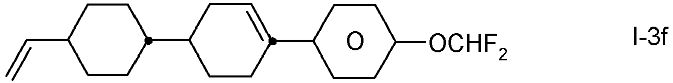 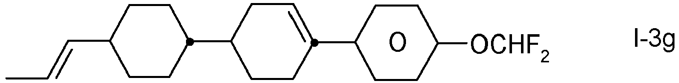 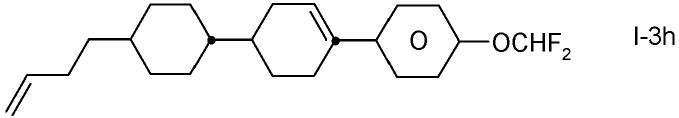 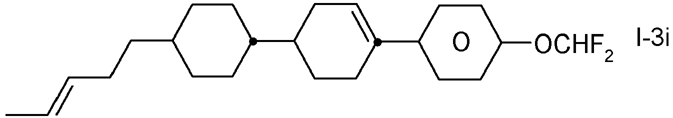 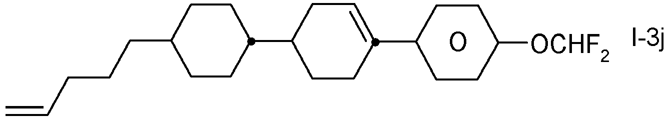 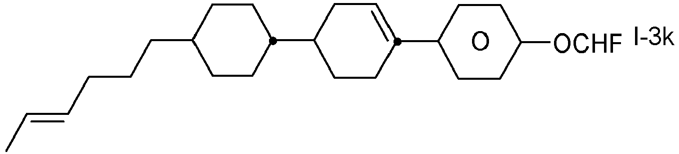 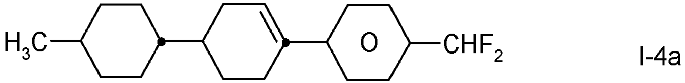 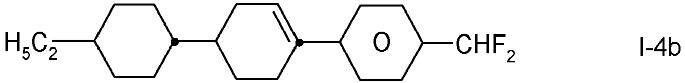 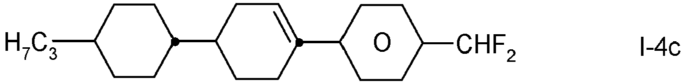 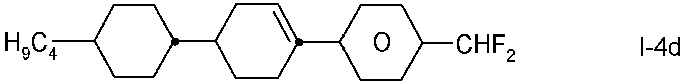 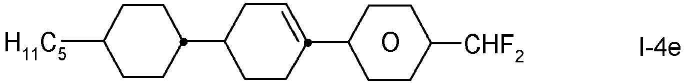 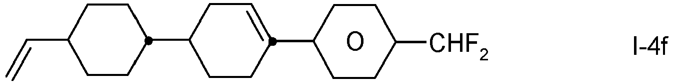 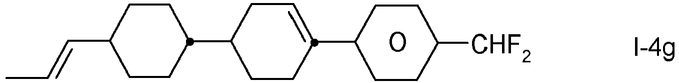 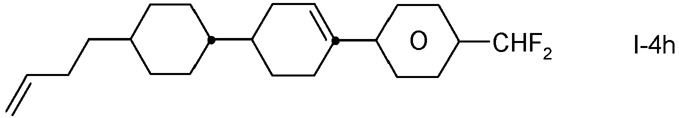 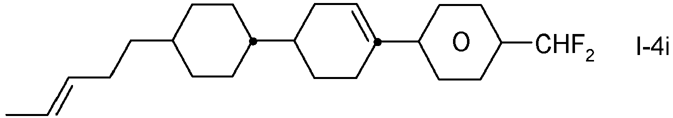 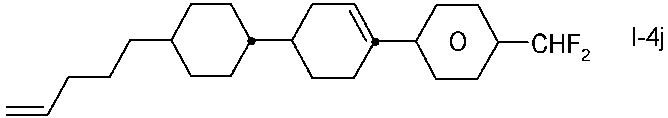 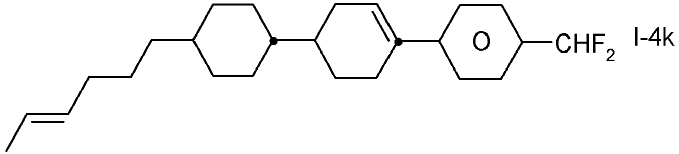 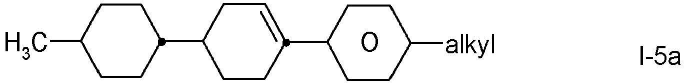 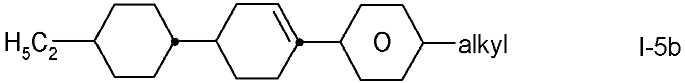 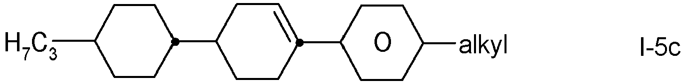 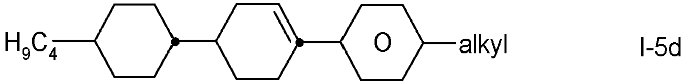 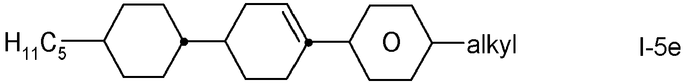 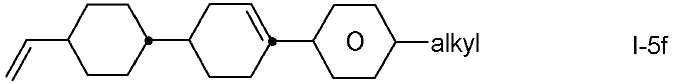 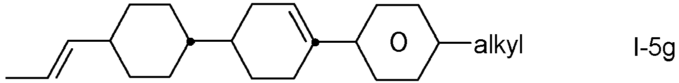 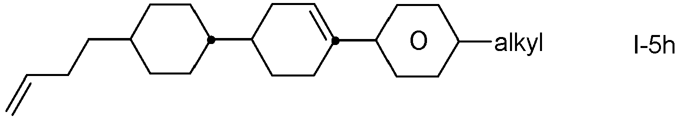 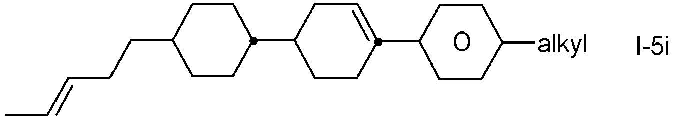 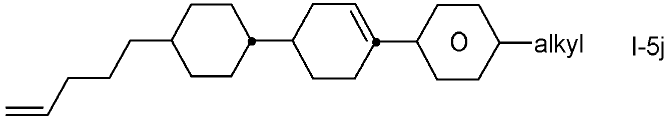 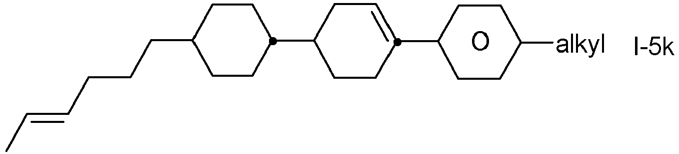 where alkyl denotes CH₃ or C₂H₅.

5. Liquid-crystalline medium according to one or more of Claims 1 to 4, **characterised in that** it additionally comprises one or more compounds selected from the compounds of the formulae, 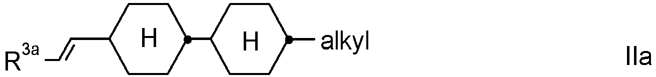 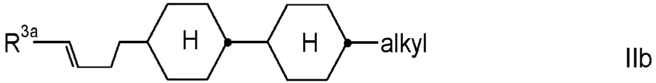 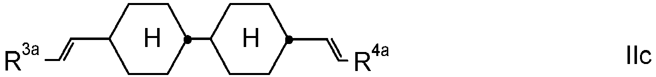 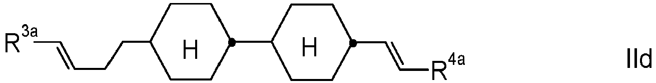 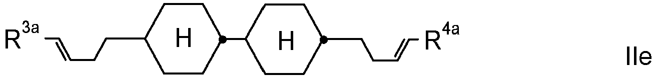 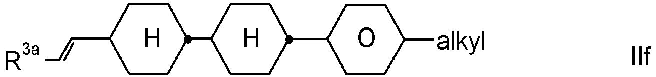 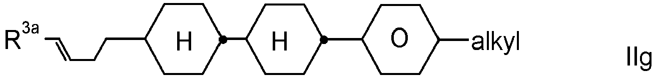 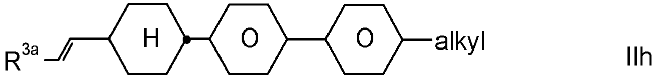 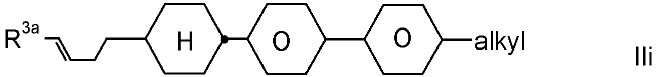 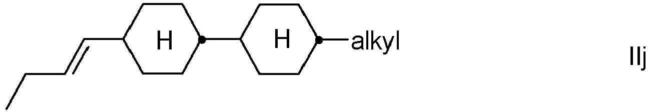 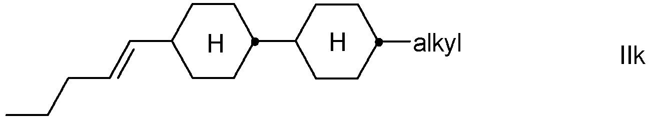 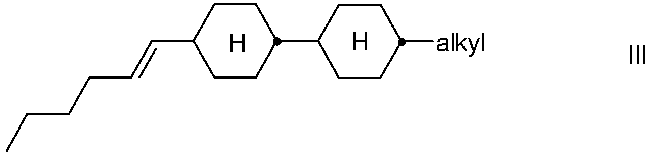 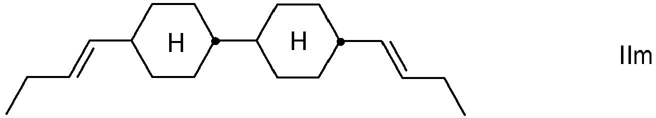 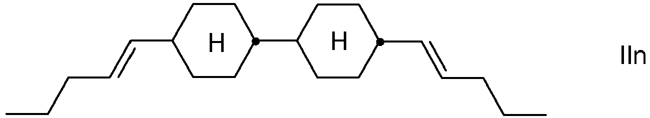 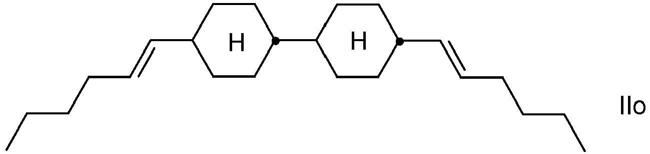 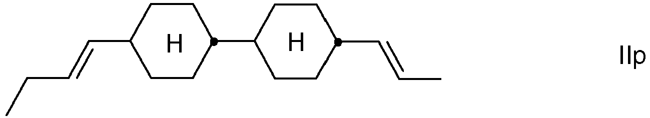 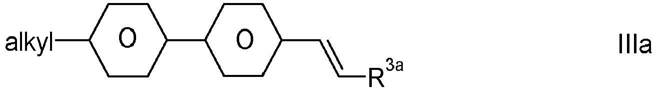 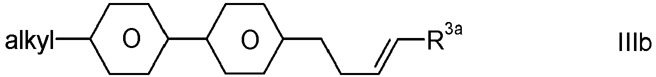 in which R^{3a} and R^{4a} in each case, independently of one another, denote H, CH₃, C₂H₅ or C₃H₇, and "alkyl" denotes a straight-chain alkyl group having 1 to 8 C atoms.

6. Liquid-crystalline medium according to one or more of Claims 1 to 5, **characterised in that** it additionally comprises one or more compounds selected from the compounds of the formulae IV to VIII, 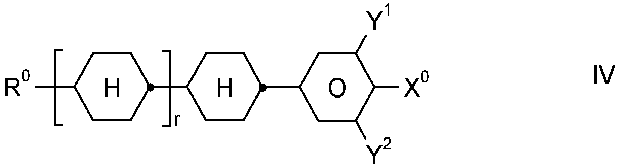 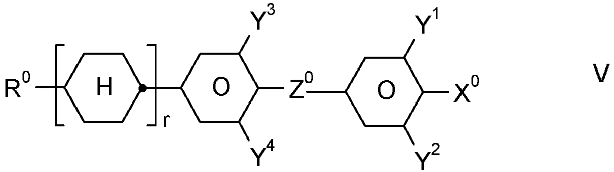 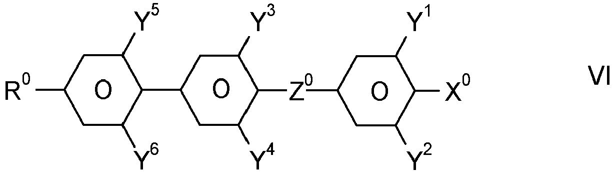 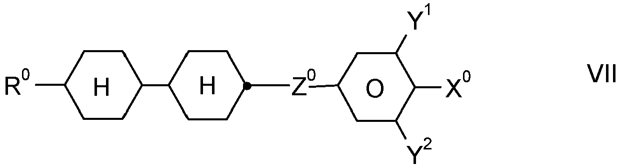 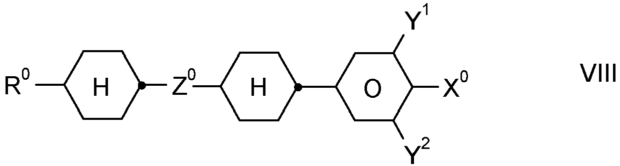 in which
R⁰ denotes an alkyl or alkoxy radical having 1 to 15 C atoms, where, in addition, one or more CH₂ groups in these radicals may in each case be replaced, independently of one another, by -C≡C-, -CF₂O-, -CH=CH-, 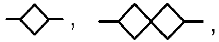 -O-, -CO-O- or -O-CO- in such a way that O atoms are not linked directly to one another, and in which, in addition, one or more H atoms may be replaced by halogen, or denotes cycloalkyl with 3 to 6 C atoms,
X⁰ denotes F, Cl, a mono- or polyfluorinated alkyl or alkoxy radical having 1 to 6 C atoms, a mono- or polyfluorinated alkenyl or alkenyloxy radical having 2 to 6 C atoms,
Y¹⁻⁶ in each case, independently of one another, denote H or F,
Z⁰ denotes -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- or -OCF₂-, in the formulae V and VI also a single bond, and
r denotes 0 or 1.

7. Liquid-crystalline medium according to one or more of Claims 1 to 6, **characterised in that** it additionally comprises one or more compounds selected from the compounds of the formulae Va to Vj, 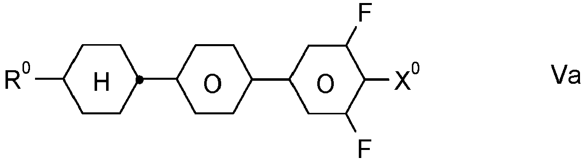 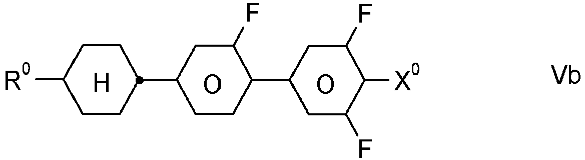 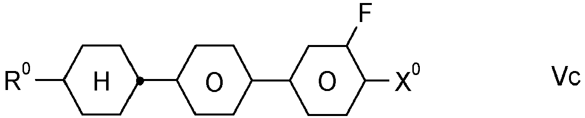 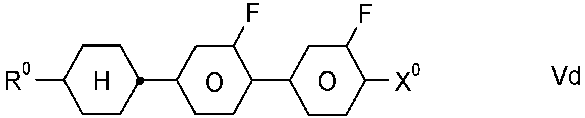 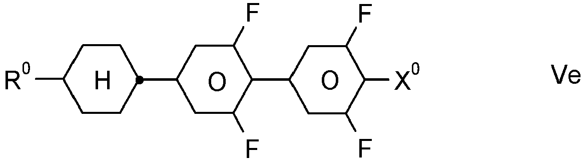 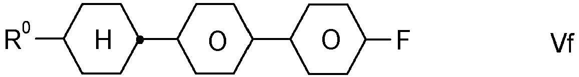 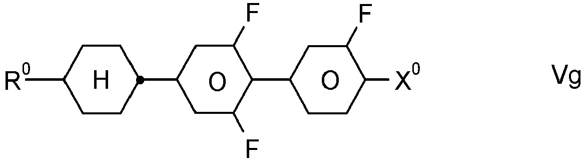 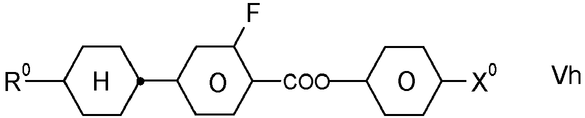 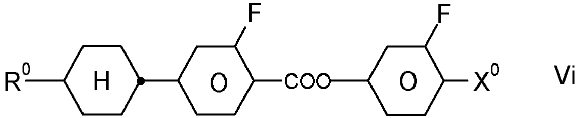 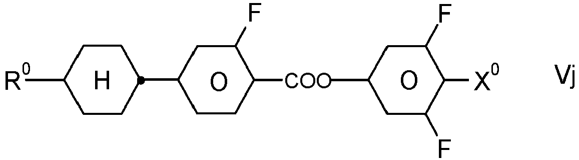 in which R⁰ and X⁰ have the meanings indicated in Claim 6.

8. Liquid-crystalline medium according to one or more of Claims 1 to 7, **characterised in that** it additionally comprises one or more compounds selected from the compounds of the formulae VI-1a to VI-1d, 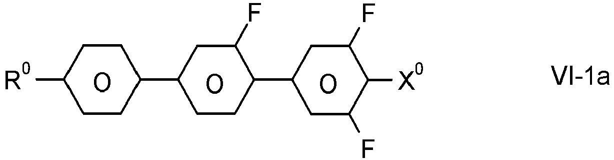 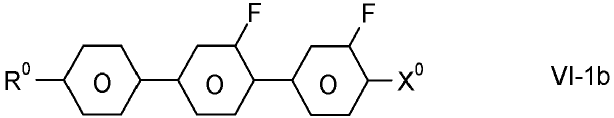 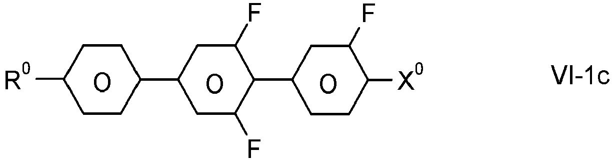 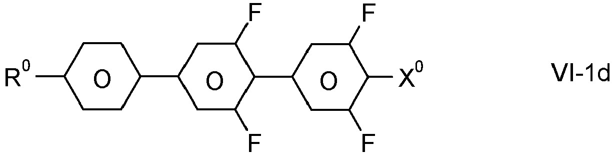 in which R⁰ and X⁰ have the meanings indicated in Claim 6.

9. Liquid-crystalline medium according to one or more of Claims 1 to 8, **characterised in that** it additionally comprises one or more compounds selected from the compounds of the formulae VI-2a to VI-2f, 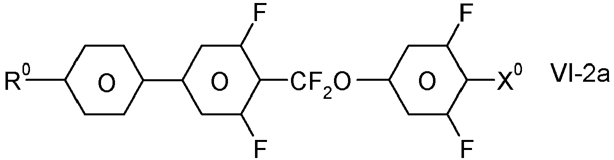 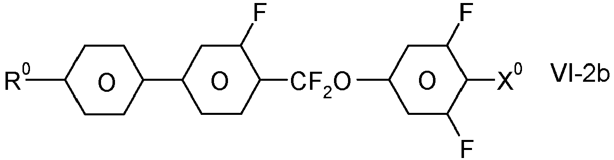 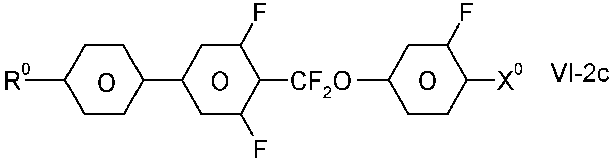 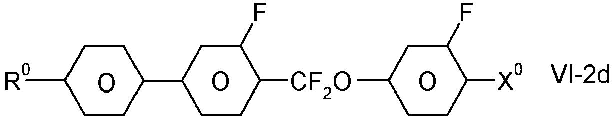 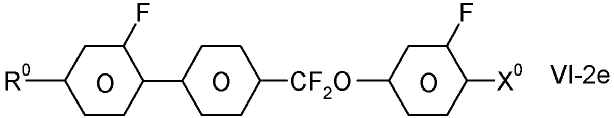 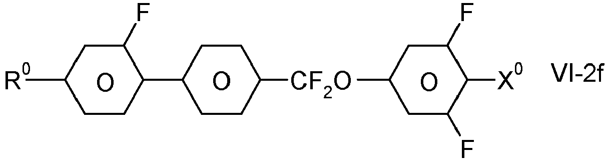 in which R⁰ and X⁰ have the meanings indicated in Claim 6.

10. Liquid-crystalline medium according to one or more of Claims 1 to 9, **characterised in that** it additionally comprises one or more compounds selected from the compounds of the formulae X and/or XI, 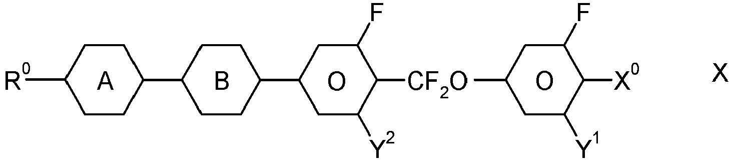 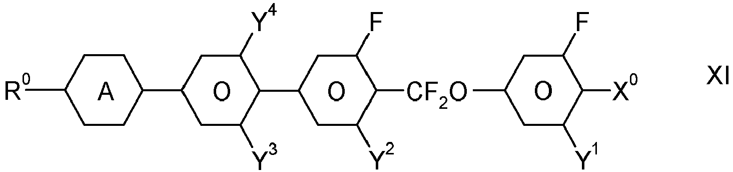 in which
R⁰ and X⁰ have the meanings indicated in Claim 6,
Y¹⁻⁴ in each case, independently of one another, denote H or F, and 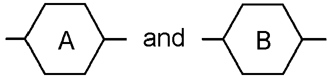
in each case, independently of one another, denote

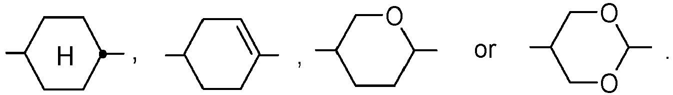

11. Liquid-crystalline medium according to one or more of Claims 1 to 10, **characterised in that** it additionally comprises one or more compounds selected from the compounds of the formula XII, 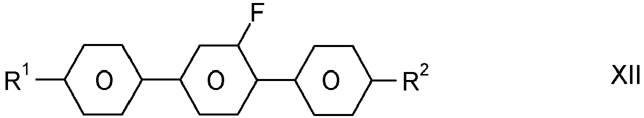 in which
R¹ and R² in each case, independently of one another, denote alkyl, alkenyl, alkoxy, oxaalkyl, fluoroalkyl or alkenyloxy, each having up to 9 C atoms, and Y¹ denotes H or F.

12. Liquid-crystalline medium according to one or more of Claims 1 to 11, **characterised in that** it additionally comprises one or more compounds selected from the compounds of the formulae XIII to XVI, 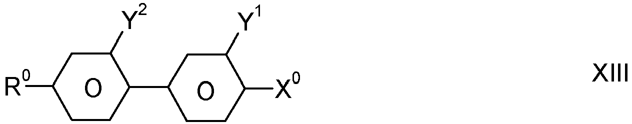 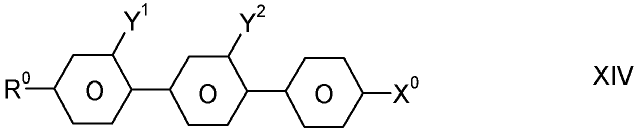 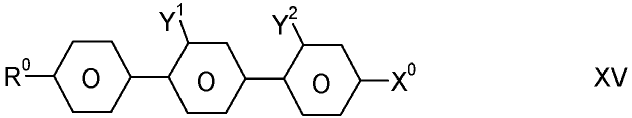 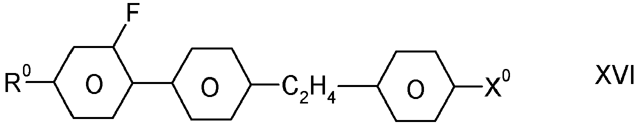 in which R⁰, X⁰, Y¹ and Y² have the meanings indicated in Claim 6.

13. Liquid-crystalline medium according to one or more of Claims 1 to 12, **characterised in that** it additionally comprises one or more compounds selected from the group of the compounds of the formulae

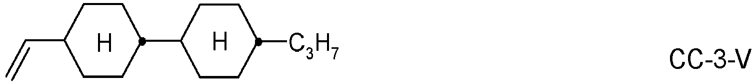

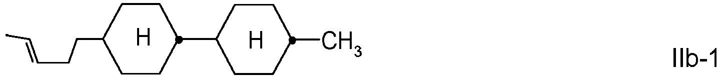

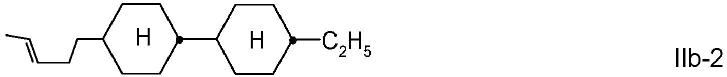

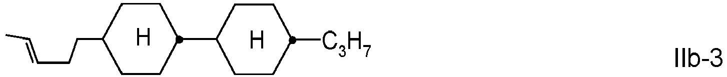

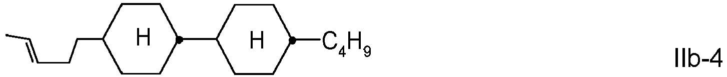

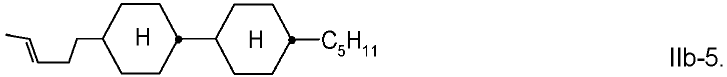

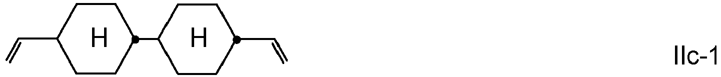

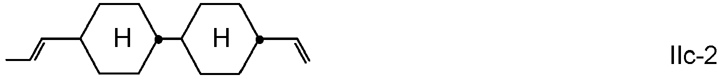

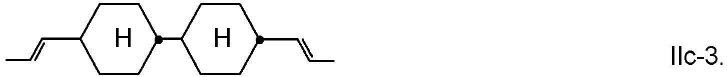

14. Liquid-crystalline medium according to one or more of Claims 1 to 13, **characterised in that** it additionally comprises one or more compounds selected from the group of the compounds of the formulae D1, D2, D3, D4 and D5, 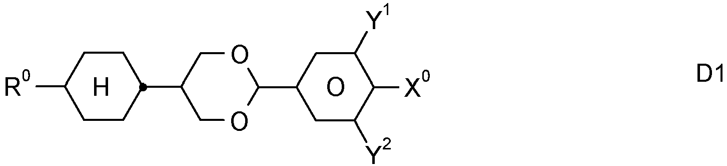 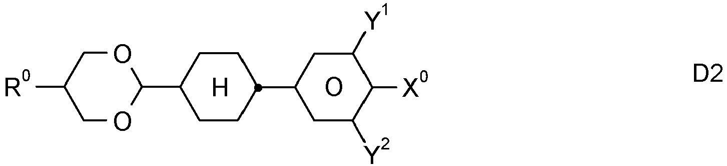 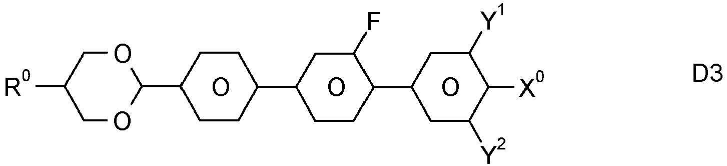 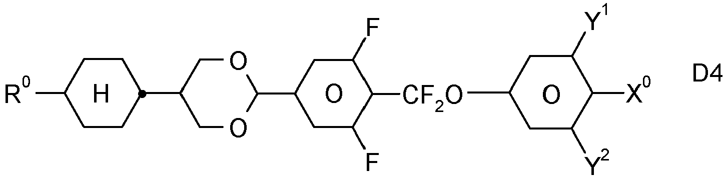 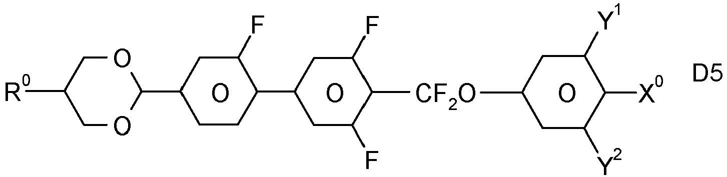 in which Y¹, Y², R⁰ and X⁰ have the meanings indicated in Claim 6.

15. Liquid-crystalline medium according to one or more of Claims 1 to 14, **characterised in that** it additionally comprises one or more compounds selected from the group of the compounds of the formulae Y-1, Y-2, Y-3 and Y-4, 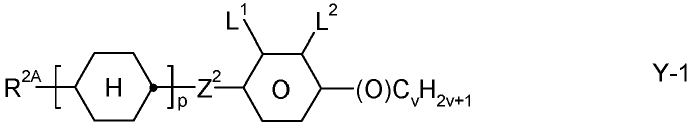 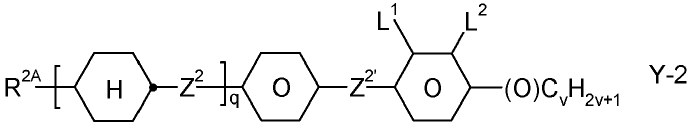 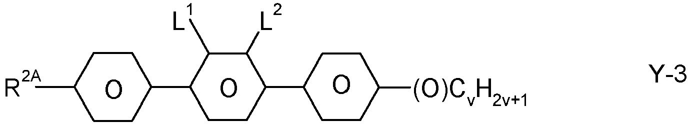 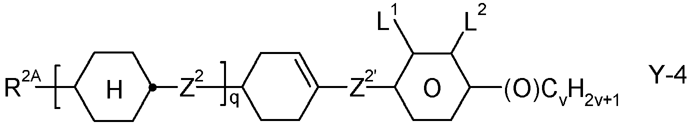 in which
R^{2A} denotes H, an alkyl or alkoxy radical having 1 to 15 C atoms, where, in addition, one or more CH₂ groups in these radicals may in each case be replaced, independently of one another, by -CH=CH-, -C≡C-, -CF₂O-, -CH=CH-, 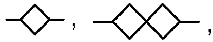 -O-, -CO-O- or -O-CO- in such a way that O atoms are not linked directly to one another, and in which, in addition, one or more H atoms may be replaced by halogen,
L¹ and L² in each case, independently of one another, denote F, Cl, CF₃ or CHF₂, preferably in each case denote F,
Z² and Z^{2'} in each case, independently of one another, denote a single bond, -CH₂CH₂-, -CH=CH-, -CF₂O-, -OCF₂-, -CH₂O-, -OCH₂-, -COO-, -OCO-, -C₂F₄-, -CF=CF- or -CH=CHCH₂O-,
p denotes 0, 1 or 2,
q denotes 0 or 1,
(O)CᵥH₂ᵥ₊₁ denotes OCᵥH₂ᵥ₊₁ or CᵥH₂ᵥ₊₁, and
v denotes 1 to 6.

16. Liquid-crystalline medium according to one or more of Claims 1 to 15, **characterised in that** it additionally comprises one or more compounds selected from the group of the compounds of the formulae BC, CR, PH-1, PH-2, BF and BS, 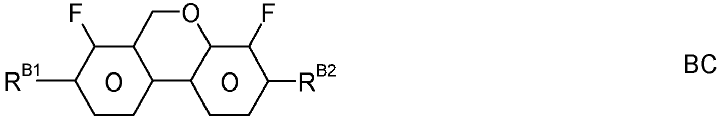 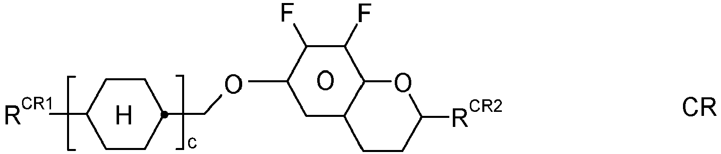 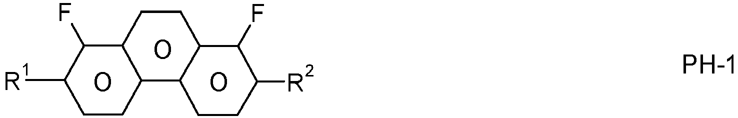 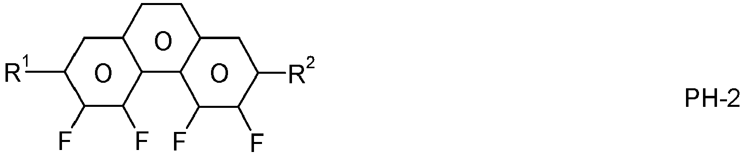 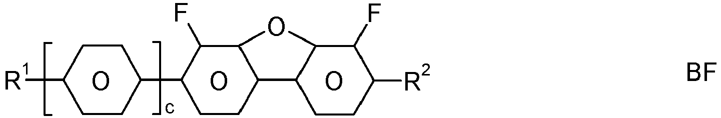 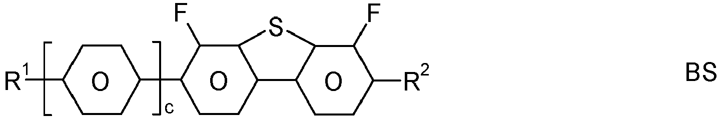 in which
R^{B1}, R^{B2}, R^{CR1}, R^{CR2}, R¹ and R² in each case, independently of one another, have the meaning of R^{2A} and
c denotes 0, 1 or 2.

17. Liquid-crystalline medium according to one or more of Claims 1 to 16, **characterised in that** it comprises 1-30% by weight of compounds of the formula I.

18. Liquid-crystalline medium according to one or more of Claims 1 to 17, **characterised in that** it additionally comprises one or more UV stabilisers and/or antioxidants.

19. Liquid-crystalline medium according to one or more of Claims 1 to 18, **characterised in that** it additionally comprises one or more polymerisable compounds.

20. Process for the preparation of a liquid-crystalline medium according to one or more of Claims 1 to 19, **characterised in that** one or more compounds of the formula I are mixed with at least one further mesogenic compound and optionally with one or more additive(s) and/or one or more polymerisable compounds.

21. Use of a liquid-crystalline medium according to one or more of Claims 1 to 19 for electro-optical purposes.

22. Use of the liquid-crystalline medium according to one or more of Claims 1 to 19 in TN, STN, TN-TFT, OCB, IPS, PS-IPS, FFS, HB-FFS and PS-FFS displays, shutter spectacles for 3D effects, LC lenses and positive VA displays.

23. Electro-optical liquid-crystal display containing a liquid-crystalline medium according to one or more of Claims 1 to 19.

## Revendications

1. Milieu cristallin liquide, **caractérisé en ce qu'**il comprend un ou plusieurs composé(s) de la formule I, 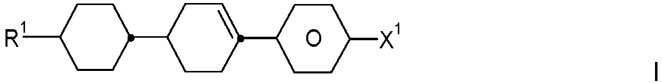 dans laquelle
R¹ représente un radical alkyle ou alcoxy qui comporte de 1 à 15 atome(s) de C, où, en outre, un ou plusieurs groupe(s) CH₂ dans ces radicaux peut/peuvent dans chaque cas être remplacé(s), de manière indépendante les uns des autres, par -C≡C-, -CF₂O-, -CH=CH-, 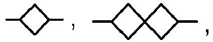 -O-, -CO-O- ou -O-CO- de telle sorte que des atomes de O ne soient pas liés directement les uns aux autres, et où, en outre, un ou plusieurs atome(s) de H peut/peuvent être remplacé(s) par halogène, et
X¹ représente un radical alkyle qui comporte de 1 à 5 atome(s) de C, OCF₃, CF₃, CHF₂, OCHF₂, OCF₂CF₃, CCF₂CHFCF₃, OCF=CF₂, OCH=CF₂ ou F,
et **en ce qu'**il comprend de façon additionnelle un ou plusieurs composé(s) des formules II et/ou III, 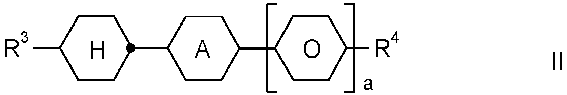 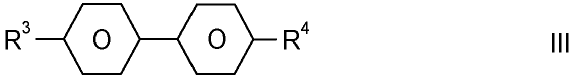 dans lesquelles
A représente 1,4-phénylène ou trans-1,4-cyclohexylène,
a représente 0 ou 1,
R³ représente alkényle qui comporte de 2 à 9 atomes de C,
et
R⁴ présente les significations qui ont été indiquées pour R¹ selon la revendication 1.

2. Milieu cristallin liquide selon la revendication 1, **caractérisé en ce que** R¹ dans la formule I représente un radical alkyle en chaîne droite, où, en outre, un ou plusieurs groupe(s) CH₂ peut/peuvent être remplacé(s) par -CH=CH-.

3. Milieu cristallin liquide selon la revendication 1 ou 2, **caractérisé en ce qu'**il comprend au moins un composé pris parmi le groupe des composés des formules I-1 à I-5 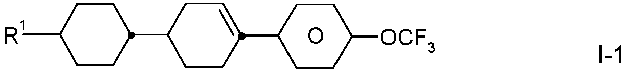 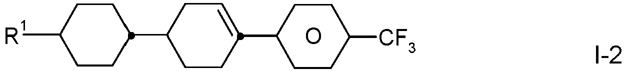 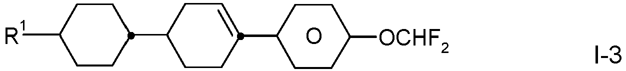 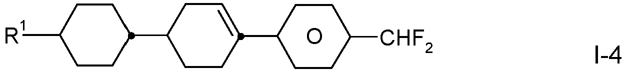 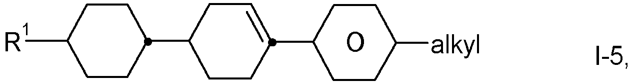 dans lesquelles R¹ présente les significations qui ont été indiquées selon la revendication 1.

4. Milieu cristallin liquide selon une ou plusieurs des revendications 1 à 3, **caractérisé en ce que** les composés sont sélectionnés parmi les formules I-1a à I-5k, 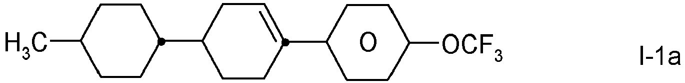 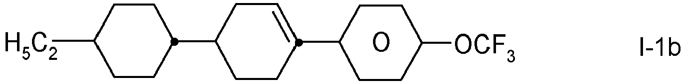 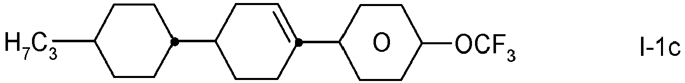 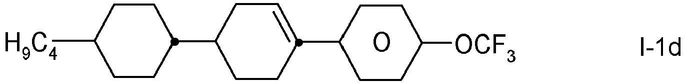 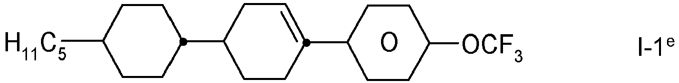 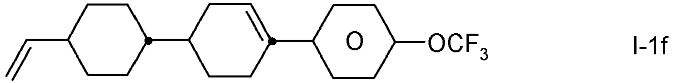 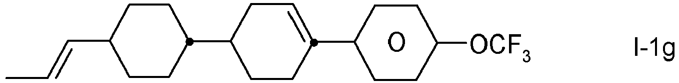 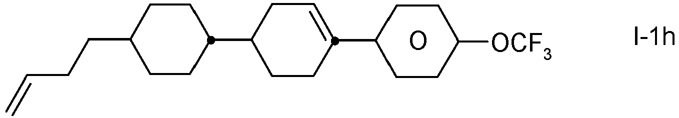 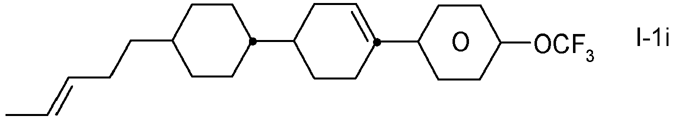 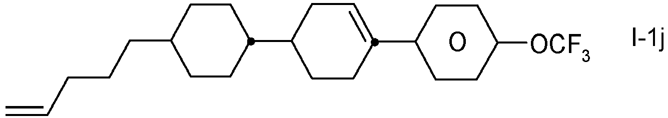 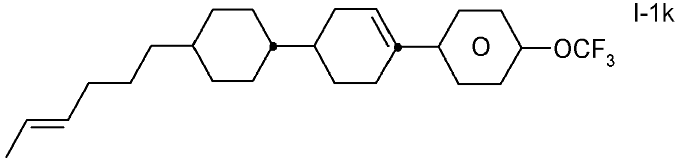 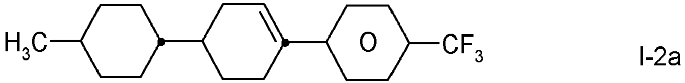 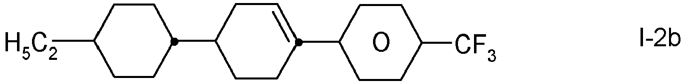 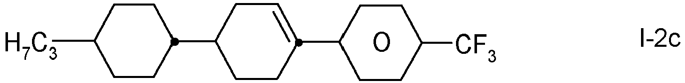 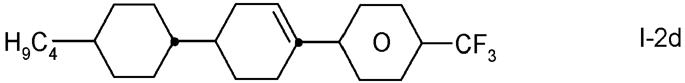 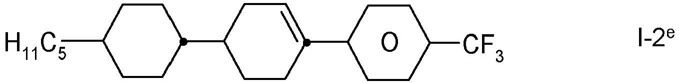 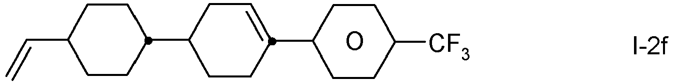 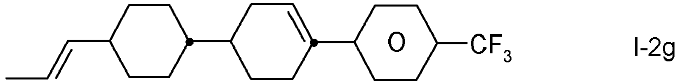 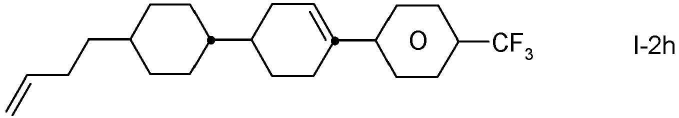 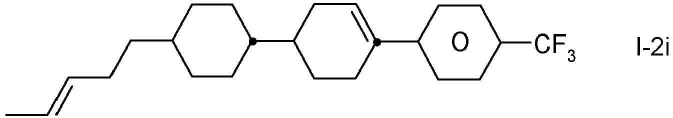 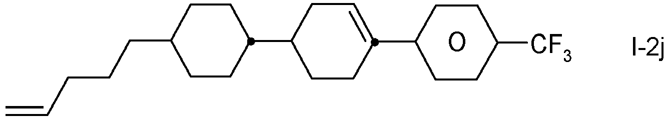 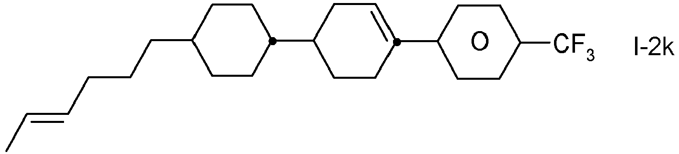 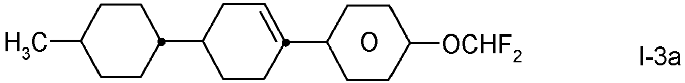 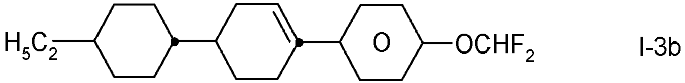 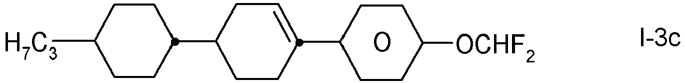 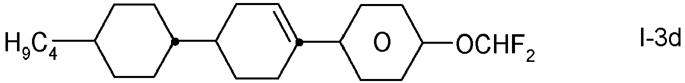 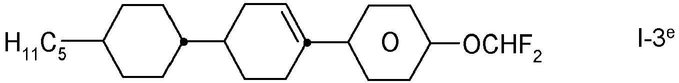 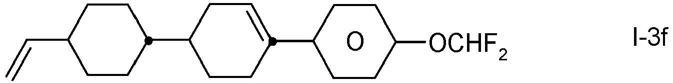 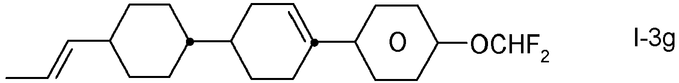 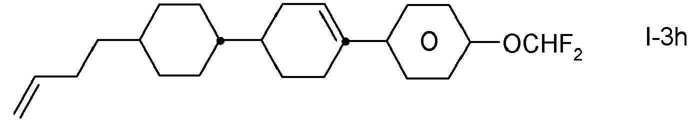 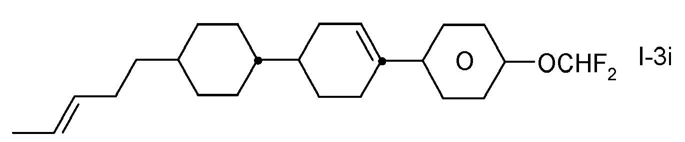 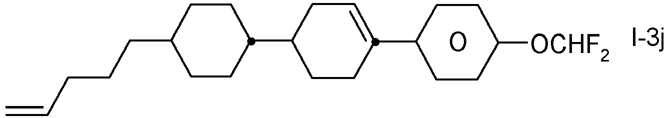 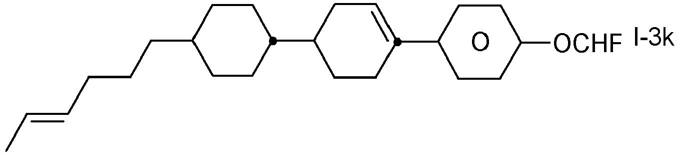 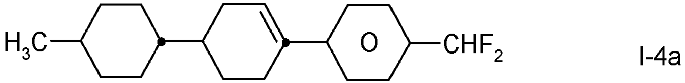 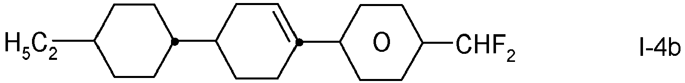 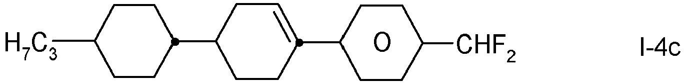 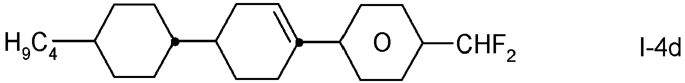 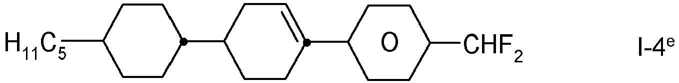 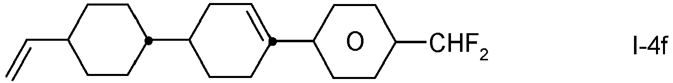 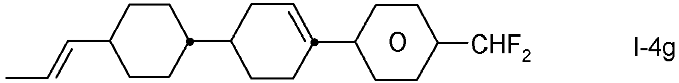 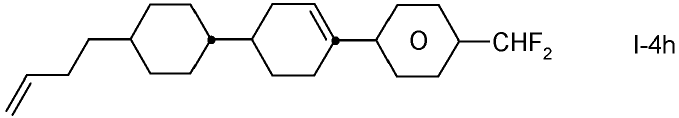 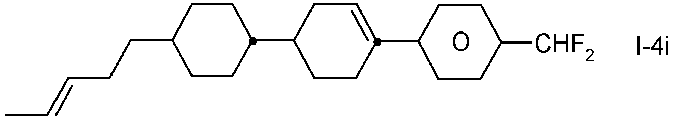 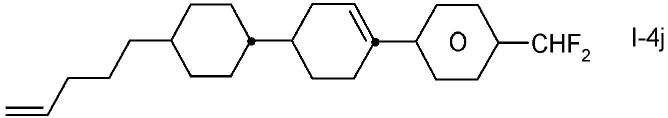 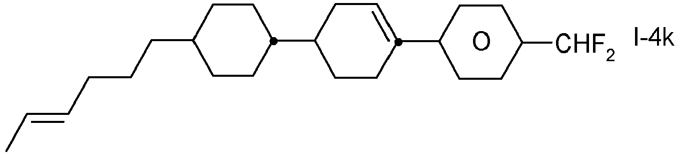 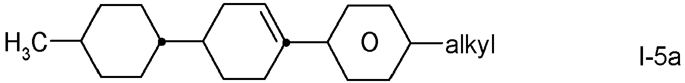 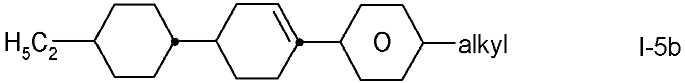 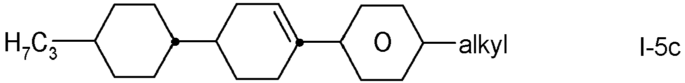 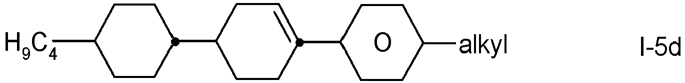 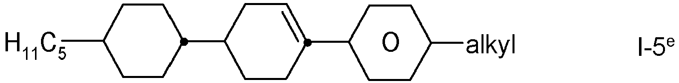 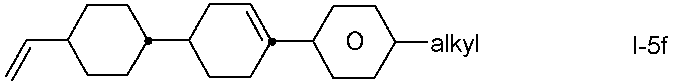 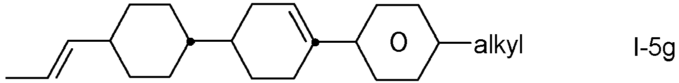 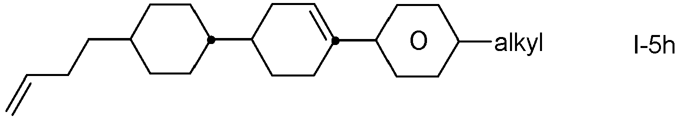 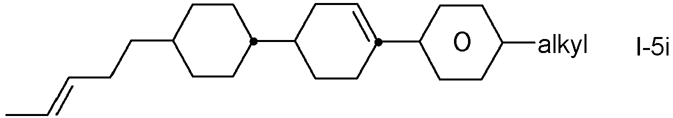 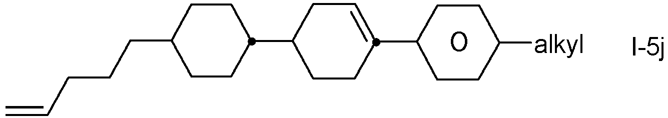 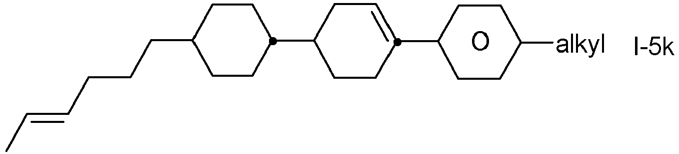 dans lesquelles alkyl représente CH₃ ou C₂H₅.

5. Milieu cristallin liquide selon une ou plusieurs des revendications 1 à 4, **caractérisé en ce qu'**il comprend de façon additionnelle un ou plusieurs composé(s) qui est/sont sélectionné(s) parmi les composés des formules, 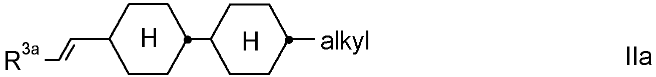 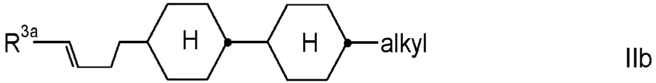 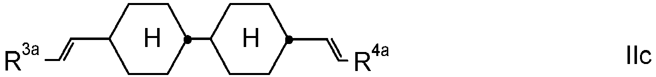 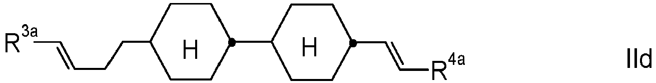 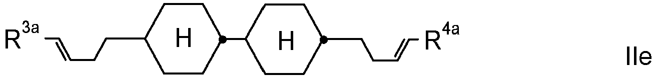 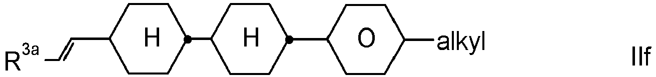 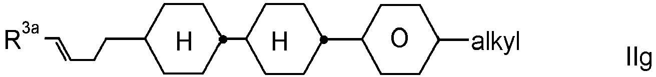 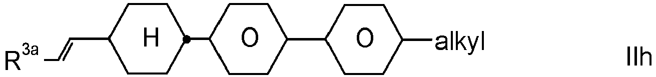 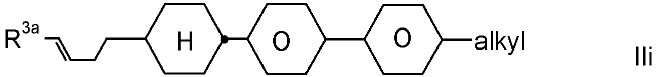 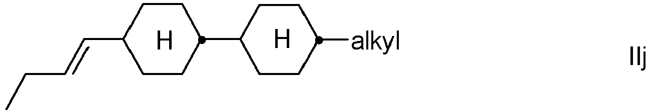 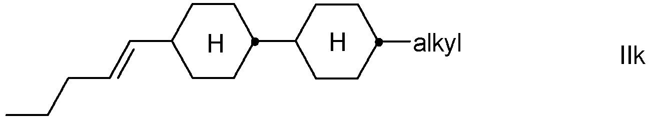 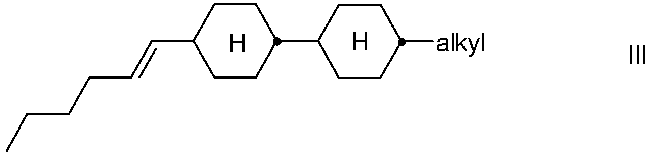 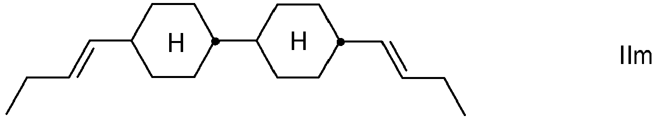 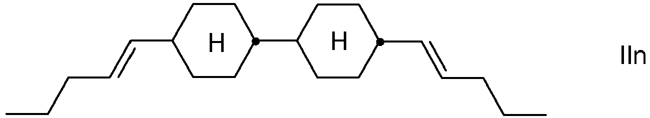 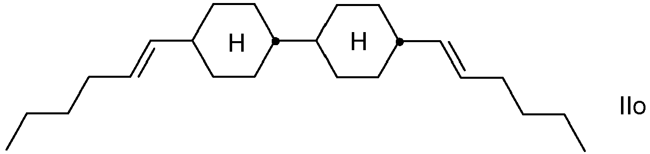 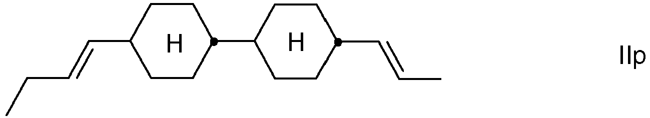 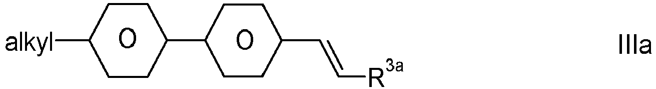 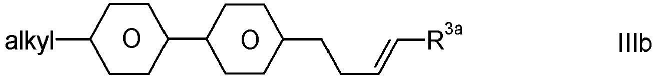 dans lesquelles R^{3a} et R^{4a} représentent dans chaque cas, de manière indépendante l'un de l'autre, H, CH₃, C₂H₅ ou C₃H₇, et "alkyl" représente un groupe alkyle en chaîne droite qui comporte de 1 à 8 atome(s) de C.

6. Milieu cristallin liquide selon une ou plusieurs des revendications 1 à 5, **caractérisé en ce qu'**il comprend de façon additionnelle un ou plusieurs composé(s) qui est/sont sélectionné(s) parmi les composés des formules IV à VIII, 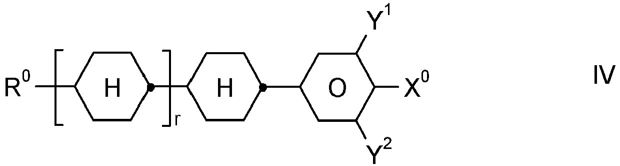 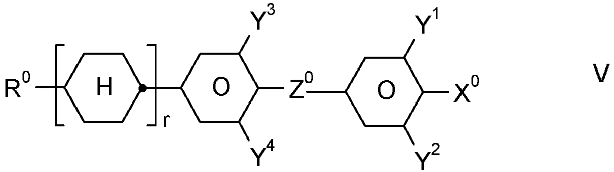 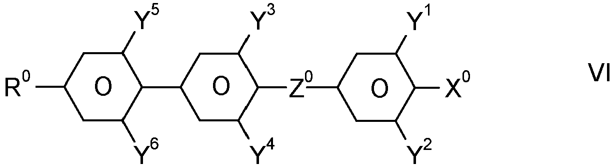 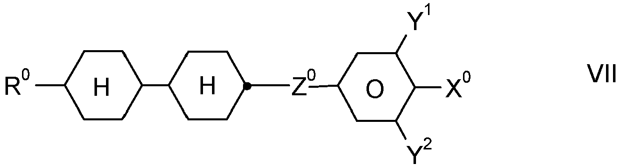 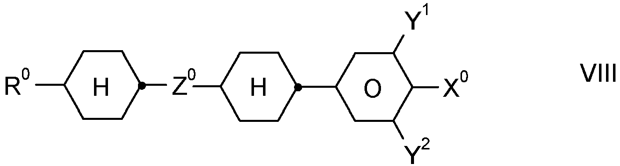 dans lesquelles
R⁰ représente un radical alkyle ou alcoxy qui comporte de 1 à 15 atome(s) de C, où, en outre, un ou plusieurs groupe(s) CH₂ dans ces radicaux peut/peuvent dans chaque cas être remplacé(s), de manière indépendante les uns des autres, par -C≡C-, -CF₂O-, -CH=CH-, 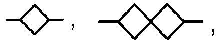 -O-, -CO-O- ou -O-CO- de telle sorte que des atomes de O ne soient pas liés directement les uns aux autres, et où, en outre, un ou plusieurs atome(s) de H peut/peuvent être remplacé(s) par halogène, ou représente cycloalkyle qui comporte de 3 à 6 atomes de C,
X⁰ représente F, Cl, un radical alkyle ou alcoxy mono- ou polyfluoré qui comporte de 1 à 6 atome(s) de C, un radical alkényle ou alkényloxy mono- ou polyfluoré qui comporte de 2 à 6 atomes de C,
Y¹⁻⁶ représentent dans chaque cas, de manière indépendante les uns des autres, H ou F,
Z⁰ représente -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O-ou -OCF₂-, dans les formules V et VI également une liaison simple, et
r représente 0 ou 1.

7. Milieu cristallin liquide selon une ou plusieurs des revendications 1 à 6, **caractérisé en ce qu'**il comprend de façon additionnelle un ou plusieurs composé(s) qui est/sont sélectionné(s) parmi les composés des formules Va à Vj, 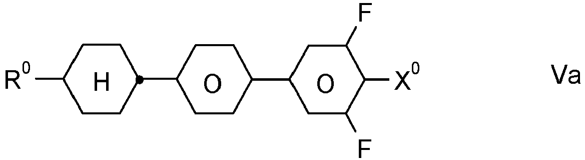 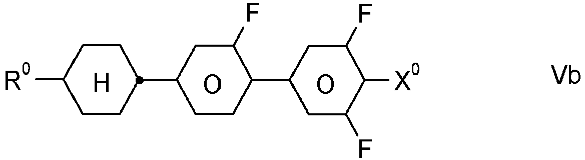 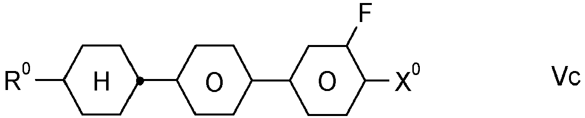 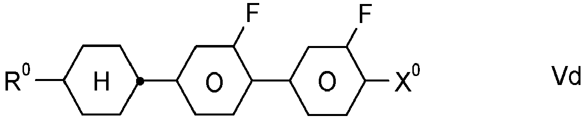 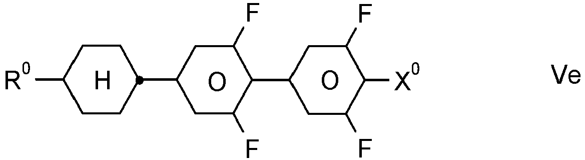 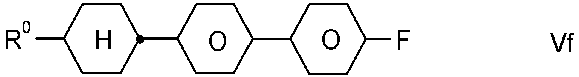 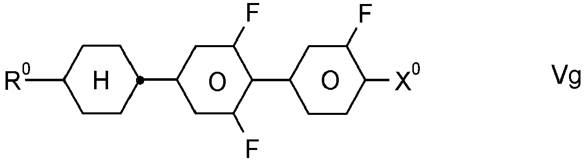 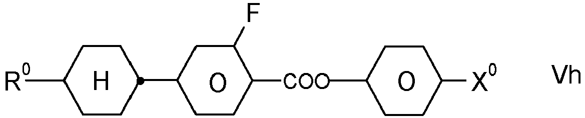 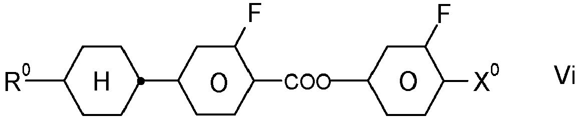 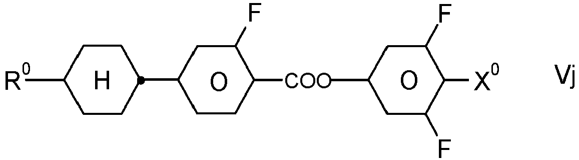 dans lesquelles R⁰ et X⁰ présentent les significations qui ont été indiquées selon la revendication 6.

8. Milieu cristallin liquide selon une ou plusieurs des revendications 1 à 7, **caractérisé en ce qu'**il comprend de façon additionnelle un ou plusieurs composé(s) qui est/sont sélectionné(s) parmi les composés des formules VI-1a à VI-1d, 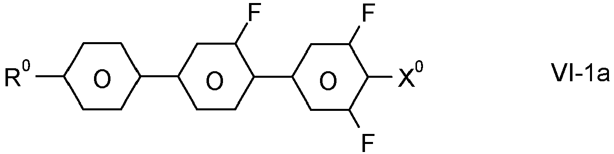 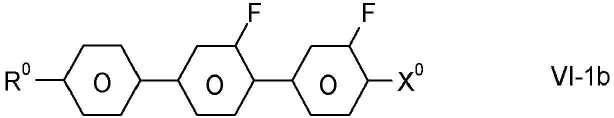 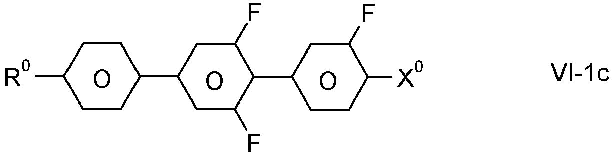 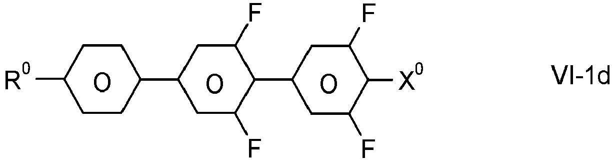 dans lesquelles R⁰ et X⁰ présentent les significations qui ont été indiquées selon la revendication 6.

9. Milieu cristallin liquide selon une ou plusieurs des revendications 1 à 8, **caractérisé en ce qu'**il comprend de façon additionnelle un ou plusieurs composé(s) qui est/sont sélectionné(s) parmi les composés des formules VI-2a à VI-2f, 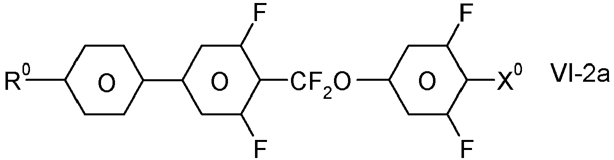 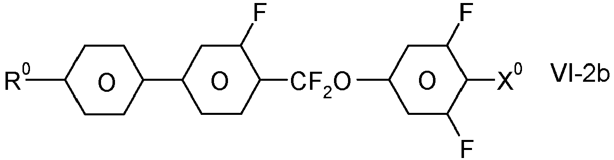 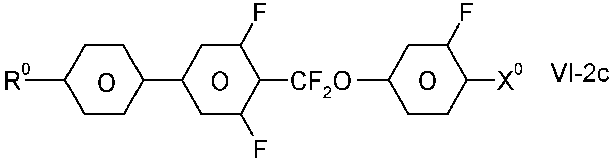 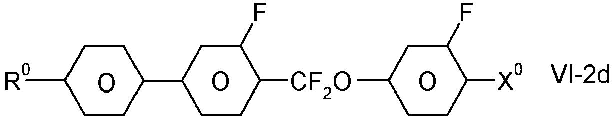 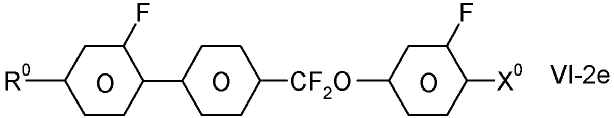 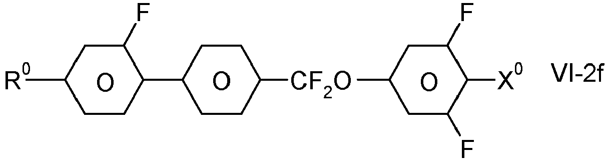 dans lesquelles R⁰ et X⁰ présentent les significations qui ont été indiquées selon la revendication 6.

10. Milieu cristallin liquide selon une ou plusieurs des revendications 1 à 9, **caractérisé en ce qu'**il comprend de façon additionnelle un ou plusieurs composé(s) qui est/sont sélectionné(s) parmi les composés des formules X et/ou XI, 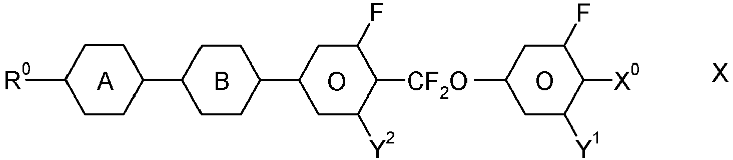 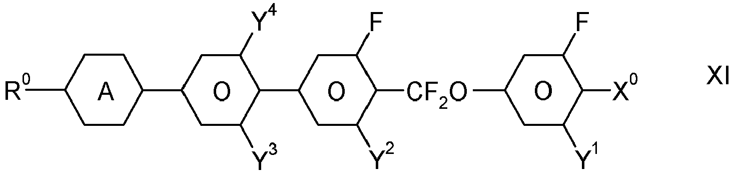 dans lesquelles
R⁰ et X⁰ présentent les significations qui ont été indiquées selon la revendication 6,
Y¹⁻⁴ représentent dans chaque cas, de manière indépendante les uns des autres, H ou F, et 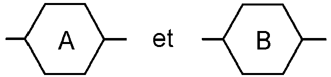 représentent dans chaque cas, de manière indépendante l'un de l'autre,

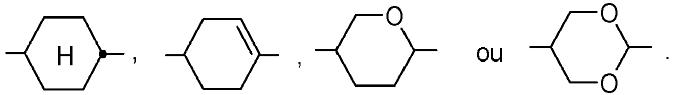

11. Milieu cristallin liquide selon une ou plusieurs des revendications 1 à 10, **caractérisé en ce qu'**il comprend de façon additionnelle un ou plusieurs composé(s) qui est/sont sélectionné(s) parmi les composés de la formule XII, 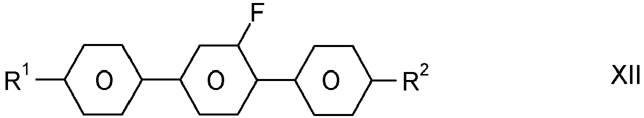 dans laquelle
R¹ et R² représentent dans chaque cas, de manière indépendante l'un de l'autre, alkyle, alkényle, alcoxy, oxaalkyle, fluoroalkyle ou alkényloxy, dont chacun comporte jusqu'à 9 atomes de C, et Y¹ représente H ou F.

12. Milieu cristallin liquide selon une ou plusieurs des revendications 1 à 11, **caractérisé en ce qu'**il comprend de façon additionnelle un ou plusieurs composé(s) qui est/sont sélectionné(s) parmi les composés des formules XIII à XVI, 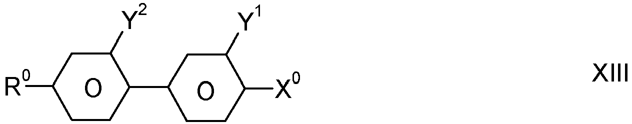 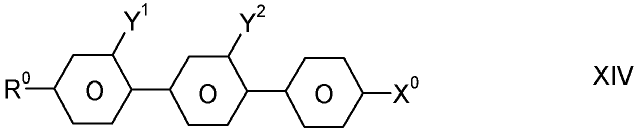 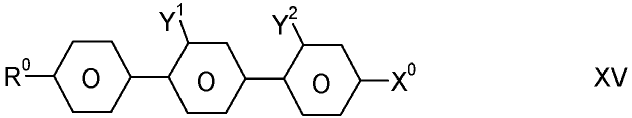 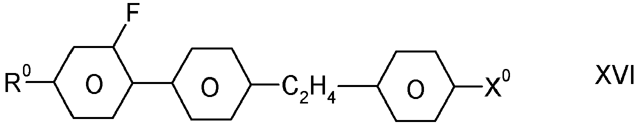 dans lesquelles R⁰, X⁰, Y¹ et Y² présentent les significations qui ont été indiquées selon la revendication 6.

13. Milieu cristallin liquide selon une ou plusieurs des revendications 1 à 12, **caractérisé en ce qu'**il comprend de façon additionnelle un ou plusieurs composé(s) qui est/sont sélectionné(s) parmi le groupe des composés des formules

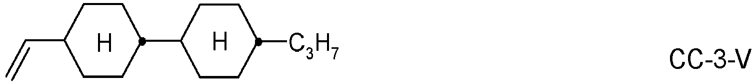

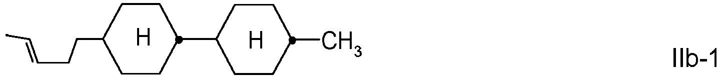

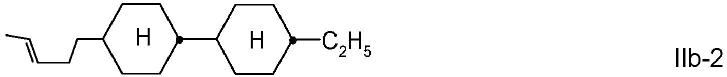

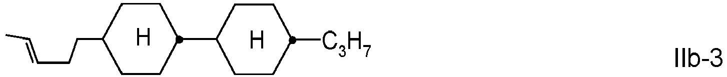

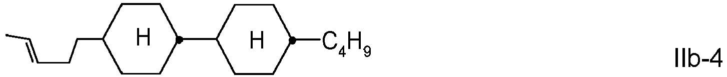

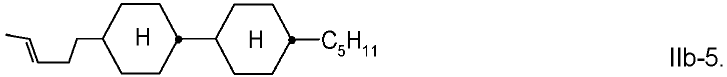

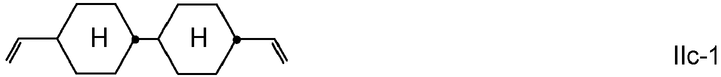

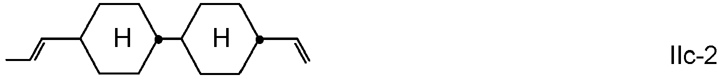

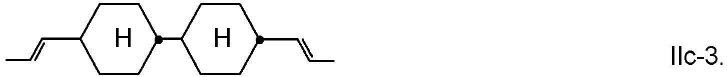

14. Milieu cristallin liquide selon une ou plusieurs des revendications 1 à 13, **caractérisé en ce qu'**il comprend de façon additionnelle un ou plusieurs composé(s) qui est/sont sélectionné(s) parmi le groupe des composés des formules D1, D2, D3, D4 et D5, 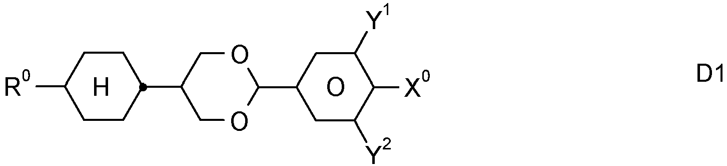 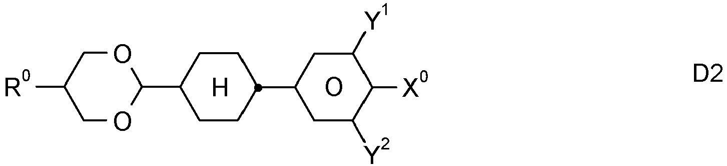 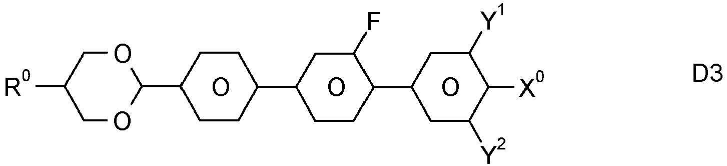 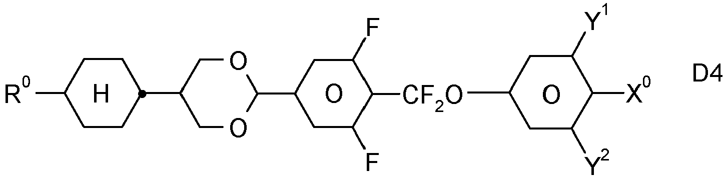 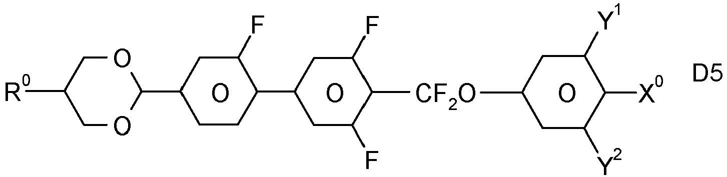 dans lesquelles Y¹, Y², R⁰ et X⁰ présentent les significations qui ont été indiquées selon la revendication 6.

15. Milieu cristallin liquide selon une ou plusieurs des revendications 1 à 14, **caractérisé en ce qu'**il comprend de façon additionnelle un ou plusieurs composé(s) qui est/sont sélectionné(s) parmi le groupe des composés des formules Y-1, Y-2, Y-3 et Y-4, 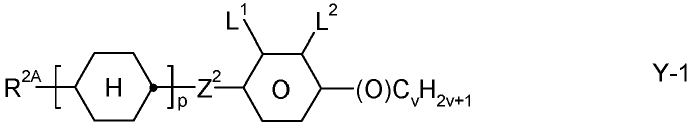 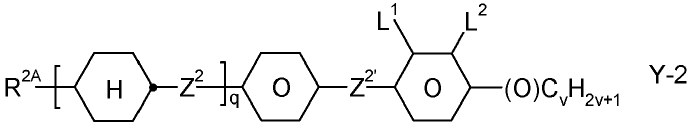 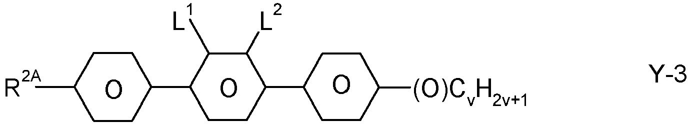 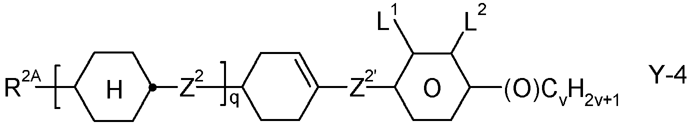 dans lesquelles
R^{2A} représente H, un radical alkyle ou alcoxy qui comporte de 1 à 15 atome(s) de C, où, en outre, un ou plusieurs groupe(s) CH₂ dans ces radicaux peut/peuvent dans chaque cas être remplacé(s), de manière indépendante les uns des autres, par -CH=CH-, -C≡C-, -CF₂O-, -CH=CH-, 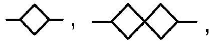 -O-, -CO-O- ou -O-CO-de telle sorte que des atomes de O ne soient pas liés directement les uns aux autres, et où, en outre, un ou plusieurs atome(s) de H peut/peuvent être remplacé(s) par halogène,
L¹ et L² représentent dans chaque cas, de manière indépendante l'un de l'autre, F, Cl, CF₃ ou CHF₂, représentent de préférence dans chaque cas F,
Z² et Z^{2'} représentent dans chaque cas, de manière indépendante l'un de l'autre, une liaison simple, -CH₂CH₂-, -CH=CH-, -CF₂O-, -OCF₂-, -CH₂O-, -OCH₂-, -COO-, -OCO-, -C₂F₄-, -CF=CF- ou -CH=CHCH₂O-,
p représente 0, 1 ou 2,
q représente 0 ou 1,
(O)CᵥH₂ᵥ₊₁ représente OCᵥH₂ᵥ₊₁ ou CᵥH₂ᵥ₊₁, et
v représente 1 à 6.

16. Milieu cristallin liquide selon une ou plusieurs des revendications 1 à 15, **caractérisé en ce qu'**il comprend de façon additionnelle un ou plusieurs composé(s) qui est/sont sélectionné(s) parmi le groupe des composés des formules BC, CR, PH-1, PH-2, BF et BS, 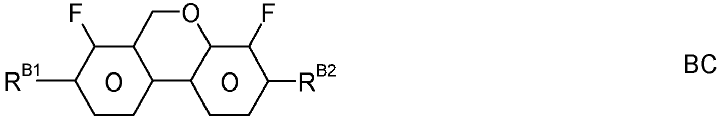 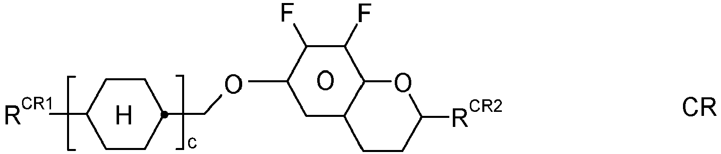 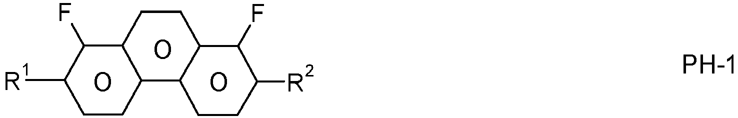 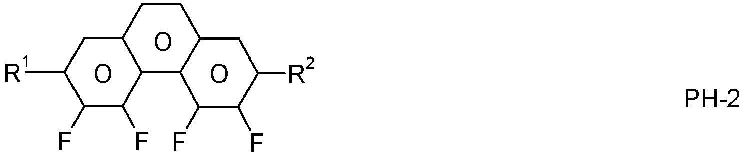 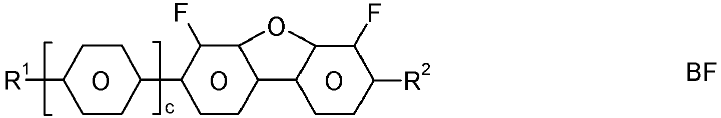 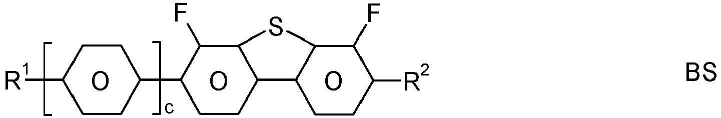 dans lesquelles
R^{B1}, R^{B2}, R^{CR1}, R^{CR2}, R¹ et R² présentent dans chaque cas, de manière indépendante les uns des autres, la signification de R^{2A} et
c représente 0, 1 ou 2.

17. Milieu cristallin liquide selon une ou plusieurs des revendications 1 à 16, **caractérisé en ce qu'**il comprend de 1 % à 30 % en poids de composés de la formule I.

18. Milieu cristallin liquide selon une ou plusieurs des revendications 1 à 17, **caractérisé en ce qu'**il comprend de façon additionnelle un ou plusieurs stabiliseur(s) UV et/ou antioxydant(s).

19. Milieu cristallin liquide selon une ou plusieurs des revendications 1 à 18, **caractérisé en ce qu'**il comprend de façon additionnelle un ou plusieurs composé(s) polymérisable(s).

20. Procédé pour la préparation d'un milieu cristallin liquide selon une ou plusieurs des revendications 1 à 19, **caractérisé en ce qu'**un ou plusieurs composé(s) de la formule I est/sont mélangé(s) avec au moins un autre composé mésogène et en option, avec un ou plusieurs additif(s) et/ou un ou plusieurs composé(s) polymérisable(s).

21. Utilisation d'un milieu cristallin liquide selon une ou plusieurs des revendications 1 à 19 à des fins électro-optiques.

22. Utilisation du milieu cristallin liquide selon une ou plusieurs des revendications 1 à 19 dans les affichages TN, STN, TN-TFT, OCB, IPS, PS-IPS, FFS, HB-FFS et PS-FFS, les lunettes à obturateur(s) pour les effets 3D, les lentilles à cristaux liquides/LC et les affichages VA positifs.

23. Affichage électro-optique à cristaux liquides contenant un milieu cristallin liquide selon une ou plusieurs des revendications 1 à 19.
